(19) Europäisches Patentamt
European Patent Office
Office européen des brevets

(11) EP 0 976 707 A1

(12) **EUROPEAN PATENT APPLICATION**
published in accordance with Art. 158(3) EPC

(43) Date of publication:
**02.02.2000 Bulletin 2000/05**

(21) Application number: **98911192.7**

(22) Date of filing: **03.04.1998**

(51) Int. Cl.$^7$: **C07C 43/18**, C07D 207/33, C07D 213/30, C07D 231/12, C07D 239/26, C07D 303/22, C07D 307/42, C07D 333/16, C07D 405/04, C07D 407/04, C07D 409/04, A01N 31/14, A01N 43/20
// A01N43/40, A01N43/54, A01N43/56

(86) International application number:
**PCT/JP98/01541**

(87) International publication number:
**WO 98/45235 (15.10.1998 Gazette 1998/41)**

(84) Designated Contracting States:
**ES FR GR IT**

(30) Priority: **07.04.1997 JP 8784297**
**11.07.1997 JP 18655497**
**06.03.1998 JP 5530298**

(71) Applicant:
**Nissan Chemical Industries, Ltd.**
**Chiyoda-ku, Tokyo 101-0054 (JP)**

(72) Inventors:
- **KAWAMURA, Yasuo;**
**Nissan Chemical Industries, Ltd.**
**Funabashi-shi; Chiba-ken 274-8507 (JP)**
- **KITA, Hiroshi;**
**Nissan Chemical Industries, Ltd.**
**Funabashi-shi; Chiba-ken 274-8507 (JP)**
- **NAKATA, Hisashi;**
**Nissan Chemical Industries, Ltd.**
**Funabashi-shi; Chiba-ken 274-8507 (JP)**
- **TAMADA, Yoshitake;**
**Nissan Chemical Industries Ltd.**
**Funabashi-shi; Chiba-ken 274-8507 (JP)**
- **HOTTA, Hiroyasu;**
**Nissan Chemical Industries, Ltd.**
**Funabashi-shi; Chiba-ken 274-8507 (JP)**
- **NISHIO, Koichi;**
**Nissan Chemical Industries, Ltd.**
**Funabashi-shi; Chiba-ken 274-8507 (JP)**
- **OHGOSHI, Akiyoshi;**
**Nissan Chemical Industries Ltd.**
**Funabashi-shi; Chiba-ken 274-8507 (JP)**
- **NAWAMAKI, Tsutomu**
**Nissan Chem. Ind., Ltd.**
**1470,**
**Saitama-ken 349-0294 (JP)**
- **WATANABE, Shigeomi;**
**Nissan Chem. Ind., Ltd.**
**Minamisaitama-gun, Saitama-ken 349-0294 (JP)**
- **NAKAHIRA, Kunimitsu;**
**Nissan Chem. Ind., Ltd.**
**Minamisaitama-gun, Saitama-ken 349-0294 (JP)**
- **OHKI, Tooru;**
**Nissan Chem. Ind., Ltd.**
**1470 Ohaza**
**Saitama-ken 349-0294 (JP)**
- **NOGUCHI, Junko;**
**Nissan Chem. Ind., Ltd.**
**1470**
**Saitama-ken 349-0294 (JP)**

(74) Representative: **HOFFMANN - EITLE**
**Patent- und Rechtsanwälte**
**Arabellastrasse 4**
**81925 München (DE)**

(54) **CYCLOALKYL BENZYL ETHER COMPOUNDS**

(57) Novel cycloalkyl benzyl ether compounds represented by the general formula (1) and agricultural chemicals, especially herbicides containing at least one of the compound of the general formula (1):

EP 0 976 707 A1

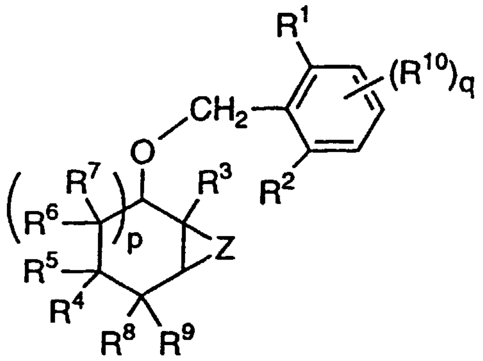

(1)

wherein $R^1$ and $R^2$ each independently represents hydrogen, halogen, alkyl, etc;
$R^3$ represents alkyl, alkenyl, haloalkyl, etc.;
$R^4$ represents hydrogen, halogen, alkyl, etc.;
$R^5$ represents halogen, cyano, azido, etc.;
$R^4$ and $R^5$ together may form alkylene chain, etc.;
$R^6$, $R^7$, $R^8$ and $R^9$ each represents hydrogen, halogen, alkyl, etc;
$R^{10}$ represents halogen, alkyl, haloalkyl, etc.;
p is 0 or 1; q is 0 to 3; and
Z represents oxygen or $CH_2$.

## Description

### Technical Field

This invention relates to novel cycloalkyl benzyl ether compounds and to agricultural chemicals, especially herbicides containing at least one of the ethers as one or more active ingredients.

### Technical Background

Compounds of 2,3-epoxy-cyclohexane-1-yl benzyl ether having herbicidal activities is described in WO No.97/02258. However, no compounds of 2,3-epoxy-cyclohexane-1-yl benzyl ethers having one or two substituents in 5-position on the cyclohexane ring have ever been known.

### Disclosure of the invention

The invention is a compound of cycloalkyl benzyl ether of the formula (1) and an agricultural chemical, especially a herbicide containing at least one of the ether:

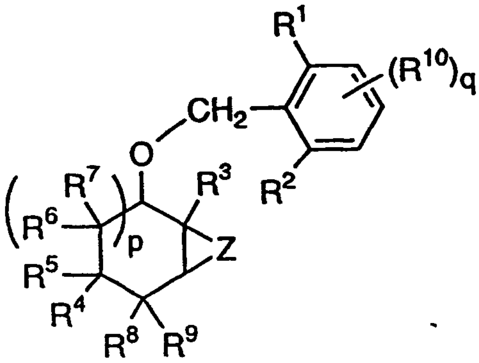

(1),

wherein $R^1$ and $R^2$ each independently represents hydrogen, halogen, $C_1$-$C_6$alkyl, $C_1$-$C_6$haloalkyl, $C_1$-$C_6$alkoxy, $C_1$-$C_6$haloalkoxy, $C_1$-$C_6$alkylthio, $C_1$-$C_6$haloalkylthio, nitro or cyano;
$R^3$ represents $C_1$-$C_6$alkyl, $C_3$-$C_6$alkenyl, $C_1$-$C_6$haloalkyl, ($C_1$-$C_4$alkoxy)$C_1$-$C_6$alkyl or the formula:

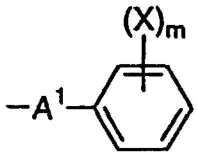

($A^1$ represents single bond or $C_1$-$C_3$alkylen chain;
X represents halogen, $C_1$-$C_6$alkyl, $C_1$-$C_6$,haloalkyl, $C_1$-$C_6$alkoxy, cyano, nitro, carboxyl or ($C_1$-$C_6$alkoxy)carbonyl;
m represents an integer of 1 to 5, and
X may be identical with or different from each other when m represents an integer 2 to 5);
$R^4$ represents hydrogen, halogen, $C_1$-$C_6$alkyl, $C_1$-$C_6$alkoxy or phenyl;
$R^5$ represents halogen, cyano, azido, $C_1$-$C_6$alkyl unsubstituted or substituted by azido, $C_2$-$C_6$alkenyl, $C_2$-$C_6$alkynyl, hydroxy, $C_1$-$C_6$alkoxy, $C_2$-$C_6$alkenyloxy, $C_2$-$C_6$alkynyloxy, mercapto, $C_1$-$C_6$alkylthio, $C_2$-$C_6$alkenylthio, $C_2$-$C_6$alkynylthio, amino, $C_1$-$C_6$alkylamino, di$C_1$-$C_6$alkylamino, $C_1$-$C_6$haloalkyl, $C_2$-$C_6$haloalkenyl, $C_2$-$C_6$haloalkynyl, $C_1$-$C_6$haloalkoxy, $C_1$-$C_6$haloalkylthio, hydroxy$C_1$-$C_6$alkyl, ($C_1$-$C_6$alkoxy)$C_1$-$C_6$alkyl, ($C_2$-$C_6$alkenyloxy)$C_1$-$C_6$alkyl, ($C_2$-$C_6$alkynyloxy)$C_1$-$C_6$alkyl, ($C_1$-$C_6$haloalkoxy)$C_1$-$C_6$alkyl, mercapto$C_1$-$C_6$alkyl, ($C_1$-$C_6$alkylthio) $C_1$-$C_6$alkyl, amino$C_1$-$C_6$alkyl, ($C_1$-$C_6$alkylamino)$C_1$-$C_6$alkyl, (di$C_1$-$C_6$alkylamino)$C_1$-$C_6$alkyl, cyano$C_1$-$C_6$alkyl, formyl$C_1$-

$C_6$alkyl, (hydroxycarbonyl)$C_1$-$C_6$alkyl, ($C_1$-$C_6$alkylcarbonyl)$C_1$-$C_6$alkyl, ($C_1$-$C_6$alkoxycarbonyl)$C_1$-$C_6$alkyl, ($C_1$-$C_6$alkoxy)($C_1$-$C_6$alkoxy)$C_1$-$C_6$alkyl, ($C_3$-$C_7$cycloalkyl) ($C_1$-$C_6$alkoxy)$C_1$-$C_6$alkyl, ($C_3$-$C_7$halocycloalkyl)($C_1$-$C_6$alkoxy)$C_1$-$C_6$alkyl, ($C_1$-$C_6$alkoxy)$C_1$-$C_6$haloalkyl, $C_3$-$C_7$cycloalkyl, $C_3$-$C_7$halocycloalkyl, $C_1$-$C_6$alkylcarboxy, epoxyethyl unsubstituted or substituted by halogen or $C_1$-$C_3$alkyl, (epoxyethyl unsubstituted or substituted by halogen or $C_1$-$C_3$alkyl)$C_1$-$C_6$alkyl, C(=W)$R^{11}$ ($R^{11}$ represents $C_2$-$C_6$alkyl, $C_1$-$C_6$haloalkyl, $C_1$-$C_6$alkoxy, $C_1$-$C_6$haloalkoxy, $C_1$-$C_6$alkylthio, $C_1$-$C_6$alkylamino or di$C_1$-$C_6$alkylamino; and W represents oxygen, sulfur or $NOR^{12}$($R^{12}$ represents $C_1$-$C_6$alkyl or $C_1$-$C_6$haloalkyl)), the formula:

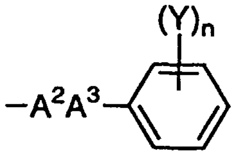

($A^2$ represents an single bond, oxygen or sulfur;
$A^3$ represents a single bond, a $C_1$-$C_3$alkylene chain or a $C_1$-$C_3$alkylene chain having hydroxy, halogen, $C_1$-$C_6$alkyl or $C_1$-$C_6$alkoxy, with a proviso that $A^2A^3$ means a single bond when $A^2$ and $A^3$ represent a single bond at the same time;
Y represents halogen, $C_1$-$C_6$alkyl, $C_1$-$C_6$haloalkyl, $C_1$-$C_6$alkoxy, $C_1$-$C_6$alkylthio, cyano, nitro, carboxyl or ($C_1$-$C_4$alkoxy) carbonyl;
n represents an integer 0 to 5, and
Y may be identical or different each other when n represents an integer 2 to 5); or the folrmula:

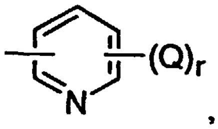 , 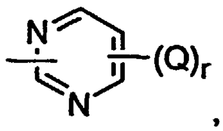 , 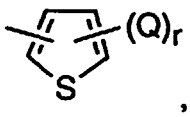 , 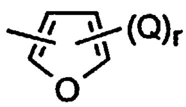 ,

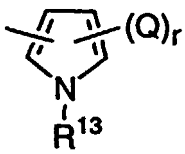 or 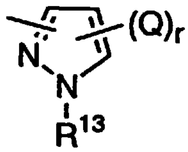

($R^{13}$ represents $C_1$-$C_6$alkyl;
Q represents halogen, $C_1$-$C_6$alkyl, $C_1$-$C_6$haloalkyl, $C_1$-$C_6$alkoxy, alkylthio, cyano, nitro, carboxyl or ($C_1$-$C_6$alkoxy)carbonyl;
r represents an integer 0 to 4; and
Q may be identical with or different from each other when r represents an integer 2 to 4);
$R^4$ and $R^5$ together may form $C_2$-$C_7$alkylene chain unsubstiuted or substituted by $C_1$-$C_6$alkyl, $C_1$-$C_6$alkoxy or halogen;
$R^6$, $R^7$, $R^8$ and $R^9$ each reprents independently hydrogen, halogen, $C_1$-$C_6$alkyl or phenyl;
$R^5$ and $R^8$ together may form $C_1$-$C_7$alkylene chain unsubstiuted or substituted by $C_1$-$C_6$alkyl;
$R^{10}$ represents halogen, $C_1$-$C_6$alkyl, $C_1$-$C_6$haloalkyl, $C_1$-$C_6$alkoxy or $C_1$-$C_6$haloalkoxy;
p is 0 or 1;
q represents an integer 0 to 3, and
$R^{10}$ each may be identical with or different from the other when q represents an integer 2 to 3; and
Z represents oxygen or $CH_2$.

As $R^1$ and $R^2$ each independently, hydrogen, halogen, $C_1$-$C_6$alkyl, $C_1$-$C_6$haloalkyl, $C_1$-$C_6$alkoxy, $C_1$-

$C_6$haloalkoxy, $C_1$-$C_6$alkylthio, $C_1$-$C_6$haloalkylthio, nitro and cyano, and preferably hydrogen, halogen and $C_1$-$C_2$ alkyl can be mentioned.

As $R^3$, $C_1$-$C_6$alkyl, $C_3$-$C_6$alkenyl, $C_1$-$C_6$haloalkyl, ($C_1$-$C_4$ alkoxy)$C_1$-$C_6$alkyl and the formula:

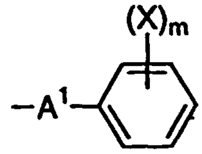

($A^1$ represents a single bond or a $C_1$-$C_3$alkylene chain;

X represents halogen, $C_1$-$C_6$alkyl, $C_1$-$C_6$ haloalkyl, $C_1$-$C_6$alkoxy, cyano, nitro, carboxyl or ($C_1$-$C_6$alkoxy)carbonyl;

m represents an integer of 0 to 5;

X may be identical with or different from the other when m represents an integer of 2 to 5), and preferably $C_1$-$C_6$alkyl can be mentioned.

As $R^4$, hydrogen, halogen, $C_1$-$C_6$alkyl, $C_1$-$C_6$alkoxy and phenyl, and preferably hydrogen and $C_1$-$C_6$alkyl can be mentioned.

As $R^5$, hydrogen, cyano, azido, $C_1$-$C_6$alkyl, azido$C_1$-$C_6$alkyl, $C_2$-$C_6$alkenyl, $C_2$-$C_6$alkynyl, hydroxy, $C_1$-$C_6$alkoxy, $C_2$-$C_6$alkenyloxy, $C_2$-$C_6$alkynyloxy, mercapto, $C_1$-$C_6$alkylthio, $C_2$-$C_6$alkenylthio, $C_2$-$C_6$alknylthio, amino, $C_1$-$C_6$alkylamino, di$C_1$-$C_6$alkylamino, $C_1$-$C_6$haloalkyl, $C_2$-$C_6$haloalkenyl, $C_2$-$C_6$haloalkynyl, $C_1$-$C_6$haloalkoxy, $C_1$-$C_6$haloalkylthio, hydroxy$C_1$-$C_6$alkyl, ($C_1$-$C_6$alkoxy)$C_1$-$C_6$alkyl, ($C_2$-$C_6$alkenyloxy)$C_1$-$C_6$alkyl, ($C_2$-$C_6$alkynyloxy)$C_1$-$C_6$alkyl, ($C_1$-$C_6$haloalkoxy)$C_1$-$C_6$alkyl, mercapto$C_1$-$C_6$alkyl, ($C_1$-$C_6$alkylthio)$C_1$-$C_6$alkyl, amino$C_1$-$C_6$alkyl, ($C_1$-$C_6$alkylamino)$C_1$-$C_6$alkyl, (di$C_1$-$C_6$alkylamino)$C_1$-$C_6$alkyl, cyano$C_1$-$C_6$alkyl, formyl$C_1$-$C_6$alkyl, (hydroxycarbonyl)$C_1$-$C_6$alkyl, ($C_1$-$C_6$alkylcarbonyl)$C_1$-$C_6$alkyl, ($C_1$-$C_6$alkoxycarbonyl)$C_1$-$C_6$alkyl, ($C_1$-$C_6$alkoxy)($C_1$-$C_6$alkoxy)$C_1$-$C_6$alkyl, ($C_3$-$C_7$cycloalkyl)($C_1$-$C_6$alkoxy)$C_1$-$C_6$alkyl, ($C_3$-$C_7$halocycloalkyl)($C_1$-$C_6$alkoxy)$C_1$-$C_6$alkyl, ($C_1$-$C_6$alkoxy)$C_1$-$C_6$haloalkyl, $C_3$-$C_7$cycloalkyl, $C_3$-$C_7$halocycloalkyl, $C_1$-$C_6$alkylcarboxy, epoxyethyl unsubstituted or substituted by halogen or $C_1$-$C_3$alkyl, (epoxyethyl unsubstituted or substituted by halogen or $C_1$-$C_3$alkyl)$C_1$-$C_6$alkyl, C(=W)$R^{11}$ (as $R^{11}$, $C_2$-$C_6$alkyl, $C_1$-$C_6$haloalkyl, $C_1$-$C_6$alkoxy, $C_1$-$C_6$haloalkoxy, $C_1$-$C_6$alkylthio, $C_1$-$C_6$alkylamino and di$C_1$-$C_6$alkylamino can be mentioned; as W, oxygen, sulfur and $NOR^{12}$ can be mentioned;

as $R^{12}$, $C_1$-$C_6$alkyl and $C_1$-$C_6$haloalkyl can be mentioned), the formula:

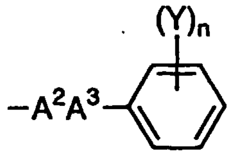

($A^2$ represents a single bond, oxygen or sulfur;

$A^3$ represents a single bond, a $C_1$-$C_3$alkylene chain or a $C_1$-$C_3$alkylene chain substituted by hydroxy, halogen, $C_1$-$C_6$alkyl or $C_1$-$C_6$alkoxy (with a proviso that $A^2$ $A^3$ means a single bond when $A^2$ and $A^3$ represent a single bond at the same time.),

Y represents halogen, $C_1$-$C_6$alkyl, $C_1$-$C_6$haloalkyl, $C_1$-$C_6$alkoxy, $C_1$-$C_6$alkylthio, cyano, nitro, carboxyl or ($C_1$-$C_4$alkoxy) carbonyl;

n represents an integer 0 to 5, and

Y may be identical with or different from each other when n represents an integer 2 to 5); and the folrmula:

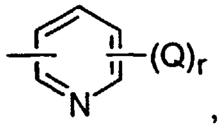, 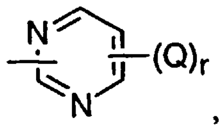, 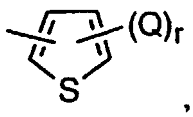, 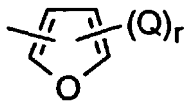,

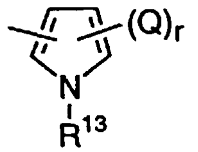 and 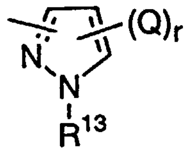 .

($R^{13}$ represents $C_1$-$C_6$alkyl;

Q represents halogen, $C_1$-$C_6$alkyl, $C_1$-$C_6$haloalkyl, $C_1$-$C_6$alkoxy, alkylthio, cyano, nitro, carboxyl or ($C_1$-$C_6$alkoxy)carbonyl;

r represents an integer 0 to 4, and

Q may be identical with or different from each other when r represents an integer 2 to 4) can be mentioned.

As $R^4$ and $R^5$ together, $C_2$-$C_7$alkylene chain unsubstiuted or substituted by $C_1$-$C_6$alkyl, $C_1$-$C_6$alkoxy and halogen can be mentioned.

As $R^6$, $R^7$, $R^8$ and $R^9$, for each independently, hydrogen, halogen, $C_1$-$C_6$alkyl and phenyl can be mentioned.

As $R^5$ and $R^8$ together, $C_1$-$C_7$alkylene chain unsubstiuted or substituted by $C_1$-$C_6$alkyl can be mentioned.

As $R^{10}$, halogen, $C_1$-$C_6$alkyl, $C_1$-$C_6$haloalkyl, $C_1$-$C_6$alkoxy and $C_1$-$C_6$haloalkoxy can be mentioned.

As p, 0 and 1 can be mentioned.

As q, integers 0 to 3 can be mentioned, and $R^{10}$ may be identical with or different from each other when q represents an integer 2 to 3.

As Z , oxygen and $CH_2$ can be mentioned.

More illustrative substituents are mentioned below and in the substituents, Me is methyl, Et is ethyl, n-Pr is n-propyl, i-Pr is isopropyl, n-Bu is n-butyl, i-Bu is isobutyl, s-Bu is sec-butyl, t-Bu is tert-butyl, n-Pen is n-pentyl, n-Hex is n-hexyl, c-Pr is cyclopropyl, c-Bu is cyclobutyl, c-Pen is cyclopentyl, c-Hex is cyclohexyl, c-Hep is cycloheptyl and e-Et is epoxyethyl.

As $R^1$ and $R^2$, for each independently, the following radicals can be mentioned:

H, F, Cl, Br, I, Me, Et, n-Pr, i-Pr, $CHF_2$, $CF_3$, $C_2F_5$, $C_3F_7$, $CCl_3$, $CH_2Cl$, OMe, OEt, On-Pr, Oi-Pr, $OCF_3$, $OCCl_3$, $OCHF_2$, $OCHCl_2$, $OCHBr_2$, $OC_2F_5$, $OCH_2CF_3$, $OCH_2CCl_3$, SMe, SEt, Sn-Pr, Si-Pr, $SCF_3$, $SCCl_3$, $SCHF_2$, $SCHCl_2$, $SCHBr_2$, $SC_2F_5$, $SCH_2CF_3$, $SCH_2CCl_3$, $NO_2$ and CN.

As $R^3$, the following radicals can be mentioned:

Me, Et, n-Pr, i-Pr, n-Bu, i-Bu, s-Bu, t-Bu, n-Pen, CHMe(n-Pr), $CH_2$s-Bu, $(CH_2)_2$i-Pr, $CH(Et)_2$, $C(Me)_2$Et, CHMe(i-Pr), $CH_2$t-Bu, n-Hex, CHMe(n-Bu), $CH_2$CHMe(n-Pr), $(CH_2)_2$s-Bu, $(CH_2)_3$i-Pr, $C(Me)_2$n-Pr, CHMe(s-Bu), CHMe(i-Bu), $CH_2C(Me)_2Et$, $CH_2$CHMe(i-Pr), $(CH_2)_2$t-Bu, CHEt(n-Pr), $CH_2CH(Et)_2$, $CMe(Et)_2$, CHEt(i-Pr), $C(Me)_2$i-Pr, CHMe(t-Bu), $CMe=CH_2$, $CH_2CH=CR_2$, CH=CHMe, CH=CHEt, $CH_2$CH=CHMe, $(CH_2)_2CH=CH_2$, $CEt=CH_2$, CMe=CHMe, $CH=C(Me)_2$, $CHMeCH=CH_2$, $CH_2CMe=CH_2$, CMe=CHEt, CH=CMe(Et), CH=CH(i-Pr), CHMeCH=CHMe, $CH_2$CMe=CHMe, $CH_2CH=CMe_2$, $CHMeCH_2CH=CH_2$, $CH_2CHMeCH=CH_2$, $(CH_2)_2CMe=CH_2$, $CH_2CEt=CH_2$, $CHEtCH=CH_2$, CEt=CHMe, $CMe=CMe_2$, $CMe_2CH=CH_2$, $CHMeCMe=CH_2$, C(i-Pr)=$CH_2$, C(n-Pr)=$CH_2$, $CH_2$CH=CH(n-Pr), $(CH_2)_2$CH=CHEt, $(CH_2)_3$CH=CHMe, $(CH_2)_4CH=CH_2$, $CH_2$CMe=CHEt, $CH_2$CH=CMe(Et), $CH_2$CH=CH(i-Pr), $CHMeCH_2$CH=CHMe, $CH_2$CHMeCH=CHMe, $(CH_2)_2$CMe=CHMe, $(CH_2)_2CH=CMe_2$, $CHMe(CH_2)_2CH=CH_2$, $CH_2CHMeCH_2CH=CH_2$, $(CH_2)_2CHMeCH=CH_2$, $(CH_2)_3CMe=CH_2$, $CH_2$CH=CMe(Et), $CH_2CMe=CMe_2$, $CH_2Cl$, $CH_2Br$, $CF_3$, $CCl_3$, $(CH_2)_2F$, $(CH_2)_2Cl$, $(CH_2)_2Br$, $(CH_2)_2I$, $C_2F_5$, CHMeCl, CHMeBr, $CHClCH_2Cl$, $CHBrCH_2Br$, $(CH_2)_3Cl$, $(CH_2)_3Br$, $(CH_2)_3F$, $CMeClCH_2Cl$, $CMeBrCH_2Br$, $CH_2CHClCH_2Cl$, $CH_2CHBrCH_2Br$, $(CH_2)_4F$, $(CH_2)_4Cl$, $CH_2$CHClCHClMe, $(CH_2)_2CHClCH_2Cl$, $CHMeCHClCH_2Cl$, $CH_2CMeClCH_2Cl$, $(CH_2)_5F$, $(CH_2)_5Cl$, $(CH_2)_5Br$, $CH_2CHClCMe_2Cl$, $CHMeCH_2CHClCH_2Cl$, $(CH_2)_2CMeClCH_2Cl$, $(CH_2)_6Cl$, $CH_2$CHClCHCl(n-Pr), $CH_2$CHClCHCl(i-Pr), $CH_2OMe$, $CH_2OEt$, $CH_2$On-Pr, $CH_2$Oi-Pr, $CH_2$On-Bu, $CH_2$Oi-Bu, $CH_2$Os-Bu, $CH_2$Ot-Bu, $(CH_2)_2OMe$, $(CH_2)_2OEt$, $(CH_2)_2$On-Pr, $(CH_2)_2$Oi-Pr, $(CH_2)_2$On-Bu, $(CH_2)_2$Oi-Bu, $(CH_2)_2$Os-Bu, $(CH_2)_2$Ot-Bu, CHMeOMe, CHMeOEt, CHMeOn-Pr, CHMeOi-Pr, CHMeOn-Bu, CHMeOi-Bu, CHMeOs-Bu, CHMeOt-Bu, $(CH_2)_3OMe$, $(CH_2)_3OEt$, $(CH_2)_3$On-Pr, $(CH_2)_3$Oi-Pr, $(CH_2)_3$On-Bu, $(CH_2)_3$Oi-Bu, $(CH_2)_3$Os-Bu, $(CH_2)_3$Ot-Bu, $CHMeCH_2OMe$, $CHMeCH_2OEt$, $CHMeCH_2$On-Pr, $CHMeCH_2$Oi-Pr, $CHMeCH_2$On-Bu, $CHMeCH_2$Oi-Bu, $CHMeCH_2$Os-Bu, $CHMeCH_2$Ot-Bu, $CH_2$CHMeOMe,

CH$_2$CHMeOEt, CH$_2$CHMeOn-Pr, CH$_2$CHMeOi-Pr, CH$_2$CHMeOn-Bu, CH$_2$CHMeOi-Bu, CH$_2$CHMeOS-Bu, CH$_2$CHMeOt-Bu, CMe$_2$OMe, CMe$_2$OEt, CMe$_2$On-Pr, CMe$_2$Oi-Pr, CMe$_2$On-Bu, CMe$_2$Oi-Bu, CMe$_2$Os-Bu, CMe$_2$Ot-Bu, (CH$_2$)$_4$OMe, (CH$_2$)$_4$OEt, (CH$_2$)$_4$On-Pr, (CH$_2$)$_4$Oi-Pr, (CH$_2$)$_4$On-Bu, (CH$_2$)$_4$Oi-Bu, -(CH$_2$)$_4$Os-Bu, (CH$_2$)$_4$Ot-Bu, CHMe(CH$_2$)$_2$OMe, CHMe(CH$_2$)$_2$OEt, CHMe(CH$_2$)$_2$On-Pr, CHMe(CH$_2$)$_2$Oi-Pr, CHMe(CH$_2$)$_2$On-Bu, CHMe(CH$_2$)$_2$Oi-Bu, CHMe(CH$_2$)$_2$Os-Bu, CHMe(CH$_2$)$_2$Ot-Bu, CH$_2$CHMeCH$_2$OMe, CH$_2$CHMeCH$_2$OEt, CH$_2$CHMeCH$_2$On-Pr, CH$_2$CHMeCH$_2$Oi-Pr, CH$_2$CHMeCH$_2$On-Bu, CH$_2$CHMeCH$_2$Oi-Bu, CH$_2$CHMeCH$_2$Os-Bu, CH$_2$CHMeCH$_2$Ot-Bu, (CH$_2$)$_2$CHMeOMe, (CH$_2$)$_2$CHMeOEt, (CH$_2$)$_2$CHMeOn-Pr, (CH$_2$)$_2$CHMeOi-Pr, (CH$_2$)$_2$CHMeOn-Bu, (CH$_2$)$_2$CHMeOi-Bu, (CH$_2$)$_2$CHMeOs-Bu, (CH$_2$)$_2$CHMeOt-Bu, CMe$_2$CH$_2$OMe, CMe$_2$CH$_2$OEt, CMe$_2$CH$_2$On-Pr, CMe$_2$CH$_2$Oi-Pr, CMe$_2$CH$_2$On-Bu, CMe$_2$CH$_2$Oi-Bu, CMe$_2$CH$_2$Os-Bu, CMe$_2$CH$_2$Ot-Bu, (CH$_2$)$_5$OMe, (CH$_2$)$_5$OEt, (CH$_2$)$_5$On-Pr, (CH$_2$)$_5$Oi-Pr, (CH$_2$)$_5$On-Bu, (CH$_2$)$_5$Oi-Bu, (CH$_2$)$_5$Os-Bu, (CH$_2$)$_5$Ot-Bu, CHMe(CH$_2$)$_3$OMe, CHMe(CH$_2$)$_3$OEt, CHMe(CH$_2$)$_3$On-Pr, CHMe(CH$_2$)$_3$Oi-Pr, CHMe(CH$_2$)$_3$On-Bu, CHMe(CH$_2$)$_3$Oi-Bu, CHMe(CH$_2$)$_3$Os-Bu, CHMe(CH$_2$)$_3$Ot-Bu, CHEt(CH$_2$)$_2$OMe, CHEt(CH$_2$)$_2$OEt, CHEt(CH$_2$)$_2$On-Pr, CHEt(CH$_2$)$_2$Oi-Pr, CHEt(CH$_2$)$_2$On-Bu, CHEt(CH$_2$)$_2$Oi-Bu, CHEt(CH$_2$)$_2$OS-Bu, CHEt(CH$_2$)$_2$Ot-Bu, CMe$_2$(CH$_2$)$_2$OMe, CMe$_2$(CH$_2$)$_2$OEt, CMe$_2$(CH$_2$)$_2$On-Pr, CMe$_2$(CH$_2$)$_2$Oi-Pr, CMe$_2$(CH$_2$)$_2$On-Bu, CMe$_2$(CH$_2$)$_2$Oi-Bu, CMe$_2$(CH$_2$)$_2$Os-Bu, CMe$_2$(CH$_2$)$_2$Ot-Bu, (CH$_2$)$_6$OMe, (CH$_2$)$_6$OEt, (CH$_2$)$_6$On-Pr, (CH$_2$)$_6$Oi-Pr, (CH$_2$)$_6$On-Bu, (CH$_2$)$_6$Oi-Bu, (CH$_2$)$_6$Os-Bu, (CH$_2$)$_6$Ot-Bu, CHMe(CH$_2$)$_4$OMe, CHMe(CH$_2$)$_4$OEt, CHMe(CH$_2$)$_4$On-Pr, CHMe(CH$_2$)$_4$Oi-Pr, CHMe(CH$_2$)$_4$On-Bu, CHMe(CH$_2$)$_4$Oi-Bu, CHMe(CH$_2$)$_4$Os-Bu, CHMe(CH$_2$)$_4$Ot-Bu and the formula:

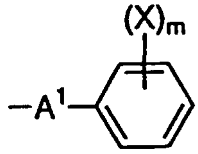

As A$^1$, the following radicals can be mentioned:
a single bond, CH$_2$, (CH$_2$)$_2$, CHMe, (CH$_2$)$_3$, CHMeCH$_2$, CH$_2$CHMe and CMe$_2$.

As X, the following radicals can be mentioned:
F, Cl, Br, I, Me, Et, n-Pr, i-Pr, n-Bu, i-Bu, s-Bu, t-Bu, CF$_3$. CH$_2$Cl, CH$_2$Br, CCl$_3$, CHF$_2$, (CH$_2$)$_2$F, (CH$_2$)$_2$Cl, (CH$_2$)$_2$Br, C$_2$F$_5$, OMe, OEt, On-Pr, Oi-Pr, On-Bu, Oi-Bu, Os-Bu, Ot-Bu, CN, NO$_2$, CO$_2$H, CO$_2$Me, CO$_2$Et, CO$_2$n-Pr, CO$_2$i-Pr, CO$_2$n-Bu, CO$_2$i-Bu, CO$_2$s-Bu and CO$_2$t-Bu.

As R$^4$, the following radicals can be mentioned:
H, F, Cl, Br, I, Me, Et, n-Pr, i-Pr, n-Bu, i-Bu, s-Bu, t-Bu, n-Pen, CHMe(n-Pr), CH$_2$s-Bu, (CH$_2$)$_2$i-Pr, CH(Et)$_2$, C(Me)$_2$Et, CHMe(i-Pr), CH$_2$t-Bu, n-Hex, CHMe(n-Bu), CH$_2$CHMe(n-Pr), (CH$_2$)$_2$s-Bu, (CH$_2$)$_3$i-Pr, C(Me)$_2$n-Pr, CHMe(s-Bu), CHMe(i-Bu), CH$_2$C(Me)$_2$Et, CH$_2$CHMe(i-Pr), (CH$_2$)$_2$t-Bu, CHEt(n-Pr), CH$_2$CH(Et)$_2$, CMe(Et)$_2$, CHEt(i-Pr), C(Me)$_2$i-Pr, CHMe(t-Bu), OMe, OEt, On-Pr, Oi-Pr, On-Bu, Oi-Bu, Os-Bu, Ot-Bu and phenyl.

As R$^5$, the following radicals can be mentioned:
F, Cl, Br, I, CN, N$_3$, Me, Et, n-Pr, i-Pr, n-Bu, i-Bu, s-Bu, t-Bu, n-Pen, CHMe(n-Pr), CH$_2$s-Bu, (CH$_2$)$_2$i-Pr, CH(Et)$_2$, C(Me)$_2$Et, CHMe(i-Pr), CH$_2$t-Bu, n-Hex, CHMe(n-Bu), CH$_2$CHMe(n-Pr), (CH$_2$)$_2$s-Bu, (CH$_2$)$_3$i-Pr, C(Me)$_2$n-Pr, CHMe(s-Bu), CHMe(i-Bu), CH$_2$C(Me)$_2$Et, CH$_2$CHMe(i-Pr), (CH$_2$)$_2$t-Bu, CHEt(n-Pr), CH$_2$CH(Et)$_2$, CMe(Et)$_2$, CHEt(i-Pr), C(Me)$_2$i-Pr, CHMe(t-Bu), CHMe(CH$_2$N$_3$), CH=CH$_2$, CMe=CH$_2$, CH$_2$CH=CH$_2$, CH=CHMe, CH=CHEt, CH$_2$CH=CHMe, (CH$_2$)$_2$CH=CH$_2$, CHEt=CH$_2$, CMe=CHMe, CH=CMe$_2$, CHMeCH=CH$_2$, CH$_2$CMe=CH$_2$, CMe=CHEt, CMe=CH(n-Pr), CMe=CH(i-Pr), CMe=CH(n-Bu), CH=CMe(Et), CH=CH(i-Pr), CHMeCH=CHMe, CH$_2$CMe=CHMe, CH$_2$CH=CMe$_2$, CHMeCH$_2$CH=CH$_2$, CH$_2$CHMeCH=CH$_2$, (CH$_2$)$_2$CMe=CH$_2$, CH$_2$CEt=CH$_2$, CHEtCH=CH$_2$, CEt=CHMe, CMe=CMe$_2$, CMe$_2$CH=CH$_2$, CHMeCMe=CH$_2$, C(i-Pr)=CH$_2$, C(n-Pr)CH$_2$, CH$_2$CH=CH(n-Pr), (CH$_2$)$_2$CH=CHEt, (CH$_2$)$_3$CH=CHMe, (CH$_2$)$_4$CH=CH$_2$, CH$_2$CMe=CHEt, CH$_2$CH=CMe(Et), CH$_2$CH=CH(i-Pr), CHMeCH$_2$CH=CHMe, CH$_2$CHMeCH=CHMe, (CH$_2$)$_2$CMe=CHMe, (CH$_2$)$_2$CH=CMe$_2$, CHMe(CH$_2$)$_2$CH=CH$_2$, CH$_2$CHMeCH$_2$CH=CH$_2$, (CH$_2$)$_2$CHMeCH=CH$_2$, (CH$_2$)$_3$CMe=CH$_2$, CH$_2$CH=CMe(Et), CH$_2$CMe=CMe$_2$, C≡CH, C≡CMe, C≡CEt, C≡Cn-Pr, C≡Ci-Pr, C≡Cn-Bu, C≡Ci-Bu, C≡Cs-Bu, C≡Ct-Bu, CH$_2$C≡CH, CH$_2$C≡CMe, CH$_2$C≡CEt, CH$_2$C≡Cn-Pr, CH$_2$C≡Ci-Pr, CH(Me)C≡CH, CH(Me)C≡CMe, CH(Me)C≡ CEt, CH(Et)C≡CH, CH(Et)C≡CMe, C(Me)$_2$C≡CH, C(Me)$_2$C≡CMe, C(Me)(Et)C≡CH, (CH$_2$)$_2$C≡CH, (CH$_2$)$_2$C≡CMe, (CH$_2$)$_2$C≡CEt, CH(Me)CH$_2$C≡CH, CH(Me)CH$_2$C≡CMe, CH(Et)CH$_2$C≡CH, C(Me$_2$)CH$_2$C≡CH, CH$_2$CH(Me)C≡CH, CH$_2$CH(Me)C≡CMe, CH$_2$C(Me)$_2$C≡CH, CH$_2$CH(Et)C≡CH, (CH$_2$)$_3$C≡CH, (CH$_2$)$_3$C≡CMe, CH(Me)(CH$_2$)$_2$C≡CH, CH$_2$CH(Me)CH$_2$C≡CH, (CH$_2$)$_2$CH(Me)C≡CH, (CH$_2$)$_4$C≡CH, CH$_2$Cl, CH$_2$Br, CF$_3$, CCl$_3$, (CH$_2$)$_2$F, (CH$_2$)$_2$Cl, (CH$_2$)$_2$Br, (CH$_2$)$_2$I, C$_2$F$_5$, CHMeCl, CHMeBr, CHClCH$_2$Cl, CHBrCH$_2$Br, (CH$_2$)$_3$Cl, (CH$_2$)$_3$Br, (CH$_2$)$_3$I, CMeClCH$_2$Cl, CMeBrCH$_2$Br, CH$_2$CHClCH$_2$Cl, CH$_2$CHBrCH$_2$Br, (CH$_2$)$_4$F, (CH$_2$)$_4$Cl, CH$_2$CHClCHClMe, (CH$_2$)$_2$CHClCH$_2$Cl, CHMeCHClCH$_2$Cl, CH$_2$CMeClCH$_2$Cl, (CH$_2$)$_5$F, (CH$_2$)$_5$Cl, (CH$_2$)$_5$Br,

$CH_2CHClCMe_2Cl$, $CHMeCH_2CHClCH_2Cl$, $(CH_2)_2CMeClCH_2Cl$, $(CH_2)_6Cl$, $CH_2CHClCHCl$(n-Pr), $CH_2CHClCHCl$(i-Pr), $CHMeCH_2Cl$, $CHMeCH_2Br$, $CHMeCH_2I$, $CMe_2Cl$, $CMe_2Br$, $CMe_2I$, $CH_2OMe$, $CH_2OEt$, $CH_2$On-Pr, $CH_2$Oi-Pr, $CH_2$On-Bu, $CH_2$Oi-Bu, $CH_2$Os-Bu, $CH_2$Ot-Bu, $(CH_2)_2OMe$, $(CH_2)_2OEt$, $(CH_2)_2$On-Pr, $(CH_2)_2$Oi-Pr, $(CH_2)_2$On-Bu, $(CH_2)_2$Oi-Bu, $(CH_2)_2$Os-Bu, $(CH_2)_2$Ot-Bu, CHMeOMe, CHMeOEt, CHMeOn-Pr, CHMeOi-Pr, CHMeOn-Bu, CHMeOi-Bu, CHMeOs-Bu, CHMeOt-Bu, $(CH_2)_3OMe$, $(CH_2)_3OEt$, $(CH_2)_3$On-Pr, $(CH_2)_3$Oi-Pr, $(CH_2)_3$On-Bu, $(CH_2)_3$Oi-Bu, $(CH_2)_3$Os-Bu, $(CH_2)_3$Ot-Bu, $CHMeCH_2OMe$, $CHMeCH_2OEt$, $CHMeCH_2$On-Pr, $CHMeCH_2$Oi-Pr, $CHMeCH_2$On-Bu, $CHMeCH_2$Oi-Bu, $CHMeCH_2$Os-Bu, $CHMeCH_2$Ot-Bu, $CH_2CHMeOMe$, $CH_2CHMeOEt$, $CH_2$CHMeOn-Pr, $CH_2$CHMeOi-Pr, $CH_2$CHMeOn-Bu, $CH_2$CHMeOi-Bu, $CH_2$CHMeOs-Bu, $CH_2$CHMeOt-Bu, $CMe_2OMe$, $CMe_2OEt$, $CMe_2$On-Pr, $CMe_2$Oi-Pr, $CMe_2$On-Bu, $CMe_2$Oi-Bu, $CMe_2$Os-Bu, $CMe_2$Ot-Bu, $(CH_2)_4OMe$, $(CH_2)_4OEt$, $(CH_2)_4$On-Pr, $(CH_2)_4$Oi-Pr, $(CH_2)_4$On-Bu, $(CH_2)_4$Oi-Bu, $(CH_2)_4$Os-Bu, $(CH_2)_4$Ot-Bu, $CHMe(CH_2)_2OMe$, $CHMe(CH_2)_2OEt$, $CHMe(CH_2)_2$On-Pr, $CHMe(CH_2)_2$Oi-Pr, $CHMe(CH_2)_2$On-Bu, $CHMe(CH_2)_2$Oi-Bu, $CHMe(CH_2)_2$Os-Bu, $CHMe(CH_2)_2$Ot-Bu, $CH_2CHMeCH_2OMe$, $CH_2CHMeCH_2OEt$, $CH_2CHMeCH_2$On-Pr, $CH_2CHMeCH_2$Oi-Pr, $CH_2CHMeCH_2$On-Bu, $CH_2CHMeCH_2$Oi-Bu, $CH_2CHMeCH_2$Os-Bu, $CH_2CHMeCH_2$Ot-Bu, $(CH_2)_2CHMeOMe$, $(CH_2)_2CHMeOEt$, $(CH_2)_2$CHMeOn-Pr, $(CH_2)_2$CHMeOi-Pr, $(CH_2)_2$CHMeOn-Bu, $(CH_2)_2$CHMeOi-Bu, $(CH_2)_2$CHMeOs-Bu, $(CH_2)_2$CHMeOt-Bu, $CMe_2CH_2OMe$, $CMe_2CH_2OEt$, $CMe_2CH_2$On-Pr, $CMe_2CH_2$Oi-Pr, $CMe_2CH_2$On-Bu, $CMe_2CH_2$Oi-Bu, $CMe_2CH_2$Os-Bu, $CMe_2CH_2$Ot-Bu, $(CH_2)_5OMe$, $(CH_2)_5OEt$, $(CH_2)_5$On-Pr, $(CH_2)_5$Oi-Pr, $(CH_2)_5$On-Bu, $(CH_2)_5$Oi-Bu, $(CH_2)_5$Os-Bu, $(CH_2)_5$Ot-Bu, $CHMe(CH_2)_3OMe$, $CHMe(CH_2)_3OEt$, $CHMe(CH_2)_3$On-Pr, $CHMe(CH_2)_3$Oi-Pr, $CHMe(CH_2)_3$On-Bu, $CHMe(CH_2)_3$Oi-Bu, $CHMe(CH_2)_3$Os-Bu, $CHMe(CH_2)_3$Ot-Bu, $CHEt(CH_2)_2OMe$, $CHEt(CH_2)_2OEt$, $CHEt(CH_2)_2$On-Pr, $CHEt(CH_2)_2$Oi-Pr, $CHEt(CH_2)_2$On-Bu, $CHEt(CH_2)_2$Oi-Bu, $CHEt(CH_2)_2$Os-Bu, $CHEt(CH_2)_2$Ot-Bu, $CMe_2(CH_2)_2OMe$, $CMe_2(CH_2)_2OEt$, $CMe_2(CH_2)_2$On-Pr, $CMe_2(CH_2)_2$Oi-Pr, $CMe_2(CH_2)_2$On-Bu, $CMe_2(CH_2)_2$Oi-Bu, $CMe_2(CH_2)_2$Os-Bu, $CMe_2(CH_2)_2$Ot-Bu, $(CH_2)_6OMe$, $(CH_2)_6OEt$, $(CH_2)_6$On-Pr, $(CH_2)_6$Oi-Pr, $(CH_2)_6$On-Bu, $(CH_2)_6$Oi-Bu, $(CH_2)_6$Os-Bu, $(CH_2)_6$Ot-Bu, $CHMe(CH_2)_4OMe$, $CHMe(CH_2)_4OEt$, $CHMe(CH_2)_4$On-Pr, $CHMe(CH_2)_4$Oi-Pr, $CHMe(CH_2)_4$On-Bu, $CHMe(CH_2)_4$Oi-Bu, $CHMe(CH_2)_4$Os-Bu, $CHMe(CH_2)_4$Ot-Bu, $CH_2OCH=CH_2$, $CH_2OCH=CHMe$, $CH_2OCH=CMe_2$, $CH_2OCMe=CH_2$, $CH_2OCMe=CHMe$, $CH_2OCMe=CMe_2$, $CH_2OCH_2CH=CH_2$, $CHMeOCH=CH_2$, CHMeOCH=CHMe, $CHMeOCH=CMe_2$, $CHMeOCMe=CH_2$, CHMeOCMe=CHMe, $CHMeOCMe=CMe_2$, $CHMeOCH_2CH=CH_2$, $CMe_2OCH=CH_2$, $CMe_2OCH=CHMe$, $CMe_2OCH=CMe_2$, $CMe_2OCMe=CH_2$, $CMe_2OCMe=CHMe$, $CMe_2OCMe=CMe_2$, $CMe_2OCH_2CH=CH_2$, $(CH_2)_2OCH=CH_2$, $CH_2OC\equiv CH$, $CH_2OC\equiv CMe$, $CHMeOC\equiv CH$, $CHMeOC\equiv CMe$, $CMe_2OC\equiv CH$, $CMe_2OC\equiv CMe$, $CH_2OCH_2C\equiv CH$, $CHMeOCH_2C\equiv CH$, $CMe_2OCH_2C\equiv CH$, $CH_2OCF_3$, $CH_2OCHF_3$, $CH_2OC_2F_5$, $CH_2OCH_2CF_3$, $CH_2OCCl_3$, $CH_2OCHCl_2$, $CH_2OCH_2Cl$, $CH_2OCH_2CCl_3$, $CHMeOCF_3$, $CHMeOCHF_2$, $CHMeOC_2F_5$, $CHMeOCH_2CF_3$, $CHMeOCCl_3$, $CHMeOCHCl_2$, $CHMeOCH_2Cl$, $CHMeOCHBr_2$, $CHMeOCH_2CCl_3$, $CMe_2OCF_3$, $CMe_2OCHF_2$, $CMe_2OC_2F_5$, $CMe_2OCMe_2CF_3$, $CMe_2OCCl_3$, $CMe_2OCHCl_2$, $CMe_2OCH_2Cl$, $OMe_2OCH_2CCl_3$, $CMe_2OCH_2CF_3$, $CH_2SMe$, $CH_2SEt$, $CH_2$Sn-Pr, $CH_2$Si-Pr, $CH_2$Sn-Bu, $CH_2$Si-Bu, $CH_2$Ss-Bu, $CH_2$St-Bu, $(CH_2)_2SMe$, $(CH_2)_2SEt$, $(CH_2)_2$Sn-Pr, $(CH_2)_2$Si-Pr, $(CH_2)_2$Sn-Bu, $(CH_2)_2$Si-Bu, $(CH_2)_2$Ss-Bu, $(CH_2)_2$St-Bu, CHMeSMe, CHMeSEt, CHMeSn-Pr, CHMeSi-Pr, CHMeSn-Bu, CHMeSi-Bu, CHMeSs-Bu, CHMeSt-Bu, $(CH_2)_3SMe$, $(CH_2)_3SEt$, $(CH_2)_3$Sn-Pr, $(CH_2)_3$Si-Pr, $(CH_2)_3$Sn-Bu, $(CH_2)_3$Si-Bu, $(CH_2)_3$Ss-Bu, $(CH_2)_3$St-Bu, $CHMeCH_2SMe$, $CHMeCH_2SEt$, $CHMeCH_2$Sn-Pr, $CHMeCH_2$Si-Pr, $CHMeCH_2$Sn-Bu, $CHMeCH_2$Si-Bu, $CHMeCH_2$Ss-Bu, $CHMeCH_2$St-Bu, $CH_2CHMeSMe$, $CH_2CHMeSEt$, $CH_2$CHMeSn-Pr, $CH_2$CHMeSi-Pr, $CH_2$CHMeSn-Bu, $CH_2$CHMeSi-Bu, $CH_2$CHMeSs-Bu, $CH_2$CHMeSt-Bu, $CMe_2SMe$, $CMe_2SEt$, $CMe_2$Sn-Pr, $CMe_2$Si-Pr, $CMe_2$Sn-Bu, $CMe_2$Si-Bu, $CMe_2$Ss-Bu, $CMe_2$St-Bu, $(CH_2)_4SMe$, $(CH_2)_4SEt$, $(CH_2)_4$Sn-Pr, $(CH_2)_4$Si-Pr, $(CH_2)_4$Sn-Bu, $(CH_2)_4$Si-Bu, $(CH_2)_4$Ss-Bu, $(CH_2)_4$St-Bu, $CHMe(CH_2)_2SMe$, $CHMe(CH_2)_2SEt$, $CHMe(CH_2)_2$Sn-Pr, $CHMe(CH_2)_2$Si-Pr, $CHMe(CH_2)_2$Sn-Bu, $CHMe(CH_2)_2$Si-Bu, $CHMe(CH_2)_2$Ss-Bu, $CHMe(CH_2)_2$St-Bu, $CH_2CHMeCH_2SMe$, $CH_2CHMeCH_2SEt$, $CH_2CHMeCH_2$Sn-Pr, $CH_2CHMeCH_2$Si-Pr, $CH_2CHMeCH_2$Sn-Bu, $CH_2CHMeCH_2$Si-Bu, $CH_2CHMeCH_2$Ss-Bu, $CH_2CHMeCH_2$St-Bu, $(CH_2)_2CHMeSMe$, $(CH_2)_2CHMeSEt$, $(CH_2)_2$CHMeSn-Pr, $(CH_2)_2$CHMeSi-Pr, $(CH_2)_2$CHMeSn-Bu, $(CH_2)_2$CHMeSi-Bu, $(CH_2)_2$CHMeSs-Bu, $(CH_2)_2$CHMeSt-Bu, $CMe_2CH_2SMe$, $CMe_2CH_2SEt$, $CMe_2CH_2$Sn-Pr, $CMe_2CH_2$Si-Pr, $CMe_2CH_2$Sn-Bu, $CMe_2CH_2$Si-Bu, $CMe_2CH_2$Ss-Bu, $CMe_2CH_2$St-Bu, $(CH_2)_5SMe$, $(CH_2)_5SEt$, $(CH_2)_5$Sn-Pr, $(CH_2)_5$Si-Pr, $(CH_2)_5$Sn-Bu, $(CH_2)_5$Si-Bu, $(CH_2)_5$Ss-Bu, $(CH_2)_5$St-Bu, $CHMe(CH_2)_3SMe$, $CHMe(CH_2)_3SEt$, $CHMe(CH_2)_3$Sn-Pr, $CHMe(CH_2)_3$Si-Pr, $CHMe(CH_2)_3$Sn-Bu, $CHMe(CH_2)_3$Si-Bu, $CHMe(CH_2)_3$Ss-Bu, $CHMe(CH_2)_3$St-Bu, $CHEt(CH_2)_2SMe$, $CHEt(CH_2)_2SEt$, $CHEt(CH_2)_2$Sn-Pr, $CHEt(CH_2)_2$Si-Pr, $CHEt(CH_2)_2$Sn-Bu, $CHEt(CH_2)_2$Si-Bu, $CHEt(CH_2)_2$Ss-Bu, $CHEt(CH_2)_2$St-Bu, $CMe_2(CH_2)_2SMe$, $CMe_2(CH_2)_2SEt$, $CMe_2(CH_2)_2$Sn-Pr, $CMe_2(CH_2)_2$Si-Pr, $CMe_2(CH_2)_2$Sn-Bu, $CMe_2(CH_2)_2$Si-Bu, $CMe_2(CH_2)_2$Ss-Bu, $CMe_2(CH_2)_2$St-Bu, $(CH_2)_6SMe$, $(CH_2)_6SEt$, $(CH_2)_6$Sn-Pr, $(CH_2)_6$Si-Pr, $(CH_2)_6$Sn-Bu, $(CH_2)_6$Si-Bu, $(CH_2)_6$Ss-Bu, $(CH_2)_6$St-Bu, $CHMe(CH_2)_4SMe$, $CHMe(CH_2)_4SEt$, $CHMe(CH_2)_4$Sn-Pr, $CHMe(CH_2)_4$Si-Pr, $CHMe(CH_2)_4$Sn-Bu, $CHMe(CH_2)_4$Si-Bu, $CHMe(CH_2)_4$Ss-Bu, $CHMe(CH_2)_4$St-Bu, $CH_2NHMe$, $CH_2NHEt$, $CH_2$NHn-Pr, $CH_2$NHi-Pr, CH(Me)NHMe, CH(Me)NHEt, CH(Me)NHn-Pr, CH(Me)NHi-Pr, $CMe_2NHMe$, $CMe_2NHEt$, $CMe_2$NHn-Pr, $CMe_2$NHi-Pr, $(CH_2)_2NHMe$, $(CH_2)_2NHEt$, $(CH_2)_2$NHn-Pr, $(CH_2)_2$NHi-Pr, $CH_2NMe_2$, $CH_2NMe$(Et), $CH_2NMe$(n-Pr), $CH_2NMe$(i-Pr), $CH_2NEt_2$, $CH_2NEt$(n-Pr), $CH_2NEt$(i-Pr), $CH_2$Nn-$Pr_2$, $CH_2$Nn-Pr(i-Pr), $CH_2$Ni-$Pr_2$, $CH(Me)NMe_2$, CH(Me)NMe(Et), CH(Me)NMe(n-Pr), CH(Me)NMe(i-Pr), $CH(Me)NEt_2$, CH(Me)NEt(n-Pr), CH(Me)NEt(i-Pr), CH(Me)Nn-$Pr_2$, CH(Me)Nn-Pr(i-Pr), CH(Me)Ni-$Pr_2$, $CMe_2NMe_2$, $CMe_2NMe$(Et), $CMe_2NMe$(n-Pr), $CMe_2NMe$(i-Pr), $CMe_2NEt_2$, $CMe_2NEt$(n-Pr), $CMe_2NEt$(i-Pr), $CMe_2$Nn-$Pr_2$, $CMe_2$Nn-Pr(i-Pr), $CMe_2$Ni-$Pr_2$, $(CH_2)_2NMe_2$,

(CH$_2$)$_2$NMe(Et), (CH$_2$)$_2$NMe(n-Pr), (CH$_2$)$_2$NMe(i-Pr), (CH$_2$)$_2$NEt$_2$, (CH$_2$)$_2$NEt(n-Pr), (CH$_2$)$_2$NEt(i-Pr), (CH$_2$)$_2$Nn-Pr$_2$, (CH$_2$)$_2$Nn-Pr(i-Pr), (CH$_2$)$_2$Ni-Pr$_2$, CH$_2$CN, CHMeCN, C(Me)$_2$CN, (CH$_2$)$_2$CN, CHMeCH$_2$CN, CMe$_2$CH$_2$CN, CH$_2$CHMeCN, CH$_2$CMe$_2$CN, CHMeCHMeCN, CMe$_2$CHMeCN, CHMeCMe$_2$CN, CMe$_2$CMe$_2$CN, (CH$_2$)$_3$CN, CH$_2$CH(O), CHMeCH(O), C(Me)$_2$CH(O), (CH$_2$)$_2$CH(O), CHMeCH$_2$CH(O), CMe$_2$CH$_2$CH(O), CH$_2$CHMeCH(O), CH$_2$CMe$_2$CH(O), CHMeCHMeCH(O), CMe$_2$CHMeCH(O), CHMeCMe$_2$CH(O), CMe$_2$CMe$_2$CH(O), (CH$_2$)$_3$CH(O). CH$_2$CO$_2$H, CHMeCO$_2$H, C(Me)$_2$CO$_2$H, (CH$_2$)$_2$CO$_2$H, CHMeCH$_2$CO$_2$H, CMe$_2$CH$_2$CO$_2$H, CH$_2$CHMeCO$_2$H, CH$_2$CMe$_2$CO$_2$H, CHMeCHMeCO$_2$H, CMe$_2$CHMeCO$_2$H, CHMeCMe$_2$CO$_2$H, CMe$_2$CMe$_2$CO$_2$H, (CH$_2$)$_3$CO$_2$H, CH$_2$C(O)Me, CHMeC(O)Me, C(Me)$_2$C(O)Me, (CH$_2$)$_2$C(O)Me, CHMeCH$_2$C(O)Me, CMe$_2$CH$_2$C(O)Me, CH$_2$CHMeC(O)Me, CH$_2$CMe$_2$C(O)Me, CHMeCHMeC(O)Me, CMe$_2$CHMeC(O)Me, CHMeCMe$_2$C(O)Me, CMe$_2$CMe$_2$C(O)Me, (CH$_2$)$_3$C(O)Me, CH$_2$C(O)Et, CHMeC(O)Et, C(Me)$_2$C(O)Et, (CH$_2$)$_2$C(O)Et, CHMeCH$_2$C(O)Et, CMe$_2$CH$_2$C(O)Et, CH$_2$CHMeC(O)Et, CH$_2$CMe$_2$C(O)Et, CHMeCHMeC(O)Et, CMe$_2$CHMeC(O)Et, CHMeCMe$_2$C(O)Et, CMe$_2$CMe$_2$C(O)Et, (CH$_2$)$_3$C(O)Et, CH$_2$C(O)n-Pr, CHMeC(O)n-Pr, C(Me)$_2$C(O)n-Pr, (CH$_2$)$_2$C(O)n-Pr, CHMeCH$_2$C(O)n-Pr, CMe$_2$CH$_2$C(O)n-Pr, CH$_2$CHMeC(O)n-Pr, CH$_2$CMe$_2$C(O)n-Pr, CHMeCHMeC(O)n-Pr, CMe$_2$CHMeC(O)n-Pr, CHMeCMe$_2$C(O)n-Pr, CMe$_2$CMe$_2$C(O)n-Pr, (CH$_2$)$_3$C(O)n-Pr, CH$_2$C(O)i-Pr, CHMeC(O)i-Pr, C(Me)$_2$C(O)i-Pr, (CH$_2$)$_2$C(O)i-Pr, CHMeCH$_2$C(O)i-Pr, CMe$_2$CH$_2$C(O)i-Pr, CH$_2$CHMeC(O)i-Pr, CH$_2$CMe$_2$C(O)i-Pr, CHMeCHMeC(O)i-Pr, CMe$_2$CHMeC(O)i-Pr, CHMeCMe$_2$C(O)i-Pr, CMe$_2$CMe$_2$C(O)i-Pr, (CH$_2$)$_3$C(O)i-Pr, CH$_2$C(O)n-Bu, CHMeC(O)n-Bu, C(Me)$_2$C(O)n-Bu, (CH$_2$)$_2$C(O)n-Bu, CHMeCH$_2$C(O)n-Bu, CMe$_2$CH$_2$C(O)n-Bu, CH$_2$CHMeC(O)n-Bu, CH$_2$CMe$_2$C(O)n-Bu, CHMeCHMeC(O)n-Bu, CMe$_2$CHMeC(O)n-Bu, CHMeCMe$_2$C(O)n-Bu, CMe$_2$CMe$_2$C(O)n-Bu, (CH$_2$)$_3$C(O)n-Bu, CH$_2$CO$_2$Me, CHMeCO$_2$Me, C(Me)$_2$CO$_2$Me, (CH$_2$)$_2$CO$_2$Me, CHMeCH$_2$CO$_2$Me, CMe$_2$CH$_2$CO$_2$Me, CH$_2$CHMeCO$_2$Me, CH$_2$CMe$_2$CO$_2$Me, CHMeCHMeCO$_2$Me, CMe$_2$CHMeCO$_2$Me, CHMeCMe$_2$CO$_2$Me, CMe$_2$CMe$_2$CO$_2$Me, (CH$_2$)$_3$CO$_2$Me, CH$_2$CO$_2$Et, CHMeCO$_2$Et, C(Me)$_2$CO$_2$Et, (CH$_2$)$_2$CO$_2$Et, CHMeCH$_2$CO$_2$Et, CMe$_2$CH$_2$CO$_2$Et, CH$_2$CHMeCO$_2$Et, CH$_2$CMe$_2$CO$_2$Et, CHMeCHMeCO$_2$Et, CMe$_2$CHMeCO$_2$Et, CHMeCMe$_2$CO$_2$Et, CMe$_2$CMe$_2$CO$_2$Et, (CH$_2$)$_3$CO$_2$Et, CH$_2$CO$_2$n-Pr, CHMeCO$_2$n-Pr, C(Me)$_2$CO$_2$n-Pr, (CH$_2$)$_2$CO$_2$n-Pr, CHMeCH$_2$CO$_2$n-Pr, CMe$_2$CH$_2$CO$_2$n-Pr, CH$_2$CHMeCO$_2$n-Pr, CH$_2$CMe$_2$CO$_2$n-Pr, CHMeCHMeCO$_2$n-Pr, CMe$_2$CHMeCO$_2$n-Pr, CHMeCMe$_2$CO$_2$n-Pr, CMe$_2$CMe$_2$CO$_2$n-Pr, (CH$_2$)$_3$CO$_2$n-Pr, CH$_2$CO$_2$i-Pr, CHMeCO$_2$i-Pr, C(Me)$_2$CO$_2$i-Pr, (CH$_2$)$_2$CO$_2$i-Pr, CHMeCH$_2$CO$_2$i-Pr, CMe$_2$CH$_2$CO$_2$i-Pr, CH$_2$CHMeCO$_2$i-Pr, CH$_2$CMe$_2$CO$_2$i-Pr, CHMeCHMeCO$_2$i-Pr, CMe$_2$CHMeCO$_2$i-Pr, CHMeCMe$_2$CO$_2$i-Pr, CMe$_2$CMe$_2$CO$_2$i-Pr, (CH$_2$)$_3$CO$_2$i-Pr, CH$_2$CO$_2$n-Bu, CHMeCO$_2$n-Bu, C(Me)$_2$CO$_2$n-Bu, (CH$_2$)$_2$CO$_2$n-Bu, CHMeCH$_2$CO$_2$n-Bu, CMe$_2$CH$_2$CO$_2$n-Bu, CH$_2$CHMeCO$_2$n-Bu, CH$_2$CMe$_2$CO$_2$n-Bu, CHMeCHMeCO$_2$n-Bu, CMe$_2$CHMeCO$_2$n-Bu, CHMeCMe$_2$CO$_2$n-Bu, CMe$_2$CMe$_2$CO$_2$n-Bu, (CH$_2$)$_3$CO$_2$n-Bu, CH$_2$OCH$_2$OMe, CHMeOCH$_2$OMe, CMe$_2$OCH$_2$OMe, CH$_2$OCHMeOMe, CH$_2$OCMe$_2$OMe, CHMeOCHMeOMe, CMe$_2$OCHMeOMe, CHMeOCMe$_2$OMe, CMe$_2$OCMe$_2$OMe, CH$_2$OCH$_2$OEt, CHMeOCH$_2$OEt, CMe$_2$OCH$_2$OEt, CH$_2$OCHMeOEt, CH$_2$OCMe$_2$OEt, CHMeOCHMeOEt, CMe$_2$OCHMeOEt, CHMeOCMe$_2$OEt, CMe$_2$OCMe$_2$OEt, CH$_2$OCH$_2$On-Pr, CHMeOCH$_2$On-Pr, CMe$_2$OCH$_2$On-Pr, CH$_2$OCHMeOn-Pr, CH$_2$OCMe$_2$On-Pr, CHMeOCHMeOn-Pr, CMe$_2$OCHMeOn-Pr, CHMeOCMe$_2$On-Pr, CMe$_2$OCMe$_2$On-Pr, CH$_2$OCH$_2$Oi-Pr, CHMeOCH$_2$Oi-Pr, CMe$_2$OCH$_2$Oi-Pr, CH$_2$OCHMeOi-Pr, CH$_2$OCMe$_2$Oi-Pr, CHMeOCHMeOi-Pr, CMe$_2$OCHMeOi-Pr, CHMeOCMe$_2$Oi-Pr, CMe$_2$OCMe$_2$Oi-Pr, (CH$_2$)$_2$OCH$_2$OMe, CH$_2$O(CH$_2$)$_2$OMe, CHMeO(CH$_2$)$_2$OMe, (CH$_2$)$_2$O(CH$_2$)$_2$OME, (CH$_2$)$_2$OCH$_2$OEt, CH$_2$O(CH$_2$)$_2$OEt, (CH$_2$)$_2$O(CH$_2$)$_2$OEt, (CH$_2$)$_2$OCH$_2$On-Pr, CH$_2$O(CH$_2$)$_2$On-Pr, (CH$_2$)$_2$O(CH$_2$)$_2$On-Pr, (CH$_2$)$_2$OCH$_2$Oi-Pr, CH$_2$O(CH$_2$)$_2$Oi-Pr, (CH$_2$)$_2$O(CH$_2$)$_2$Oi-Pr, CH$_2$O(CH$_2$)$_3$OMe, CH$_2$O(CH$_2$)$_3$OEt, CH$_2$O(CH$_2$)$_3$On-Pr, CH$_2$O(CH$_2$)$_3$Oi-Pr, CH$_2$OCH$_2$c-Pr, CH$_2$OCH$_2$c-Bu, CH$_2$OCH$_2$c-Pen, CHMeOCH$_2$c-Pr, CHMeOCH$_2$c-Bu, CHMeOCH$_2$c-Pen, CMe$_2$OCH$_2$c-Pr, CMe$_2$OCH$_2$c-Bu, CMe$_2$OCH$_2$c-Pen, CH$_2$OCHMec-Pr, CH$_2$OCHMec-Bu, CH$_2$OCHMec-Pen, CH$_2$OCMe$_2$c-Pr, CH$_2$OCMe$_2$c-Bu, CH$_2$OCMe$_2$c-Pen, CHMeOCHMec-Pr, CHMeOCHMec-Bu, CHMeOCHMec-Pen, CMe$_2$OCHMec-Pr, CMe$_2$OCHMec-Bu, CMe$_2$OCHMec-Pen, CMe$_2$OCMe$_2$c-Pr, CMe$_2$OCMe$_2$c-Bu, CMe$_2$OCMe$_2$c-Pen, (CH$_2$)$_2$OCH$_2$c-Pr, CH$_2$O(CH$_2$)$_2$c-Pr, (CH$_2$)$_2$O(CH$_2$)$_2$c-Pr, (CH$_2$)$_2$OCH$_2$c-Bu, CH$_2$O(CH$_2$)$_2$c-Bu, (CH$_2$)$_2$O(CH$_2$)$_2$c-Bu, (CH$_2$)$_2$OCH$_2$c-Pen, CH$_2$O(CH$_2$)$_2$c-Pen, (CH$_2$)$_2$O(CH$_2$)$_2$c-Pen, CH$_2$OCH$_2$(2-F-c-Pr), CH$_2$OCH$_2$(2,3-F$_2$-c-Pr), CH$_2$OCH$_2$ (2,2-F$_2$-c-Pr), CH$_2$OCH$_2$(2-Cl-c-Pr), CH$_2$OCH$_2$(2,3-Cl$_2$-c-Pr). CH$_2$OCH$_2$(2,2-Cl$_2$-c-Pr), CH$_2$OCH$_2$(2-Br-c-Pr), CH$_2$OCH$_2$(2,3-Br$_2$-c-Pr), CH$_2$OCH$_2$(2,2-Br$_2$-c-Pr), CHMeOCH$_2$(2-F-c-Pr), CHMeOCH$_2$(2,2-F$_2$-c-Pr), CHMeOCH$_2$(2-Cl-c-Pr), CHMeOCH$_2$(2,2-Cl$_2$-c-Pr), CHMeOCH$_2$(2-Br-c-Pr), CHMeOCH$_2$(2,2-Br$_2$-c-Pr), CMe$_2$OCH$_2$(2-F-c-Pr), CMe$_2$OCH$_2$(2,2-F$_2$-c-Pr), CMe$_2$OCH$_2$(2-Cl-c-Pr), CMe$_2$OCH$_2$(2,2-Cl$_2$-c-Pr), CMe$_2$OCH$_2$(2-Br-c-Pr), CMe$_2$OCH$_2$(2,2-Br$_2$-c-Pr), CH$_2$OCHMe(2-F-c-Pr), CH$_2$OCHMe(2,2-F$_2$-c-Pr), CH$_2$OCHMe(2-Cl-c-Pr), CH$_2$OCHMe(2,2-Cl$_2$-c-Pr), CH$_2$OCHMe(2-Br-c-Pr), CH$_2$OCHMe(2,2-Br$_2$-c-Pr), CH$_2$OCMe$_2$(2-F-c-Pr), CH$_2$OCMe$_2$ (2,2-F$_2$-c-Pr), CH$_2$OCMe$_2$(2-Cl-c-Pr), CH$_2$OCMe$_2$(2,2-Cl$_2$-c-Pr), CH$_2$OCMe$_2$(2-Br-c-Pr), CH$_2$OCMe$_2$(2,2-Br$_2$-c-Pr), CHMeOCHMe(2-F-c-Pr), CHMeOCHMe(2,2-F$_2$-c-Pr), CHMeOCHMe(2-Cl-c-Pr), CHMeOCHMe(2,2-Cl$_2$-c-Pr), CHMeOCHMe(2-Br-c-Pr), CHMe-OCHMe(2,3-Br$_2$-c-Pr), CHMeOCMe$_2$(2-F-c-Pr), CHMeOCMe$_2$(2,2-F$_2$-c-Pr), CHMeOCMe$_2$(2-Cl-c-Pr), CHMeOCMe$_2$(2,2-Cl$_2$-c-Pr), CHMeOCMe$_2$(2-Br-c-Pr), CHMeOCMe$_2$(2,2-Br$_2$-c-Pr), CMe$_2$OCMe$_2$(2-F-c-Pr), CMe$_2$OCMe$_2$(2,2-F$_2$-c-Pr), CMe$_2$OCMe$_2$(2-Cl-c-Pr), CMe$_2$OCMe$_2$(2,2-Cl$_2$-c-Pr), CMe$_2$OCMe$_2$(2-Br-c-Pr), CMe$_2$OCMe$_2$(2,2-Br$_2$-c-Pr), OH, OMe, OEt, On-Pr, Oi-Pr, On-Bu, Oi-Bu, Os-Bu, Ot-Bu, On-Pen, OCHMe(n-Pr), OCH$_2$s-Bu, O(CH$_2$)$_2$i-Pr, OCH(Et)$_2$, OC(Me)$_2$Et, OCHMe(i-Pr), OCH$_2$t-Bu, On-Hex, OCHMe(n-Bu), OCH$_2$CHMe(n-Pr), O(CH$_2$)$_2$s-Bu, O(CH$_2$)$_3$i-Pr, OC(Me)$_2$n-Pr, OCHMe(s-Bu), OCHMe(i-Bu), OCH$_2$C(Me)$_2$Et, OCH$_2$CHMe(i-Pr), O(CH$_2$)$_2$t-

Bu, OCHEt(n-Pr), $OCH_2CH(Et)_2$, $OCMe(Et)_2$, OCHEt(i-Pr), $OC(Me)_2$i-Pr, OCHMe(t-Bu), $OCH=CH_2$, $OCMe=CH_2$, $OCH_2CH=CH_2$, OCH=CHMe, OCH=CHEt, $OCH_2CH=CHMe$, $O(CH_2)_2CH=CH_2$, $OCHEt=CH_2$, OCMe=CHMe, $OCH=CMe_2$, $OCHMeCH=CH_2$, $OCH_2CMe=CH_2$, OCMe=CHEt, OCH=CMe(Et), OCH=CH(i-Pr), OCHMeCH=CHMe, $OCH_2CMe=CHMe$, $OCH_2CH=CMe_2$, $OCHMeCH_2CH=CH_2$, $OCH_2CHMeCH=CH_2$, $O(CH_2)_2CMe=CH_2$, $OCH_2CEt=CH_2$, $OCHEtCH=CH_2$, OCEt=CHMe, $OCMe=CMe_2$, $OCMe_2CH=CH_2$, $OCHMeCMe=CH_2$, $OC(i-Pr)=CH_2$, $OC(n-Pr)=CH_2$, $OCH_2CH=CH(n-Pr)$, $O(CH_2)_2CH=CHEt$, $O(CH_2)_3CH=CHMe$, $O(CH_2)_4CH=CH_2$, $OCH_2CMe=CHEt$, $OCH_2CH=CMe(Et)$, $OCH_2CH=CH(i-Pr)$, $OCHMeCH_2CH=CHMe$, $OCH_2CHMeCH=CHMe$, $O(CH_2)_2CMe=CHMe$, $O(CH_2)_2CH=CMe_2$, $OCHMe(CH_2)_2CH=CH_2$, $OCH_2CHMeCH_2CH=CH_2$, $O(CH_2)_2CHMeCH=CH_2$, $O(CH_2)_3CMe=CH_2$, $OCH_2CH=CMe(Et)$, $OCH_2CMe=CMe_2$, OC≡CH, OC≡CMe, OC≡CEt, OC≡Cn-Pr, OC≡Ci-Pr, OC≡Cn-Bu, OC≡Ci-Bu, OC≡Cs-Bu, OC≡Ct-Bu, $OCH_2C$≡CH, $OCH_2C$≡CMe, $OCH_2C$≡CEt, $OCH_2C$≡Cn-Pr, $OCH_2C$≡Ci-Pr, OCH(Me)C≡CH, OCH(Me)C≡CMe, OCH(Me)C≡CEt, OCH(Et)C≡CH, OCH(Et)C≡CMe, $OC(Me)_2C$≡CH, $OC(Me)_2C$≡CMe, OC(Me)(Et)C≡CH, $O(CH_2)_2C$≡CH, $O(CH_2)_2C$≡CMe, $O(CH_2)_2C$≡CEt, $OCH(Me)CH_2C$≡CH, $OCH(Me)CH_2C$≡CMe, $OCH(Et)CH_2C$≡CH, $OC(Me)_2CH_2C$ ≡CH, $OCH_2CH(Me)C$≡CH, $OCH_2CH(Me)C$≡CMe, $OCH_2C(Me)_2C$≡CH, $OCH_2CH(Et)C$≡CH, $O(CH_2)_3C$≡CH, $O(CH_2)_3C$≡CMe, $OCH(Me)(CH_2)_2C$≡CH, $OCH_2CH(Me)CH_2C$≡CH, $O(CH_2)_2CH(Me)C$≡CH, $O(CH_2)_4C$≡CH, $OCH_2Cl$, $OCH_2Br$, $OCF_3$, $OCHF_2$, $OCHCl_2$, $OCH_2CF_3$, $OCH_2CCl_3$, $OCCl_3$, $O(CH_2)_2F$, $O(CH_2)_2Cl$, $O(CH_2)_2Br$, $O(CH_2)_2I$, $OC_2F_5$, OCHMeCl, OCHMeBr, $OCHClCH_2Cl$, $OCHBrCH_2Br$, $O(CH_2)_3Cl$, $O(CH_2)_3Br$, $OCMeClCH_2Cl$, $OCMeBrCH_2Br$, $OCH_2CHClCH_2Cl$, $OCH_2CHBrCH_2Br$, $O(CH_2)_4F$, $O(CH_2)_4Cl$, $OCH_2CHClCHCMe$, $O(CH_2)_2CHClCH_2Cl$, $OCHMeCHClCH_2Cl$, $OCH_2CMeClCH_2Cl$, $O(CH_2)_5F$, $O(CH_2)_5Cl$, $O(CH_2)_5Br$, $OCH_2CHClCMe_2Cl$, $OCHMeCH_2CHClCH_2Cl$, $O(CH_2)_2CMeClCH_2Cl$, $O(CH_2)_6Cl$, $OCH_2CHClCHCl$(n-Pr), $OCH_2CHClCHCl$(i-Pr), OC(O)Me, OC(O)Et, OC(O)n-Pr, OC(O)i-Pr, OC(O)n-Bu, OC(O)i-Bu, OC(O)s-Bu, OC(O)t-Bu, OC(O)n-Pen, epoxyethyl (hereinafter referred to as "e-Et"), 1-Me-e-Et, 2-Me-e-Et, 1,2-$Me_2$-e-Et, 2,3-$Me_2$-e-Et, 2,2-$Me_2$-e-Et, 1,2,2-$Me_3$-e-Et, 1,2,3-$Me_3$-e-Et, 2,2,3-$Me_3$-e-Et, 1,2,2,3-$Me_4$-e-Et, 2,2,3,3-$Me_4$-e-Et, 1,2,2,3,3-$Me_5$-e-Et, 1-Me-2-Cl-e-Et, 1,2-$Me_2$-2-Cl-e-Et, 1,3-$Me_2$-2-Cl-e-Et, 2-Cl-1,2,3-$Me_3$-e-Et, 2-Cl-Me-1,2,3,3-$Me_4$-e-Et, 1-Cl-2-Me-e-Et, 1-Cl-2,2-$Me_2$-e-Et, 1-Cl-2,3-$Me_2$-e-Et, 1-Cl-2,2,3-$Me_3$-e-Et, 1-Cl-2,2,3,3-$Me_4$-e-Et, 1,2-$Cl_2$-2-Me-e-Et, 1,2-$Cl_2$-3-Me-e-Et, 1,2-$Cl_2$-2,3-$Me_2$-e-Et, 1,2-$Cl_2$-3,3-$Me_2$-e-Et, 1,2-$Cl_2$-2,3, 4-$Me_3$-e-Et, 1,2,2-$Cl_3$-3-Me-e-Et, 1,2,2-$Cl_3$-3,3-$Me_2$-e-Et, 1,2,3-$Cl_3$-2-Me-e-Et, 1,2,3-$Cl_3$-3-Me-e-Et, 1,2,3-$Cl_3$-2,3-$Me_2$-e-Et, 1,2,2,3-$Cl_4$-3-Me-e-Et, $CH_2$-e-Et, $CH_2$(1-Me-e-Et), $CH_2$(2-Me-e-Et), $CH_2$(1,2-$Me_2$-e-Et), $CH_2$(2,3-$Me_2$-e-Et), $CH_2$(2,2-$Me_2$-e-Et), $CH_2$(1,2,2-$Me_3$-e-Et), $CH_2$(1,2,3-$Me_3$-e-Et), $CH_2$(2,2,3-$Me_3$-e-Et), $CH_2$(1,2,2,3-$Me_4$-e-Et), $CH_2$(2,2,3,3-$Me_4$-e-Et), $CH_2$(1,2,2,3,3-$Me_5$-e-Et), $CH_2$(1-Me-2-Cl-e-Et), $CH_2$(1,2-$Me_2$-2-Cl-e-Et), $CH_2$(1,3-$Me_2$-2-Cl-e-Et), $CH_2$(2-Cl-1,2,3-$Me_3$-e-Et), $CH_2$(2-Cl-Me-1,2,3,3-$Me_4$-e-Et), $CH_2$(1-Cl-2-Me-e-Et), $CH_2$(1-Cl-2,2-$Me_2$-e-Et), $CH_2$(1-Cl-2,3-$Me_2$-e-Et), $CH_2$(1-Cl-2,2,3-$Me_3$-e-Et), $CH_2$(1-Cl-2,2,3,3-$Me_4$-e-Et), $CH_2$(1,2-$Cl_2$-2-Me-e-Et), $CH_2$(1,2-$Cl_2$-3-Me-e-Et), $CH_2$(1,2-$Cl_2$-2,3-$Me_2$-e-Et), $CH_2$(1,2-$Cl_2$-3,3-$Me_2$-e-Et), $CH_2$(1,2-$Cl_2$-2,3,4-$Me_3$-e-Et), $CH_2$(1,2,2-$Cl_3$-3-Me-e-Et), $CH_2$(1,2,2-$Cl_3$-3,3-$Me_2$-e-Et), $CH_2$(1,2,3-$Cl_3$-2-Me-e-Et), $CH_2$(1,2,3-$Cl_3$-3-Me-e-Et), $CH_2$(1,2,3-$Cl_3$-2,3-$Me_2$-e-Et), $CH_2$(1,2,2,3-$Cl_4$-3-Me-e-Et), CHMe-e-Et, CHMe(1-Me-e-Et), CHMe(2-Me-e-Et), CHMe(1,2-$Me_2$-e-Et), CHMe(2,3-$Me_2$-e-Et), CHMe(2,2-$Me_2$-e-Et), CHMe(1,2,2-$Me_3$-e-Et), CHMe(1,2,3-$Me_3$-e-Et), CHMe(2,2,3-$Me_3$-e-Et), CHMe(1,2,2,3-$Me_4$-e-Et), CHMe(2,2,3,3-$Me_4$-e-Et), CHMe(1,2,2,3,3-$Me_5$-e-Et), CHMe(1-Me-2-Cl-e-Et), CHMe(1,2-$Me_2$-2-Cl-e-Et), CHMe(1,3-$Me_2$-2-Cl-e-Et), CHMe(2-Cl-1), CHMe(2,3-$Me_3$-e-Et), CHMe(2-Cl-Me-1,2,3,3-$Me_4$-e-Et), CHMe(1-Cl-2-Me-e-Et), CHMe(1-Cl-2,2-$Me_2$-e-Et), CHMe(1-Cl-2,3-$Me_2$-e-Et), CHMe(1-Cl-2,2,3-$Me_3$-e-Et), CHMe(1-Cl-2,2,3,3-$Me_4$-e-Et), CHMe(1,2-$Cl_2$-2-Me-e-Et), CHMe(1,2-$Cl_2$-3-Me-e-Et), CHMe(1,2-$Cl_2$-2,3-$Me_2$-e-Et), CHMe(1,2-$Cl_2$-3,3-$Me_2$-e-Et), CHMe(1,2-$Cl_2$-2,3,4-$Me_3$-e-Et), CHMe(1,2,2-$Cl_3$-3-Me-e-Et), CHMe(1,2,2-$Cl_3$-3,3-$Me_2$-e-Et), CHMe(1,2,3-$Cl_3$-2-Me-e-Et), CHMe(1,2,3-$Cl_3$-3-Me-e-Et), CHMe(1,2,3-$Cl_3$-2,3-$Me_2$-e-Et), CHMe(1,2,2,3-$Cl_4$-3-Me-e-Et), $CMe_2$-e-Et, $CMe_2$(1-Me-e-Et), $CMe_2$(2-Me-e-Et), $CMe_2$(1,2-$Me_2$-e-Et), $CMe_2$(2,3-$Me_2$-e-Et), $CMe_2$(2,2-$Me_2$-e-Et), $CMe_2$(1,2,2-$Me_3$-e-Et), $CMe_2$(1,2,3-$Me_3$-e-Et),$CMe_2$(2,2,3-$Me_3$-e-Et), $CMe_2$(1,2,2,3-$Me_4$-e-Et), $CMe_2$(2,2,3,3-$Me_4$-e-Et), $CMe_2$(1,2,2,3,3-$Me_5$-e-Et), $CMe_2$(1-Me-2-Cl-e-Et), $CMe_2$(1,2-$Me_2$-2-Cl-e-Et), $CMe_2$(1,3-$Me_2$-2-Cl-e-Et), $CMe_2$(2-Cl-1,2,3-$Me_3$-e-Et), $CMe_2$(2-Cl-Me-1,2,3,3-$Me_4$-e-Et), $CMe_2$(1-Cl-2-Me-e-Et), $CMe_2$(1-Cl-2,2-$Me_2$-e-Et), $CMe_2$(1-Cl-2,3-$Me_2$-e-Et), $CMe_2$(1-Cl-2,2,3-$Me_3$-e-Et), $CMe_2$(1-Cl-2,2,3,3-$Me_4$-e-Et), $CMe_2$(1,2-$Cl_2$-2-Me-e-Et), $CMe_2$(1,2-$Cl_2$-3-Me-e-Et), $CMe_2$(1,2-$Cl_2$-2,3-$Me_2$-e-Et), $CMe_2$(1,2-$Cl_2$-3,3-$Me_2$-e-Et), $CMe_2$(1,2-$Cl_2$-2,3,4-$Me_3$-e-Et), $CMe_2$(1,2,2-$Cl_3$-3-Me-e-Et), $CMe_2$(1,2,2-$Cl_3$-3,3-$Me_2$-e-Et), $CMe_2$(1,2,3-$Cl_3$-2-Me-e-Et), $CMe_2$(1,2,3-$Cl_3$-3-Me-e-Et), $CMe_2$(1,2,3-$Cl_3$-2,3-$Me_2$-e-Et), $CMe_2$(1,2,2,3-$Cl_4$-3-Me-e-Et), $(CH_2)_2$(1-Me-e-Et), $(CH_2)_2$(2-Me-e-Et), $(CH_2)_2$(1,2-$Me_2$-e-Et), $(CH_2)_2$(2,3-$Me_2$-e-Et), $(CH_2)_2$(2,2-$Me_2$-e-Et), $(CH_2)_2$(1,2,2-$Me_3$-e-Et), $(CH_2)_2$(1,2,3-$Me_3$-e-Et), $(CH_2)_2$(2,2,3-$Me_3$-e-Et), $(CH_2)_2$(1,2,2,3-$Me_4$-e-Et), $(CH_2)_2$(2,2,3,3-$Me_4$-e-Et), $(CH_2)_2$(1,2,2,3,3-$Me_5$-e-Et), $(CH_2)_2$(1-Me-2-Cl-e-Et), $(CH_2)_2$(1,2-$Me_2$-2-Cl-e-Et), $(CH_2)_2$(1,3-$Me_2$-2-Cl-e-Et), $(CH_2)_2$(2-Cl-1,2,3-$Me_3$-e-Et), $(CH_2)_2$(2-Cl-Me-1,2,3, 3-$Me_4$-e-Et), $(CH_2)_2$(1-Cl-2-Me-e-Et), $(CH_2)_2$(1-Cl-2,2-$Me_2$-e-Et), $(CH_2)_2$(1-Cl-2,3-$Me_2$-e-Et), $(CH_2)_2$(1-Cl-2,2,3-$Me_3$-e-Et), $(CH_2)_2$(1-Cl-2,2,3,3-$Me_4$-e-Et), $(CH_2)_2$(1,2-$Cl_2$-2-Me-e-Et), $(CH_2)_2$(1,2-$Cl_2$-3-Me-e-Et), $(CH_2)_2$(1,2-$Cl_2$-2,3-$Me_2$-e-Et), $(CH_2)_2$(1,2-$Cl_2$-3,3-$Me_2$-e-Et), $(CH_2)_2$(1,2-$Cl_2$-2,3,4-$Me_3$-e-Et), $(CH_2)_2$(1,2,2-$Cl_3$-3-Me-e-Et), $(CH_2)_2$(1,2,2-$Cl_3$-3,3-$Me_2$-e-Et), $(CH_2)_2$(1,2,3-$Cl_3$-2-Me-e-Et), $(CH_2)_2$(1,2,3-$Cl_3$-3-Me-e-Et), $(CH_2)_2$(1,2,3-$Cl_3$-2,3-$Me_2$-e-Et), $(CH_2)_2$(1,2,2,3-$Cl_4$-3-Me-e-Et), SH, SMe, SEt, Sn-Pr, Si-Pr, Sn-Bu, Si-Bu, Ss-Bu, St-Bu, Sn-Pen, SCHMe(n-Pr), $SCH_2$s-Bu, $S(CH_2)_2$i-Pr, $SCH(Et)_2$, $SC(Me)_2Et$, SCHMe(i-Pr), $SCH_2$t-Bu, Sn-Hex, SCHMe(n-Bu), $SCH_2CHMe$(n-Pr), $S(CH_2)_2$s-Bu, $S(CH_2)_3$i-Pr, $SC(Me)_2$n-Pr, SCHMe(s-Bu), SCHMe(i-Bu),

$SCH_2C(Me)_2Et$, $SCH_2CHMe(i-Pr)$, $S(CH_2)_2t-Bu$, SCHEt(n-Pr), $SCH_2CH(Et)_2$, $SCMe(Et)_2$, SCHEt(i-Pr), $SC(Me)_2i-Pr$, SCHMe(t-Bu), $SCH=CH_2$, $SCMe=CH_2$, $SCH_2CH=CH_2$, SCH=CHMe, SCH=CHEt, $SCH_2CH=CHMe$, $S(CH_2)_2CH=CH_2$, $SCHEt=CH_2$, SCMe=CHMe, $SCH=CMe_2$, $SCHMeCH=CH_2$, $SCH_2CMe=CH_2$, SCMe=CHEt, SCH=CMe(Et), SCH=CH(i-Pr), SCHMeCH=CHMe, $SCH_2CMe=CHMe$, $SCH_2CH=CMe_2$, $SCHMeCH_2CH=CH_2$, $SCH_2CHMeCH=CH_2$, $S(CH_2)_2CMe=CH_2$, $SCH_2CEt=CH_2$, $SCHEtCH=CH_2$, SCEt=CHMe, $SCMe=CMe_2$, $SCMe_2CH=CH_2$, $SCHMeCMe=CH_2$, $SC(i-Pr)=CH_2$, $SC(n-Pr)=CH_2$, $SCH_2CH=CH(n-Pr)$, $S(CH_2)_2CH=CHEt$, $S(CH_2)_3CH=CHMe$, $S(CH_2)_4CH=CH_2$, $SCH_2CMe=CHEt$, $SCH_2CH=CMe(Et)$, $SCH_2CH=CH(i-Pr)$, $SCHMeCH_2CH=CHMe$, $SCH_2CHMeCH=CHMe$, $S(CH_2)_2CMe=CHMe$, $S(CH_2)_2CH=CMe_2$, $SCHMe(CH_2)_2CH=CH_2$, $SCH_2CHMeCH_2CH=CH_2$, $S(CH_2)_2CHMeCH=CH_2$, $S(CH_2)_3CMe=CH_2$, $SCH_2CH=CMe(Et)$, $SCH_2CMe=CMe_2$, SC≡CH, SC≡CMe, SC≡CEt, SC≡Cn-Pr, SC≡Ci-Pr, SC≡Cn-Bu, SC≡Ci-Bu, SC≡Cs-Bu, SC≡Ct-Bu, $SCH_2C\equiv CH$, $SCH_2C\equiv CMe$, $SCH_2C\equiv CEt$, $SCH_2C\equiv Cn-Pr$, $SCH_2C\equiv Ci-Pr$, SCH(Me)C≡CH, SCH(Me)C≡CMe, SCH(Me)C≡CEt, SCH(Et)C≡CH, SCH(Et)C≡CMe, $SC(Me)_2C\equiv CH$, $SC(Me)_2C\equiv CMe$, SC(Me)(Et)C≡CH, $S(CH_2)_2C\equiv CH$, $S(CH_2)_2C\equiv CMe$, $S(CH_2)_2C\equiv CEt$, $SCH(Me)CH_2C\equiv CH$, $SCH(Me)CH_2C\equiv CMe$, $SCH(Et)CH_2C\equiv CH$, $SC(Me)_2CH_2C\equiv CH$, $SCH_2CH(Me)C\equiv CH$, $SCH_2CH(Me)C\equiv CMe$, $SCH_2C(Me)_2C\equiv CH$, $SCH_2CH(Et)C\equiv CH$, $S(CH_2)_3C\equiv CH$, $S(CH_2)_3C\equiv CMe$, $SCH(Me)(CH_2)_2C\equiv CH$, $SCH_2CH(Me)CH_2C\equiv CH$, $S(CH_2)_2CH(Me)C\equiv CH$, $S(CH_2)_4C\equiv CH$, $SCH_2Cl$, $SCH_2Br$, $SCF_3$, $SCHF_2$, $SCHCl_2$, $SCH_2CF_3$, $SCH_2CCl_3$, $SCCl_3$, $S(CH_2)_2F$, $S(CH_2)_2Cl$, $S(CH_2)_2Br$, $S(CH_2)_2I$, $SC_2F_5$, SCHMeCl, SCHMeBr, $SCHClCH_2Cl$, $SCHBrCH_2Br$, $S(CH_2)_3Cl$, $S(CH_2)_3Br$, $SCMeClCH_2Cl$, $SCMeBrCH_2Br$, $SCH_2CHClCH_2Cl$, $SCH_2CHBrCH_2Br$, $S(CH_2)_4F$, $S(CH_2)_4Cl$, $SCH_2CHClCHClMe$, $S(CH_2)_2CHClCH_2Cl$, $SCHMeCHClCH_2Cl$, $SCH_2CMeClCH_2Cl$, $S(CH_2)_5F$, $S(CH_2)_5Cl$, $S(CH_2)_5Br$, $SCH_2CHClCMe_2Cl$, $SCHMeCH_2CHClCH_2Cl$, $S(CH_2)_2CMeClCH_2Cl$, $S(CH_2)_6Cl$, $SCH_2CHClCHCl(n-Pr)$, $SCH_2CHClCHCl(i-Pr)$, $NH_2$, NHMe, NHEt, NHn-Pr, NHi-Pr, NHn-Bu, NHi-Bu, NHs-Bu, NHt-Bu, NHn-Pen, NHCHMe(n-Pr), $NHCH_2s-Bu$, $NH(CH_2)_2i-Pr$, $NHCH(Et)_2$, $NHC(Me)_2Et$, NHCHMe(i-Pr), $NHCH_2t-Bu$, NHn-Hex, NHCHMe(n-Bu), $NHCH_2CHMe(n-Pr)$, $NH(CH_2)_2s-Bu$, $NH(CH_2)_3i-Pr$, $NHC(Me)_2n-Pr$, NHCHMe(s-Bu), NHCHMe(i-Bu), $NHCH_2C(Me)_2Et$, $NHCH_2CHMe(i-Pr)$, $NH(CH_2)_2t-Bu$, NHCHEt(n-Pr), $NHCH_2CH(Et)_2$, $NHCMe(Et)_2$, NHCHEt(i-Pr), $NHC(Me)_2i-Pr$, NHCHMe(t-Bu), $NMe_2$, $NEt_2$, $N(n-Pr)_2$, $N(i-Pr)_2$, $N(n-Bu)_2$, $N(i-Bu)_2$, $N(s-Bu)_2$, $N(t-Bu)_2$, $N(n-Pen)_2$, NMe(Et), NMe(n-Pr), NMe(i-Pr), NMe(n-Bu), NMe(s-Bu), NMe(i-Bu), NMe(t-Bu), NMe(n-Pen), NEt(n-Pr), NEt(i-Pr), NEt(n-Bu), NEt(s-Bu), NEt(i-Bu), NEt(t-Bu), NEt(n-Pen), Nn-Pr(i-Pr), Nn-Pr(n-Bu), NI-Pr(n-Bu), Ni-Pr(s-Bu), Ni-Pr(i-Bu), Ni-Pr(t-Bu), Ni-Pr(n-Pen), Nn-Bu(s-Bu), Nn-Bu(i-Bu), Nn-Bu(t-Bu), Nn-Bu(n-Pen), Ns-Bu(i-Bu), Ns-Bu(t-Bu), Ns-Bu(n-Pen), Ni-Bu(t-Bu), Ni-Bu(n-Pen), Nt-Bu(n-Pen), $CH_2OH$, CH(OH)Me, CH(OH)Et, CH(OH)n-Pr, CH(OH)i-Pr, $CH_2SH$, CH(SH)Me, CH(SH)Et, CH(SH)n-Pr, CH(SH)i-Pr, $CMe_2SH$, $CH_2NH_2$, $CH(NH_2)Me$, $CH(NH_2)Et$, $CH(NH_2)n-Pr$, $CH(NH_2)i-Pr$, $CMe_2NH_2$, CH=CHCl, CCl=CHCl, CBr=CHBr, $CH=CCl_2$ , $CH=CBr_2$ , CMe=CHCl, CMe=CHBr, $CH_2CCl=CHCl$, CHMeCCl=CHCl, CHMeCBr=CHBr, $CHMeCH=CBr_2$, $CHMeCH=CCl_2$, $CMe_2CCl=CHCl$, $CMe_2CBr=CHBr$, CHEtCCl=CHCl, CHEtCBr=CHBr, CHn-PrCCl=CHCl, CMe(Et)CCl=CHCl, $C(CH_2Cl)=CH_2$, $C(CH_2Br)=CH_2$, $C(CH_2I)=CH_2$, C≡CCl, C≡CBr, C≡CI, $CH_2C\equiv CCl$, $CH_2C\equiv CBr$, $CH_2C\equiv CI$, CH(Me)C≡CCl, CH(Me)C≡CBr, CH(Me)C≡CI, CH(Et)C=CCl, CH(Et)C≡CBr, CH(Et)C≡CI, $C(Me)_2C\equiv CCl$, $C(Me)_2C\equiv CBr$, $C(Me)_2C\equiv CI$, CHClC≡CH, $CHClCH_2OMe$, $CHBrCH_2OEt$, $CH(OMe)CH_2F$, $CH(OMe)CH_2Cl$, $CH(OMe)CH_2Br$, $CMe(OMe)CH_2Cl$, $CMe(OMe)CH_2Br$, $CMe(OMe)CH_2I$, $CMe(OEt)CH_2Cl$, $CMe(OEt)CH_2Br$, $CMe(Oi-Pr)CH_2Cl$, $CMe(Oi-Pr)CH_2Br$, $CMe(On-Bu)CH_2Cl$, $CMe(On-Bu)CH_2Br$, $CEt(OMe)CH_2Cl$, c-Pr, 1-Me-c-Pr, 2-Me-c-Pr, $1,2-Me_2-c-Pr$, $2,2-Me_2-c-Pr$, $2,3-Me_2-c-Pr$, c-Bu, 1-Me-c-Bu, c-Pen, 1-Me-c-Pen, 2-Me-c-Pen, 3-Me-c-Pen, c-Hex, 1-Me-c-Hex, 2-Me-c-Hex, 4-Me-c-Hex, c-Hep, 1-Cl-c-Pr, 1-Br-c-Pr, 2-F-1-Me-c-Pr, 2-Cl-1-Me-c-Pr, 2-Br-1-Me-c-Pr, $2,2-F_2-1-Me-c-Pr$, $2,2-Cl_2-1-Me-c-Pr$, $2,2-Br_2-1-Me-c-Pr$, 1-Cl-c-Bu, 1-Cl-c-Pen, 2-Cl-1-Me-c-Pen, 1-Cl-c-Hex, 1-Br-c-Hex, $1,2-Cl_2-c-Hex$, $1,2-Br_2-c-Hex$, 2-Cl-1-Me-c-Hex, 4-Br-1-Me-c-Hex, $N_3$, $C(=W)R^{11}$, and the formulas:

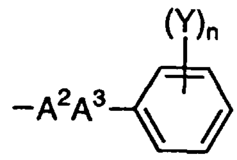

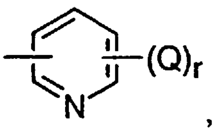 , 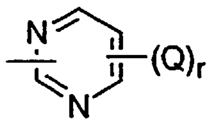 , 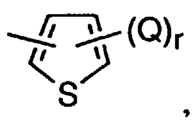 , 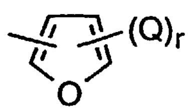 ,

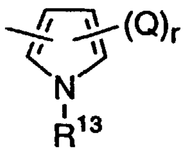 and 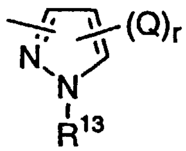

As $R^{11}$, the following radicals can be mentioned:
Et, n-Pr, i-Pr, n-Bu, i-Bu, s-Bu, t-Bu, n-Pen, $CF_3$, $CH_2Cl$, $CH_2Br$, $CCl_3$, $CHF_2$, $(CH_2)_2F$, $(CH_2)_2Cl$, $(CH_2)_2Br$, $C_2F_5$, OMe, OEt, On-Pr, Oi-Pr, On-Bu, Oi-Bu, Os-Bu, Ot-Bu, $OCF_3$, $OCH_2CF_2$, $OCH_2CCl_3$, SMe, SEt, Sn-Pr, Si-Pr, Sn-Bu, Si-Bu, Ss-Bu, St-Bu, NHMe, NHEt, NHn-Pr, NHi-Pr, NHn-Bu, NHt-Bu, $NMe_2$, NMe(Et), NMe(n-Pr), NMe(i-Pr), NMe(n-Bu), NMe(i-Bu), NMe(s-Bu), NMe(t-Bu), $NEt_2$, NEt(n-Pr) and $N(n\text{-}Pr)_2$.

As W, the following radicals can be mentioned:
O, S, NOMe, NOEt, NOn-Pr, NOi-Pr, NOn-Bu, NOi-Bu, NOs-Bu, NOt-Bu, NOn-Pen, $NOCF_3$, $NOCH_2Cl$, $NOCH_2Br$, $NOCCl_3$, $NOCHF_2$, $NO(CH_2)_2F$, $NO(CH_2)_2Cl$ and $NO(CH_2)_2Br$.

As $A^2$, single bond, O and S can be mentioned.

As $A^3$, the following radicals can be mentioned:
single bond, $CH_2$, $(CH_2)_2$, CHMe, $(CH_2)_3$, $CHMeCH_2$, $CH_2CHMe$, $CMe_2$, CH(OH), CMe(OH), CH(OMe), CMe(OMe), CH(OEt), CMe(OEt), CHF, CF(Me), CHCl, CCl(Me), CHBr and CBr(Me).

As Y, the following radicals can be mentioned:
F, Cl, Br, I, Me, Et, n-Pr, i-Pr, n-Bu, i-Bu, s-Bu, t-Bu, $CF_3$, $CH_2Cl$, $CH_2Br$, $CCl_3$, $CHF_2$, $(CH_2)_2F$, $(CH_2)_2Cl$, $(CH_2)_2Br$, $C_2F_5$, OMe, OEt, On-Pr, Oi-Pr, On-Bu, Oi-Bu, Os-Bu, Ot-Bu, SMe, SEt, Sn-Pr, Si-Pr, Sn-Bu, Si-Bu, Ss-Bu, St-Bu, CN, $NO_2$, $CO_2H$, $CO_2Me$, $CO_2Et$, $CO_2$n-Pr, $CO_2$i-Pr, $CO_2$n-Bu, $CO_2$i-Bu, $CO_2$s-Bu and $CO_2$t-Bu.

As $R^{13}$, the following radicals can be mentioned:
Me, Et, n-Pr, i-Pr, n-Bu, i-Bu, s-Bu, t-Bu and n-Pen.

As Q, the following radicals can be mentioned:
F, Cl, Br, I, Me, Et, n-Pr, i-Pr, n-Bu, i-Bu, s-Bu, t-Bu, $CF_3$, $CH_2Cl$, $CH_2Br$, $CCl_3$, $CHF_2$, $(CH_2)_2F$, $(CH_2)_2Cl$, $(CH_2)_2Br$, $C_2F_5$, OMe, OEt, On-Pr, Oi-Pr, On-Bu, Oi-Bu, Os-Bu, Ot-Bu, SMe, SEt, Sn-Pr, Si-Pr, Sn-Bu, Si-Bu, Ss-Bu, St-Bu, CN, $NO_2$, $CO_2H$, $CO_2Me$, $CO_2Et$, $CO_2$n-Pr, $CO_2$i-Pr, $CO_2$n-Bu, $CO_2$i-Bu, $CO_2$s-Bu and $CO_2$t-Bu.

As $R^4$ and $R^5$ together, the following radicals can be mentioned:
$(CH_2)_2$, $(CH_2)_3$, $(CH_2)_4$, $(CH_2)_5$, $CCl_2CHMe$, $CBr_2CHMe$, $CCl_2CHEt$ and $CBr_2CHEt$.

As $R^6$, $R^7$, $R^8$ and $R^9$, for each independently, the following radicals can be mentioned:
hydrogen, F, Cl, Br, I, Me, Et, n-Pr, i-Pr, n-Bu, i-Bu, s-Bu, t-Bu and phenyl.

As $R^5$ and $R^8$ together, $CH_2$, $(CH_2)_2$, $(CH_2)_3$, $(CH_2)_4$ and $(CH_2)_5$ can be mentioned.

As $R^{10}$, the following radicals can be mentioned:
H, F, Cl, Br, I, Me, Et, n-Pr, i-Pr, $CHF_2$, $CF_3$, $C_2F_5$, $C_3F_7$, $CCl_3$, $CH_2Cl$, OMe, OEt, On-Pr, Oi-Pr, $OCF_3$, $OCCl_3$, $OCHF_2$, $OCHCl_2$, $OCHBr_2$, $OC_2F_5$, $OCH_2CF_3$ and $OCH_2CCl_3$.

As Z, O and $CH_2$ can be mentioned.

The compounds of the present invention can be used as active ingredients of herbicides employed in crop lands, paddy fields or uncultivated fields by any method of soil treatment, soil incorporation treatment or foliage treatment.

As objective weeds in croplands, i.e. cropland weeds, the following weeds can be mentioned: the weeds of

Solanaceae family such as Solanum nigrum, Datura stramonium, etc. ; the weeds of Malvaceae family such as Abutilon theophrasti, Sida spinosa, etc.; the weeds of Convolvulaceae family such as Ipomoea spps. involving Ipomoea purpurea, and Calystegia spps. ; the weeds of Amaranthaceae family such as Amaranthus lividus, Amaranthus retroflexus, etc. ; the weeds of Compositae family Xanthium pensylvanicum, Ambrosia artemisiaefolia, Helianthus annuus, Galinsoga ciliata, Cirsium arvense, Senecio vulgaris, Erigeron annus, etc. ; the weeds of Cruciferae family such as Rorippa indica, Sinapis arvensis, Capsella Bursapastoris, etc. ; the weed of Polygonaceae family such as Polygonum Blumei, Polygonum convolvulus, etc. ; the weeds of Portulacaceae family such as Portulaca oleracea etc. ; the weeds of Chenopodiaceae family such as Chenopodium album, Chenopodium ficifolium, Kochia scoparia, etc. ; the weeds of Caryophyllaceae family such as Stellaria media etc. ; the weeds of Scrophulariaceae family such as Veronica persica etc. ; the weeds of Commelinaceae family such as Commelina communis etc. ; the weeds of Labiatae family such as Lamium amplexicaule, Lamium purpureum, etc. ; the weeds of Euphorbiaceae family such as Euphorbia supina, Euphorbia maculata, etc. ; the weeds of Rubiaceae family such as Galium spurium, Rubia akane, etc. ;the weeds of Violaceae family such as Viola mandshurica, etc. ; broad-leaved weeds for example the weeds of Leguminosae family such as Sesbania exaltata, Cassia obtusifolia, etc., Graminaceous weeds such as Sorgham bicolor, Panicum dichotomiflorum, ,Sorghum halepense, Echinochloa crus-galli var. crus-galli, Echinochloa crus-galli var. praticola, Echinochloa utilis, Digitaria adscendens, Avenafatua, Eleusine indica, Setaria viridis, Alopecurus aegualis, etc. ; Cyperaceous weeds such as Cyperus rotundus, Cyperus esculentus, etc..

As objective weeds in paddy fields, i.e. paddy weeds, for example, the following weeds can be mentioned: the weeds of Alismataceae family such as Alisma canaliculatum, Sagittaria trifolia, Sagittaria pygmaea, etc. ; the weeds of Cyperaceae family such as Cyperus difformis, Cyperus serotinus, Scirpus juncoides, Eleocharis kuroguwai, etc. ; the weeds of Scrothulariaceae family such as Lindemia pyxidaria; the weeds of Potenderiaceae family such as Monochoria vaginalis; the weeds of Potamogetonaceae family such as Potamogeton distinctus; the weeds of Lythraceae family such as Rotala indica, ; the weeds of Gramineae family such as Echinochloa crus-galli.

As the objective crops for the hebicide containing at least one compound of the present invention, for example important crops such as wheat, barley, maize, soybean, rice, cotton, beet, sorghum, etc. can be mentioned.

The herbicide containing at least one compound of the present invention are also useful as a defoliant.

The compound of the present invention can be synthesized by the method shown in the following Scheme 1. In the scheme 1, $R^1$, $R^2$, $R^3$, $R^4$, $R^5$, $R^6$, $R^7$, $R^8$, $R^9$, $R^{10}$,n ,p ,q and Z represent the same meanings as mentioned above, Hal represents halogen, and Q represents hydrogen, alkylsulfonyl or arylsulfonyl.

Scheme 1

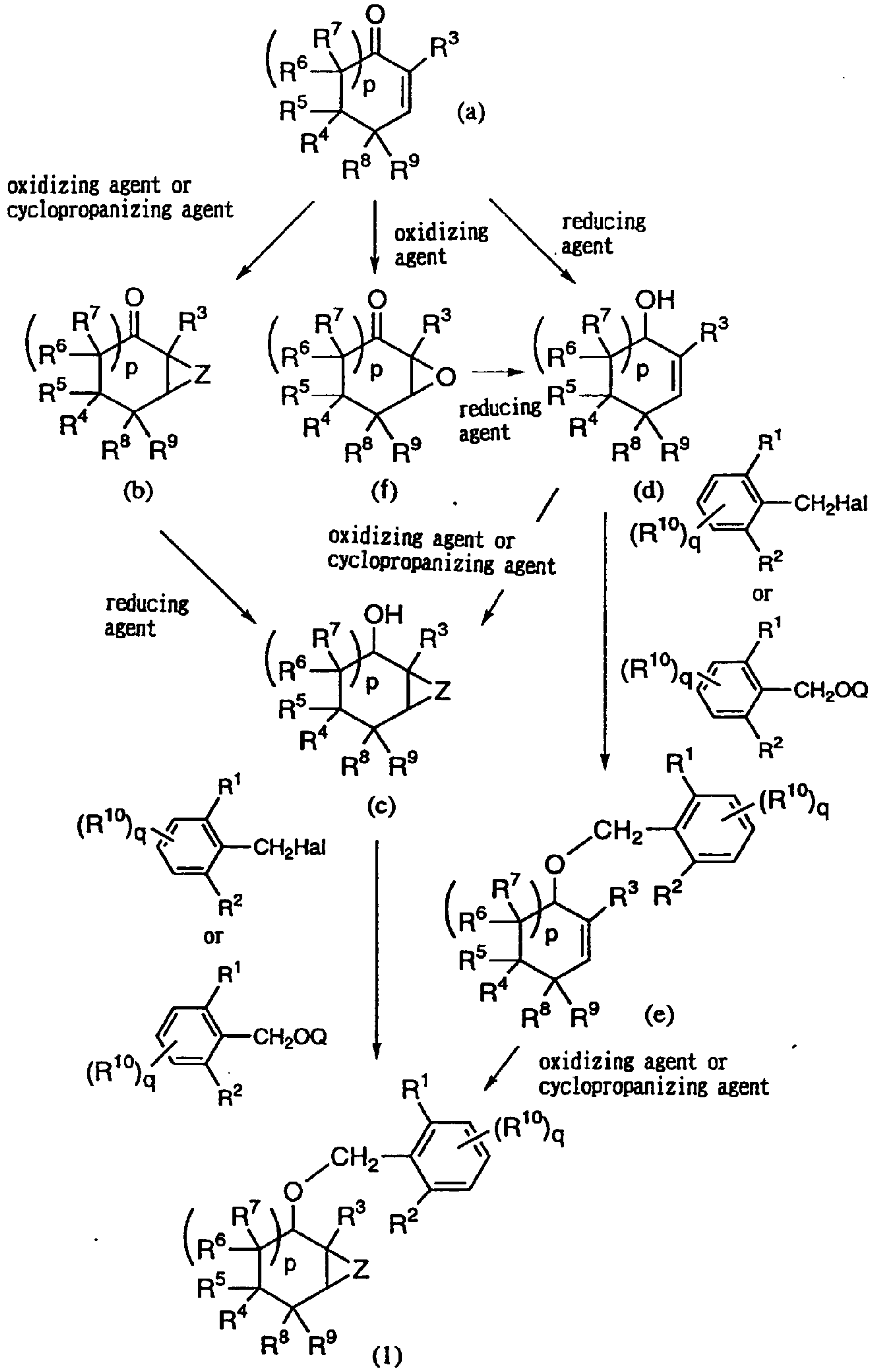


Scheme 1 illustrates four types of synthetic routes starting from an α,β -unsaturated cyclic ketone (a). One represents a method involving reacting an α,β -unsaturated cyclic ketone (a) with an oxidizing agent or a cyclopropanizing

agent to synthesize a compound (b) and reducing ketone with a reducing agent to synthesize a cyclic alcohol compound (c), and finally reacting the cyclic alcohol compound (c) with a benzyl halide, a benzyl alcohol or a benzyl alcohol sulfonic acid ester to synthesize the compound of the present invention (1).

Another one represents a synthetic route involving reducing an α,β -unsaturated cyclic ketone (a) with a reducing agent to reduce ketone first to synthesize an unsaturated alcohol (d), reacting it with an oxidizing agent or a cyclopropanizing agent to synthesize a cyclic alcohol compound (c), and then leading to the compound of the present invention (1).

Still another one represents a method involving reducing an α,β -unsaturated cyclic ketone (a) to synthesize an unsaturated alcohol (d), reacting it with a benzyl halide, benzyl alcohol, or benzyl alcohol sulfonic acid ester to synthesize a benzyl ether compound (e), and reacting the benzyl ether compound (e) with an oxidizing agent or a cyclopropanizing agent to synthesize the compound of the present invention (1).

The final one represents a synthetic route involving oxidizing an α,β -unsaturated cyclic ketone (a) with an oxidizing agent to synthesize an epoxy compound (f), subjecting the epoxy compound (f) to epoxy ring opening reaction with a reducing agent to synthesize an unsaturated alcohol (d), reacting it with an oxidizing agent or a cyclopropanizing agent to synthesize a cyclic alcohol compound (c), and then leading to the compound of the present invention (1).

When purification of the compound of the present invention is needed, it can be separated and purified by any purification method such as recrystallization and column chromatography.

The compound of the present invention contains an asymmetric carbon and includes all the stereoisomers, examples of which include the following stereoisomers.

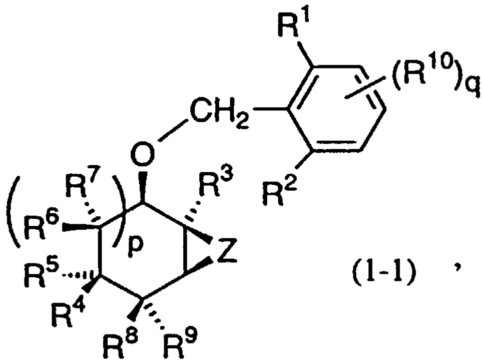
(1-1) ,

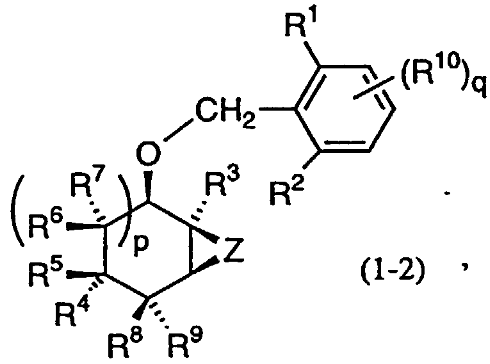
(1-2) ,

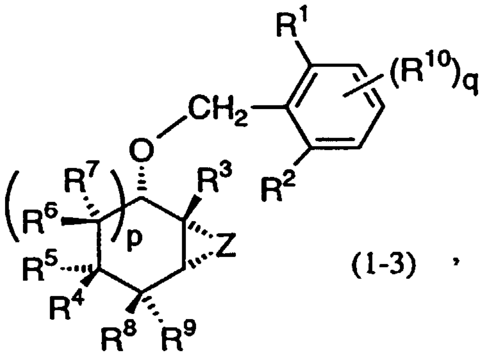
(1-3) ,

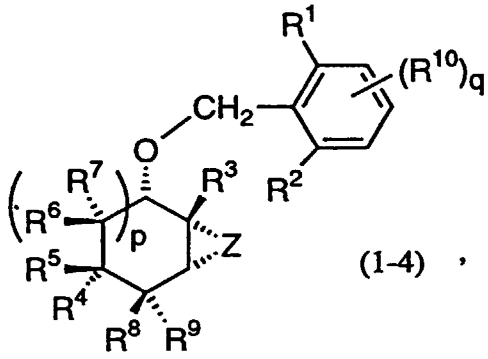
(1-4) ,

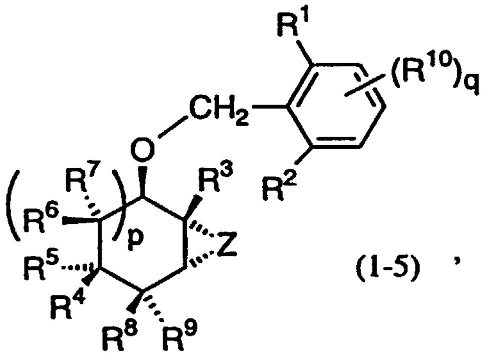
(1-5) ,

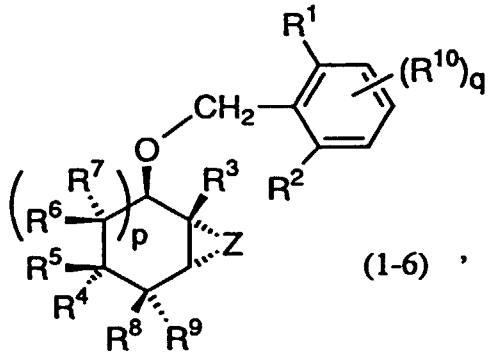
(1-6) ,

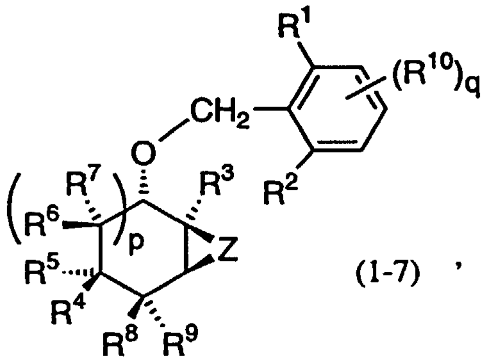
(1-7) ,

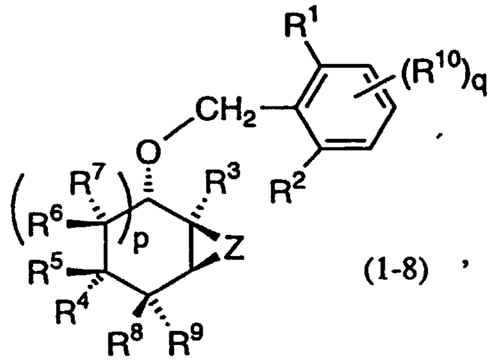
(1-8) ,

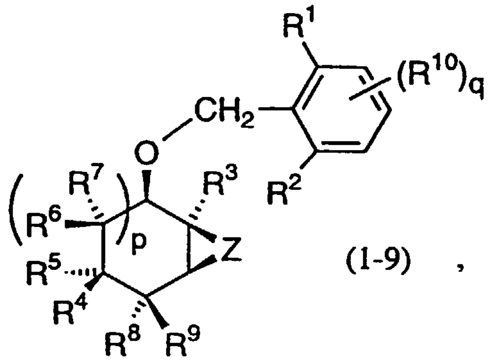
(1-9) ,

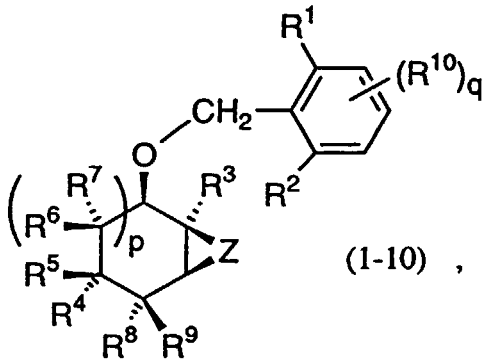
(1-10) ,

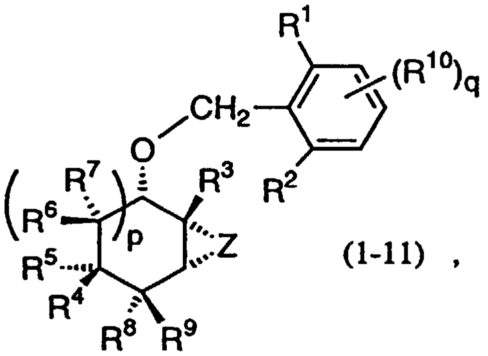
(1-11) ,

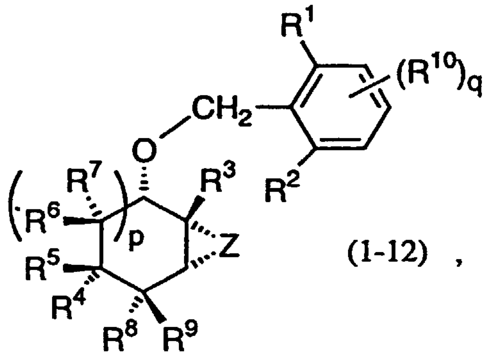
(1-12) ,

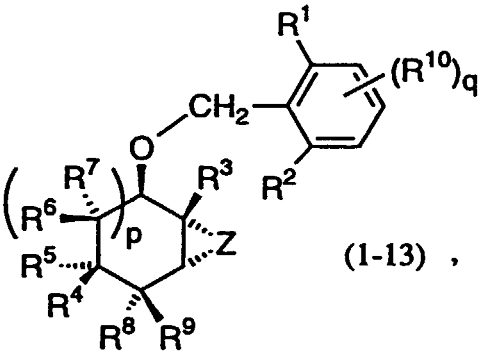
(1-13) ,

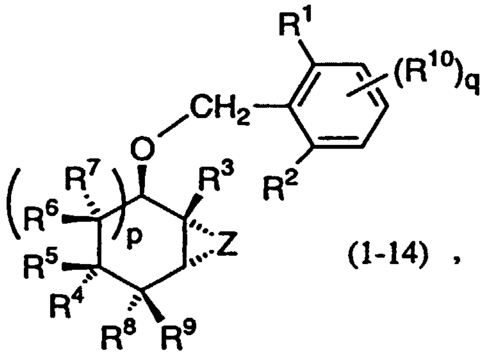
(1-14) ,

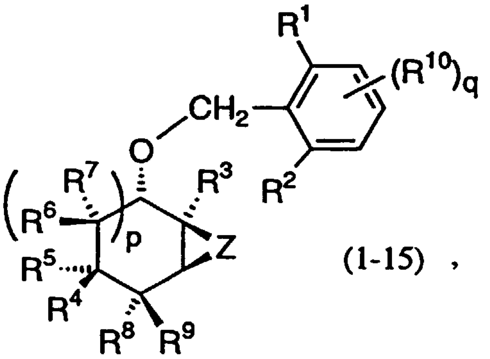
(1-15) ,

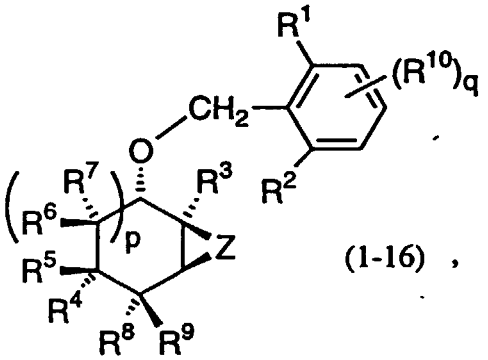
(1-16) ,

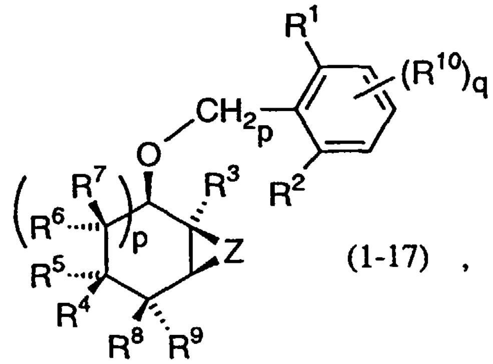

(1-17) ,

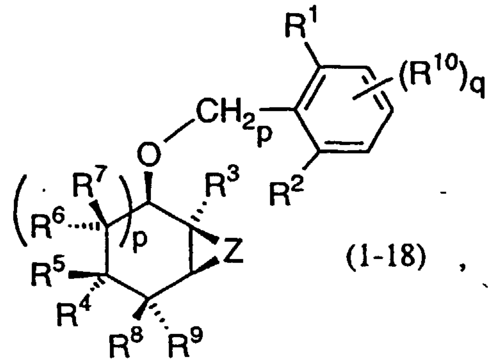

(1-18) ,

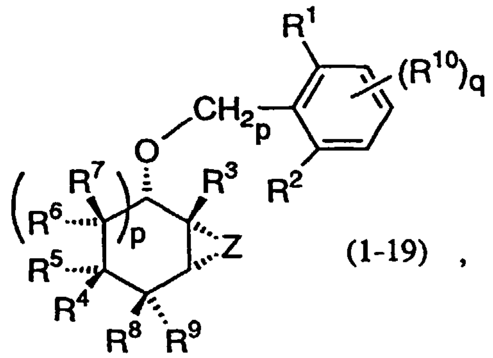

(1-19) ,

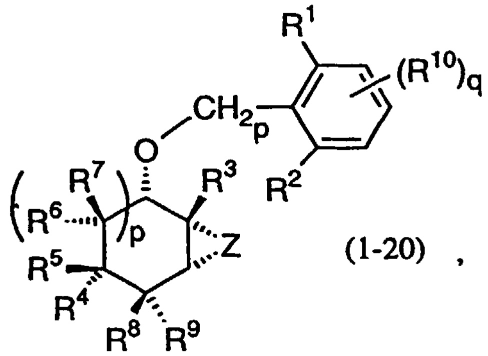

(1-20) ,

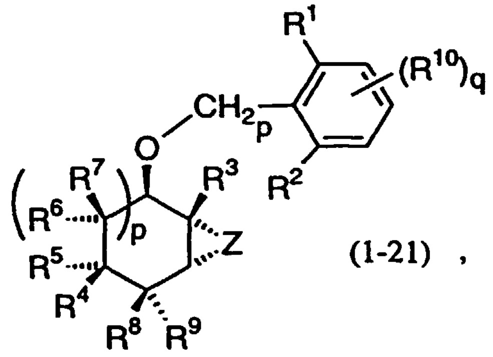

(1-21) ,

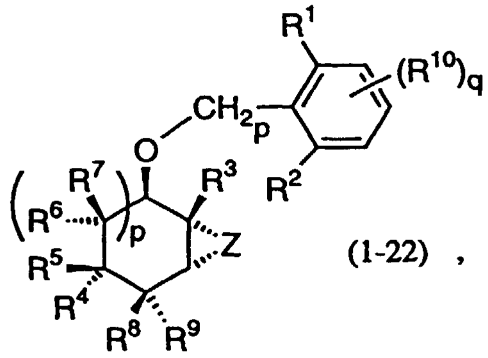

(1-22) ,

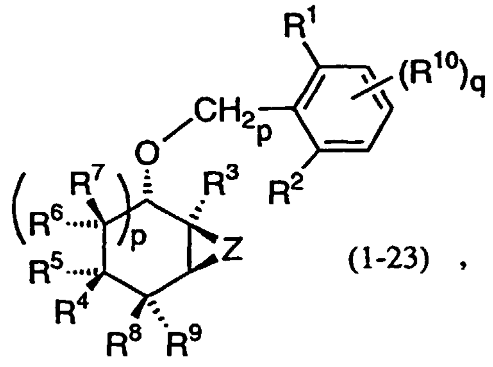

(1-23) ,

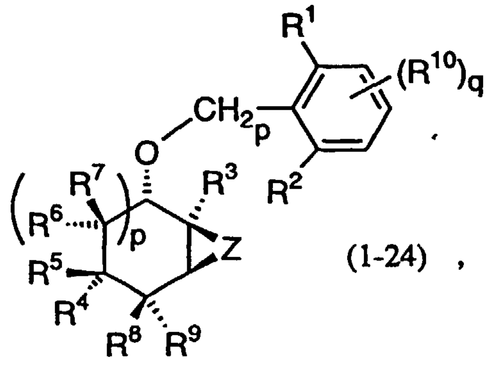

(1-24) ,

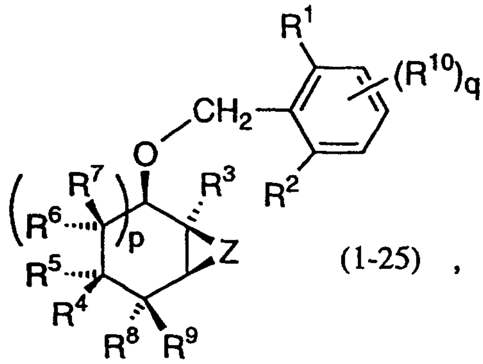
(1-25) ,

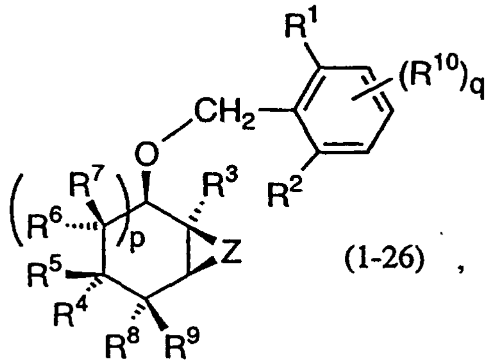
(1-26) ,

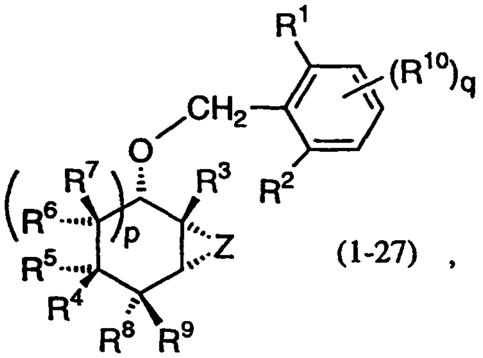
(1-27) ,

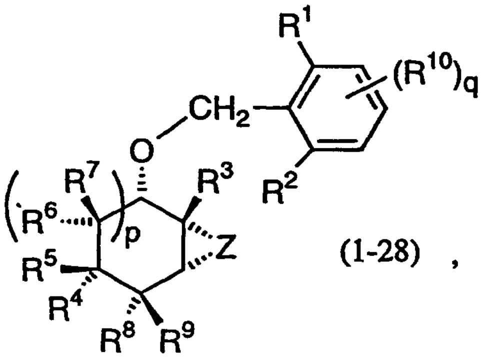
(1-28) ,

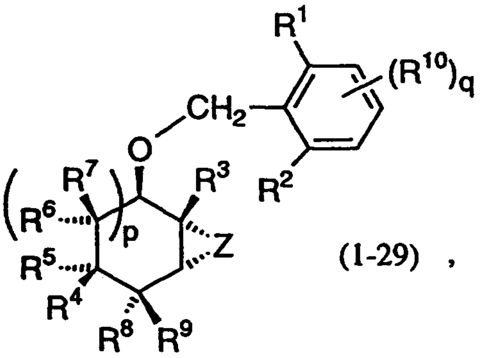
(1-29) ,

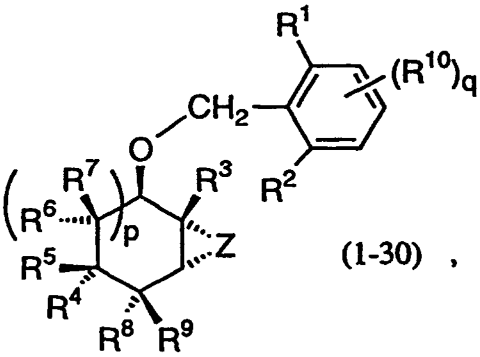
(1-30) ,

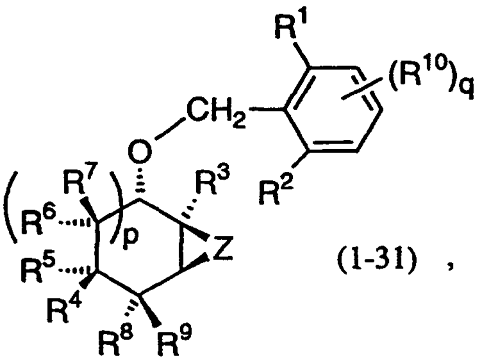
(1-31) ,

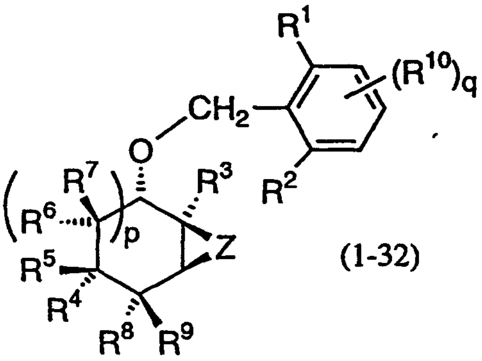
(1-32)

And when the compound of the present invention can has geometrical isomers, the compound of the present invention also involves the cis and trans isomers thereof. As the preferable stereoisomers among those illustrated just above , those of the formulas (1-1), (1-2), (1-3), (1-4), (1-9), (1-10), (1-11), (1-12), (1-17), (1-18), (1-19), (1-20), (1-25), (1-26), (1-27) and (1-28) can be mentioned.

BEST MODE FOR CARRYING OUT THE INVENTION

Hereafter, synthetic examples of the present invention will be described concretely as examples. However, the present invention should not be construed as being limited thereto. [Example 1] Synthesis of 2-(2-methylbenzyloxy)-4-isopropenyl-1-methyl-7-oxabicyclo[4.1.0]heptane (Compound No. A-4)

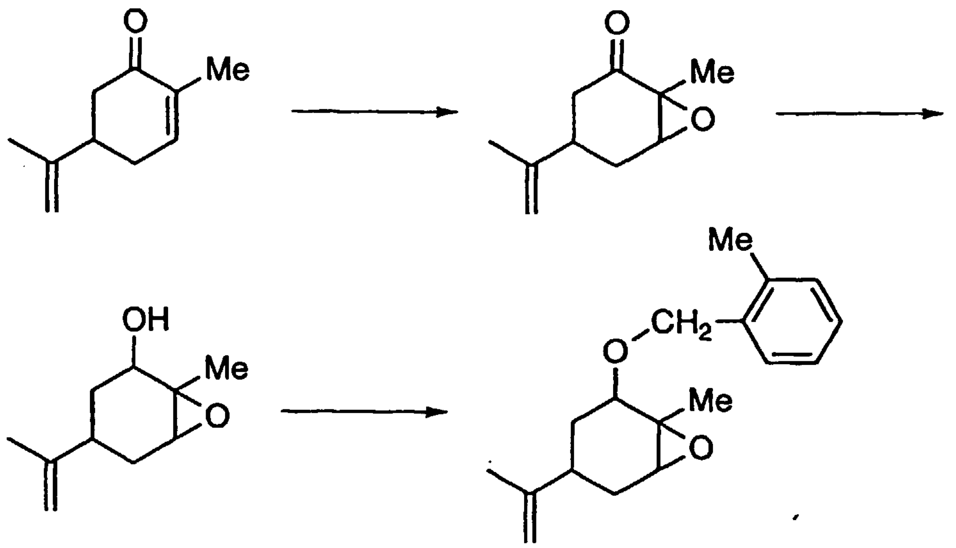

(1)A 40 ml ethanol solution of 7 g of (S)-carvone was cooled to 0 °C and 18 g of 30% hydrogen peroxide water was added thereto. Then, to the solution was portionwise dropped 2 ml of aqueous 6M sodium hydroxide solution. After the dropping, the mixture was stirred at 0°C to room temperature for 3 hours. The reaction mixture was poured into saturated saline and extracted with ethyl acetate. The organic layer was washed with water and dried over anhydrous sodium sulfate and then the solvent was distilled off under reduced pressure to obtain 6.4 g of (1S,4S,6S)-1-methyl-4-isopropenyl-7-oxabicyclo[4.1.0]heptane-2-one.
$^{1}$H-NMR($CDCl_3$) δ (ppm): 1.42(s, 3H), 1.72(s, 3H), 1.85-2.07 (m, 2H), 2.35-2.78(m, 3H), 3.45(d, 1H), 4.72(s, 1H), 4.79(s, 1H)
(2)To 6.4 g of (1S,4S,6S)-1-methyl-4-isopropenyl-7-oxabicyclo[4.1.0]heptane-2-one were added 50 ml of methanol and 10 ml of water and the mixture was cooled to 0 °C. To this was portionwise added 1.4 g of sodium borohydride and then the mixture was stirred at 0 °C to room temperature for 4 hours. The reaction mixture was poured into water and rendered weakly acidic by addition of an aqueous hydrochloric acid solution and extracted with ethyl acetate. The organic layer was washed with saturated saline and dried over anhydrous sodium sulfate. Distilling off the solvent under reduced pressure afforded 5.2 g of (1S,2RS,4S,6S)-2-hydroxy-4-isopropenyl-1-methyl-7-oxabicyclo[4.1.0]heptane as a mixture of diastereomers.
$^{1}$H-NMR($CDCl_3$)δ (ppm): (1.40(s),1.43(s),3H), 1.71(s,3H), 1.1-2.3(m, 5H), 3.12-3.15, 3.28-3.30(m, 1H), 3.85-3.96(m, 1H), 4.68-4.75(m, 2H)
(3)Thirty ml of tetrahydrofuran solution of (1S,2RS,4S,6S) -2-hydroxy-4-isopropenyl-1-methyl-7-oxabicyclo[4.1.0]heptane was cooled to 0°C and 0.5 g of 60% sodium hydride was added thereto. The mixture was stirred at 0°C to room temperature for 30 minutes. The solution was cooled to 0 °C and 5 ml of a tetrahydrofuran solution of 1.8 g of 2-methylbenzyl bromide was dropped. After the dropping, the mixture was stirred at room temperature for 15 minutes and under reflux with heating for 2 hours. The reaction mixture was poured into water and extracted with ethyl acetate. The organic layer was washed with saturated saline and dried over anhydrous sodium sulfate. Thereafter, the solvent was distilled off under reduced pressure. Purification of the crude product by thin layer chromatography afforded 0.5 g of diastereomer component of the title compound.

[Example 2] Synthesis of (1S, 2R, 4S, 6S)-2-(2-fluorobenzyloxy)-1-methyl-4-(1-methyl-2,2-dichlorocyclopropyl)-7-oxabicyclo[4.1.0]heptane (Compounds Nos. A-34-1 and A-34-2)

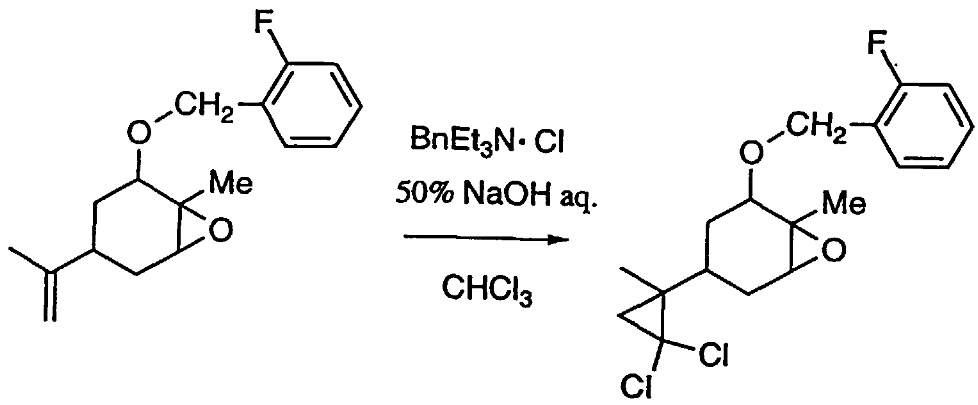


To 15 ml of a chloroform solution of 1.1 g of (1S, 2R, 4S, 6S)-2-(2-fluorobenzyloxy)-4-isopropenyl-1-methyl-7-oxabicyclo[4.1.0]heptane (Compound No. A-5) synthesized according to the example 1 were added 8 ml of an aqueous 50% sodium hydroxide solution and 0.1 g of benzyltriethylammonium chloride and the mixture was stirred at room temperature overnight. Water was added to the reaction solution and the mixture was extracted with chloroform. The organic layer was washed with water and dried over anhydrous sodium sulfate. Thereafter, the solvent was distilled off under reduced pressure. The crude product obtained was purified by liquid chromatography to obtain 1.1 g of the title compound as a mixture of diastereomers. Further, 0.5 g of the mixture of diastereomers was purified by thin layer chromatography ($SiO_2$, n-hexane/chloroform/ether = 4/1/1), to obtain 160 mg of diastereomer component 1 (Compound No. A-34-1) and 290 mg of diastereomer component 2 (Compound No. A-34-2).

[Example 3] Synthesis of 4-(2-chlorophenyl)-2-(2-fluorobenzyloxy)-1-methyl-7-oxabicyclo[4.1.0]heptane (Compound No. A-86-1)

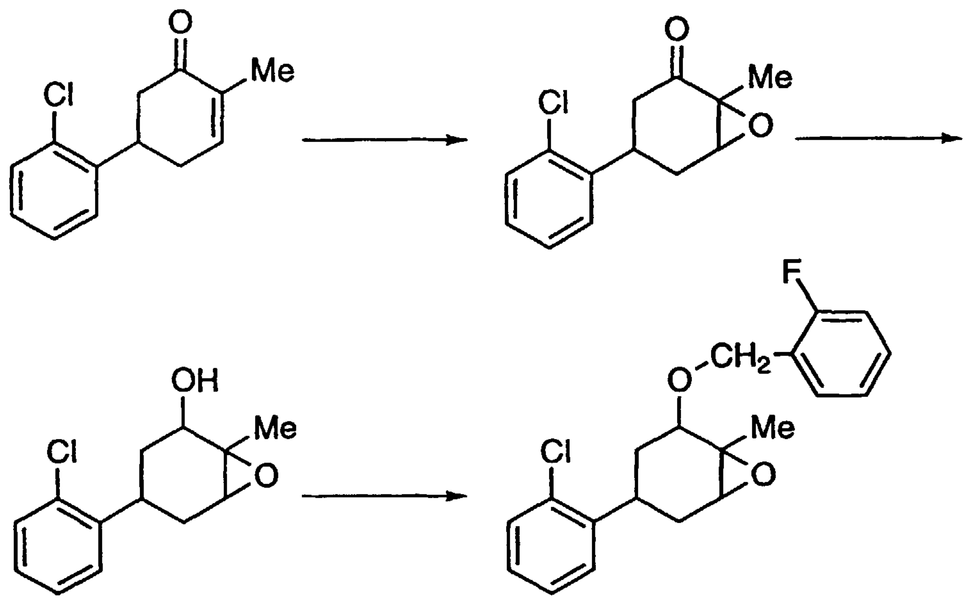

(1) A 10 ml methanol solution of 1 g of 4-(2-chlorophenyl)-1-methyl-6-cyclohexene-2-one was cooled to 0°C and 1.56 g of 30% hydrogen peroxide water was added thereto. Then, to the solution was portionwise dropped aqueous 0.1 g sodium hydroxide solution. After the dropping, the mixture was stirred at 0°C to room temperature overnight. The reaction mixture was poured into water and extracted with ethyl acetate. The organic layer was washed with water and then with saturated saline and dried over anhydrous sodium sulfate. Thereafter, the solvent was distilled off under reduced pressure to obtain 0.83 g of 4-(2-chlorophenyl)-1-methyl-7-oxabicyclo[4.1.0]heptane-2-one.

(2) A 15 ml tetrahydrofuran solution of 1 g of 4-(2 chlorophenyl)-1-methyl-7-oxabicyclo[4.1.0]heptane-2-one was cooled to -78 °C and 6.3 ml of 1.0 M tetrahydrofuran solution of L-Selectolide was portionwise dropped thereto. After the dropping, the temperature was elevated from -78 °C to room temperature and the mixture was stirred overnight. The reaction mixture was poured into a mixture of hydrogen peroxide water and aqueous sodium hydroxide solution and extracted with ethyl acetate. The organic layer was washed with water and then with saturated saline and dried over anhydrous sodium sulfate. Thereafter, the solvent was distilled off under reduced pressure to obtain 1.0 g of 4-(2-chlorophenyl)-2-hydroxy-1-methyl-7-oxabicyclo[4.1.0]heptane.

(3) A 15 ml tetrahydrofuran solution of 1 g of 4-(2-chlorophenyl)-2-hydroxy-1-methyl-7-oxabicyclo[4.1.0]-heptane was cooled to 0°C and 0.5 g of 60% sodium hydride was added thereto. The mixture was stirred at 0°C to room temperature for 30 minutes. To the solution was dropped 0.78 g of 2-fluorobenzyl bromide. After the dropping, the mixture was stirred under reflux with heating for 3 hours. The reaction mixture was poured into water and extracted with ethyl acetate. The organic layer was washed with water and then with saturated saline and dried over anhydrous sodium sulfate. Thereafter, the solvent was distilled off under reduced pressure. Purification of the crude product by thin layer chromatography afforded 0.41 g of the title compound.

[Example 4] Synthesis of 4-(2-chlorophenyl)-2-(2-fluorobenzyloxy)-1-methyl-7-oxabicyclo[4.1.0]heptane (Compound No. A-86-2)

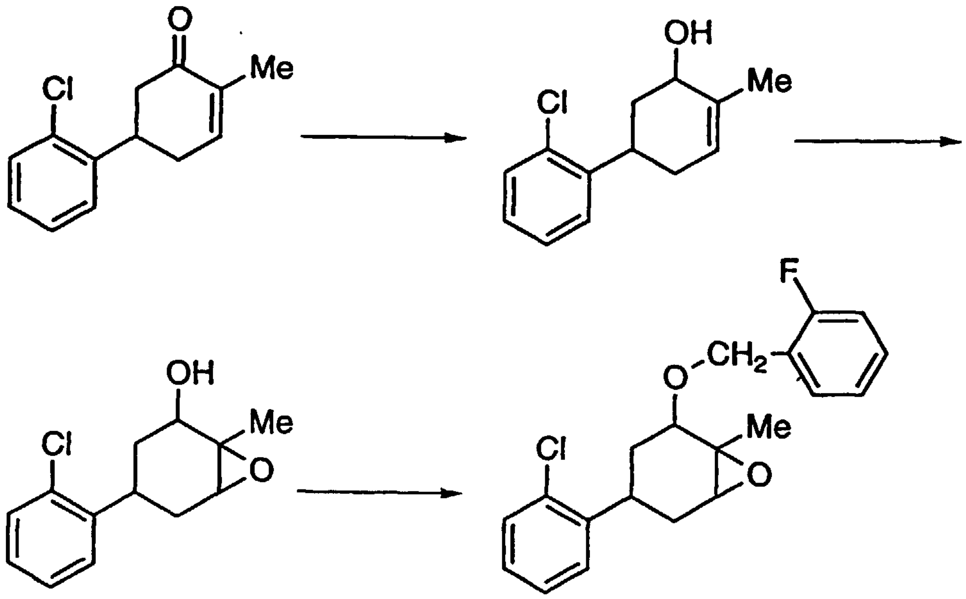

(1) A 20 ml diethyl ether solution of 0.35 g of lithium aluminum hydride was cooled to -78°C and a 20 ml diethyl ether solution of 2.2 g of 4-(2-chlorophenyl)-1-methyl-6-cyclohexene-2-one was portionwise dropped thereto. After the dropping, the mixture was stirred at -78 °C for 2 hours. The reaction mixture was poured into aqueous hydrochloric acid solution and extracted with ethyl acetate. The organic layer was washed with water and then with saturated saline and dried over anhydrous sodium sulfate. Thereafter, the solvent was distilled off under reduced pressure to obtain 2.2 g of 4-(2-chlorophenyl)-2-hydroxy-1-methyl-6-cyclohexene.
(2) A 30 ml methylene chloride solution of 2.2 g of 4-(2-chlorophenyl)-2-hydroxy-1-methyl-6-cyclohexene was cooled to 0 °C and 2.56 g of m-chloroperbenzoic acid was portionwise added thereto and stirred at 0 °C for 4 hours. Then, the reaction mixture was poured into water and extracted with ethyl acetate. The organic layer was washed with water and then with saturated saline and dried over anhydrous sodium sulfate. Thereafter, the solvent was distilled off under reduced pressure to obtain 2.4 g of 4-(2-chlorophenyl)-2-hydroxy-1-methyl-7-oxabicyclo[4.1.0]-heptane.
(3) A 15 ml tetrahydrofuran solution of 0.52 g of 4-(2-chlorophenyl)-2-hydroxy-1-methyl-7-oxabicyclo[4.1.0]-heptane was cooled to 0 °C and 0.26 g of 60% sodium hydride was added thereto. The mixture was stirred at 0 °C to room temperature for 30 minutes. To the solution was dropped 0.41 g of 2-fluorobenzyl bromide. After the dropping, the mixture was stirred under reflux with heating for 3 hours. The reaction mixture was poured into water and extracted with ethyl acetate. The organic layer was washed with water and then with saturated saline and dried over anhydrous sodium sulfate. Thereafter, the solvent was distilled off under reduced pressure. Purification of the crude product by thin layer chromatography afforded 0.49 g of the title compound.

[Example 5] Synthesis of 4-acetyloxy-2-(2-fluorobenzyloxy)-1-methyl-7-oxabicyclo[4.1.0]heptane (Compound No. A-53)

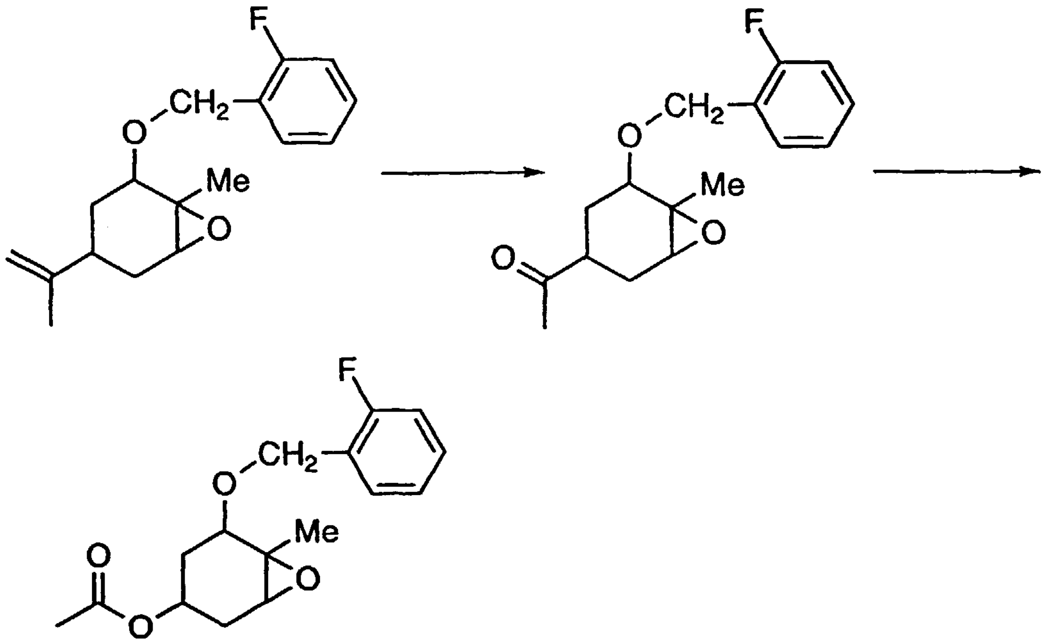

(1) A mixed solution of a 30 ml tetrahydroxyfuran solution of 1.1 g of (1R,2S,4R,6R)-2-(2-fluorobenzyloxy)-4-isopropenyl-1-methyl-7-oxabicyclo[4.1.0]heptane (Compound No. A-18) and 20 ml of water was cooled to 0 °C, to which was dropped 0.2 ml of an aqueous 4% osmium tetroxide solution. After the dropping, the mixture was stirred at 0°C for 5 minutes. Then, 2.1 g of sodium periodate was added thereto and the mixture was stirred at 0°C to room temperature overnight. After the reaction solvent was distilled off under reduced pressure, the residue was extracted with ethyl acetate. The organic layer was washed with an aqueous sodium bisulfite solution, and then with water and dried over anhydrous sodium sulfate. Thereafter, the solvent was distilled off under reduced pressure. Purification of the crude product by thin layer chromatography afforded 0.92 g of 4-acetyl-2-(2-fluorobenzyloxy)-1-methyl-7-oxabicyclo[4.1.0]heptane

(2) A 15 ml methylene chloride solution of 0.5 g of 4-acetyl-2-(2-fluorobenzyloxy)-1-methyl-7-oxabicyclo[4.1.0]heptane was ice cooled, to which was added 0.5 g of m-chloroperbenzoic acid and the mixture was stirred at 0 °C to room temperature for 3 days. After being treated with an aqueous sodium hydrogen carbonate solution and an aqueous sodium thiosulfate solution, the reaction mixture was extracted with chloroform. The organic layer was washed with water and dried over anhydrous sodium sulfate. Thereafter, the solvent was distilled off under reduced pressure. Purification of the crude product by thin layer chromatography afforded 0.37 g of the title compound.

[Example 6] Synthesis of 2-(2-fluorobenzyloxy)-4-hydroxy-1-methyl-7-oxabicyclo[4.1.0]heptane (Compound No. A-56)

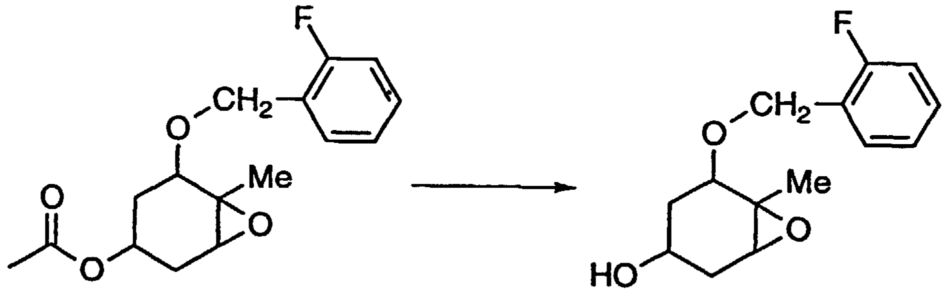

To a 30 ml methanol solution of 1.8 g of 4-acetyloxy-2-(2-fluorobenzyloxy)-1-methyl-7-oxabicyclo[4.1.0]heptane (Compound No. A-53) obtained in Example 5 was added a catalytic amount of sodium methoxide and the mixture was stirred at room temperature overnight. The reaction mixture was poured into water and extracted with ethyl acetate. The organic layer was washed with water and dried over anhydrous sodium sulfate. Thereafter, the solvent was distilled off under reduced pressure to obtain 1.35 g of the title compound.

[Example 7] Synthesis of 2-(2-fluorobenzyloxy)-4-methoxy-1-methyl-7-oxabicyclo[4.1.0]heptane (Compound No. A-57)

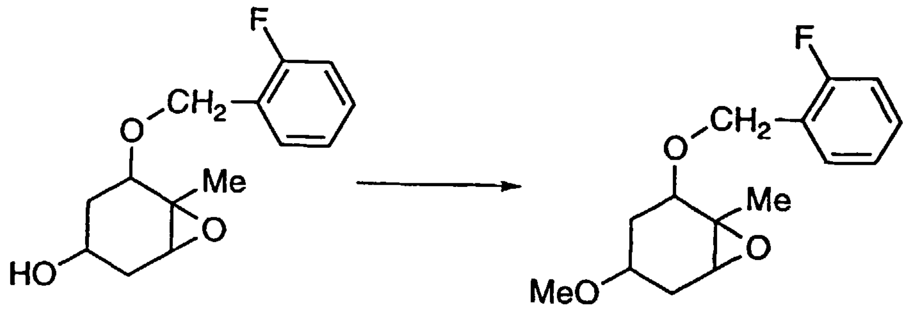

A mixed solution of a 8 ml N,N-dimethylformamide solution of 0.5 g of 2-(2-fluorobenzyloxy)-4-hydroxy-1-methyl-7-oxabicyclo[4.1.0]heptane (Compound No. A-56) obtained in Example 6 and 4 ml of tetrahydrofuran was cooled to 0 °C, to which was added 0.12 g of 60% sodium hydride and the mixture was stirred for 10 minutes. To this solution was dropped methyl iodide and the mixture was stirred at 0 °C to room temperature for 3 days. The reaction mixture was poured into water and extracted with ethyl acetate. The organic layer was washed with water and dried over anhydrous sodium sulfate. Thereafter, the solvent was distilled off under reduced pressure. Purification of the crude product by thin layer chromatography afforded 0.47 g of the title compound.

[Example 8] Synthesis of 1-(n-butyl)-3-methyl-2-(2-methylbenzyloxy)-6-oxabicyclo[3.1.0]hexane (Compounds Nos. B-4-1, B-4-2, B-4-3, and B-4-4)

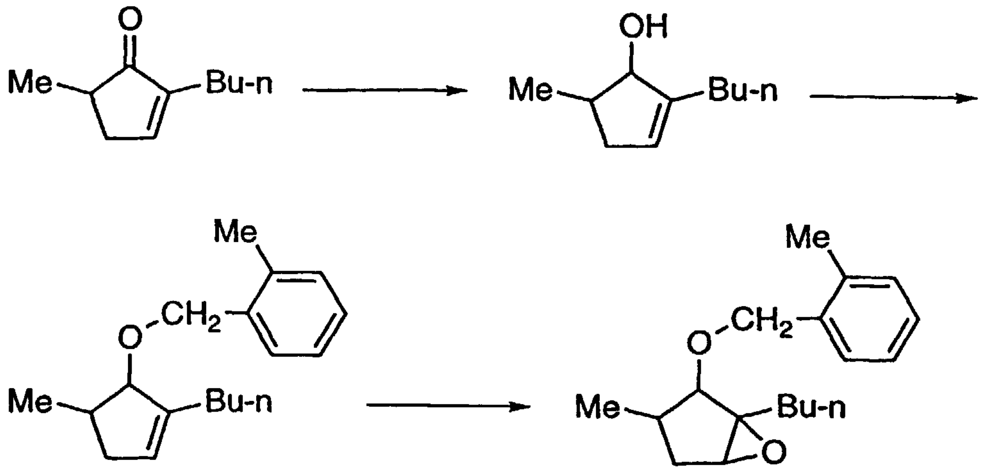

(1) A 50 ml benzene solution of 3.6 g of 1-(n-butyl)-3-methyl-5-cyclopentene-2-one was cooled to 0°C, to which was slowly dropped 23 ml of a toluene solution of 1.0 M diisobutylaluminum hydride over 1.5 hours. After the dropping, the mixture was stirred at 0°C for 1 hour. The reaction mixture was poured into 150 ml of water, to which was added 50 ml of 1N hydrochloric acid. Then, the mixture was extracted with ethyl acetate. The organic layer was washed with water and dried over anhydrous sodium sulfate. Thereafter, the solvent was distilled off under reduced pressure. Purification of the crude product by thin layer chromatography afforded 2.4 g of 1-(n-butyl)-2-hydroxy-3-methyl-6-cyclopentene.

(2) A 25 ml tetrahydrofuran solution of 2.4 g of 1-(n-butyl)-2-hydroxy-3-methyl-6-cyclopentene was cooled to 0°C, to which was added 0.65 g of 60% sodium hydride. The mixture was stirred at 0 °C for 15 minutes. To the solution was dropped a 5 ml tetrahydrofuran solution of 2.6 g of 2-methylbenzyl bromide. After the dropping, the mixture was stirred for 2 hours under reflux with heating and then at room temperature overnight. The reaction mixture was poured into water and extracted with ethyl acetate. The organic layer was washed with water and dried over anhydrous sodium sulfate. Thereafter, the solvent was distilled off under reduced pressure. Purification of the crude product by thin layer chromatography afforded two types of diastereomers (A) and (B) of 1-(n-butyl)-3-methyl-2-(2-methylbenzyloxy)-5-cyclopentene, i.e., 1.9 g of diastereomer (A) and 0.7 g of diastereomer (B).

(3) A 30 ml methylene chloride solution of 1.5 g of the diastereomer (A) obtained in (2) was cooled to 0°C and 1.2 g of m-chloroperbenzoic acid was added thereto and stirred at 0°C to room temperature overnight. Then, the reaction mixture was poured into an aqueous sodium hydrogen carbonate solution and extracted with chloroform. The organic layer was washed with water and dried over anhydrous sodium sulfate. Thereafter, the solvent was distilled off under reduced pressure. Separation and purification of the crude product by thin layer chromatography afforded 0.73 g of (B-4-1) and 0.2 g of (B-4-2), the title compounds.

(4) A 20 ml methylene chloride solution of 0.7 g of the diastereomer (B) obtained in (2) was cooled to 0°C and 0.6 g of m-chloroperbenzoic acid was added thereto and stirred at 0°C to room temperature overnight. Then, the reaction mixture was poured into an aqueous sodium hydrogen carbonate solution and extracted with chloroform. The organic layer was washed with water and dried over anhydrous sodium sulfate. Thereafter, the solvent was distilled off under reduced pressure. Separation and purification of the crude product by thin layer chromatography afforded 0.15 g of (B-4-3) and 0.28 g of (B-4-4), the title compounds.

[Example 9] Synthesis of 2-(2-fluorobenzyloxy)-4-isopropenyl-1-methyl-7-bicyclo[4.1.0]-heptane (Compound No. C-1)

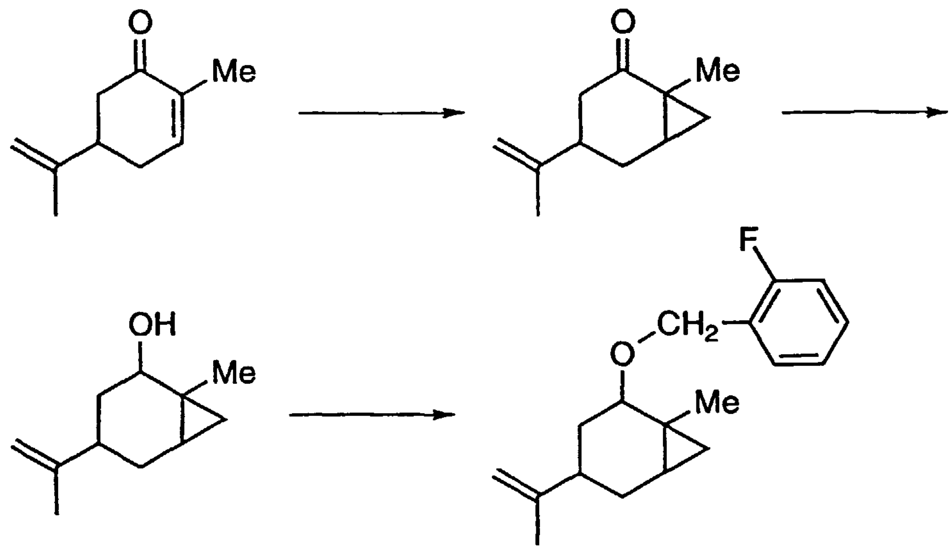

(1) A 18 ml dimethyl sulfoxide solution of 1.75 g of trimethylsulfoxonium iodide and 0.35 g of 60% sodium hydride was stirred at room temperature for 3 hours. At 0 °C, 1.08 g of (S)-carvone was added to the solution and the mixture was stirred at 0°C to room temperature overnight. The reaction mixture was poured into water and extracted with diethyl ether. The organic layer was washed with water and dried over anhydrous sodium sulfate. Thereafter, the solvent was distilled off under reduced pressure to obtain 1.46 g of 2-oxo-4-isopropenyl-1-methyl-7-bicyclo[4.1.0]heptane.

(2) A 22 ml methanol solution of 1.46 g of 2-oxo-4-isopropenyl-1-methyl-7-bicyclo[4.1.0]heptane was cooled to -78 °C and 11.6 ml of 1.0 M L-Selectolide (trade name) was slowly dropped thereto. After the dropping, the temperature was slowly elevated from -78 °C to room temperature and the mixture was stirred overnight. To the reaction mixture was dropped 30% hydrogen peroxide and the mixture was stirred at room temperature overnight. The reaction mixture was poured into a dilute aqueous sodium hydroxide solution and extracted with diethyl ether. The organic layer was washed with water and dried over anhydrous sodium sulfate. Thereafter, the solvent was distilled off under reduced pressure to obtain 1.27 g of 2-hydroxy-4-isopropenyl-1-methyl-7-bicyclo[4.1.0]heptane.

(3) A 20 ml tetrahydrofuran solution of 1.27 g of 2-hydroxy-4-isopropenyl-1-methyl-7-bicyclo[4.1.0]heptane was cooled to 0°C and 0.92 g of 60% sodium hydride was added thereto. The mixture was stirred at 0°C for 30 minutes. To the solution was added 1.59 g of 2-fluorobenzyl bromide. The mixture was stirred under reflux with heating for 3 hours. The reaction mixture was poured into a saturated aqueous ammonium chloride solution and extracted with n-hexane. The organic layer was washed with water and dried over anhydrous sodium sulfate. Thereafter, the solvent was distilled off under reduced pressure. Purification of the crude product by thin layer chromatography afforded 0.76 g of the title compound.

[Example 10] Synthesis of 2-(4-(2-(2-fluorobenzyloxy)-1-methyl-7-oxabicyclo[4.1.0]heptyl))-1-propanol (Compound No. A-100)

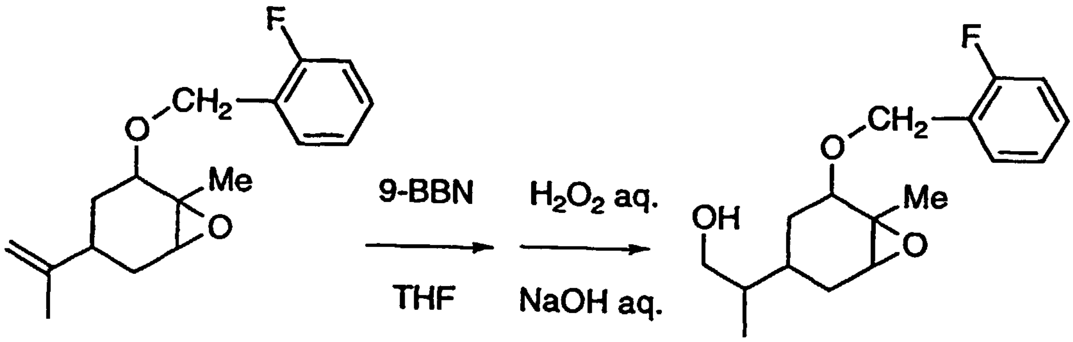


To 20 ml of a tetrahydrofuran solution of 3 g of 2-(2-fluorobenzyloxy)-4-isopropenyl-1-methyl-7-oxabicyclo[4.1.0] heptane was dropped 30 ml of a 0.5M tetrahydrofuran solution of 9-BBN under ice cooling and after the dropping, the mixture was stirred at room temperature overnight. The reaction mixture was ice cooled, to which were added 2 ml of ethanol, 30 ml of an aqueous 6 M sodium hydroxide, and 35 ml of 30% hydrogen peroxide water and the mixture was stirred at 0°C to room temperature overnight. The reaction mixture was extracted with ethyl acetate. The organic layer was washed with water and dried over anhydrous sodium sulfate. Thereafter, the solvent was distilled off under reduced pressure. Purification of the crude product by thin layer chromatography afforded 2.6 g of the title compound as a mixture of diastereomers.

[Example 11] Synthesis of 4-(1-bromo-1-bromomethylethyl)-2-(2-fluorobenzyloxy)-1-methyl-7-oxabicyclo[4.1.0]heptane (Compound No. A-101)

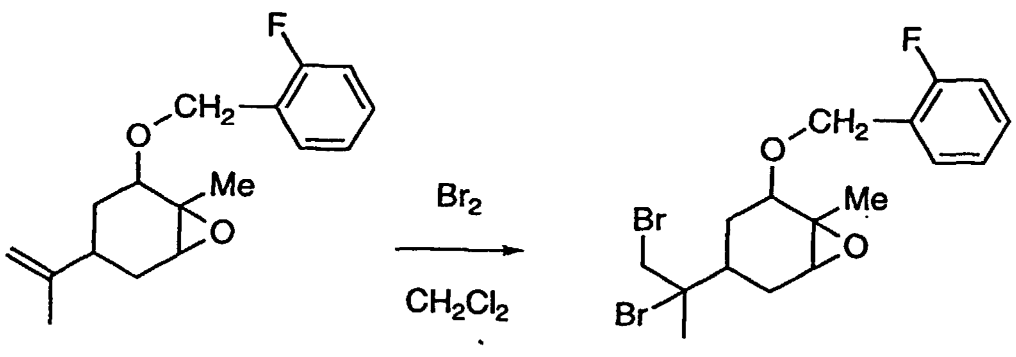


A 50 ml methylene chloride solution of 2.8 g of 2-(2-fluorobenzyloxy)-4-isopropenyl-1-methyl-7-oxabicyclo[4.1.0] heptane was cooled to -78 °C and 6 ml methylene chloride solution of 1 g of bromine was dropped thereto. After the dropping, the mixture was stirred at -78 °C for 5 minutes and then the reaction mixture was poured into an aqueous sodium hydrogen carbonate solution and extracted with chloroform. The organic layer was washed with an aqueous sodium thiosulfate solution and then with water. After drying over anhydrous sodium sulfate, the solvent was distilled off under reduced pressure. Purification of the crude product by thin layer chromatography afforded 0.9 g of the title compound as a mixture of diastereomers.

[Example 12] Synthesis of 4-(1-bromomethylethyl)-2-(2-fluorobenzyloxy)-1-methyl-7-oxabicyclo[4.1.0]heptane (Compound No. A-104)

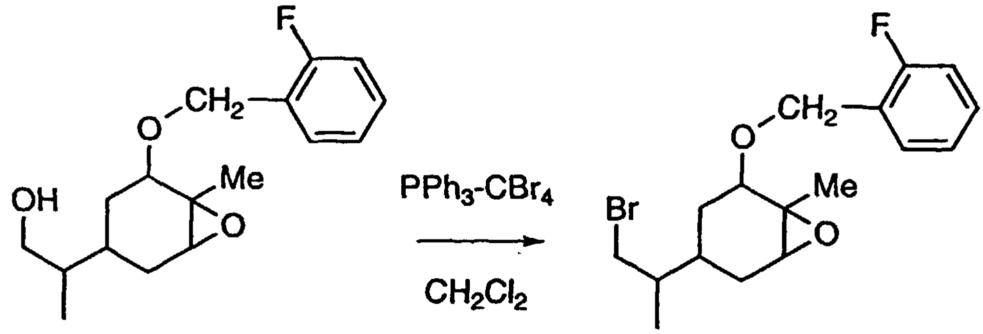


To a 30 ml methylene chloride solution of 0.7 g of 2-(4-(2-(2-fluorobenzyloxy)-1-methyl-7-oxabicyclo[4.1.0]heptyl))-1-propanol were added 0.8 g of triphenylphosphine and 1.3 g of carbon tetrabromide and the mixture was stirred at room temperature for 30 minutes. The reaction mixture was poured into an aqueous sodium hydrogen carbonate solution and extracted with chloroform. The organic layer was washed with water and dried over anhydrous sodium sulfate. Then, the solvent was distilled off under reduced pressure. Purification of the crude product by thin layer chromatography afforded 0.63 g of the title compound as a mixture of diastereomers.

[Example 13] Synthesis of 4-(1-azidomethylethyl)-2-(2-fluorobenzyloxy)-1-methyl-7-oxabicyclo[4.1.0]heptane (Compound No. A-112)

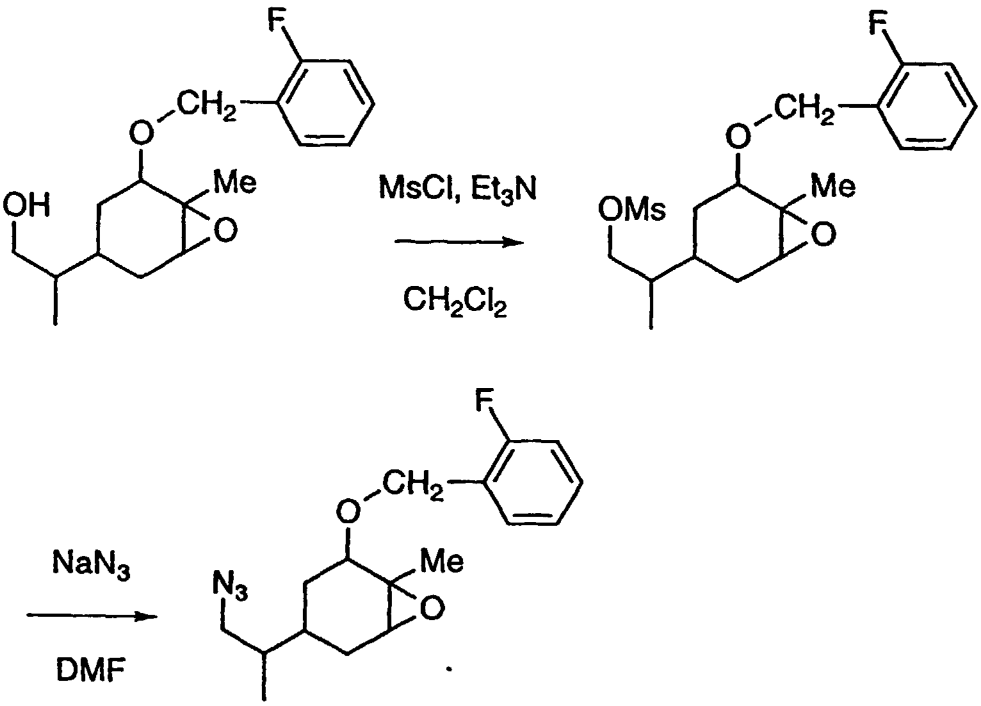


To a 15 ml methylene chloride solution of 0.6 g of 2-(4-(2-(2-fluorobenzyloxy)-1-methyl-7-oxabicyclo[4.1.0]heptyl))-1-propanol were added 0.4 g of triethylamine and 0.35 g of methanesulfonyl chloride under ice cooling and the mixture was stirred for 1 hour. The reaction mixture was poured into an aqueous sodium hydrogen carbonate solution and extracted with chloroform. The organic layer was washed with water and dried over anhydrous sodium sulfate. Then, the solvent was distilled off under reduced pressure to obtain 0.75 g of 2-(4-(2-(2-fluorobenzyloxy)-1-methyl-7-oxabicyclo[4.1.0]heptyl))propyl methanesulfonate.

To a 20 ml dimethylformamide solution of 0.6 g of 2-(4-(2-(2-fluorobenzyloxy)-1-methyl-7-oxabicyclo[4.1.0]heptyl))propyl methanesulfonate was added 0.6 g of sodium azide and the mixture was heated to 70°C and then stirred for 4 hours. The reaction mixture was poured into water and extracted with ethyl acetate.

The organic layer was washed with water and dried over anhydrous sodium sulfate. Thereafter, the solvent was distilled off under reduced pressure. Purification of the crude product by thin layer chromatography afforded 0.41 g of the title compound as a mixture of diastereomers.

[Example 14] Synthesis of 2-(2-fluorobenzyloxy)-1-methyl-7-oxabicyclo[4.1.0]heptane-4-spiro-1'-(2',2'-dichloro-3'-methyl) cyclopropane (Compound No. A-117)

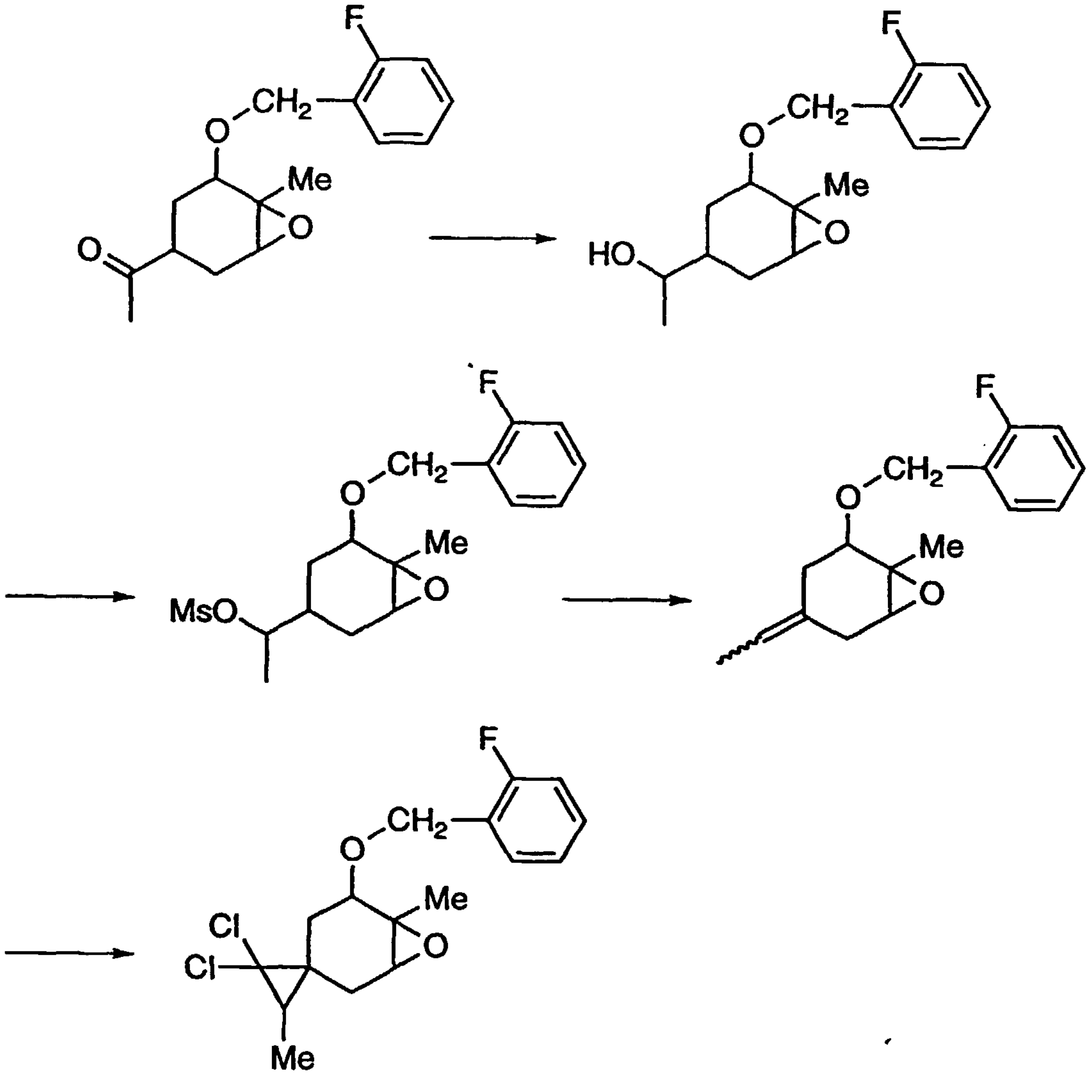

To a 20 ml ethanol solution of 1.4 g of 4-acetyl-2-(2 fluorobenzyloxy)-1-methyl-7-oxabicyclo[4.1.0]heptane was added 100 mg of sodium borohydride under ice cooling and the mixture was stirred at 0°C to room temperature for 4 hours. The reaction mixture was poured into water and adjusted to pH 5 to 6 with an aqueous hydrochloric acid and then extracted with ethyl acetate. The organic layer was washed with water and dried over anhydrous sodium sulfate. Then, the solvent was distilled off under reduced pressure to obtain 1.4 g of 1-(4-(2-(2-fluorobenzyloxy)-1-methyl-7-oxabicyclo[4.1.0]heptyl))-1-ethanol as a mixture of diastereomers.

To a 20 ml methylene chloride solution of 1 g of 1-(4-(2-(2-fluorobenzyloxy)-1-methyl-7-oxabicyclo[4.1.0]heptyl))-1-ethanol was added 0.5 g of triethylamine and 0.5 g of methanesulfonyl chloride under ice cooling and the mixture was stirred for 2 hours. The reaction mixture was poured into an aqueous sodium hydrogen carbonate solution and extracted with chloroform. The organic layer was washed with water and dried over anhydrous sodium sulfate. Thereafter, the solvent was distilled off under reduced pressure to obtain 1.2 g of 1-(4-(2-(2-fluorobenzyloxy)-1-methyl-7-oxabicyclo[4.1.0]heptyl))ethyl methanesulfonate as a mixture of diastereomers.

To a 15 ml dimethylformamide solution of 0.6 g of 1-(4-(2-(2-fluorobenzyloxy)-1-methyl-7-oxabicyclo[4.1.0]heptyl))ethyl methanesulfonate was added 5 ml of 1.0M tetrahydrofuran solution of tetrabutylammonium fluoride and the

mixture was stirred at 70 °C for 5 hours. The reaction mixture was poured into an aqueous sodium hydrogen carbonate solution and extracted with ethyl acetate. The organic layer was washed with water and dried over anhydrous sodium sulfate. Thereafter, the solvent was distilled off under reduced pressure. Purification of the crude product by thin layer chromatography afforded 0.3 g of 4-ethylidene-2-(2-fluorobenzyloxy)-1-methyl-7-oxabicyclo[4.1.0]heptane as a mixture of E-form and Z-form.

To a 10 ml chloroform solution of 0.3 g of 4-ethylidene-2-(2-fluorobenzyloxy)-1-methyl-7-oxabicyclo[4.1.0]heptane were added 5 ml of aqueous 50% sodium hydroxide solution and a catalytic amount of benzyltriethylammonium chloride and the mixture was stirred at room temperature for 3 days. The reaction mixture was poured into water and extracted with chloroform. The organic layer was washed with water and dried over anhydrous sodium sulfate. Thereafter, the solvent was distilled off under reduced pressure. Purification of the crude product by thin layer chromatography afforded 0.26 g of the title compound as a mixture of diastereomers.

[Example 15] Synthesis of 4-(1-chloro-1-methylethyl)-2-(2-fluorobenzyloxy)-1-methyl-7-oxabicyclo[4.1.0]heptane (Compound No. A-106)

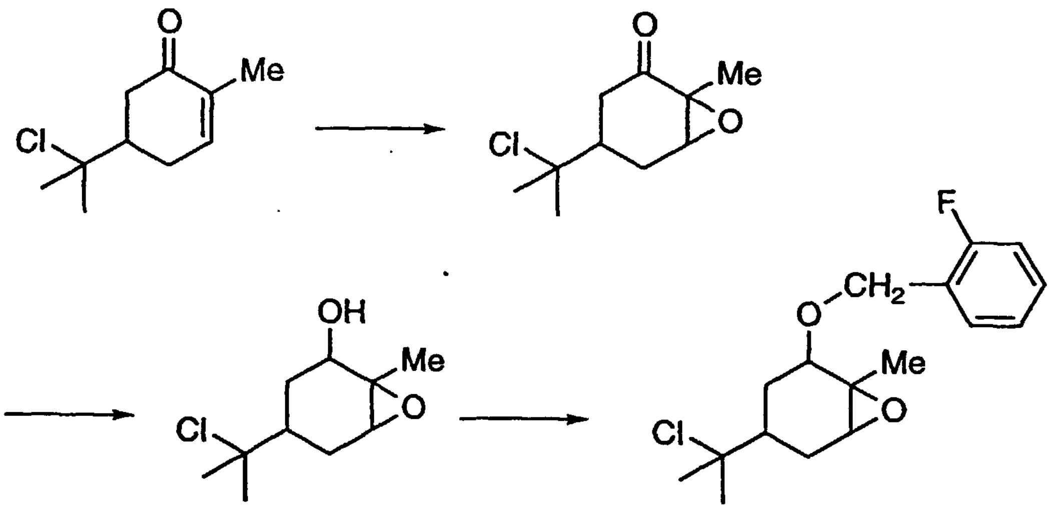

To a 30 ml methanol solution of 4.6 g of 5-(1-chloro-1-methylethyl)-2-methylcyclohexenone were added 10 g of 30% hydrogen peroxide water and a 1.2 ml aqueous solution of 0.6 g of sodium hydroxide under ice cooling and the mixture was stirred at 0°C to room temperature for 2 hours. The reaction mixture was poured into an aqueous sodium chloride solution and extracted with ethyl acetate. The organic layer, was washed with water and dried over anhydrous sodium sulfate. Then, the solvent was distilled off under reduced pressure to obtain 4.4 g of 4-(1-chloro-1-methylethyl)-1-methyl-7-oxabicyclo[4.1.0] heptane-2-one.

To a 40 ml tetrahydrofuran solution of 4.4 g of 4-(1-chloro-1-methylethyl)-1-methyl-7-oxabicyclo[4.1.0]heptane-2-one was cooled to -78°C and 27 ml of 1.0 M tetrahydrofuran solution of LS-selectolide (trade name) was dropped thereto. After the dropping, the mixture was stirred at -78 °C to room temperature for 2 days. The reaction mixture was cooled to -78 °C and 10 ml of ethanol and 20 ml of 30% hydrogen peroxide water, and 20 ml of aqueous 6N sodium hydroxide solution were added thereto. The mixture was stirred at -78°C to room temperature for 4 hours. Potassium carbonate was added to the reaction mixture, which was then extracted with ethyl acetate. The organic layer was washed with water and dried over anhydrous sodium sulfate. Thereafter, the solvent was distilled off under reduced pressure to obtain 4.1 g of 4-(1-chloro-1-methylethyl)-2-hydroxy-1-methyl-7-oxabicyclo[4.1.0]heptane.

To a 20 ml tetrahydrofuran solution of 2 g of 4-(1-chloro-1-methylethyl)-2-hydroxy-1-methyl-7-oxabicyclo[4.1.0]heptane was added 0.6 g of 60% sodium hydroxide under ice cooling and the mixture was stirred at 0 °C to room temperature for 15 minutes. Then, 2 g of 2-fluorobenzyl bromide was added thereto and the mixture was stirred under reflux with heating for 1 hour. The reaction mixture was poured into water and extracted with ethyl acetate. The organic layer was washed with water and dried over anhydrous sodium sulfate. Thereafter, the solvent was distilled off

under reduced pressure. Purification of the crude product by thin layer chromatography afforded 1.4 g of the title compound.

[Example 16] Synthesis of 4-bromo-2-(2-fluorobenzyloxy)-1-methyl-7-oxabicyclo[4.1.0]heptane (Compound No. A-114)

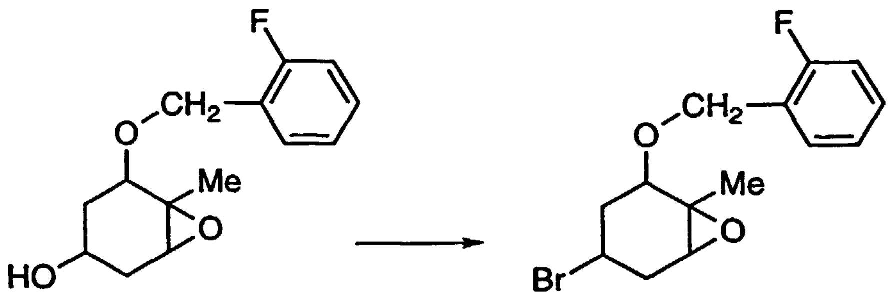

To a 40 ml toluene suspension of 1 g of 2-(2-fluorobenzyloxy)-4-hydroxy-1-methyl-7-oxabicyclo[4.1.0]heptane were added 0.7 g of imidazole, 2.6 g of triphenylphosphine, and 1.3 g of bromine and the mixture was stirred at room temperature overnight. The reaction mixture was poured into an aqueous sodium hydrogen carbonate solution and extracted with ethyl acetate. The organic layer was washed with water and dried over anhydrous sodium sulfate. Thereafter, the solvent was distilled off under reduced pressure. Purification of the crude product by thin layer chromatography afforded 0.58 g of the title compound.

[Example 17] Synthesis of 4-azido-2-(2-fluorobenzyloxy)-1-methyl-7-oxabicyclo[4.1.0]heptane (Compound No. A-116)

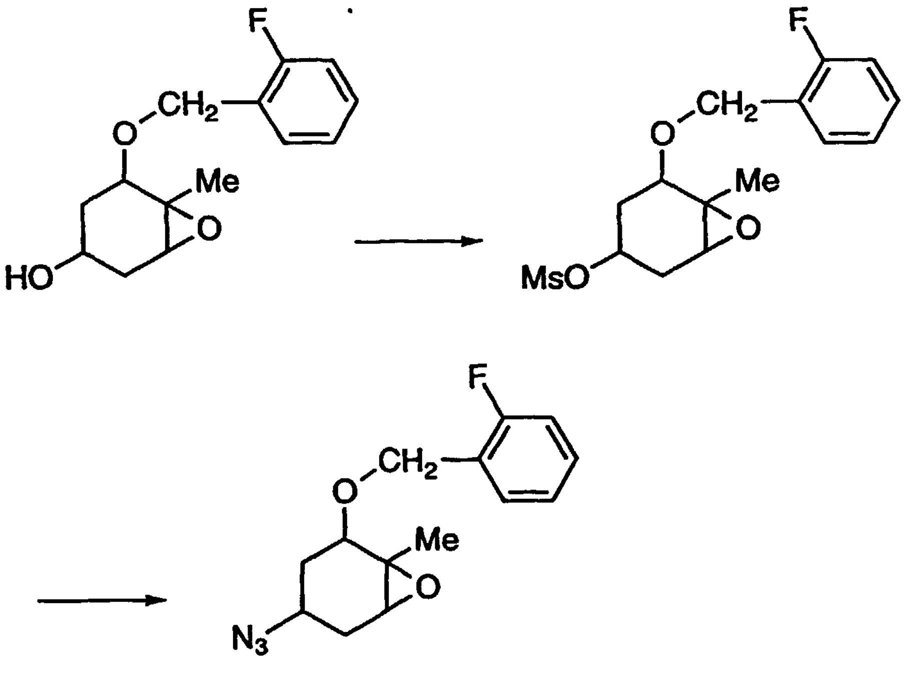

To a solution of 1 g of 2-(2-fluorobenzyloxy)-4-hydroxy-1-methyl-7-oxabicyclo[4.1.0]heptane in 20 ml methylene chloride and 4 ml of dimethylformamide were added 0.6 g of triethylamine and 0.5 g of methanesulfonyl chloride under ice cooling and the mixture was stirred for 1 hour. The reaction mixture was poured into an aqueous sodium hydrogen carbonate solution and extracted with chloroform. The organic layer was washed with water and dried over anhydrous sodium sulfate. Thereafter, the solvent was distilled off under reduced pressure to obtain 1.3 g of 4-(2-(2-fluorobenzyloxy)-1-methyl-7-oxabicyclo[4.1.0]heptyl) methanesulfonate.

To a 15 ml dimethylformamide solution of 1.6 g of 4-(2-(2-fluorobenzyloxy)-1-methyl-7-oxabicyclo[4.1.0]heptyl) methanesulfonate was added 0.6 g of sodium azide and the mixture was heated to 80 °C and stirred for 2 hours. The reaction mixture was poured into water and extracted with ethyl acetate. The organic layer was washed with water and dried over anhydrous sodium sulfate. Thereafter, the solvent was distilled off under reduced pressure. Purification of the crude product by thin layer chromatography afforded 0.43 g of the title compound.

The compounds of the present invention are shown for their chemical structures in the Table 1 and for their physical data in the Table 2, and which compounds were synthesized in the examples described above or synthesized in the manners similar to that shown in the Scheme 1 or to that of the examples described above: And in the Table 1, Me represents methyl, Et represents ethyl. i-Pr represents isopropyl, c-Pr represents cyclopropyl and Ph represent phenyl.

Table 1

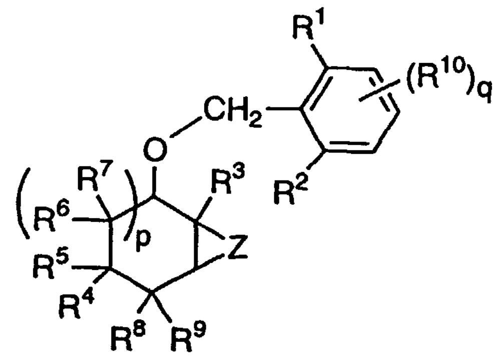

Table 1

| compound No. | $R^1$ | $R^2$ | $R^3$ | $R^4$ | $R^5$ | $(R^{10})_q$ | starting material |
|---|---|---|---|---|---|---|---|
| (in case $R^6 = R^7 = R^8 = R^9 = H$, Z = 0 and p = 1) | | | | | | | |
| A-1 | H | H | Me | H | $CMe=CH_2$ | H | (R)-carvone |
| A-2 | H | H | Me | H | $CMe=CH_2$ | H | (S)-carvone |
| A-3 | Me | H | Me | H | $CMe=CH_2$ | H | (R)-carvone |
| A-4 | Me | H | Me | H | $CMe=CH_2$ | H | (S)-carvone |
| A-5 | F | H | Me | H | $CMe=CH_2$ | H | (S)-carvone |
| A-6 | Cl | H | Me | H | $CMe=CH_2$ | H | (R)-carvone |
| A-7 | Me | H | Me | H | i-Pr | H | (S)-carvone |
| A-8 | F | H | Me | H | i-Pr | H | (S)-carvone |
| A-9 | Me | H | Me | H | i-Pr | H | (R)-carvone |
| A-10 | F | H | Me | H | i-Pr | H | (R)-carvone |
| A-11 | F | F | Me | H | i-Pr | H | (R)-carvone |
| A-12 | F | Cl | Me | H | i-Pr | H | (S)-carvone |
| A-13 | Cl | H | Me | H | i-Pr | H | (S)-carvone |
| A-14 | F | F | Me | H | $CMe=CH_2$ | H | (S)-carvone |
| A-15 | Cl | H | Me | H | $CMe=CH_2$ | H | (S)-carvone |
| A-16 | F | Cl | Me | H | $CMe=CH_2$ | H | (S)-carvone |
| A-17 | F | F | Me | H | i-Pr | H | (S)-carvone |
| A-18 | F | H | Me | H | $CMe=CH_2$ | H | (R)-carvone |
| A-19 | Me | H | Me | Me | Me | H | |
| A-20 | F | H | Me | Me | Me | H | |
| A-21 | Me | H | Et | Me | Me | H | |
| A-22 | F | H | Et | Me | Me | H | |
| A-23-1 (diastereomer-1) | F | H | Me | H | 2,2-$Br_2$-1-Me-c-Pr | H | A-18 |
| A-23-2 (diastereomer-2) | F | H | Me | H | 2,2-$Br_2$-1-Me-c-Pr | H | A-18 |
| A-25 | Me | H | $CH_2CH=CH_2$ | H | $CH_2OMe$ | | |
| A-28-1 (diastereomer-1) | Cl | H | Me | H | 2,2-$Cl_2$-1-Me-c-Pr | H | A-15 |
| A-28-2 (diastereomer-2) | Cl | H | Me | H | 2,2-$Cl_2$-1-Me-c-Pr | H | A-15 |
| A-29-1 (diastereomer-1) | Cl | H | Me | H | 2,2-$Br_2$-1-Me-c-Pr | H | A-15 |
| A-29-2 (diastereomer-2) | Cl | H | Me | H | 2,2-$Br_2$-1-Me-c-Pr | H | A-15 |

Table 1 (continued)

| compound No. | $R^1$ | $R^2$ | $R^3$ | $R^4$ | $R^5$ | $(R^{10})_q$ | starting material |
|---|---|---|---|---|---|---|---|
| A-30-1 (diastereomer-1) | F | F | Me | H | 2,2-$Cl_2$-1-Me-c-Pr | H | A-14 |
| A-30-2 (diastereomer-2) | F | F | Me | H | 2,2-$Cl_2$-1-Me-c-Pr | H | A-14 |
| A-31-1 (diastereomer-1) | F | F | Me | H | 2,2-$Br_2$-1-Me-c-Pr | H | A-14 |
| A-31-2 (diastereomer-2) | F | F | Me | H | 2,2-$Br_2$-1-Me-c-Pr | H | A-14 |
| A-34-1 (diastereomer-1) | F | H | Me | H | 2,2-$Cl_2$-1-Me-c-Pr | H | A-5 |
| A-34-2 (diastereomer-2) | F | H | Me | H | 2,2-$Cl_2$-1-Me-c-Pr | H | A-5 |
| A-35-1 (diastereomer-1) | F | H | Me | H | 2,2-$Br_2$-1-Me-c-Pr | H | A-5 |
| A-35-2 (diastereomer-2) | F | H | Me | H | 2,2-$Br_2$-1-Me-c-Pr | H | A-5 |
| A-36-1 (diastereomer-1) | F | H | Me | H | 2,2-$Cl_2$-1-Me-c-Pr | H | A-18 |
| A-36-2 (diastereomer-2) | F | H | Me | H | 2,2-$Cl_2$-1-Me-c-Pr | H | A-18 |
| A-37-1 (diastereomer-1) | Me | H | Me | H | 2,2-$Cl_2$-1-Me-c-Pr | H | A-4 |
| A-37-2 (diastereomer-2) | Me | H | Me | H | 2,2-$Cl_2$-1-Me-c-Pr | H | A-4 |
| A-38-1 (diastereomer-1) | Me | H | Me | H | 2,2-$Br_2$-1-Me-c-Pr | H | A-4 |
| A-38-2 (diastereomer-2) | Me | H | Me | H | 2,2-$Br_2$-1-Me-c-Pr | H | A-4 |
| A-39-1 (diastereomer-1) | F | Cl | Me | H | 2,2-$Cl_2$-1-Me-c-Pr | H | A-16 |
| A-39-2 (diastereomer-2) | F | Cl | Me | H | 2,2-$Cl_2$-1-Me-c-Pr | H | A-16 |
| A-40-1 (diastereomer-1) | F | Cl | Me | H | 2,2-$Br_2$-1-Me-c-Pr | H | A-16 |
| A-40-2 (diastereomer-2) | F | Cl | Me | H | 2,2-$Br_2$-1-Me-c-Pr | H | A-16 |
| A-43 | F | H | Me | H | 1-Me-epoxyethyl | H | A-5 |

Table 1 (continued)

| compound No. | $R^1$ | $R^2$ | $R^3$ | $R^4$ | $R^5$ | $(R^{10})_q$ | starting material |
|---|---|---|---|---|---|---|---|
| A-44 | F | H | Me | H | CH(Me)OH | H | A-5 |
| A-45 | F | H | Me | H | CH(Me)OH | H | A-18 |
| A-46 | F | H | Me | H | $CH(Me)OCH_2CH=CH_2$ | H | A-18 |
| A-47 | F | H | Me | H | $CH(Me)OCH_2$2,2-$Cl_2$-c-Pr | H | A-18 |
| A-48 | F | H | Me | H | $C(Me)(OMe)CH_2Br$ | H | A-5 |
| A-49 | F | H | Me | H | $C(Me)(OMe)CH_2Br$ | H | A-18 |
| A-50 | Me | H | $CH_2CH=CH_2$ | H | $CH_2OH$ | H | |
| A-51 | F | H | Me | H | CH(Me)OMe | H | A-18 |
| A-52 | F | H | Me | H | $CH(Me)OCH_2OMe$ | H | A-18 |
| A-53 | F | H | Me | H | OC(O)Me | H | A-18 |
| A-54 | F | H | Me | H | $C(Me)(OEt)CH_2Br$ | H | A-5 |
| A-55 | Cl | Cl | Me | H | $CMe=CH_2$ | H | (S)-carvone |
| A-56 | F | H | Me | H | OH | H | A-18 |
| A-57 | F | H | Me | H | OMe | H | A-18 |
| A-58 | F | H | Me | H | OEt | H | A-18 |
| A-59 | F | H | Me | H | $OCH_2Ph$ | H | A-18 |
| A-60 | F | H | Me | H | On-Bu | H | A-18 |
| A-61-1 (diastereomer-1) | Cl | Cl | Me | H | 2,2-$Cl_2$-1-Me-c-Pr | H | A-55 |
| A-61-2 (diastereomer-2) | Cl | Cl | Me | H | 2,2-$Cl_2$-1-Me-c-Pr | H | A-55 |
| A-62 | F | H | Me | H | OPh | H | A-18 |
| A-63-1 (diastereomer-1) | H | H | Me | H | 2,2-$Cl_2$-1-Me-c-Pr | H | A-2 |
| A-63-2 (diastereomer-2) | H | H | Me | H | 2,2-$Cl_2$-1-Me-c-Pr | H | A-2 |
| A-64-1 (diastereomer-1) | H | H | Me | H | 2,2-$Br_2$-1-Me-c-Pr | H | A-2 |
| A-64-2 (diastereomer-2) | H | H | Me | H | 2,2-$Br_2$-1-Me-c-Pr | H | A-2 |
| A-65 | F | H | Me | Et | Et | H | |
| A-66 | F | H | Me | H | OC(O)Me | H | A-5 |
| A-67 | F | H | Me | H | OH | H | A-5 |
| A-68 | F | H | Me | H | OMe | H | A-5 |
| A-69 | F | H | Me | H | On-Bu | H | A-5 |
| A-70 | F | H | Me | H | $OCH_2Ph$ | H | A-5 |
| A-71 | F | H | Me | H | $OCH_2$-(2-Cl-Ph) | H | A-5 |

Table 1 (continued)

| compound No. | $R^1$ | $R^2$ | $R^3$ | $R^4$ | $R^5$ | $(R^{10})_q$ | starting material |
|---|---|---|---|---|---|---|---|
| A-72 | F | H | Me | H | OPh | H | A-5 |
| A-73 | F | H | Me | H | $OCH_2$-(3-Cl-Ph) | H | A-5 |
| A-74 | F | H | Me | H | CMe=CHMe (E/Z mixture) | H | A-18 |
| A-75 | F | H | Me | H | C(Me)(OH)Ph | H | A-18 |
| A-76 (an enantiomer of A-18) | F | H | Me | H | $CMe=CH_2$ | H | |
| A-77 | F | H | Me | H | i-Pr | H | (R)-carvone |
| A-78-1 | F | H | Me | H | Ph | H | |
| A-78-2 | F | H | Me | H | Ph | H | |
| A-79 | Me | H | Et | H | Ph | H | |
| A-80 | F | H | Et | H | Ph | H | |
| A-81 | F | H | Me | H | 2,2-$Cl_2$-1-Me-c-Pr | H | A-76 |
| A-82 | F | H | Me | H | 2,2-$Br_2$-1-Me-c-Pr | H | A-76 |
| A-83 (an enantiomer of A-34-1 ) | F | H | Me | H | 2,2-$Cl_2$-1-Me-c-Pr | H | |
| A-84 (an enantiomer of A-35-1 ) | F | H | Me | H | 2,2-$Br_2$-1-Me-c-Pr | H | |
| A-85-1 | F | H | Me | H | n-Bu | H | |
| A-85-2 | F | H | Me | H | n-Bu | H | |
| A-86-1 | F | H | Me | H | 2-Cl-Ph | H | |
| A-86-2 | F | H | Me | H | 2-Cl-Ph | H | |
| A-87-1 | F | H | Me | H | 4-Cl-Ph | H | |
| A-87-2 | F | H | Me | H | 4-Cl-Ph | H | |
| A-88 | F | H | Me | H | $CMe=CH_2$ | 3-F | (S)-carvone |
| A-89 | F | H | Me | H | 2,2-$Br_2$-1-Me-c-Pr | 3-F | A-88 |
| A-90 | F | H | Me | H | $CMe=CH_2$ | 4-F | (S)-carvone |
| A-91 | F | H | Me | H | 2,2-$Br_2$-1-Me-c-Pr | 4-F | A-90 |
| A-92 | H | H | Me | H | 2,2-$Cl_2$-1-Me-c-Pr | 3-F | (S)-carvone |
| A-93 | H | H | Me | H | 2,2-$Cl_2$-1-Me-c-Pr | 4-F | (S)-carvone |
| A-94 | $CF_3$ | H | Me | H | 2,2-$Br_2$-1-Me-c-Pr | H | (S)-carvone |
| A-95 | F | F | Me | H | $CMe=CH_2$ | H | (R)-carvone |
| A-96 | F | H | Me | H | CMe=CHMe (E/Z mixture) | H | A-5 |
| A-97 | OMe | H | Me | H | $CMe=CH_2$ | H | (S)-carvone |
| A-98 | CN | H | Me | H | $CMe=CH_2$ | H | (S)-carvone |

Table 1 (continued)

| compound No. | $R^1$ | $R^2$ | $R^3$ | $R^4$ | $R^5$ | $(R^{10})_q$ | starting material |
|---|---|---|---|---|---|---|---|
| A-99 | F | H | Me | H | 2-thienyl | H | |
| A-100 | F | H | Me | H | $CHMeCH_2OH$ | H | A-5 |
| A-101 | F | H | Me | H | $CBrMeCH_2Br$ | H | A-5 |
| A-102 | F | H | Me | H | $CBrMeCH_2Br$ | H | A-18 |
| A-103 | F | H | Me | H | $CHMeCH_2Cl$ | H | A-5 |
| A-104 | F | H | Me | H | $CHMeCH_2Br$ | H | A-5 |
| A-105 | F | H | Me | H | $CHMeCH_2I$ | H | A-5 |
| A-106 | F | H | Me | H | $CMe_2Cl$ | H | A-5 |
| A-107 | F | F | Me | H | $CMe_2Cl$ | H | A-5 |
| A-108 | $CF_3$ | H | Me | H | $CMe{=}CH_2$ | H | (S)-carvone |
| A-109 | F | H | Me | H | 2-Me-Ph | H | |
| A-110 | F | H | Me | H | 2,6-$Cl_2$-Ph | H | |
| A-111 | F | H | Me | H | $CHMeCH_2F$ | H | A-5 |
| A-112 | F | H | Me | H | $CHMeCH_2N_3$ | H | A-5 |
| A-113 | F | H | Me | H | CHMeC(O)H | H | A-5 |
| A-114 | F | H | Me | H | Br | H | A-18 |
| A-115 | F | H | Me | H | I | H | A-18 |
| A-116 | F | H | Me | H | $N_3$ | H | A-18 |
| A-117 | F | H | Me | $-CCl_2CHMe-$ | | H | |
| A-118 | F | H | Me | H | 2-OMe-Ph | H | |
| A-119 | F | H | Me | H | 2-$CF_3$-Ph | H | |
| A-120 | F | H | Me | H | $CHMeCO_2H$ | H | A-5 |
| A-121 | F | H | Me | H | CHMeI | H | A-5 |
| A-122 | F | H | Me | H | S-Ph | H | A-18 |
| A-123 | F | H | Me | H | $CHMeCH_2CN$ | H | A-5 |
| A-124 | F | H | Me | H | CN | H | A-18 |
| A-125 | F | H | Me | H | $CHMeCH{=}CBr_2$ | H | A-5 |
| A-126 | F | H | Me | H | F | H | A-18 |
| A-127 | F | H | Me | H | 2,4-$Cl_2$-Ph | H | |
| A-128 | F | H | Me | H | 3-Cl-Ph | H | |
| A-129 | F | F | Me | H | $CHMeCH_2Br$ | H | A-14 |

Table 1 (continued)

| compound No. | $R^1$ | $R^2$ | $R^3$ | $R^4$ | $R^5$ | $R^6$ | $(R^{10})_q$ | starting material |
|---|---|---|---|---|---|---|---|---|
| (in case $R^6 \neq H$, $R^7 = R^8 = R^9 = H$, $Z = 0$ and $p = 1$) | | | | | | | | |
| A-24 | F | H | Me | H | Me | Me | H | |
| A-26 | F | H | Me | H | Ph | Me | H | |
| A-27 | Me | H | Me | H | Ph | Me | H | |
| A-32 | Me | H | Me | H | Et | Me | H | |
| A-33 | F | H | Me | H | Et | Me | H | |
| A-41 | Me | H | Et | H | Ph | Et | H | |
| A-42 | F | H | Et | H | Ph | Et | H | |

Table 1 (continued)

| compound No. | $R^1$ | $R^2$ | $R^3$ | $R^4$ | $R^5$ | $R^8$ | $R^9$ | $(R^{10})_q$ | starting material |
|---|---|---|---|---|---|---|---|---|---|
| (in case $Z = 0$ and $p = 0$) | | | | | | | | | |
| B-1 | Me | H | Me | H | n-Bu | H | H | H | |
| B-2-1 | Me | H | Me | H | $CH_2Ph$ | H | H | H | |
| B-2-2 | Me | H | Me | H | $CH_2Ph$ | H | H | H | |
| B-3-1 | Me | H | n-Bu | H | Me | Me | H | H | |
| B-3-2 | Me | H | n-Bu | H | Me | Me | H | H | |
| B-4-1 | Me | H | n-Bu | H | Me | H | H | H | |
| B-4-2 | Me | H | n-Bu | H | Me | H | H | H | |
| B-4-3 | Me | H | n-Bu | H | Me | H | H | H | |
| B-4-4 | Me | H | n-Bu | H | Me | H | H | H | |
| B-5-1 | Me | H | Et | H | $(CH_2)_3$ | | H | H | |
| B-5-2 | Me | H | Et | H | $(CH_2)_3$ | | H | H | |
| B-5-3 | Me | H | Et | H | $(CH_2)_3$ | | H | H | |

Table 1 (continued)

| compound No. | $R^1$ | $R^2$ | $R^3$ | $R^4$ | $R^5$ | $(R^{10})_q$ | starting material |
|---|---|---|---|---|---|---|---|
| (in case $R^6 = R^7 = R^8 = R^9 = H$, $Z = CH_2$ and $p = 1$) | | | | | | | |
| C-1 | F | H | Me | H | $CMe{=}CH_2$ | H | (S)-carvone |

Table 2

| compound No. | | physical quality |
|---|---|---|
| A-1 | | viscous liquid |
| | $^1$H-NMR δ(ppm) ($CDCl_3$) | 1.34(s, 3H), 1.38-1.45(m, 1H), 1.70(s, 3H), 1.65-1.82(m, 2H), 2.10-2.18(m, 1H), 2.34-2.42(m, 1H), 3.10(br s, 1H), 3.72(dd, 1H), 4.58-4.76(m, 4H), 7.26-7.38(m, 5H) |
| A-2 | | viscous liquid |
| | $^1$H-NMR δ(ppm) ($CDCl_3$) | 1.34(s, 3H), 1.38-1.45(m, 1H), 1.70(s, 3H), 1.65-1.82(m, 2H), 2.10-2.18(m, 1H), 2.34-2.42(m, 1H), 3.10(br s, 1H), 3.72(dd, 1H), 4.58-4.76(m, 4H), 7.25-7.39(m, 5H) |
| A-3 | | viscous liquid |
| | $^1$H-NMR δ(ppm) ($CDCl_3$) | 1.33(s, 3H), 1.38-1.45(m, 1H), 1.70(s, 3H), 1.65-1.82(m, 2H), 2.10-2.20(m, 1H), 2.35-2.40(m, 1H), 2.40(s, 3H), 3.08-3.10(m, 1H), 3.72(dd, 1H), 4.54-4.76(m, 4H), 7.17-7.40(m, 4H) |
| A-4 | | viscous liquid |
| | $^1$H-NMR δ(ppm) ($CDCl_3$) | 1.33(s, 3H), 1.38-1.45(m, 1H), 1.70(s, 3H), 1.65-1.82(m, 2H), 2.10-2.20(m, 1H), 2.35-2.40(m, 1H), 2.40(s, 3H), 3.08-3.10(m, 1H), 3.72(dd, 1H), 4.54-4.76(m, 4H), 7.17-7.40(m, 4H) |
| A-5 | | viscous liquid |
| | $^1$H-NMR δ(ppm) ($CDCl_3$) | 1.35(s, 3H), 1.44-1.51(m, 1H), 1.71(s, 3H), 1.70-1.83(m, 2H), 2.10-2.16(m, 1H), 2.34-2.42(m, 1H), 3.09-3.11(m, 1H), 3.74(dd, 1H), 4.63-4.77(m, 4H), 7.0-7.5(m, 4H) |

Table 2 (continued)

| compound No. | | physical quality |
|---|---|---|
| A-6 | | viscous liquid |
| | $^1$H-NMR δ(ppm) (CDCl$_3$) | 1.40(s,3H), 1.44-1.52(m,1H), 1.72(s,3H), 1.70-1.86(m,2H), 2.10-2.18(m,1H), 2.36-2.44(m,1H), 3.11-3.13(m,1H), 3.79(dd,1H), 4.66 -4.77(m,4H), 7.21-7.35(m,3H), 7.57-7.60(m,1H) |
| A-7 | | viscous liquid |
| | $^1$H-NMR δ(ppm) (CDCl$_3$) | 0.85(d,3H), 0.86(d,3H), 1.08-1.18(m,1H), 1.33(s,3H), 1.38-1.60(m,3H), 1.70-1.78(m,1H), 2.04-2.10(m,1H), 2.39(s,3H), 3.02-3.04(m,1H), 3.75(dd,1H), 4.57(d,1H), 4.67(d,1H), 7.15-7.40(m,4H) |
| A-8 | | viscous liquid |
| | $^1$H-NMR δ(ppm) (CDCl$_3$) | 0.86(d,6H), 1.13-1.22(m,1H), 1.35(s,3H), 1.40-1.60(m,3H), 1.72-1.80(m,1H), 2.04-2.10(m,1H), 3.04-3.06(m,1H), 3.78(dd,1H), 4.66(d,1H), 4.73(d,1H), 7.02 -7.07(m,1H), 7.12-7.16(m,1H), 7.25-7.31(m,1H), 7.42-7.47(m,1H) |
| A-9 | | viscous liquid |
| | $^1$H-NMR δ(ppm) (CDCl$_3$) | 0.85(d,3H), 0.86(d,3H), 1.08-1.18(m,1H), 1.33(s,3H), 1.38-1.60(m,3H), 1.70-1.78(m,1H), 2.04-2.10(m,1H), 2.39(s,3H), 3.02-3.04(m,1H), 3.75(dd,1H), 4.57(d,1H), 4.67(d,1H), 7.15-7.40(m,4H) |
| A-10 | | viscous liquid |
| | $^1$H-NMR δ(ppm) (CDCl$_3$) | 0.86(d,6H), 1.13-1.22(m,1H), 1.35(s,3H), 1.40-1.60(m,3H), 1.72-1.80(m,1H), 2.04-2.10(m,1H), 3.04-3.06(m,1H), 3.78(dd,1H), 4.66(d,1H), 4.73(d,1H), 7.02 -7.07(m,1H), 7.12-7.16(m,1H), 7.25-7.31(m,1H), 7.42-7.47(m,1H) |

Table 2 (continued)

| compound No. | | physical quality |
|---|---|---|
| A-11 | | viscous liquid |
| | $^1$H-NMR δ(ppm) (CDCl$_3$) | 0.87(d, 6H), 1.16-1.58(m, 4H), 1.30(s, 3H), 1.68-1.80(m, 1H), 2.00-2.08(m, 1H), 3.02-3.04(m, 1H), 3.76(dd, 1H), 4.62(d, 1H), 4.75(d, 1H), 6.85 -6.95(2H), 7.25-7.35(m, 1H) |
| A-12 | | viscous liquid |
| | $^1$H-NMR δ(ppm) (CDCl$_3$) | 0.86(d, 6H), 1.16-1.67(m, 4H), 1.32(s, 3H). 1.79-1.83(m, 1H), 2.02-2.07(m, 1H), 3.03(t, 1H), 3.76(dd, 1H), 4.68(d, 1H), 4.81(d, 1H), 6.96 -7.01(m, 1H), 7.18-7.26(m, 2H) |
| A-13 | | viscous liquid |
| | $^1$H-NMR δ(ppm) (CDCl$_3$) | 0.86(d, 6H), 1.14-1.22(m, 1H), 1.40(s, 3H). 1.40-1.57(m, 3H), 1.77-1.81(m, 1H), 2.07-2.11(m, 1H), 3.06(s, 1H), 3.83(t, 1H), 4.72(dd, 2H), 7.19 -7.34(m, 3H), 7.59-7.61(m, 1H) |
| A-14 | | viscous liquid |
| | $^1$H-NMR δ(ppm) (CDCl$_3$) | 1.31(s, 3H), 1.47-1.54(m, 1H), 1.72(s, 3H). 1.70-1.84(m, 2H), 2.07-2.12(m, 1H), 2.33-2.40(m, 1H), 3.08-3.10(dd, 1H), 3.70-3.72(dd, 1H), 4.60-4.78(m, 4H), 6.85 -6.91(m, 2H), 7.22-7.31(m, 1H) |
| A-15 | | viscous liquid |
| | $^1$H-NMR δ(ppm) (CDCl$_3$) | 1.40(s, 3H), 1.44-1.52(m, 1H), 1.72(s, 3H). 1.70-1.77(m, 1H), 1.81-1.86(m, 1H), 2.11-2.17(m, 1H), 2.36-2.43(m, 1H), 3.11-3.12(dd, 1H), 3.77-3.80(dd, 1H), 4.66 -4.77(m, 4H), 7.21-7.35(m, 3H), 7.57-7.60(m, 1H) |

Table 2 (continued)

| compound No. | | physical quality |
|---|---|---|
| A-16 | | viscous liquid |
| | $^1$H-NMR δ(ppm) (CDCl$_3$) | 1.32(s, 3H), 1.48-1.55(m, 1H), 1.72(s, 3H), 1.68-1.78(m, 1H), 1.81-1.87(m, 1H), 2.07-2.13(m, 1H), 2.35-2.41(m, 1H), 3.08-3.10(dd, 1H), 3.70-3.73(dd, 1H), 4.66 -4.83(m, 4H), 6.96-7.02(m, 1H), 7.18-7.26(m, 2H) |
| A-17 | | viscous liquid |
| | $^1$H-NMR δ(ppm) (CDCl$_3$) | 0.86-0.88(d, 6H), 1.17-1.24(m, 1H), 1.31(s, 3H), 1.38-1.55(m, 3H), 1.75-1.80(d, 1H). 2.03-2.06(d, 1H), 3.03(s, 1H), 3.75-3.77(t, 1H), 4.61-4.64(d, 1H), 4.74-4.77(d, 1H), 6.87-6.92(m, 2H), 7.23-7.30(m, 1H) |
| A-18 | | viscous liquid |
| | $^1$H-NMR δ(ppm) (CDCl$_3$) | 1.40(s, 3H), 1.67(s, 3H), 1.3-2.2(m, 5H), 3.40(d, 1H), 3.76(dd, 1H), 4.60-4.78(m, 4H), 7.00-7.07(m, 1H), 7.12-7.16(m, 1H), 7.24-7.33(m, 1H), 7.42-7.51(m, 1H) |
| A-19 | | viscous liquid |
| | $^1$H-NMR δ(ppm) (CDCl$_3$) | 0.83(s, 3H), 0.88(s, 3H), 1.37(s, 3H), 1.55-1.61(m, 4H), 2.35(s, 3H), 2.89-2.98(t, 1H), 3.63-3.91(dd, 1H), 4.33-4.52(d, 1H), 4.60-4.79(d, 1H), 7.08-7.45(m, 4H) |
| A-20 | | viscous liquid |
| | $^1$H-NMR δ(ppm) (CDCl$_3$) | 0.83(s, 3H), 0.90(s, 3H), 1.37(s, 3H), 1.50-1.63(m, 4H), 2.90-2.99(t, 1H), 3.66-3.94(dd, 1H), 4.65(s, 2H), 6.82-7.65(m, 4H) |

Table 2 (continued)

| compound No. | | physical quality |
|---|---|---|
| A-21 | | viscous liquid |
| | $^1$H-NMR δ(ppm) (CDCl$_3$) | 0.83(s, 3H), 0.92(s, 3H), 0.74-1.68(m, 8H), 1.86-2.33(m, 1H), 2.35(s, 3H), 2.90-2.99(t, 1H), 3.77-4.04(dd, 1H), 4.32-4.80(dd, 2H), 7.06-7.43(m, 4H) |
| A-22 | | viscous liquid |
| | $^1$H-NMR δ(ppm) (CDCl$_3$) | 0.83(s, 3H), 0.90(s, 3H), 0.78-1.65(m, 8H), 1.78-2.45(m, 1H), 2.90-3.00(t, 1H), 3.77-4.04(dd, 1H), 4.40-4.83(t, 2H), 6.78-7.61(m, 4H) |
| A-23-1 | | viscous liquid |
| | $^1$H-NMR δ(ppm) (CDCl$_3$) | 1.26(s, 3H), 1.37(s, 3H), 1.2-2.0(m, 7H), 3.05(d, 1H), 3.72(dd, 1H), 4.64(d, 1H), 4.81(d, 1H), 7.02-7.07(m, 1H), 7.11-7.17(m, 1H), 7.24-7.31(m, 1H), 7.45-7.52(m, 1H) |
| A-23-2 | | viscous liquid |
| | $^1$H-NMR δ(ppm) (CDCl$_3$) | 1.25(s, 3H), 1.40(s, 3H), 1.2-2.2(m, 7H), 3.08(d, 1H), 3.71(dd, 1H), 4.62(d, 1H), 4.76(d, 1H), 7.01-7.07(m, 1H), 7.11-7.17(m, 1H), 7.25-7.30(m, 1H), 7.45-7.51(m, 1H) |
| A-24 | | viscous liquid |
| | $^1$H-NMR δ(ppm) (CDCl$_3$) (diastereomer mixture) | 0.82-1.00(m), 1.42(s), 1.42(s), 1.45(s), 1.2-2.2(m), 2.97-2.99(m), 3.43(d), 3.64-3.66(m), 4.53-4.86(m), 7.0-7.6(m) |

Table 2 (continued)

| compound No. | | physical quality |
|---|---|---|
| A-25 | | viscous liquid |
| | $^1$H-NMR δ(ppm) (CDCl$_3$) | 1.28-1.35(m, 1H), 1.50-1.56(m, 1H), 1.76-1.80(m, 1H), 2.05-2.11(m, 3H), 2.40(s, 3H), 2.56-2.62(dd, 1H), 3.07(s, 1H), 3.15-3.23(m, 2H), 3.30(s, 3H), 3.84-3.86(t, 1H), 4.48-4.51(d, 1H), 4.66-4.69(d, 1H), 4.86-4.90(d, 1H), 4.98-5.01(d, 1H), 5.58-5.69(m, 1H), 7.15-7.26(m, 3H), 7.33-7.35(d, 1H) |
| A-26 | | viscous liquid |
| | $^1$H-NMR δ(ppm) (CDCl$_3$) (diastereomer mixture) | 0.71-0.73(m), 0.87(d), 1:40(s), 1.48(s), 1.51(s), 1.75-2.80(m), 3.07(d), 3.12(dd), 3.16(d), 3.59(d), 3.77(d), 3.89(d), 4.56-4.92(m), 7.0-7.6(m) |
| A-27 | | viscous liquid |
| | $^1$H-NMR δ(ppm) (CDCl$_3$) (diastereomer mixture) | 0.70-0.73(m), 0.88(d), 1.40(s), 1.46(s), 1.49(s), 1.8-2.8(m), 3.06(d), 3.11(dd), 3.14(d), 3.60(d), 3.76(d), 3.86(d), 4.4-4.9(m), 7.1-7.5(m) |
| A-28-1 | | m.p. 102-104 ℃ |
| | $^1$H-NMR δ(ppm) (CDCl$_3$) | 1.14(s, 2H), 1.18(s, 3H), 1.32-1.40(m, 4H), 1.66-1.76(m, 2H), 1.81-1.88(dt, 1H), 2.30-2.34(d, 1H), 3.11(s, 1H), 3.86-3.88(d, 1H), 4.71(s, 2H), 7.20-7.35(m, 3H), 7.55-7.57(d, 1H) |
| A-28-2 | | viscous liquid |
| | $^1$H-NMR δ(ppm) (CDCl$_3$) | 1.17-1.24(m, 5H), 1.29-1.37(m, 1H), 1.40(s, 3H), 1.70-1.76(m, 1H), 1.88-1.95(dt, 1H), 2.03-2.07(d, 2H), 3.13(s, 1H), 3.86-3.87(d, 1H), 4.67-4.78(dd, 2H), 7.18-7.33(m, 3H), 7.56-7.58(d, 1H) |

Table 2 (continued)

| compound No. | | physical quality |
|---|---|---|
| A-29-1 | | m.p. 70-74 ℃ |
| | $^1$H-NMR δ (ppm) (CDCl$_3$) | 1.21(s, 3H), 1.30-1.37(m, 1H), 1.33(s, 2H), 1.39(s, 3H), 1.65-1.75(q, 2H), 1.84-1.91(dt, 1H), 2.38-2.41(d, 1H), 3.13(s, 1H), 3.86-3.88(d, 1H), 4.71(s, 2H), 7.20-7.35(m, 3H), 7.55-7.57(d, 1H) |
| A-29-2 | | viscous liquid |
| | $^1$H-NMR δ (ppm) (CDCl$_3$) | 1.21-1.43(m, 9H), 1.67-1.76(m, 1H), 1.89-1.96(m, 1H), 2.03-2.13(m, 2H), 3.13(s, 1H), 3.86-3.88(d, 1H), 4.69-4.82(dd, 2H), 7.17-7.35(m, 3H), 7.58-7.60(d, 1H) |
| A-30-1 | | m.p. 156-160 ℃ |
| | $^1$H-NMR δ (ppm) (CDCl$_3$) | 1.16-1.41(m, 9H), 1.63-1.70(m, 1H), 1.76-1.85(m, 2H), 2.29-2.32(d, 1H), 3.10(s, 1H), 3.80-3.81(d, 1H), 4.60-4.74(dd, 2H), 6.87-6.91(m, 2H), 7.23-7.31(m, 1H) |
| A-30-2 | | viscous liquid |
| | $^1$H-NMR δ (ppm) (CDCl$_3$) | 1.17-1.36(m, 9H), 1.66-1.73(m, 1H), 1.83-1.91(m, 1H), 2.02-2.08(m, 2H), 3.11(s, 1H), 3.80-3.82(d, 1H), 4.55-4.86(dd, 2H), 6.87-6.92(m, 2H), 7.23-7.30(m, 1H) |
| A-31-1 | | m.p. 172-176 ℃ |
| | $^1$H-NMR δ (ppm) (CDCl$_3$) | 1.22-1.42(m, 9H), 1.60-1.87(m, 3H), 2.35-2.38(d, 1H), 3.10(s, 1H), 3.79-3.80(d, 1H), 4.59-4.73(dd, 2H), 6.87-6.91(m, 2H), 7.26-7.31(m, 1H) |

Table 2 (continued)

| compound No. | | physical quality |
|---|---|---|
| A-31-2 | | viscous liquid |
| | $^1$H-NMR δ(ppm) (CDCl$_3$) | 1.23-1.42(m, 9H), 1.66-1.72(m, 1H), 1.85-1.93(m, 1H), 2.02-2.13(m, 2H), 3.11(s, 1H), 3.80-3.82(d, 1H), 4.57-4.89(dd, 2H), 6.86-6.90(m, 2H), 7.23-7.29(m, 1H) |
| A-32 | | viscous liquid |
| | $^1$H-NMR δ(ppm) (CDCl$_3$) (diastereomer mixture) | 0.8-2.2(m), 1.40(s), 1.43(s), 2.37(s), 2.38(s), 2.40(s), 2.96-3.04(m), 3.44(d), 3.58-3.66(m), 4.40-4.78(m), 7.1-7.5(m) |
| A-33 | | viscous liquid |
| | $^1$H-NMR δ(ppm) (CDCl$_3$) (diastereomer mixture) | 0.98(d), 1.41(s), 1.45(s), 0.8-2.2(m), 2.98-3.04(m), 3.43(d), 3.63-3.66(m), 4.55-4.84(m), 7.0-7.6(m) |
| A-34-1 | | m.p. 128-131 ℃ |
| | $^1$H-NMR δ(ppm) (CDCl$_3$) | 1.18(s, 3H), 1.33(s, 3H), 1.12-1.40(m, 3H), 1.65-1.88(m, 3H), 2.28-2.36(m, 1H), 3.11(brs, 1H), 3.83(dd, 1H), 4.66(d, 1H), 4.69(d, 1H), 7.00-7.06(m, 1H), 7.11-7.15(m, 1H), 7.24-7.33(m, 1H), 7.43-7.52(m, 1H) |
| A-34-2 | | viscous liquid |
| | $^1$H-NMR δ(ppm) (CDCl$_3$) | 1.21(s, 3H), 1.35(s, 3H), 1.16-1.38(m, 3H), 1.68-1.76(m, 1H), 1.85-1.94(m, 1H), 2.02-2.09(m, 2H), 3.13(brs, 1H), 3.82(dd, 1H), 4.67(d, 1H), 4.74(d, 1H), 7.00-7.07(m, 1H), 7.11-7.15(m, 1H), 7.24-7.33(m, 1H), 7.43-7.52(m, 1H) |

Table 2 (continued)

| compound No. | | physical quality |
|---|---|---|
| A-35-1 | | m.p. 122-125 ℃ |
| | $^1$H-NMR δ(ppm) (CDCl$_3$) | 1.21(s, 3H), 1.33(s, 3H), 1.30-1.40(m, 3H), 1.63-1.75(m, 2H), 1.81-1.90(m, 1H), 2.36-2.43(m, 1H), 3.12(brs, 1H), 3.82(dd, 1H), 4.66(d, 1H), 4.68(d, 1H), 7.01-7.06(m, 1H), 7.12-7.17(m, 1H), 7.26-7.29(m, 1H), 7.47-7.51(m, 1H) |
| A-35-2 | | viscous liquid |
| | $^1$H-NMR δ(ppm) (CDCl$_3$) | 1.25(s, 3H), 1.34(s, 3H), 1.24-1.44(m, 3H), 1.68-1.76(m, 1H), 1.88-1.96(m, 1H), 2.03-2.14(m, 2H), 3.12(brs, 1H), 3.82(dd, 1H), 4.69(d, 1H), 4.77(d, 1H), 7.01-7.05(m, 1H), 7.10-7.14(m, 1H), 7.23-7.30(m, 1H), 7.48-7.54(m, 1H) |
| A-36-1 | | viscous liquid |
| | $^1$H-NMR δ(ppm) (CDCl$_3$) | 1.22(s, 3H), 1.40(s, 3H), 1.18-1.40(m, 3H), 1.45-1.64(m, 1H), 1.68-1.75(m, 1H), 1.81-1.88(m, 1H), 2.07-2.16(m, 1H), 3.06(d, 1H), 3.71(dd, 1H), 4.62(d, 1H), 4.76(d, 1H), 7.02-7.06(m, 1H), 7.14-7.18(m, 1H), 7.24-7.30(m, 1H), 7.45-7.50(m, 1H) |
| A-36-2 | | m.p. 60-65 ℃ |
| | $^1$H-NMR δ(ppm) (CDCl$_3$) | 1.23(s, 3H), 1.37(s, 3H), 1.15-1.94(m, 7H), 3.06(d, 1H), 3.71(dd, 1H), 4.63(d, 1H), 4.79(d, 1H), 7.02-7.07(m, 1H), 7.11-7.16(m, 1H), 7.23-7.29(m, 1H), 7.45-7.50(m, 1H) |

Table 2 (continued)

| compound No. | | physical quality |
|---|---|---|
| A-37-1 | | m.p. 68-72 ℃ |
| | $^1$H-NMR δ (ppm) (CDCl$_3$) | 1.17(s, 3H), 1.34(s, 3H), 1.10-1.39(m, 3H), 1.64-1.72(m, 2H), 1.79-1.88(m, 1H), 2.28-2.36(m, 1H), 2.38(s, 3H), 3.10(brs, 1H), 3.81(dd, 1H), 4.62(s, 2H), 7.10-7.35(m, 4H) |
| A-37-2 | | viscous liquid |
| | $^1$H-NMR δ (ppm) (CDCl$_3$) | 1.21(s, 3H), 1.33(s, 3H), 1.16-1.34(m, 3H), 1.68-1.76(m, 1H), 1.85-1.93(m, 1H), 2.01-2.08(m, 2H), 2.39(s, 3H), 3.11-3.13(m, 1H), 3.79(dd, 1H), 4.53(d, 1H), 4.73(d, 1H), 7.15-7.40(m, 4H) |
| A-38-1 | | viscous liquid |
| | $^1$H-NMR δ (ppm) (CDCl$_3$) | 1.20(s, 3H), 1.35(s, 3H), 1.20-1.35(m, 3H), 1.63-1.72(m, 2H), 1.81-1.90(m, 1H), 2.38(s, 3H), 2.36-2.42(m, 1H), 3.10-3.13(m, 1H), 3.81(dd, 1H), 4.61(d, 1H), 4.62(d, 1H), 7.15-7.40(m, 4H) |
| A-38-2 | | viscous liquid |
| | $^1$H-NMR δ (ppm) (CDCl$_3$) | 1.25(s, 3H), 1.33(s, 3H), 1.20-1.44(m, 3H), 1.68-1.76(m, 1H), 1.87-1.96(m, 1H), 2.02-2.14(m, 2H), 2.40(s, 3H), 3.11(brs, 1H), 3.79(dd, 1H), 4.56(d, 1H), 4.76(d, 1H), 7.13-7.40(m, 4H) |
| A-39-1 | | m.p. 147-150 ℃ |
| | $^1$H-NMR δ (ppm) (CDCl$_3$) | 1.20(s, 3H), 1.33(s, 3H), 1.16-1.42(m, 3H) 1.62-1.71(m, 1H), 1.78-1.88(m, 2H), 2.26-2.34(m, 1H), 3.09-3.11(m, 1H), 2.31(dd, 1H), 4.68(dd, 1H), 4.79(dd, 1H), 6.97-7.02(m, 1H), 7.20-7.27(m, 2H) |

Table 2 (continued)

| compound No. | | physical quality |
|---|---|---|
| A-39-2 | | viscous liquid |
| | $^1$H-NMR δ(ppm) (CDCl$_3$) | 1.22(s, 3H), 1.31(s, 3H), 1.16-1.38(m, 3H), 1.65-1.74(m, 1H), 1.83-1.92(m, 1H), 2.00-2.11(m, 2H), 3.10-3.12(m, 1H), 4.62(dd, 1H), 4.62(dd, 1H), 4.92(dd, 1H), 6.97-7.01(m, 1H), 7.20-7.27(m, 2H) |
| A-40-1 | | m.p. 130-135 ℃ |
| | $^1$H-NMR δ(ppm) (CDCl$_3$) | 1.23(s, 3H), 1.33(s, 3H), 1.20-1.90(m, 6H), 2.34-2.42(m, 1H), 3.10-3.12(m, 1H), 3.81(dd, 1H), 4.68(dd, 1H), 4.78(dd, 1H), 6.97-7.02(m, 1H), 7.16-7.27(m, 2H) |
| A-40-2 | | viscous liquid |
| | $^1$H-NMR δ(ppm) (CDCl$_3$) | 1.25(s, 3H), 1.31(s, 3H), 1.20-1.43(m, 3H), 1.66-1.74(m, 1H), 1.86-1.94(m, 1H), 2.01-2.07(m, 1H), 2.11-2.17(m, 1H), 3.10-3.12(m, 1H), 3.83(dd, 1H), 4.64(dd, 1H), 4.97(dd, 1H), 6.96-7.02(m, 1H), 7.16-7.27(m, 2H) |
| A-41 (diastereomer mixture) | | viscous liquid |
| | $^1$H-NMR δ(ppm) (CDCl$_3$) | 0.64(t), 0.72(t), 0.99(t), 1.00(t), 0.90-2.55(m), 2.36(s), 2.37(s), 2.78-2.88(m), 3.06(d), 3.12-3.15(m), 3.99(d), 4.03(d), 4.04(d), 4.48(d), 4.66(d), 4.87(d), 7.11-7.46(m) |
| A-42 (diastereomer mixture) | | viscous liquid |
| | $^1$H-NMR δ(ppm) (CDCl$_3$) | 0.65(t), 0.75(t), 0.99(t), 1.01(t), 0.90-2.55(m), 2.78-2.88(m), 3.07(m), 3.12-3.16(m), 4.00(d), 4.03(d), 4.64(d), 4.69(d), 4.76(d), 4.91(d), 6.98-7.56(m) |

Table 2 (continued)

| compound No. | | physical quality |
|---|---|---|
| A-43 (diastereomer mixture) | | viscous liquid |
| $^1$H-NMR δ(ppm) (CDCl$_3$) | 1.26(s), 1.35(s), 1.15-1.60(m), 1.84-1.94(m), 2.04-2.20(m), 2.49-2.64(m), 3.05(dd), 3.08(dd), 3.76-3.84(m), 4.63-4.75(m), 7.00-7.05(m), 7.10-7.16(m), 7.22-7.30(m), 7.50-7.55(m) | |
| A-44 (diastereomer mixture) | | viscous liquid |
| $^1$H-NMR δ(ppm) (CDCl$_3$) | 1.17(d), 1.18(d), 1.36(s), 1.20-2.18(m), 3.05-3.10(m), 3.52-3.68(m), 3.80(dd), 3.86(dd), 4.64-4.78(m), 6.98-7.05(m), 7.11-7.17(m), 7.25-7.30(m), 7.45-7.55(m) | |
| A-45 (diastereomer mixture) | | viscous liquid |
| $^1$H-NMR δ(ppm) (CDCl$_3$) | 1.16(d), 1.40(s), 1.10-2.15(m), 3.10-3.20(m), 3.48-3.68(m), 3.71-3.78(m), 4.60-4.80(m), 7.00-7.05(m), 7.10-7.18(m), 7.25-7.32(m), 7.45-7.55(m) | |
| A-46 (diastereomer mixture) | | viscous liquid |
| $^1$H-NMR δ(ppm) (CDCl$_3$) | 1.08(d), 1.09(d), 1.39(s), 1.20-2.10(m), 3.02-3.04(m), 3.14-3.26(m), 3.68-3.75(m), 3.82-3.92(m), 4.00-4.08(m), 4.62(d), 4.75(d), 5.02-5.30(m), 5.85-5.96(m), 7.00-7.05(m), 7.10-7.17(m), 7.24-7.32(m), 7.45-7.55(m) | |
| A-47 (diastereomer mixture) | | viscous liquid |
| $^1$H-NMR δ(ppm) (CDCl$_3$) | 1.40(s), 1.06-2.15(m), 3.00-3.80(m), 4.58-4.80(m), 7.00-7.05(m), 7.10-7.18(m), 7.24-7.32(m), 7.45-7.55(m) | |
| A-48 (diastereomer mixture) | | viscous liquid |
| $^1$H-NMR δ(ppm) (CDCl$_3$) | 1.18(s), 1.20(s), 1.34(s), 1.10-2.32(m), 3.08-3.12(m), 3.19(s), 3.20(s), 3.36-3.52(m), 3.79-3.84(m), 4.65-4.76(m), 7.00-7.05(m), 7.10-7.17(m), 7.23-7.33(m), 7.45-7.55(m) | |

Table 2 (continued)

| compound No. | | physical quality |
|---|---|---|
| A-49 (diastereomer mixture) | | viscous liquid |
| $^1$H-NMR δ(ppm) ($CDCl_3$) | 1.14(s), 1.17(s), 1.39(s), 1.40(s), 1.25-2.30(m), 3.04-3.07(m), 3.20(s), 3.22(s), 3.26-3.80(m), 4.58-4.82(m), 7.00-7.06(m), 7.10-7.18(m), 7.23-7.33(m), 7.45-7.55(m) | |
| A-50 | | viscous liquid |
| $^1$H-NMR δ(ppm) ($CDCl_3$) | 1.20-1.28(m, 1H), 1.47-1.53(m, 2H), 1.80-1.86(m, 1H), 1.92-1.97(m, 1H), 2.10-2.15(m, 2H), 2.39(s, 3H), 2.57(dd, 1H), 3.09(s, 1H), 3.39-3.46(m, 2H), 3.86(t, 1H), 4.50-4.53(d, 1H), 4.65-4.68(d, 1H), 4.87-4.92(d, 1H), 5.02-5.05(d, 1H), 5.59-5.70(m, 1H), 7.15-7.23(m, 3H), 7.32-7.34(m, 1H) | |
| A-51 (diastereomer mixture) | | viscous liquid |
| $^1$H-NMR δ(ppm) ($CDCl_3$) | 1.07(d), 1.08(d), 1.39(s), 1.20-2.02(m), 2.98-3.12(m), 3.31(s), 3.32(s), 3.69-3.75(m), 4.59-4.78(m), 7.01-7.06(m), 7.11-7.16(m), 7.24-7.33(m), 7.45-7.55(m) | |
| A-52 (diastereomer mixture) | | viscous liquid |
| $^1$H-NMR δ(ppm) ($CDCl_3$) | 1.11(d), 1.12(d), 1.39(s), 1.10-2.15(m), 3.02-3.05(m), 3.36(s), 3.70(s), 3.30-3.76(m), 4.55-4.80(m), 7.00-7.06(m), 7.11-7.17(m), 7.24-7.33(m), 7.45-7.55(m) | |
| A-53 | | viscous liquid |
| $^1$H-NMR δ(ppm) ($CDCl_3$) | 1.39(s, 3H), 1.66-1.84(m, 2H), 2.04(s, 3H), 2.50-2.12(m, 1H), 2.30-2.38(m, 1H), 2.95(dd, 1H), 3.84(dd, 1H), 4.60(d, 1H), 4.63-4.72(m, 1H), 4.74(d, 1H), 7.02-7.07(m, 1H), 7.12-7.16(m, 1H), 7.24-7.32(m, 1H), 7.45-7.55(m, 1H) | |

Table 2 (continued)

| compound No. | | physical quality |
|---|---|---|
| A-54 (diastereomer mixture) | | viscous liquid |
| | $^1$H-NMR δ(ppm) ($CDCl_3$) | 1.14(t), 1.15(t), 1.19(s), 1.20(s), 1.34(s), 1.35(s), 1.10-2.30(m), 3.07-3.10(m), 3.34-3.52(m), 3.80-3.83(m), 4.64-4.76(m), 7.02-7.07(m), 7.11-7.16(m), 7.24-7.33(m), 7.45-7.55(m) |
| A-55 | | viscous liquid |
| | $^1$H-NMR δ(ppm) ($CDCl_3$) | 1.35(s, 3H), 1.48-1.55(m, 1H), 1.73(s, 3H), 1.73-1.78(m, 1H), 1.86-1.92(m, 1H), 2.08-2.12(m, 1H), 2.38(brs, 1H), 3.10(s, 1H), 3.74(t, 1H), 4.67-4.91(m, 4H), 7.15-7.19(m, 1H), 7.30-7.32(m, 2H) |
| A-56 | | m.p. 137-141 ℃ |
| | $^1$H-NMR δ(ppm) ($CDCl_3$) | 1.37(s, 3H), 1.55-1.63(m, 1H), 1.72-1.78(m, 1H), 1.98-2.05(m, 1H), 2.19-2.26(m, 1H), 2.93(dd, 1H), 3.55-3.65(m, 1H), 3.79(dd, 1H), 4.04(d, 1H), 4.60(d, 1H), 4.73(d, 1H), 7.02-7.07(m, 1H), 7.12-7.16(m, 1H), 7.25-7.32(m, 1H), 7.45-7.55(m, 1H) |
| A-57 | | viscous liquid |
| | $^1$H-NMR δ(ppm) ($CDCl_3$) | 1.38(s, 3H), 1.44-1.61(m, 1H), 1.69-1.76(m, 1H), 2.11-2.18(m, 1H), 2.26-2.34(m, 1H), 2.94(dd, 1H), 3.14-3.22(m, 1H), 3.31(s, 3H), 3.77(dd, 1H), 4.63(d, 1H), 4.75(d, 1H), 7.02-7.07(m, 1H), 7.12-7.16(m, 1H), 7.25-7.32(m, 1H), 7.45-7.55(m, 1H) |

Table 2 (continued)

| compound No. | | physical quality |
|---|---|---|
| A-58 | | m.p. 67-69 ℃ |
| | $^1$H-NMR δ(ppm) ($CDCl_3$) | 1.19(t, 3H), 1.38(s, 3H), 1.52-1.62(m, 1H), 1.72-1.80(m, 1H), 2.16-2.18(m, 1H), 2.25-2.33(m, 1H), 2.94(dd, 1H), 3.22-3.32(m, 1H), 3.48(q, 2H), 3.77(dd, 1H), 4.62(d, 1H), 4.75(d, 1H), 7.01-7.07(m, 1H), 7.12-7.16(m, 1H), 7.25-7.33(m, 1H), 7.45-7.55(m, 1H) |
| A-59 | | viscous liquid |
| | $^1$H-NMR δ(ppm) ($CDCl_3$) | 1.37(s, 3H), 1.58-1.68(m, 1H), 1.81-1.87(m, 1H), 2.15-2.21(m, 1H), 2.26-2.34(m, 1H), 2.93(dd, 1H), 3.32-3.41(m, 1H), 3.75(dd, 1H), 4.52(s, 2H), 4.62(d, 1H), 4.74(d, 1H), 7.01-7.07(m, 1H), 7.11-7.16(m, 1H), 7.24-7.38(m, 1H), 7.45-7.54(m, 1H) |
| A-60 | | viscous liquid |
| | $^1$H-NMR δ(ppm) ($CDCl_3$) | 0.92(t, 3H), 1.38(s, 3H), 1.32-1.78(m, 6H), 2.08-2.15(m, 1H), 2.24-2.32(m, 1H), 2.93(dd, 1H), 3.20-3.28(m, 1H), 3.38-3.42(m, 2H), 3.77(dd, 1H), 4.62(d, 1H), 4.75(d, 1H), 7.01-7.06(m, 1H), 7.11-7.16(m, 1H), 7.24-7.32(m, 1H), 7.46-7.54(m, 1H) |
| A-61-1 | | m.p. 143-147 ℃ |
| | $^1$H-NMR δ(ppm) ($CDCl_3$) | 1.16-1.27(m, 2H), 1.19(s, 3H), 1.35(s, 3H), 1.37-1.43(m, 1H), 1.63-1.70(m, 1H), 1.80-1.88(m, 2H), 2.28-2.31(m, 1H), 3.10(s, 1H), 3.81-3.83(d, 1H), 4.75-4.77(d, 1H), 4.87-4.90(d, 1H), 7.16-7.20(m, 1H), 7.29-7.33(m, 2H) |

Table 2 (continued)

| compound No. | | physical quality |
|---|---|---|
| A-61-2 | | m.p. 136-138 ℃ |
| | $^1$H-NMR δ(ppm) (CDCl$_3$) | 1.15-1.27(m, 2H), 1.21(s, 3H), 1.32(s, 3H), 1.35-1.40(m, 1H), 1.70(t, 1H), 1.89(t, 1H), 2.00-2.11(m, 2H), 3.10(s, 1H), 3.84(d, 1H), 4.71(d, 1H), 5.03(d, 1H), 7.14-7.30(m, 3H) |
| A-62 | | viscous liquid |
| | $^1$H-NMR δ(ppm) (CDCl$_3$) | 1.46(s, 3H), 2.12-2.20(m, 3H), 3.05-3.08(m, 1H), 4.11(dd, 1H), 4.56-4.60(m, 3H), 4.72(d, 1H), 6.83-6.86(m, 2H), 6.94-7.16(m, 3H), 7.22-7.31(m, 3H), 7.40-7.50(m, 1H) |
| A-63-1 | | m.p. 104-106 ℃ |
| | $^1$H-NMR δ(ppm) (CDCl$_3$) | 1.17(s, 3H), 1.31(s, 3H), 1.13-1.35(m, 3H), 1.63-1.72(m, 2H), 1.78-1.87(m, 1H), 2.29-2.35(m, 1H), 3.09-3.11(m, 1H), 3.80(dd, 1H), 4.59(d, 1H), 4.67(d, 1H), 7.25-7.40(m, 5H) |
| A-63-2 | | viscous liquid |
| | $^1$H-NMR δ(ppm) (CDCl$_3$) | 1.21(s, 3H), 1.33(s, 3H), 1.17-1.33(m, 3H), 1.67-1.74(m, 1H), 1.86-1.94(m, 1H), 2.00-2.09 (m, 2H), 3.11(brs, 1H), 3.78(dd, 1H), 4.53(d, 1H), 4.75(d, 1H), 7.26-7.40(m, 5H) |
| A-64-1 | | m.p. 95- 98 ℃ |
| | $^1$H-NMR δ(ppm) (CDCl$_3$) | 1.12(s, 3H), 1.31(s, 3H), 1.18-1.35(m, 3H), 1.62-1.71(m, 2H), 1.82-1.89(m, 1H), 2.37-2.42(m, 1H), 3.09-3.11(m, 1H), 3.79(dd, 1H), 4.58(d, 1H), 4.65(d, 1H), 7.25-7.40(m, 5H) |

Table 2 (continued)

| compound No. | | physical quality |
|---|---|---|
| A-64-2 | | viscous liquid |
| | $^1$H-NMR δ(ppm) (CDCl$_3$) | 1.24(s, 3H), 1.32(s, 3H), 1.20-1.44(m, 3H), 1.68-1.75(m, 1H), 1.88-1.96(m, 1H), 2.03-2.11 (m, 2H), 3.12(brs, 1H), 3.78(dd, 1H), 4.55(d, 1H), 4.78(d, 1H), 7.26-7.41(m, 5H) |
| A-65 | | viscous liquid |
| | $^1$H-NMR δ(ppm) (CDCl$_3$) | 0.73-0.77(dt, 6H), 1.16-1.29(m, 5H), 1.39(s, 3H), 1.55-1.65(m, 3H), 2.98(d, 1H), 3.73(dd, 1H), 4.61(d, 1H), 4.73(d, 1H), 7.01-7.50(m, 4H) |
| A-66 | | viscous liquid |
| | $^1$H-NMR δ(ppm) (CDCl$_3$) | 1.46(s, 3H), 1.66-1.74(m, 1H), 2.03(s, 3H), 1.92-2.16(m, 3H), 3.00(d, 1H), 3.96(dd, 1H), 4.61(d, 1H), 4.72(d, 1H), 5.01-5.06(m, 1H), 7.01-7.06(m, 1H), 7.11-7.16(m, 1H), 7.24-7.32(m, 1H), 7.44-7.54(m, 1H) |
| A-67 | | viscous liquid |
| | $^1$H-NMR δ(ppm) (CDCl$_3$+DMSO-d$_6$) | 1.40(s, 3H), 1.52-1.59(m, 1H), 1.86-2.04(m, 3H), 3.00-3.02(m, 1H), 4.00(brs, 1H), 4.13(dd, 1H), 4.27(brs, 1H), 4.58(d, 1H), 4.72(d, 1H), 7.01-7.06(m, 1H), 7.12-7.17(m, 1H), 7.24-7.32(m, 1H), 7.45-7.52(m, 1H) |
| A-68 | | viscous liquid |
| | $^1$H-NMR δ(ppm) (CDCl$_3$) | 1.41(s, 3H), 1.57-1.64(m, 1H), 1.98-2.01(m, 2H), 2.05-2.11(m, 1H), 3.01(brs, 1H), 3.26(s, 3H), 3.47-3.50(m, 1H), 3.95(dd, 1H), 4.64(d, 1H), 4.77(d, 1H), 7.01-7.07(m, 1H), 7.12-7.16(m, 1H), 7.25-7.32(m, 1H), 7.45-7.54(m, 1H) |

Table 2 (continued)

| compound No. | | physical quality |
|---|---|---|
| A-69 | | viscous liquid |
| | $^1$H-NMR δ(ppm) (CDCl$_3$) | 0.92(t, 3H), 1.41(s, 3H), 1.30-1.62(m, 5H), 1.96-2.06(m, 3H), 3.01(dd, 1H), 3.26-3.40(m, 2H), 3.56(brs, 1H), 3.96(dd, 1H), 4.63(d, 1H), 4.73(d, 1H), 7.01-7.06(m, 1H), 7.11-7.16(m, 1H), 7.24-7.32(m, 1H), 7.46-7.52(m, 1H) |
| A-70 | | viscous liquid |
| | $^1$H-NMR δ(ppm) (CDCl$_3$) | 1.42(s, 3H), 1.56-1.66(m, 1H), 2.02-2.13(m, 3H), 3.02(dd, 1H), 3.70(brs, 1H), 4.05(dd, 1H), 4.43(d, 1H), 4.46(d, 1H), 4.64(d, 1H), 4.73(d, 1H), 7.01-7.07(m, 1H), 7.11-7.17(m, 1H), 7.23-7.38(m, 6H), 7.45-7.55(m, 1H) |
| A-71 | | viscous liquid |
| | $^1$H-NMR δ(ppm) (CDCl$_3$) | 1.43(s, 3H), 1.63-1.69(m, 1H), 2.01-2.17(m, 3H), 3.03(dd, 1H), 3.75(brs, 1H), 4.07(dd, 1H), 4.52(d, 1H), 4.53(d, 1H), 4.65(d, 1H), 4.74(d, 1H), 7.00-7.55(m, 8H) |
| A-72 | | viscous liquid |
| | $^1$H-NMR δ(ppm) (CDCl$_3$) | 1.42(s, 3H), 1.72-1.81(m, 1H), 1.92-1.99(m, 1H), 2.23-2.29(m, 1H) 2.39-2.47(m, 1H), 2.99(dd, 1H), 3.86(dd, 1H), 4.15-4.24(m, 1H), 4.62(d, 1H), 4.74(d, 1H), 6.84-6.87(m, 2H), 6.93-6.98(m, 1H), 7.01-7.07(m, 1H), 7.12-7.16(m, 1H), 7.25-7.30(m, 3H), 7.45-7.52(m, 1H) |
| A-73 | | viscous liquid |
| | $^1$H-NMR δ(ppm) (CDCl$_3$) | 1.42(s, 3H), 1.61-1.68(m, 1H), 2.02-2.11(m, 3H), 3.01-3.03(m, 1H), 3.68-3.72(m, 1H), 4.03(dd, 1H), 4.38(d, 1H), 4.43(d, 1H), 4.65(d, 1H), 4.73(d, 1H), 7.02-7.07(m, 1H), 7.12-7.17(m, 2H), 7.25-7.33(m, 4H), 7.46-7.52(m, 1H) |

Table 2 (continued)

| compound No. | | physical quality |
|---|---|---|
| A-74 (E/Z mixture) | | viscous liquid |
| $^1$H-NMR δ (ppm) ($CDCl_3$) | 1.40(s), 1.42(s), 1.54-2.02(m), 2.48-2.58(m), 3.02-3.06(m), 3.72-3.80(m), 4.58-4.80(m), 5.16-5.25(m), 7.01-7.07(m), 7.11-7.17(m), 7.25-7.32(m), 7.46-7.53(m) | |
| A-75 (diastereomer mixture) | | viscous liquid |
| $^1$H-NMR δ (ppm) ($CDCl_3$) | 1.36(s), 1.50-1.92(m), 2.92-2.98(m), 3.65-3.72(m), 4.61-4.74(m), 7.00-7.50(m) | |
| A-76 | | viscous liquid |
| $^1$H-NMR δ (ppm) ($CDCl_3$) | 1.40(s, 3H), 1.68(s, 3H), 1.48-2.05(m, 5H), 3.62(d, 1H), 3.75(dd, 1H), 4.59-4.78(m, 4H), 7.00-7.05(m, 1H), 7.10-7.15(m, 1H), 7.24-7.30(m, 1H), 7.46-7.52(m, 1H) | |
| A-77 | | viscous liquid |
| $^1$H-NMR δ (ppm) ($CDCl_3$) | 0.85(d, 6H), 1.39(s, 3H), 1.13-1.90(m, 6H), 3.02(d, 1H), 3.70(dd, 1H), 4.64(d, 1H), 4.75(d, 1H), 7.03-7.06(m, 1H), 7.11-7.16(m, 1H), 7.24-7.31(m, 1H), 7.45-7.53(m, 1H) | |
| A-78-1 | | viscous liquid |
| $^1$H-NMR δ (ppm) ($CDCl_3$) | 1.39(s, 3H), 1.60-1.68(m, 1H), 1.87-1.94(m, 1H), 1.99-2.05(m, 1H), 2.30-2.37(m, 1H), 3.05-3.13(m, 1H), 3.18(dd, 1H), 3.78(dd, 1H), 4.63(d, 1H), 4.70(d, 1H), 7.00-7.04(m, 1H), 7.11-7.35(m, 7H), 7.45-7.55(m, 1H) | |
| A-78-2 | | viscous liquid |
| $^1$H-NMR δ (ppm) ($CDCl_3$) | 1.45(s, 3H), 1.80-1.94(m, 2H), 1.96-2.01(m, 1H), 2.11-2.19(m, 1H), 2.55-2.65(m, 1H), 3.09(dd, 1H), 3.87(dd, 1H), 4.63(d, 1H), 4.75(d, 1H), 7.00-7.06(m, 1H), 7.10-7.33(m, 7H), 7.45-7.55(m, 1H) | |

Table 2 (continued)

| compound No. | | physical quality |
|---|---|---|
| A-79 | | viscous liquid |
| | $^1$H-NMR δ(ppm) (CDCl$_3$) | 0.84(t, 3H), 1.43-1.51(m, 1H), 1.53-1.60(m, 1H), 1.82-1.90(m, 2H), 2.02-2.06(m, 1H), 2.32-2.37(m, 1H), 2.35(s, 3H), 3.09(m, 1H), 3.17(s, 1H), 3.84(t, 1H), 4.47(d, 1H), 4.64(d, 1H), 7.12-7.31(m, 9H) |
| A-80 | | viscous liquid |
| | $^1$H-NMR δ(ppm) (CDCl$_3$) | 0.88(t, 3H), 1.46-1.63(m, 2H), 1.81-1.91(m, 2H), 2.04-2.08(m, 1H), 2.33-2.38(m, 1H), 3.10(m, 1H), 3.19(t, 1H), 3.67(t, 1H), 4.58(d, 1H), 4.69(d, 1H), 6.98-7.49(m, 9H) |
| A-81 (diastereomer mixture) | | viscous liquid |
| | $^1$H-NMR δ(ppm) (CDCl$_3$) | 1.22(s), 1.23(s), 1.37(s), 1.40(s), 1.18-1.93(m), 2.08-2.16(m), 3.05-3.07(m), 3.68-3.73(m), 4.61-4.81(m), 7.02-7.07(m), 7.11-7.16(m), 7.24-7.32(m), 7.45-7.54(m) |
| A-82 (diastereomer mixture) | | viscous liquid |
| | $^1$H-NMR δ(ppm) (CDCl$_3$) | 1.25(s), 1.26(s), 1.37(s), 1.40(s), 1.15-1.95(m), 2.15-2.25(m), 3.04-3.08(m), 3.68-3.74(m), 4.60-4.65(m), 4.73-4.82(m), 7.02-7.07(m), 7.11-7.16(m), 7.25-7.32(m), 7.45-7.55(m) |
| A-83 | | m.p. 129-130 ℃ |
| | $^1$H-NMR δ(ppm) (CDCl$_3$) | 1.18(s, 3H), 1.11-1.41(m, 2H), 1.33(s, 3H), 1.62-1.91(m, 3H), 2.31(d, 1H), 3.10(s, 1H), 3.82(d, 1H), 4.65(d, 1H), 4.70(d, 1H), 6.97-7.58(m, 4H) |

Table 2 (continued)

| compound No. | | physical quality |
|---|---|---|
| A-84 | | m.p. 122-125 ℃ |
| | $^1$H-NMR δ(ppm) (CDCl$_3$) | 1.21(s, 3H), 1.28-1.40(m, 2H), 1.33(d, 3H), 1.62-1.78(m, 2H), 1.79-1.92(m, 1H), 2.39(d, 1H), 3.12(s, 1H), 3.82(d, 1H), 4.66(d, 1H), 4.70(d, 1H), 7.00-7.08(m, 1H), 7.12-7.19(m, 1H), 7.24-7.32(m, 1H), 7.46-7.54(m, 1H) |
| A-85-1 | | viscous liquid |
| | $^1$H-NMR δ(ppm) (CDCl$_3$) | 0.88(t, 3H), 1.15-1.41(m, 8H), 1.35(s, 3H), 1.70-1.74(m, 2H), 2.06-2.11(m, 1H), 3.01(s, 1H), 3.76(t, 1H), 4.68(dd, 2H), 7.00-7.52(m, 4H), |
| A-85-2 | | viscous liquid |
| | $^1$H-NMR δ(ppm) (CDCl$_3$) | 0.88(t, 3H), 1.22-1.40(m, 9H), 1.38(s, 3H), 1.74-1.78(m, 1H), 1.91-1.96(m, 1H), 2.98(d, 1H), 3.70(dd, 1H), 4.68(dd, 2H), 7.00-7.50(m, 4H), |
| A-86-1 | | viscous liquid |
| | $^1$H-NMR δ(ppm) (CDCl$_3$) | 1.39(s, 3H), 1.66-1.72(m, 1H), 1.85-2.02(m, 2H), 2.34-2.39(m, 1H), 3.21(s, 1H), 3.53-3.61(m, 1H), 3.70-3.73(m, 1H), 4.66(dd, 2H), 6.94-7.55(m, 8H) |
| A-86-2 | | viscous liquid |
| | $^1$H-NMR δ(ppm) (CDCl$_3$) | 1.46(s, 3H), 1.73-1.90(m, 2H), 1.94-1.99(m, 1H), 2.22-2.30(m, 1H), 3.11(dd, 1H), 3.90(dd, 1H), 4.71(dd, 2H), 7.00-7.50(m, 8H) |

Table 2 (continued)

| compound No. | | physical quality |
|---|---|---|
| A-87-1 | | viscous liquid |
| | $^1$H-NMR δ (ppm) (CDCl$_3$) | 1.38(s, 3H), 1.54-1.61(m, 1H), 1.80-1.87(m, 1H), 1.95-2.04(m, 1H), 2.28-2.33(m, 1H), 3.06(m, 1H), 3.17(t, 1H), 3.76(t, 1H), 4.61-4.72(dd, 2H), 6.99-7.50(m, 8H) |
| A-87-2 | | m.p. 143-147 ℃ |
| | $^1$H-NMR δ (ppm) (CDCl$_3$) | 1.43(s, 3H), 1.76-1.91(m, 3H), 2.09-2.16(m, 1H), 2.54-2.62(m, 1H), 3.07(d, 1H), 3.83-3.87(dd, 1H), 4.60-4.77(dd, 2H), 7.00-7.49(m, 8H) |
| A-88 | | viscous liquid |
| | $^1$H-NMR δ (ppm) (CDCl$_3$) | 1.36(s, 3H), 1.44-1.51(m, 1H), 1.71(s, 3H), 1.72-1.82(m, 2H), 2.10-2.15(dd, 1H), 2.35-2.38(m, 1H), 3.10(t, 1H), 3.75(t, 1H), 4.64-4.77(m, 4H), 7.04-7.12(m, 2H), 7.25-7.28(m, 1H) |
| A-89 | | viscous liquid |
| | $^1$H-NMR δ (ppm) (CDCl$_3$) | 1.22-1.43(m, 9H), 1.65-2.41(m, 4H), 3.13(s, 1H), 3.83(t, 1H), 4.68-4.80(m, 2H), 7.04-7.12(m, 2H), 7.24-7.29(m, 1H), |
| A-90 | | viscous liquid |
| | $^1$H-NMR δ (ppm) (CDCl$_3$) | 1.34(s, 3H), 1.43-1.50(m, 1H), 1.69-1.81(m, 2H), 1.71(s, 3H), 2.10-2.15(dd, 1H), 2.35-2.38(m, 1H), 3.09-3.10(dd, 1H), 3.73(t, 1H), 4.57-4.77(m, 4H), 6.76-6.89(m, 2H), 7.43-7.49(m, 1H) |
| A-91 | | viscous liquid |
| | $^1$H-NMR δ (ppm) (CDCl$_3$) | 1.21-1.43(m, 9H), 1.64-2.40(m, 4H), 3.12(s, 1H), 3.81(s, 1H), 4.57-4.73(m, 2H), 6.75-6.90(m, 2H), 7.42-7.50(m, 1H) |

Table 2 (continued)

| compound No. | | physical quality |
|---|---|---|
| A-92 (diastereomer mixture) | | viscous liquid |
| $^1$H-NMR δ(ppm) (CDCl$_3$) | 1.18(s), 1.21(s), 1.35(s), 1.36(s), 1.15-1.36(m), 1.64-2.10(m), 2.30-2.36(m), 3.11-3.15(m), 3.78-3.82(m), 4.51-4.74(m), 6.94-7.35(m) | |
| A-93 (diastereomer mixture) | | viscous liquid |
| $^1$H-NMR δ(ppm) (CDCl$_3$) | 1.17(s), 1.20(s), 1.31(s), 1.33(s), 1.12-1.36(m), 1.64-2.09(m), 2.28-2.35(m), 3.09-3.13(m), 3.76-3.80(m), 4.49(d), 4.54(d), 4.62(d), 4.69(d), 7.01-7.05(m), 7.32-7.38(m) | |
| A-94 | | m.p. 124-125 ℃ |
| $^1$H-NMR δ(ppm) (CDCl$_3$) | 1.21(s, 3H), 1.24-1.27(m, 2H), 1.32-1.38(m, 3H), 1.40(s, 1H), 1.66-1.73(m, 2H), 1.83-1.91(m, 1H), 2.40(d, 1H), 3.13(s, 1H), 3.87(dd, 1H), 4.79(s, 2H), 7.35-7.39(m, 1H), 7.54-7.63(m, 2H), 7.79-7.82(m, 1H) | |
| A-95 | | viscous liquid |
| $^1$H-NMR δ(ppm) (CDCl$_3$) | 1.35(s, 3H), 1.48-1.59(m, 1H), 1.65-1.74(m, 1H), 1.70(s, 3H), 1.80-1.86(m, 1H), 1.91-2.08(m, 2H), 3.02(d, 1H), 3.73(dd, 1H), 4.72(s, 2H), 4.75(dd, 2H), 6.86-6.93(m, 2H), 7.23-7.32(m, 1H) | |
| A-96 (E/Z mixture) | | viscous liquid |
| $^1$H-NMR δ(ppm) (CDCl$_3$) | 1.35(s), 1.30-2.10(m), 2.95-3.05(m), 3.08-3.11(m), 3.72(dd), 3, 80(dd), 4.60-4.75(m), 5.18-5.25(m), 7.01-7.06(m), 7.10-7.16(m), 7.25-7.32(m), 7.46-7.52(m) | |

Table 2 (continued)

| compound No. | | physical quality |
|---|---|---|
| A-97 | | viscous liquid |
| | $^1$H-NMR δ(ppm) (CDCl$_3$) | 1.35(s, 3H), 1.41-1.50(m, 1H), 1.71(s, 3H), 1.69-1.76(m, 1H), 1.78-1.85(m, 1H), 2.28-2.35(m, 1H), 2.34-2.43(m, 1H), 3.07-3.11(m, 1H), 3.75(t, J=5Hz, 1H), 3.82(s, 3H), 4.66(dd, J=22Hz, 2H), 4.66(s, 1H), 4.76(s, 1H), 6.83-6.87(m, 1H), 6.92-6.98(m, 1H), 7.22-7.28(m, 1H), 7.45-7.49(m, 1H) |
| A-98 | | viscous liquid |
| | $^1$H-NMR δ(ppm) (CDCl$_3$) | 1.42(s, 3H), 1.49-1.57(m, 1H), 1.72(s, 3H), 1.73-1.79(m, 1H), 1.82-1.89(m, 1H), 2.11-2.18(m, 1H), 2.33-2.43(m, 1H), 3.11-3.14(m, 1H), 3.85(t, J=5Hz, 1H), 4.68(s, 1H), 4.78(s, 1H), 4.81(dd, J=13Hz, 2H), 7.36-7.41(m, 1H), 7.58-7.72(m, 3H) |
| A-99 | | viscous liquid |
| | $^1$H-NMR δ(ppm) (CDCl$_3$) | 1.37(s, 3H), 1.65-1.72(m, 1H), 1.93-2.10(m, 1H), 2.39-2.44(m, 1H), 3.14-3.15(m, 1H), 3.36-3.38(m, 1H), 3.77(dd, 1H), 4.66(dd, 2H), 6.78-6.79(m, 1H), 6.90-6.93(m, 1H), 6.99-7.03(m, 1H), 7.10-7.14(m, 2H), 7.22-7.26(m, 2H), 7.46-7.50(m, 1H) |
| A-100 (diastereomer mixture) | | viscous liquid |
| | $^1$H-NMR δ(ppm) (CDCl$_3$) | 0.89(d), 0.90(d), 1.35(s), 1.15-1.86(m), 2.02-2.12(m), 3.06(br s), 3.38-3.46(m), 3.56-3.64(m), 3.76-3.82(m), 4.67(d), 4.71(d), 7.01-7.06(m), 7.12-7.17(m), 7.24-7.31(m), 7.49-7.53(m) |

Table 2 (continued)

| compound No. | physical quality |
|---|---|
| A-101 (diastereomer mixture)<br>$^1$H-NMR δ(ppm) (CDCl$_3$) 1.36(s), 1.38(s), 1.84(s), 1.86(s), 1.20-2.35(m), 3.11-3.16(m), 3.80-4.01(m), 4.68-4.78(m), 7.01-7.07(m), 7.11-7.18(m), 7.24-7.32(m), 7.48-7.59(m) | viscous liquid |
| A-102 (diastereomer mixture)<br>$^1$H-NMR δ(ppm) (CDCl$_3$) 1.40(s), 1.41(s), 1.78(s), 1.86(s), 1.45-2.08(m), 3.06-3.08(m), 3.76-3.88(m), 4.01(d), 4.66(d), 4.74-4.80(m), 7.02-7.09(m), 7.12-7.18(m), 7.25-7.32(m), 7.46-7.52(m) | viscous liquid |
| A-103 (diastereomer mixture)<br>$^1$H-NMR δ(ppm) (CDCl$_3$) 0.99(d), 1.00(d), 1.35(s), 1.15-2.15(m), 3.06(s), 3.35-3.41(m), 3.51-3.56(m), 3.77-3.80(m), 4.65-4.75(m), 7.02-7.08(m), 7.12-7.18(m), 7.24-7.33(m), 7.45-7.52(m), | viscous liquid |
| A-104 (diastereomer mixture)<br>$^1$H-NMR δ(ppm) (CDCl$_3$) 1.01(d), 1.02(d), 1.35(s), 1.15-2.15(m), 3.06(s), 3.24-3.30(m), 3.41-3.47(m), 3.77-3.79(m), 4.65-4.76(m), 7.02-7.06(m), 7.13-7.17(m), 7.24-7.33(m), 7.46-7.52(m), | viscous liquid |
| A-105 (diastereomer mixture)<br>$^1$H-NMR δ(ppm) (CDCl$_3$) 0.98(d), 0.99(d), 1.15-1.24(m), 1.35(s), 1.35-1.54(m), 1.74-1.85(m), 2.07-2.14(m), 3.02-3.10(m), 3.26-3.30(m), 3.75-3.80(m), 4.64-4.76(m), 7.01-7.06(m), 7.13-7.17(m), 7.24-7.33(m), 7.48-7.53(m) | viscous liquid |

Table 2 (continued)

| compound No. | | physical quality |
|---|---|---|
| A-106 | | viscous liquid |
| | $^1$H-NMR δ(ppm) (CDCl$_3$) | 1.23-1.31(m, 1H), 1.37(s, 3H), 1.54(s, 3H), 1.56(s, 3H), 1.64-1.71(m, 1H), 1.92-2.00(m, 1H), 2.02-2.07(m, 1H), 2.29-2.36(m, 1H), 3.12(dd, H), 3.86(dd, 1H), 4.69(d, 1H), 4.73(d, 1H), 7.01-7.06(m, 1H), 7.12-7.16(m, 1H), 7.25-7.32(m, 1H), 7.48-7.51(m, 1H) |
| A-107 | | viscous liquid |
| | $^1$H-NMR δ(ppm) (CDCl$_3$) | 1.25-1.35(m, 1H), 1.33(s, 3H), 1.55(s, 3H), 1.57(s, 3H), 1.63-1.70(m, 1H), 1.90-1.98(m, 1H), 2.02-2.09(m, 1H), 2.28-2.34(m, 1H), 3.11(dd, H), 3.85(dd, 1H), 4.65(d, 1H), 4.75(d, 1H), 6.87-6.92(m, 2H), 7.24-7.30(m, 1H) |
| A-108 | | viscous liquid |
| | $^1$H-NMR δ(ppm) (CDCl$_3$) | 1.41(s, 3H), 1.41-1.51(m, 1H), 1.71(s, 3H), 1.71-1.78(m, 1H), 1.79-1.86(m, 1H), 2.12-2.19(m, 1H), 2.35-2.44(m, 1H), 3.10-3.14(m, 1H), 3.79(t, J=5Hz, 1H), 4.68(s, 1H), 4.77(s, 1H), 4.81(dd, J=14Hz, 2H), 7.33-7.39(m, 1H), 7.53-7.65(m, 2H), 7.82-7.86(m, 1H) |
| A-109 | | viscous liquid |
| | $^1$H-NMR δ(ppm) (CDCl$_3$) | 1.40(s, 3H), 1.53-1.64(m, 1H), 1.83-1.99(m, 2H), 2.26-2.37(m, 1H), 2.34(s, 3H), 3.19-3.22(m, 1H), 3.27-3.36(m, 1H), 3.78(t, J=4Hz, 1H), 4.67(dd, J=13Hz, 2H), 6.98-7.04(m, 1H), 7.07-7.20(m, 6H), 7.22-7.29(m, 1H), 7.46-7.52(m, 1H) |

Table 2 (continued)

| compound No. | | physical quality |
|---|---|---|
| A-110 | | viscous liquid |
| $^1$H-NMR δ(ppm) ($CDCl_3$) | 1.41(s,3H),1.82-1.89(m,1H), 2.06-2.14(m,1H),2.31-2.40(m,1H), 2.55-2.64(m,1H),3.14-3.18(m,1H), 3.19(dd,J=3Hz,1H),4.08-4.18(m,1H), 4.70(s,2H),6.96-7.32(m,6H), 7.52-7.58(m,1H) | |
| A-111 (diastereomer mixture) | | viscous liquid |
| $^1$H-NMR δ(ppm) ($CDCl_3$) | 0.93(d),1.15-1.28(m),1.35(s), 1.49-1.88(m),2.05-2.14(m),3.04-3.06(m), 3.78-3.81(m),4.18-4.45(m),4.67(d), 4.71(d),7.01-7.06(m),7.11-7.16(m), 7.24-7.32(m),7.48-7.52(m) | |
| A-112 (diastereomer mixture) | | viscous liquid |
| $^1$H-NMR δ(ppm) ($CDCl_3$) | 0.92(d),0.93(d),1.12-1.22(m),1.35(s), 1.44-1.62(m),1.71-1.84(m),2.02-2.12(m), 3.02-3.11(m),3.29-3.34(m),4.64-4.75(m), 7.01-7.06(m),7.11-7.17(m),7.24-7.32(m), 7.48-7.52(m) | |
| A-113 (diastereomer mixture) | | viscous liquid |
| $^1$H-NMR δ(ppm) ($CDCl_3$) | 1.03(d),1.05(d),1.20-1.28(m),1.36(s), 1.48-1.81(m),2.03-2.24(m),3.06-3.09(m), 3.76-3.81(m),4.64-4.74(m),7.01-7.07(m), 7.11-7.16(m),7.24-7.32(m),7.48-7.52(m), 9.57(d),9.58(d) | |
| A-114 | | viscous liquid |
| $^1$H-NMR δ(ppm) ($CDCl_3$) | 1.44(s,3H),1.91-1.97(m,1H), 2.13-2.20(m,1H),2.36-2.43(m,1H), 2.49-2.55(m,1H),3.09(d,1H),4.18(dd,1H), 4.40-4.46(m,1H),4.65(d,1H),4.74(d,1H), 7.02-7.08(m,1H),7.12-7.17(m,1H), 7.24-7.32(m,1H),7.46-7.51(m,1H) | |

Table 2 (continued)

| compound No. | | physical quality |
|---|---|---|
| A-115 | | viscous liquid |
| | $^1$H-NMR δ(ppm) (CDCl$_3$) | 1.44(s, 3H), 1.71-1.78(m, 1H), 2.08-2.16(m, 1H), 2.46-2.54(m, 1H), 2.58-2.65(m, 1H), 3.06(d, 1H), 4.08(dd, 1H), 4.44-4.50(m, 1H), 4.65(d, 1H), 4.74(d, 1H), 7.03-7.08(m, 1H), 7.13-7.17(m, 1H), 7.24-7.31(m, 1H), 7.46-7.50(m, 1H) |
| A-116 | | viscous liquid |
| | $^1$H-NMR δ(ppm) (CDCl$_3$) | 1.41(s, 3H), 1.73-1.81(m, 1H), 1.87-1.97(m, 2H), 2.13-2.19(m, 1H), 3.05(d, 1H), 3.82-3.88(m, 1H), 3.95(dd, 1H), 4.64(d, 1H), 4.73(d, 1H), 7.02-7.08(m, 1H), 7.12-7.17(m, 1H), 7.24-7.32(m, 1H), 7.46-7.51(m, 1H) |
| A-117 (diastereomer mixture) | | viscous liquid |
| | $^1$H-NMR δ(ppm) (CDCl$_3$) | 1.41(s), 1.44(s), 1.10-2.58(m), 3.05-3.12(m), 3.60(dd), 3.72(dd), 3.92(dd), 3.98(dd), 4.58-4.78(m), 7.01-7.08(m), 7.11-7.17(m), 7.24-7.33(m), 7.45-7.52(m) |
| A-118 | | viscous liquid |
| | $^1$H-NMR δ(ppm) (CDCl$_3$) | 1.38(s, 3H), 1.69-1.76(m, 1H), 1.88-1.97(m, 2H), 2.26-2.31(m, 1H), 3.17-3.18(m, 1H), 3.43-3.49(m, 1H), 3.70(t, 1H), 3.77(s, 3H), 4.64(dd, 2H), 6.82-7.24(m, 7H), 7.46-7.50(m, 1H) |
| A-119 | | viscous liquid |
| | $^1$H-NMR δ(ppm) (CDCl$_3$) | 1.41(s, 3H), 1.57-1.66(m, 1H), 1.84-1.91(m, 1H), 1.96-2.02(m, 1H), 2.33-2.38(m, 1H), 3.17-3.18(m, 1H), 3.55-3.61(m, 1H), 3.83-3.84(m, 1H), 4.66(dd, 2H), 6.97-7.02(m, 1H), 7.11-7.15(m, 1H), 7.22-7.37(m, 3H), 7.48-7.54(m, 2H), 7.62-7.64(m, 1H) |

Table 2 (continued)

| compound No. | | physical quality |
|---|---|---|
| A-120 (diastereomer mixture) | | viscous liquid |
| $^{1}$H-NMR δ(ppm) ($CDCl_3$) | 1.24(d), 1.37(d), 1.37(s), 1.38(s), 1.70-2.70(m), 3.24(dd), 3.38(dd), 4.36-4.38(m), 4.57(d), 4.58(d), 4.68(d), 7.00-7.40(m) | |
| A-121 (diastereomer mixture) | | viscous liquid |
| $^{1}$H-NMR δ(ppm) ($CDCl_3$) | 1.37(s), 1.91(d), 1.05-2.25(m), 3.05-3.12(m), 3.80-3.86(m), 4.17-4.32(m), 4.65-4.80(m), 7.01-7.05(m), 7.11-7.16(m), 7.23-7.34(m), 7.42-7.52(m) | |
| A-122 | | viscous liquid |
| $^{1}$H-NMR δ(ppm) ($CDCl_3$) | 1.41(s, 3H), 1.69-1.76(m, 1H), 1.87-1.99(m, 2H), 2.31-2.37(m, 1H), 3.07(d, 1H), 3.52-3.58(m, 1H), 4.01(dd, 1H), 4.58(d, 1H), 4.66(d, 1H), 7.01-7.06(m, 1H), 7.11-7.14(m, 1H), 7.20-7.55(m, 7H) | |
| A-123 (diastereomer mixture) | | viscous liquid |
| $^{1}$H-NMR δ(ppm) ($CDCl_3$) | 1.06(d), 1.07(d), 1.12-1.20(m), 1.36(s), 1.44-1.52(m), 1.70-1.80(m), 2.07-2.18(m), 2.33-2.40(m), 3.06-3.08(m), 3.77-3.82(m), 4.65-4.74(m), 7.01-7.05(m), 7.11-7.16(m), 7.24-7.34(m), 7.42-7.51(m) | |
| A-124 | | viscous liquid |
| $^{1}$H-NMR δ(ppm) ($CDCl_3$) | 1.41(s, 3H), 1.81-1.88(m, 1H), 1.91-1.97(m, 1H), 2.07-2.14(m, 1H), 2.25-2.32(m, 1H), 2.90-2.98(m, 1H), 3.11(dd, 1H), 3.98(dd, 1H), 4.66(d, 1H), 4.74(d, 1H), 7.04-7.09(m, 1H), 7.13-7.18(m, 1H), 7.27-7.35(m, 1H), 7.44-7.50(m, 1H) | |

Table 2 (continued)

| compound No. | | physical quality |
|---|---|---|
| A-125 (diastereomer mixture) | | viscous liquid |
| $^{1}$H-NMR δ (ppm) ($CDCl_3$) | 0.98(d), 0.99(d), 1.12-1.32(m), 1.36(s), 1.37(s), 1.44-1.52(m), 1.65-1.82(m), 2.05-2.32(m), 3.05(br s), 3.76-3.80(m), 4.63-4.75(m), 6.16(d), 6.17(d), 7.01-7.06(m), 7.12-7.17(m), 7.23-7.34(m), 7.43-7.52(m) | |
| A-126 | | viscous liquid |
| $^{1}$H-NMR δ (ppm) ($CDCl_3$) | 1.44(s, 3H), 1.60-1.78(m, 1H), 2.05-2.29(m, 3H), 3.03(dd, 1H), 4.05(dd, 1H), 4.63(d, 1H), 4.74(d, 1H), 4.78-4.93(m, 1H), 7.02-7.07(m, 1H), 7.12-7.16(m, 1H), 7.25-7.33(m, 1H), 7.44-7.51(m, 1H) | |
| A-127 | | viscous liquid |
| $^{1}$H-NMR δ (ppm) ($CDCl_3$) | 1.39(s, 3H), 1.59-1.67(m, 1H), 1.79-1.86(m, 1H), 1.94-1.99(m, 1H), 2.33-2.38(m, 1H), 3.19-3.20(m, 1H), 3.49-3.55(m, 1H), 3.72(t, 1H), 4.66(dd, 2H), 6.98-7.28(m, 5H), 7.36-7.37(m, 1H), 7.46-7.50(m, 1H) | |
| A-128 | | viscous liquid |
| $^{1}$H-NMR δ (ppm) ($CDCl_3$) | 1.38(s, 3H), 1.44-1.61(m, 1H), 1.80-1.87(m, 1H), 1.95-2.00(m, 1H), 2.27-2.32(m, 1H), 3.02-3.09(m, 1H), 3.15-3.16(m, 1H), 3.77(t, 1H), 4.66(dd, 2H), 6.98-7.06(m, 2H), 7.10-7.28(m, 5H), 7.46-7.50(m, 1H) | |

Table 2 (continued)

| compound No. | | physical quality |
|---|---|---|
| A-129 | | viscous liquid |
| $^1$H-NMR δ(ppm) (CDCl$_3$) | 1.02(d,3H),1.22-1.28(m,1H),1.31(s,3H),1.46-1.54(m,1H),1.67-1.73(m,1H),1.78-1.90(m,2H),2.06-2.12(m,1H),3.05(s,1H),3.24-3.30(m,1H),3.45-3.47(m,1H),3.76(s,1H),4.63-4.78(m,2H),6.88-6.92(m,2H),7.24-7.30(m,1H) | |
| B-1 (diastereomer mixture) | | viscous liquid |
| $^1$H-NMR δ(ppm) (CDCl$_3$) | 0.86(t),1.51(s),1.15-1.55(m),1.85-2.22(m),2.37(s),3.18(s),3.28(s),3.57(s),3.73(s),3.85(s),4.42-4.73(m),7.15-7.40(m) | |
| B-2-1 (diastereomer mixture) | | viscous liquid |
| $^1$H-NMR δ(ppm) (CDCl$_3$) | 1.46(s),1.50(s),1.55-1.62(m),1.75-1.93(m),2.21(s),2.15-2.42(m),2.32(s),2.63-2.71(m),2.82-2.89(m),3.29(s),3.40(s),3.77(d)3.96(d),4.22(d),4.48(d),4.55(d),7.00-7.50(m) | |
| B-2-2 (diastereomer mixture) | | viscous liquid |
| $^1$H-NMR δ(ppm) (CDCl$_3$) | 1.46(s),1.51(s),1.28-1.68(m),1.85-2.00(m),2.18-2.40(m),2.40(s),2.60-3.00(m),3.15(s),3.22(s),3.67(s),3.97(s),4.56-4.76(m),7.06-7.40(m) | |
| B-3-1 | | viscous liquid |
| $^1$H-NMR δ(ppm) (CDCl$_3$) | 0.87(t,3H),1.02(d,3H),1.03(d,3H),1.22-1.36(m,4H),1.48-1.58(m,2H),1.72-1.78(m,1H),2.01-2.09(m,1H),2.37(s,1H),3.16(s,1H),3.81(d,1H),4.54(d,1H),4.64(d,1H),7.15-7.40(m,4H) | |

Table 2 (continued)

| compound No. | | physical quality |
|---|---|---|
| B-3-2 | | viscous liquid |
| | $^1$H-NMR δ(ppm) (CDCl$_3$) | 0.88(t, 3H), 1.01(d, 3H), 1.09(d, 3H), 1.20-2.00(m, 8H), 2.37(s, 3H), 3.10(m, 3H), 3.56(d, 1H), 4.65(s, 2H), 7.15-7.40(m, 4H) |
| B-4-1 | | viscous liquid |
| | $^1$H-NMR δ(ppm) (CDCl$_3$) | 0.87(t, 3H), 1.05(d, 3H), 1.22-1.34(m, 4H), 1.41-1.58(m, 2H), 1.95-2.12(m, 3H), 2.38(s, 3H), 3.29(s, 1H), 3.78(d, 1H), 4.55(d, 1H), 4.65(d, 1H), 7.15-7.40(m, 4H) |
| B-4-2 | | viscous liquid |
| | $^1$H-NMR δ(ppm) (CDCl$_3$) | 0.83(t, 3H), 1.07(d, 3H), 0.85-2.40(m, 9H), 2.39(s, 3H), 3.21(s, 1H), 3.91(d, 1H), 4.46(d, 1H), 4.68(d, 1H), 7.15-7.40(m, 4H) |
| B-4-3 | | viscous liquid |
| | $^1$H-NMR δ(ppm) (CDCl$_3$) | 0.86(t, 3H), 1.01(d, 3H), 1.20-2.30(m, 9H), 2.37(s, 3H), 3.36(s, 1H), 3.52(d, 1H), 4.41(d, 1H), 4.60(d, 1H), 7.15-7.40(m, 4H) |
| B-4-4 | | viscous liquid |
| | $^1$H-NMR δ(ppm) (CDCl$_3$) | 0.88(t, 3H), 1.04(d, 3H), 1.20-2.20(m, 9H), 2.37(s, 3H), 3.20(s, 1H), 3.57(d, 1H), 4.66(s, 2H), 7.15-7.40(m, 4H) |
| B-5-1 | | viscous liquid |
| | $^1$H-NMR δ(ppm) (CDCl$_3$) | 0.90(t, 3H), 1.25-1.34(m, 1H), 1.47-1.80(m, 6H), 1.98-2.07(m, 1H), 2.33(s, 3H), 2.53-2.70(m, 2H), 3.26(s, 1H), 3.86(d, 1H), 4.48(d, 1H), 4.57(d, 1H), 7.15-7.40(m, 4H) |
| B-5-2 | | viscous liquid |
| | $^1$H-NMR δ(ppm) (CDCl$_3$) | 0.83(t, 3H), 1.40-1.98(m, 8H), 2.38(s, 3H), 2.42-2.58(m, 2H), 3.15(s, 1H), 4.00(d, 1H), 4.42(d, 1H), 4.67(d, 1H), 7.15-7.35(m, 4H) |

Table 2 (continued)

| compound No. | | physical quality |
|---|---|---|
| B-5-3 | | viscous liquid |
| | $^{1}$H-NMR δ (ppm) (CDCl$_3$) | 0.90(t, 3H), 1.22-1.32(m, 1H), 1.45-1.82(m, 6H), 1.90-2.00(m, 1H), 2.32-2.38(m, 1H), 2.36(s, 3H), 2.68-2.76(m, 1H), 3.15(s, 1H), 3.65(d, 1H), 4.52(d, 1H), 4.66(d, 1H), 7.15-7.40(m, 4H) |
| C-1 | | viscous liquid |
| | $^{1}$H-NMR δ (ppm) (CDCl$_3$) | 0.28-0.31(t, 1H), 0.38-0.42(dd, 1H), 0.90-0.95(m, 1H), 1.16(s, 3H), 1.25-1.34(m, 1H), 1.70(s, 3H), 1.76-1.88(m, 4H), 3.54-3.58(dd, 1H), 4.60-4.73(m, 4H), 7.00-7.52(m, 4H) |

The examples of the compounds of the present invention which can be synthesized in the manners similar to those shown in the Scheme 1 and to those of the examples described above are shown in the Table 3-a and 3-b and these tables also show the compounds of the example given above. The scope of the present invention should not be restricted by these compounds. And the meaning of each abbreviation used in the Table 3-a and 3-b is shown below. Me:methyl; Et:ethyl; n-Pr:n-propyl; i-Pr:isopropyl; n-Bu:n-butyl; i-Bu:isobutyl; s-Bu:sec-butyl; t-Bu:tert-butyl; n-Pen:n-pentyl ; i-Pen:isopentyl ; n-Hex:n-hexyl ; Ph:phenyl ; c-Pr: cyclopropyl; c-Bu:cyclobutyl; c-Pen:cyclopentyl; c-Hex: cyclohexyl; c-Hep:cycloheptyl; e-Et:epoxyethyl.

Table 3-a

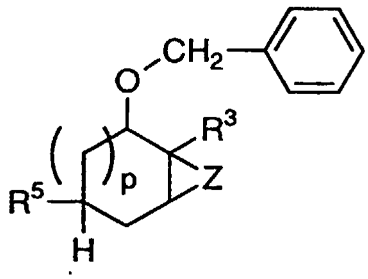 ,

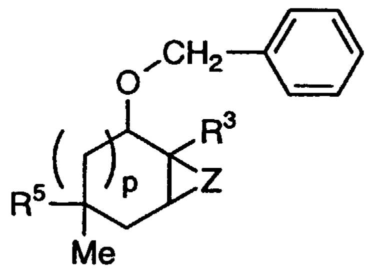 ,

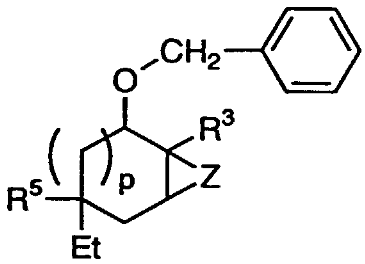 ,

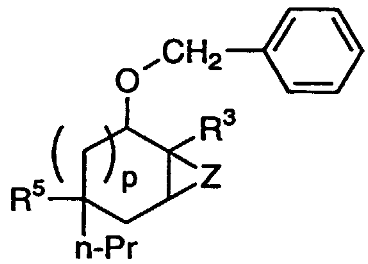 ,

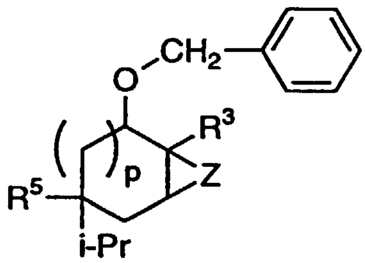 ,

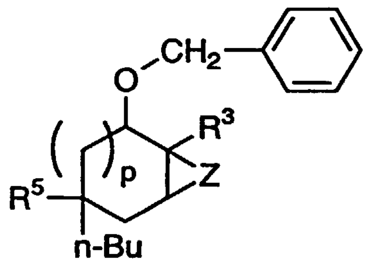 ,

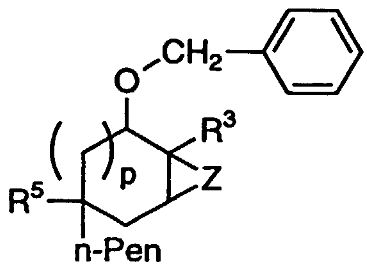 ,

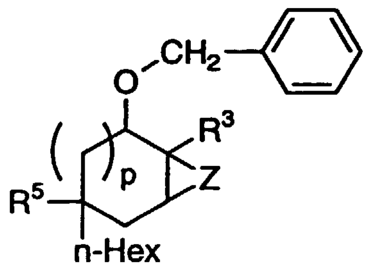 ,

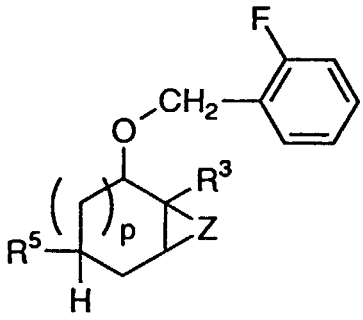 , 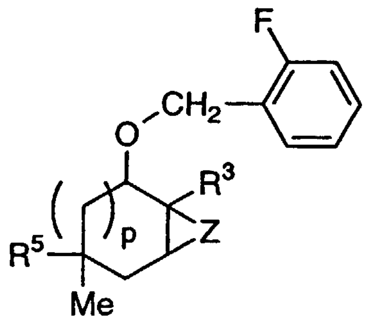 ,

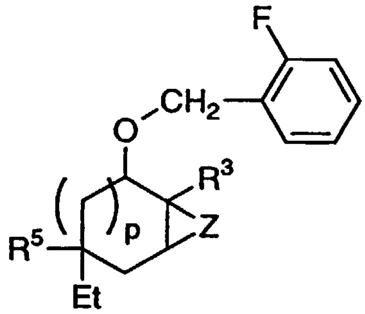 , 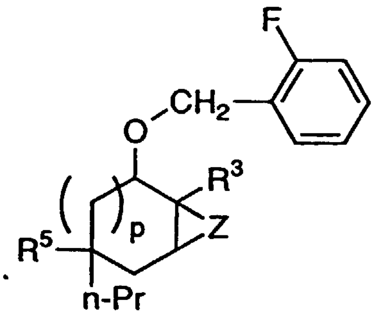 ,

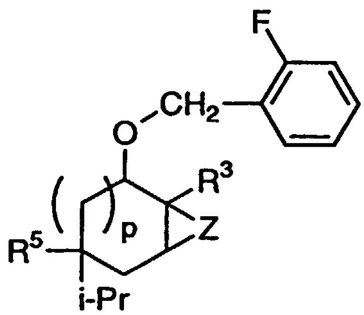 , 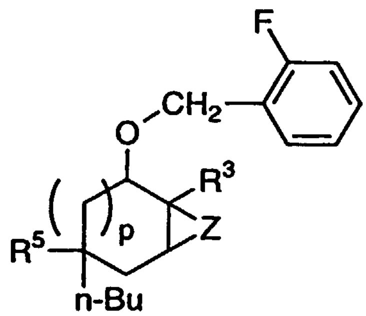 ,

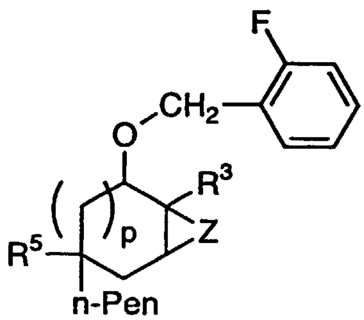 , 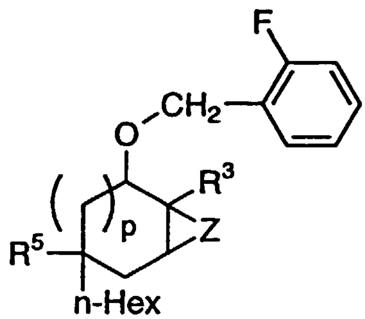 ,

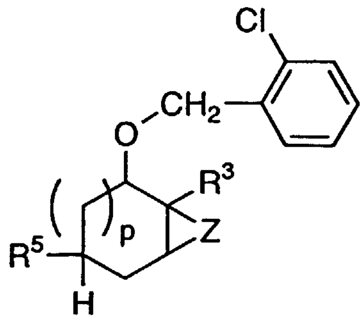 ,

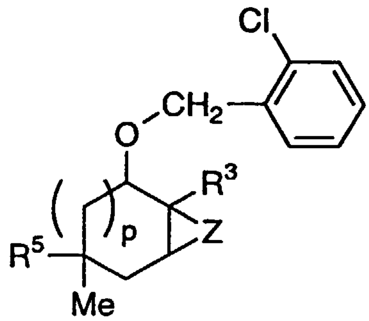 ,

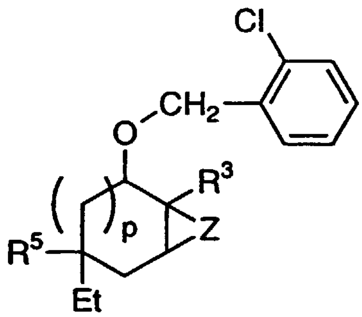 ,

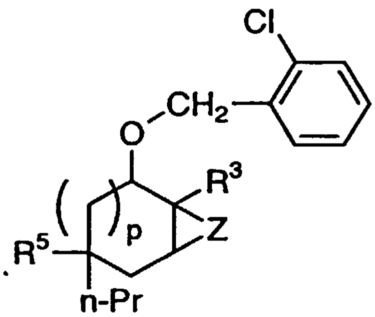 ,

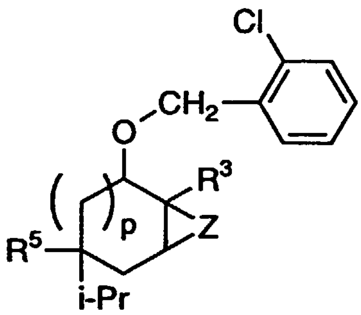 ,

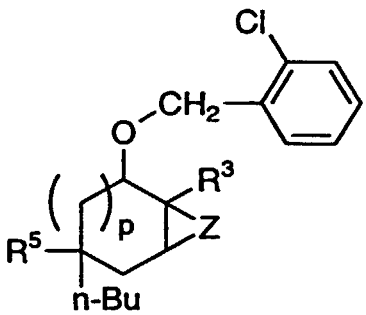 ,

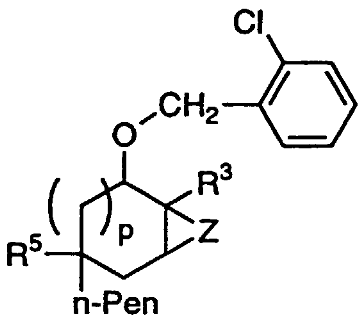 ,

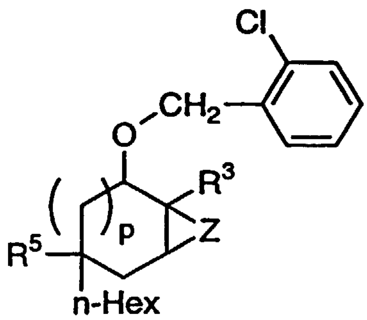 ,

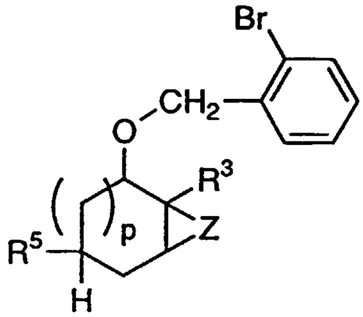

,

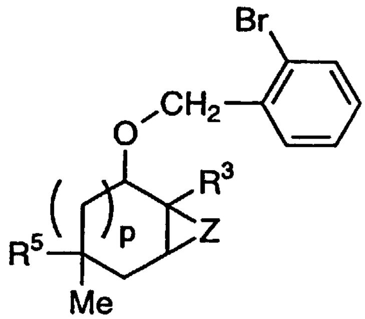

,

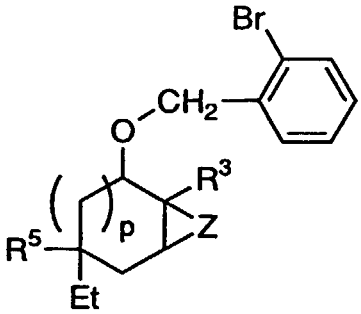

,

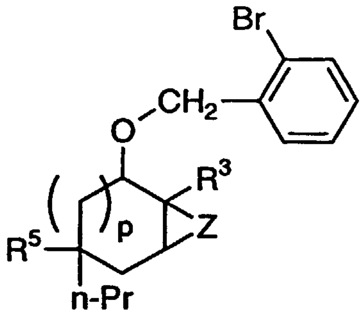

,

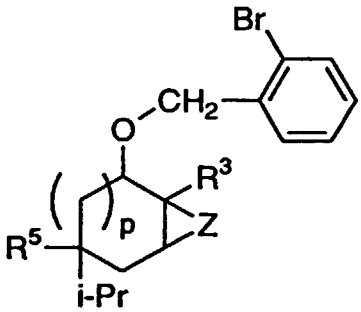

,

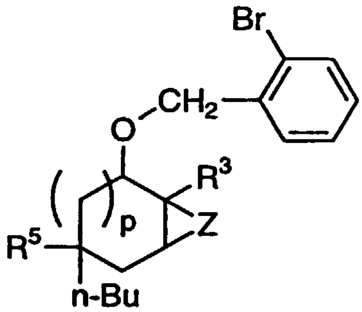

,

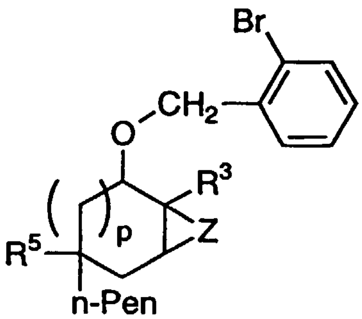

,

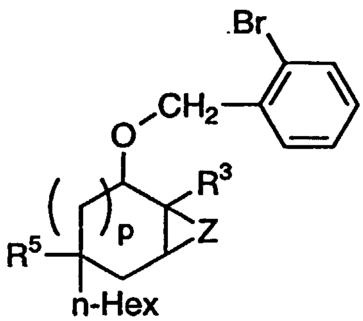

,

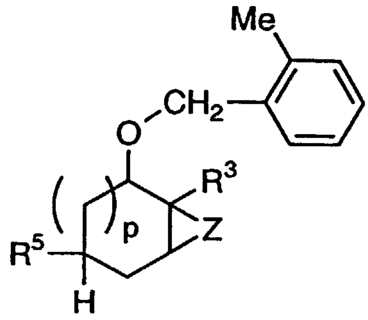
,
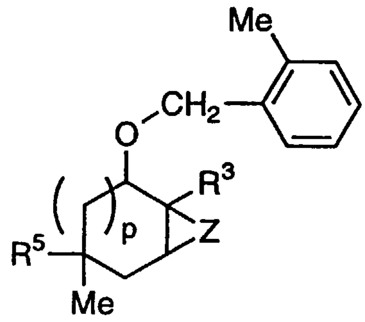
,
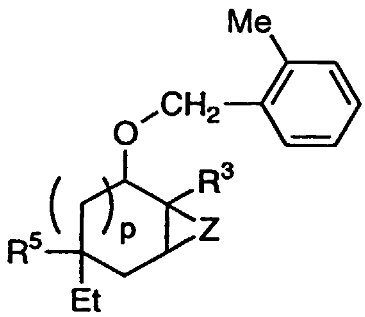
,
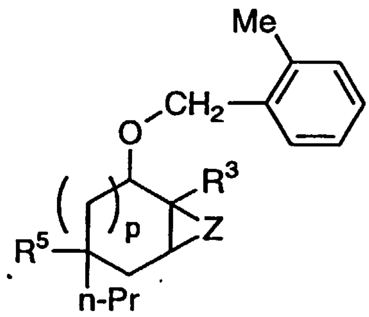
,
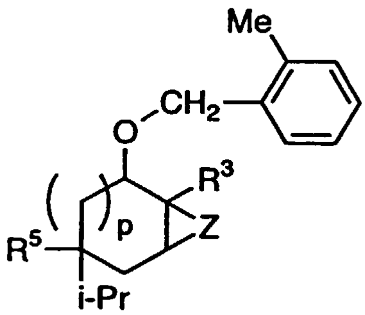
,
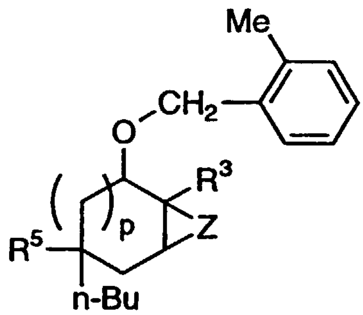
,
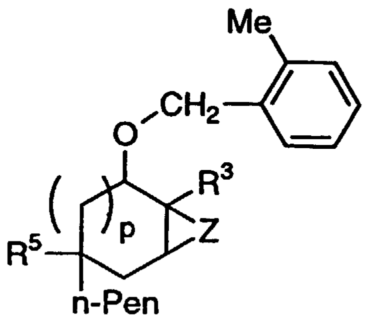
,
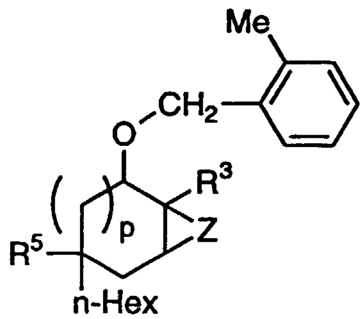
,

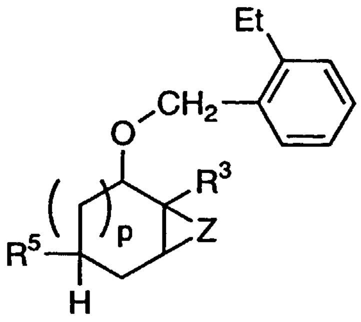 ,

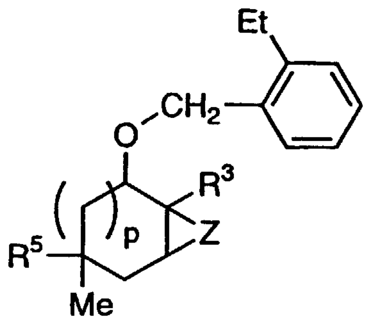 ,

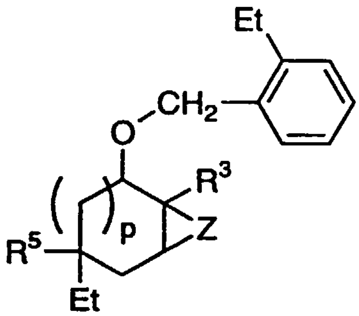 ,

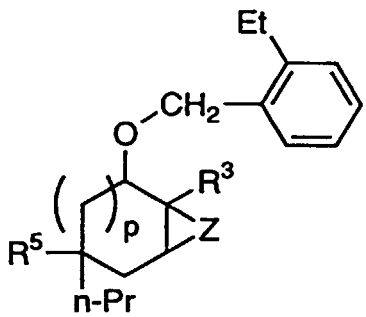 ,

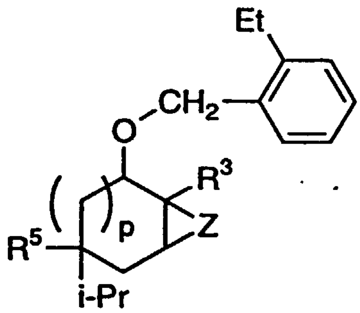 ,

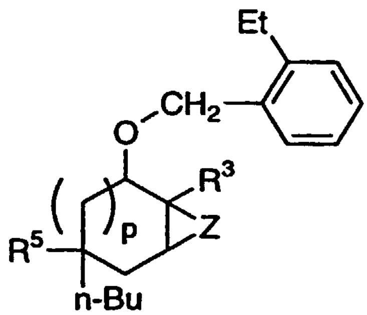 ,

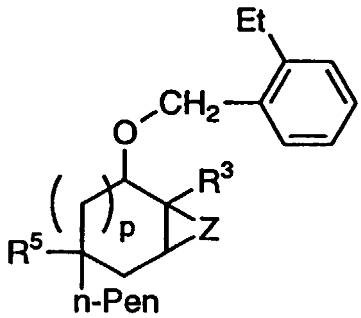 ,

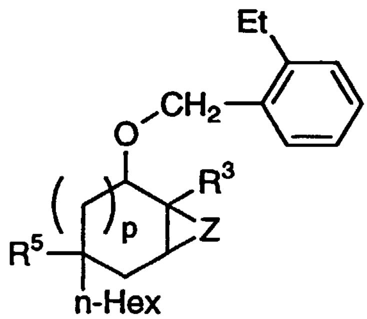 ,

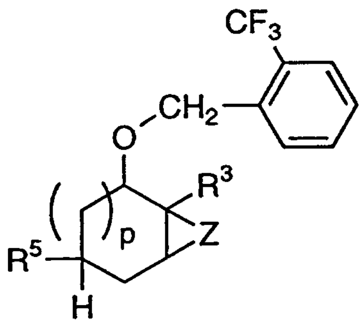

,

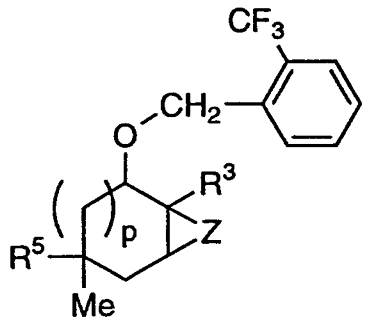

,

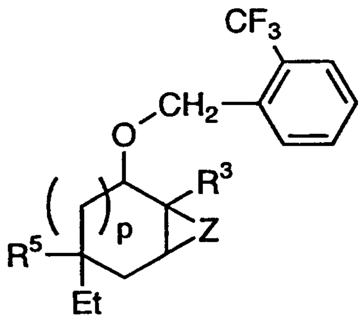

,

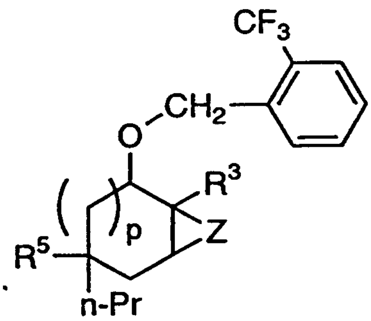

,

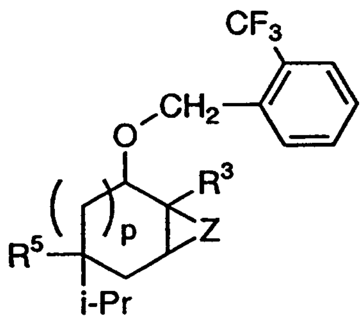

,

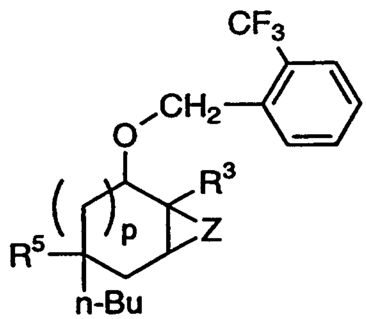

,

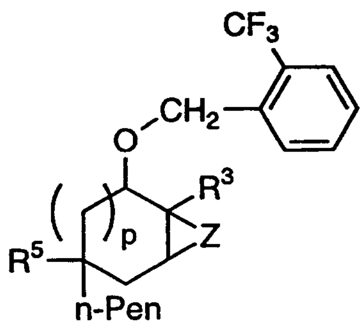

,

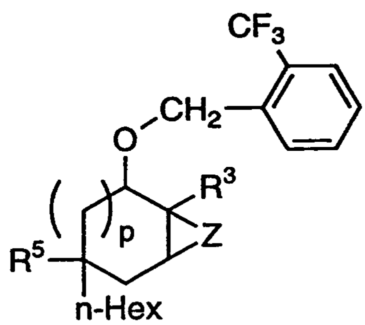

;

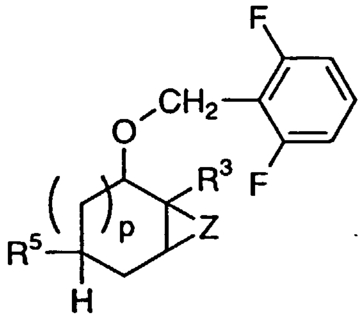 , 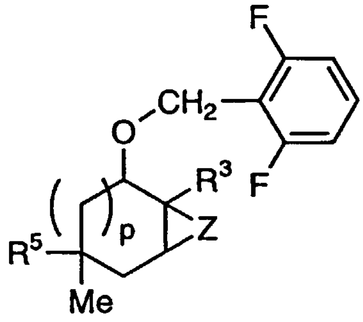 ,

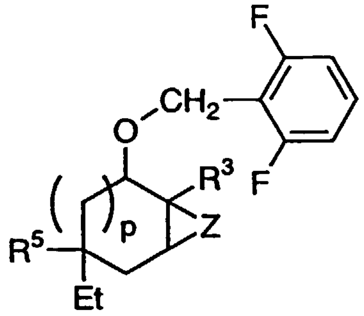 , 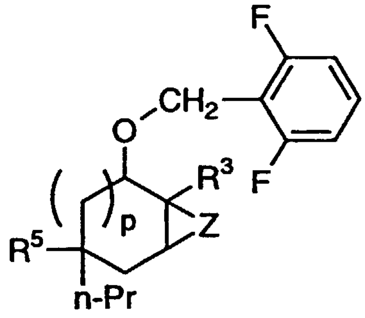 ,

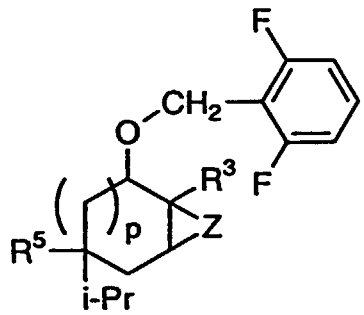 , 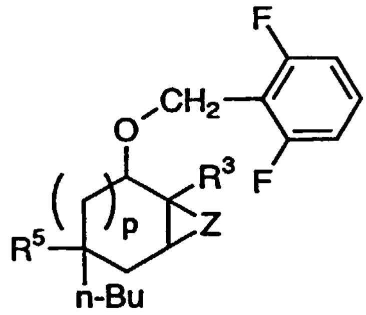 ,

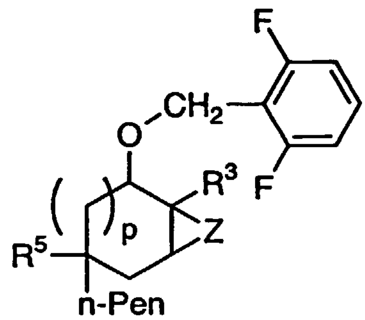 , 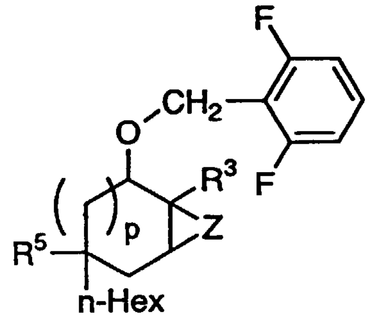 ,

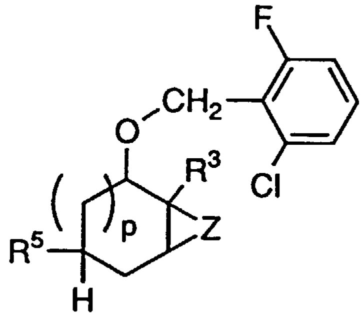 , 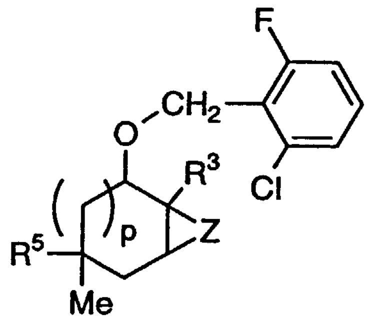 ,

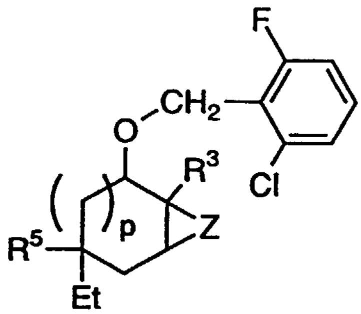 , 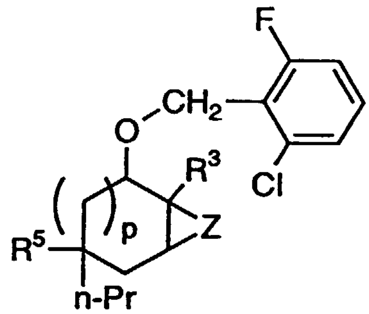 ,

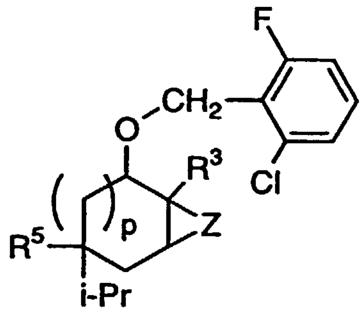 , 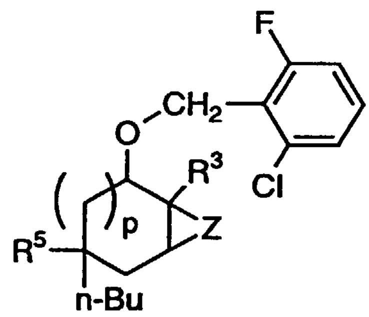 ,

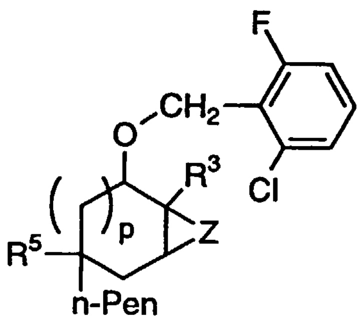 , 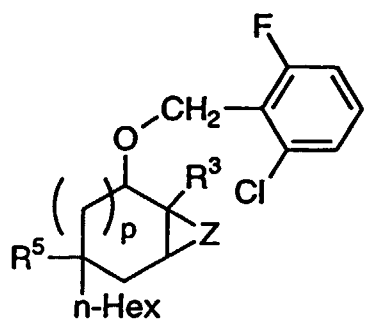 ;

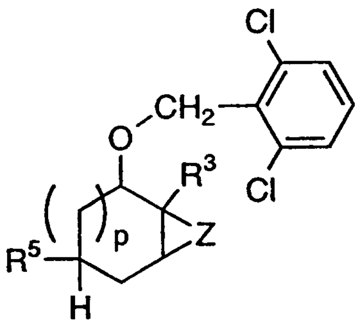
,
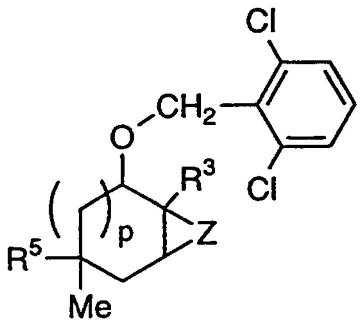
,

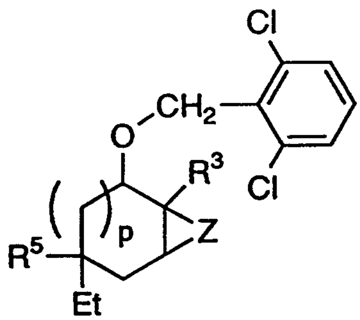
,
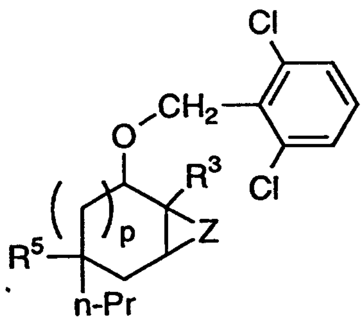
,

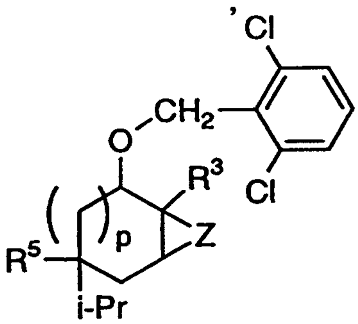
,
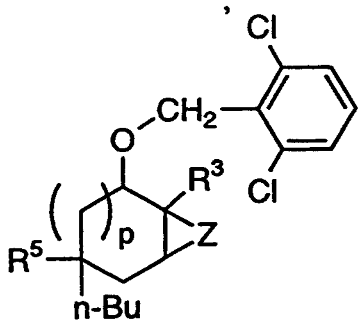
,

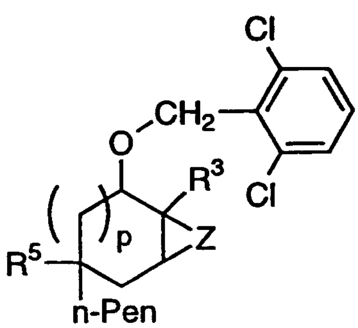
,
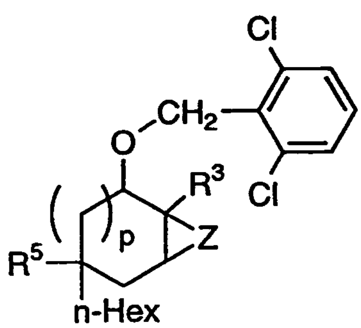
,

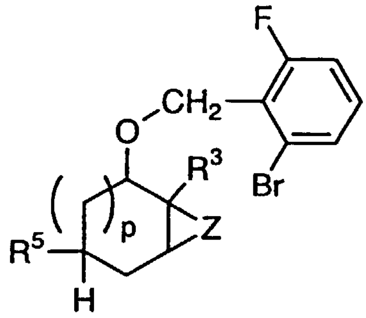

,

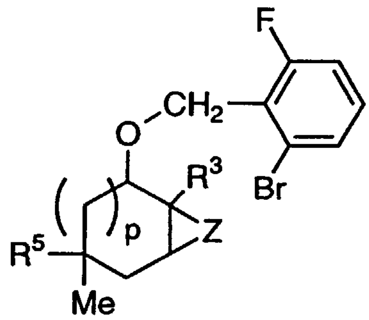

,

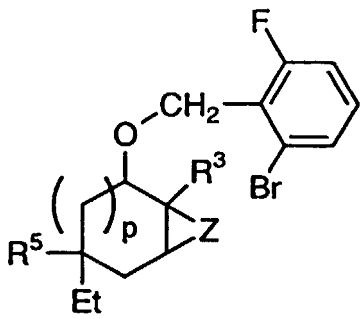

,

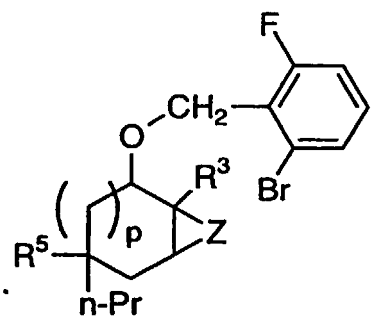

,

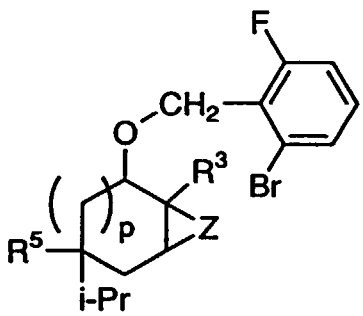

,

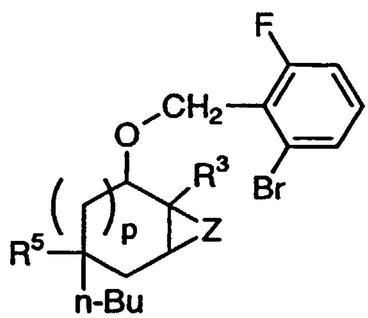

,

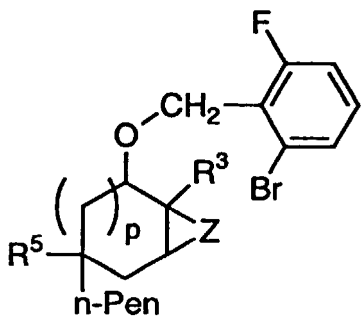

,

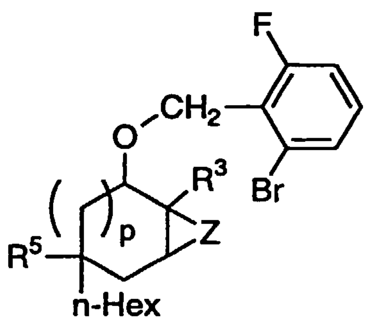

,

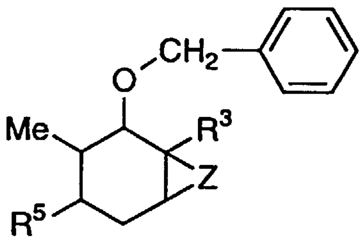
,
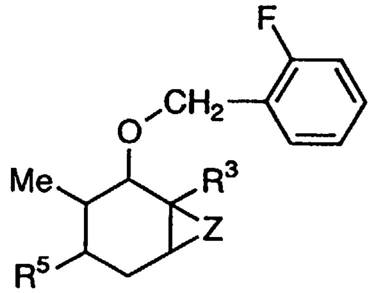
,
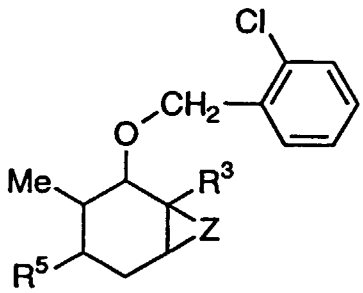
,
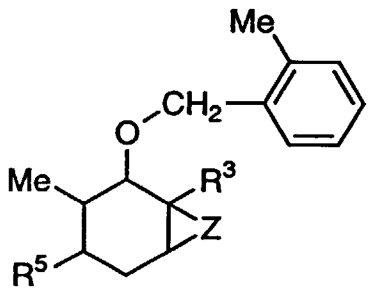
,
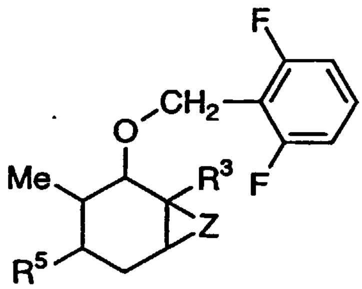
,
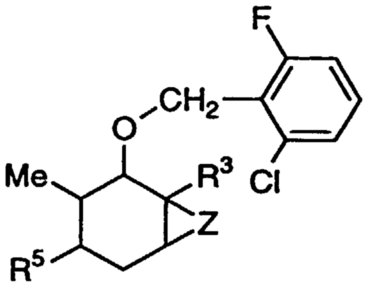
,
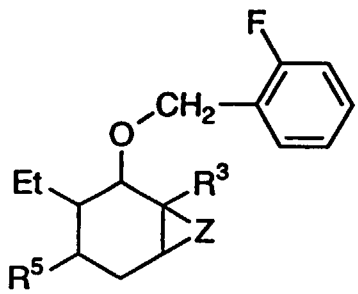
,
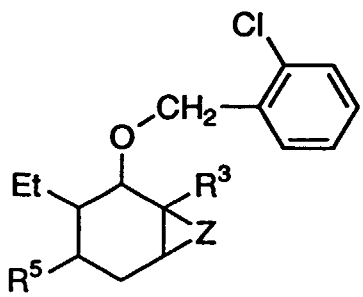
,

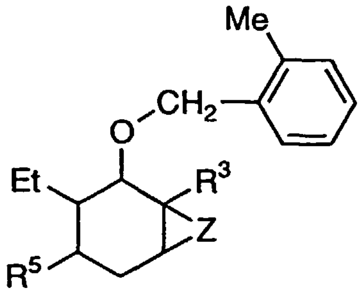

,

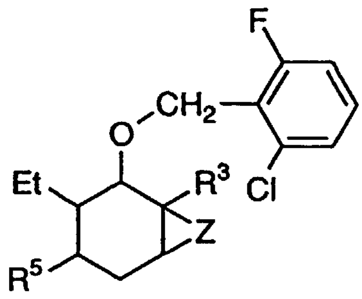

,

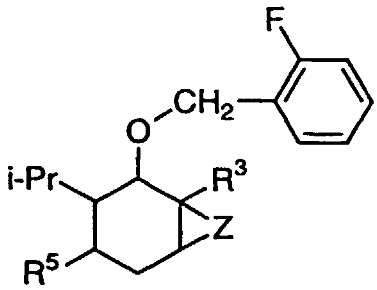

,

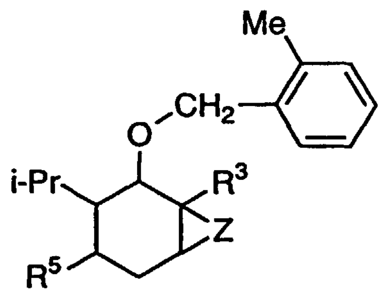

,

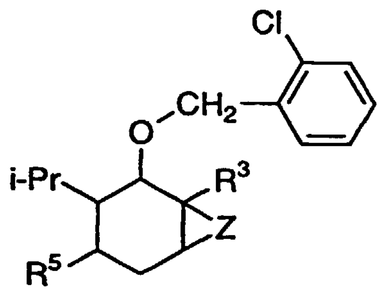

,

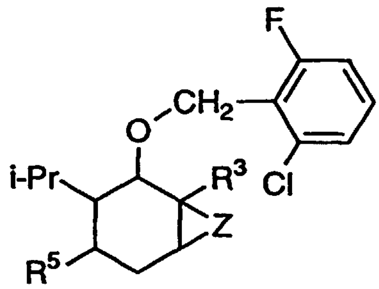

,

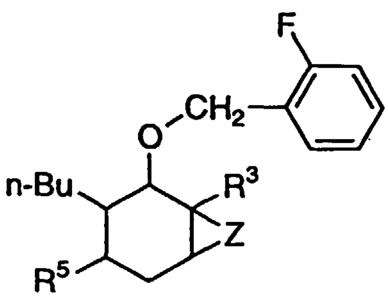

,

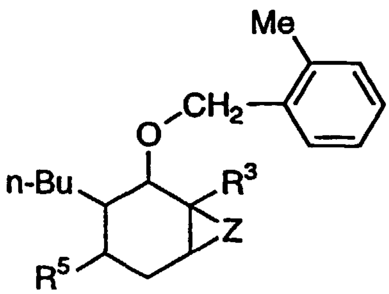

,

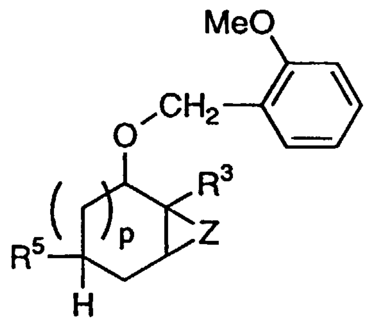 , 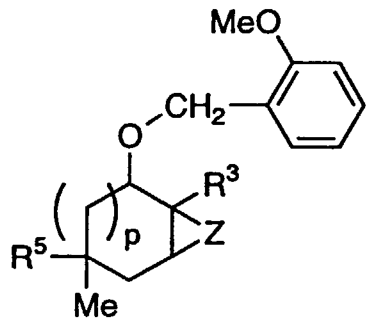 ,

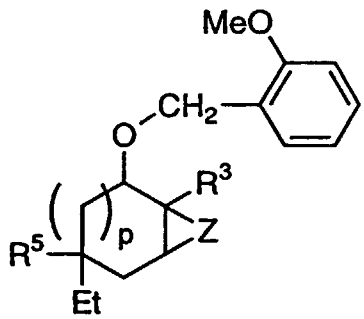 , 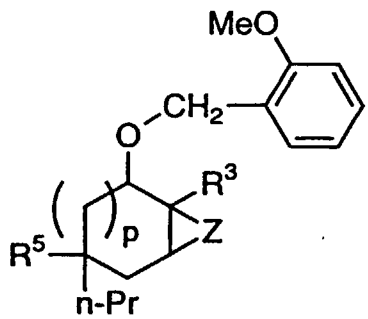 ,

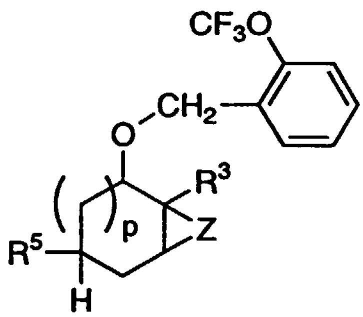 , 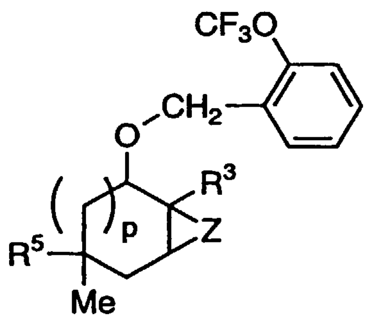 ,

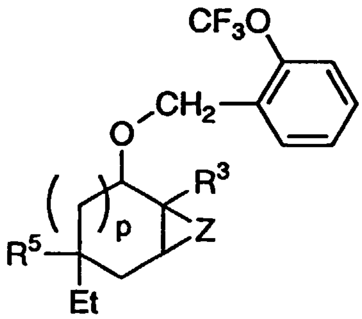 , 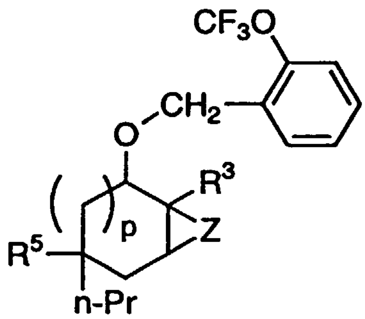 ,

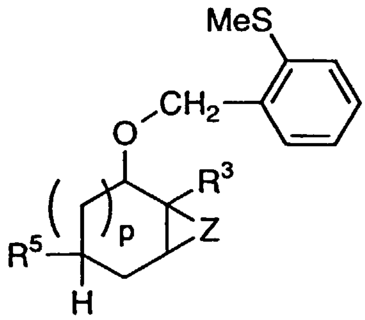 , 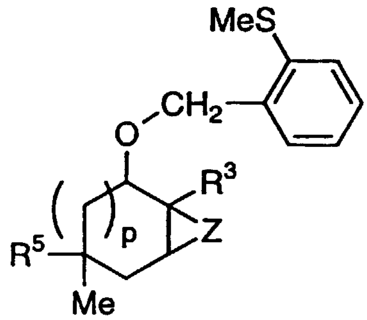 ,

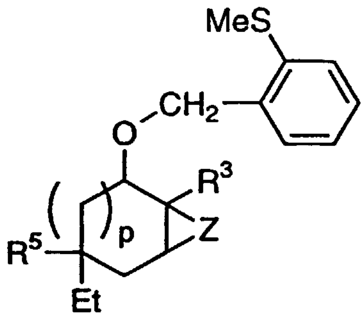 , 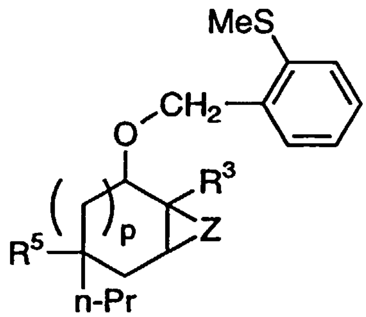 ,

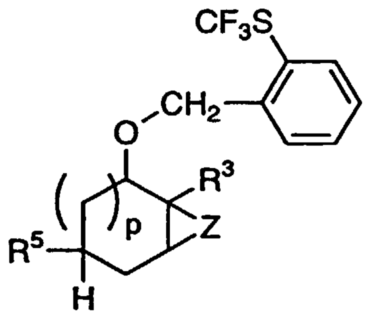 , 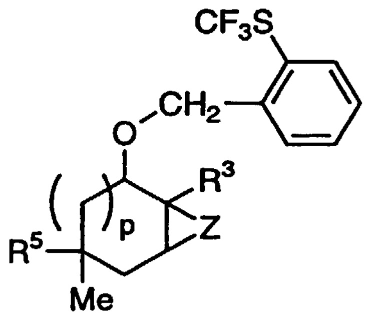 ,

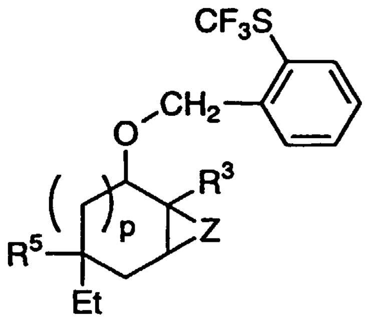 , 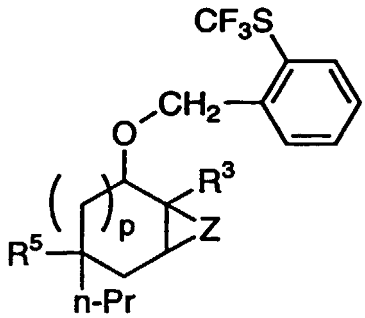 ,

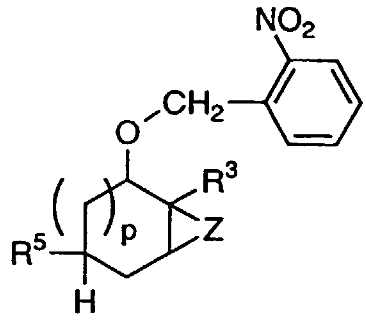

,

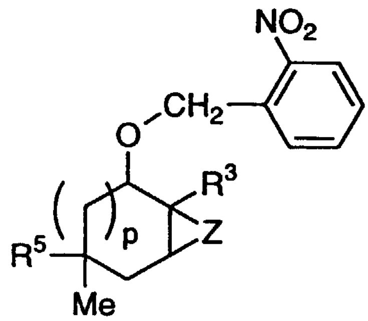

,

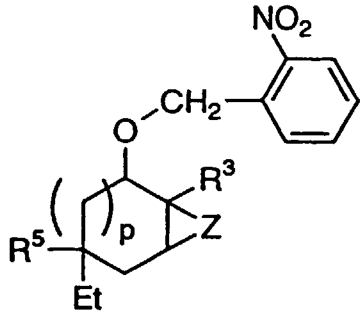

,

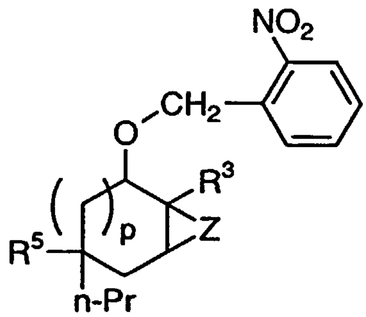

,

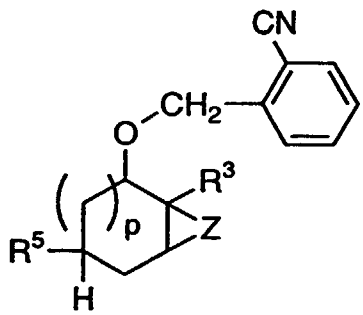

,

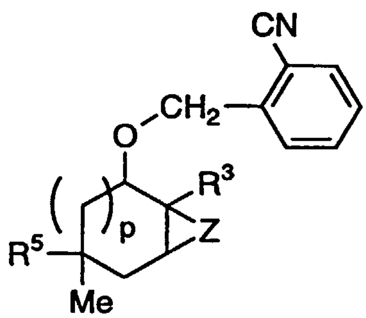

,

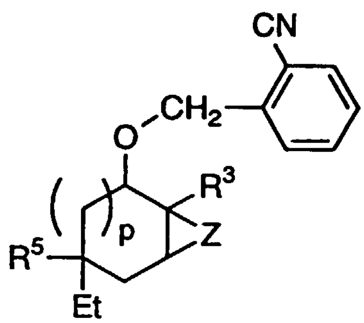

,

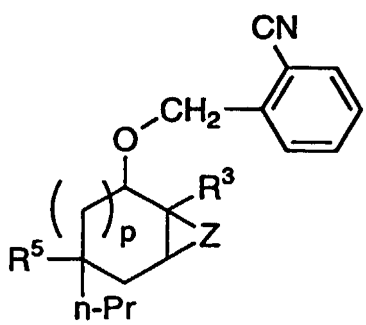

,

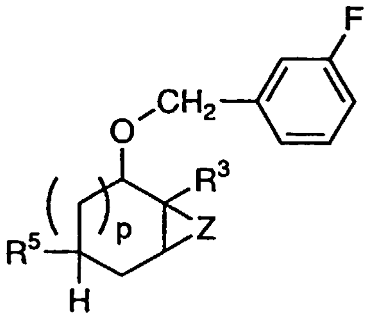
,
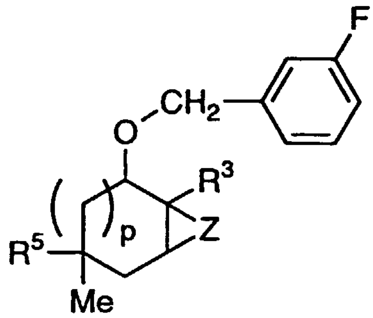
,

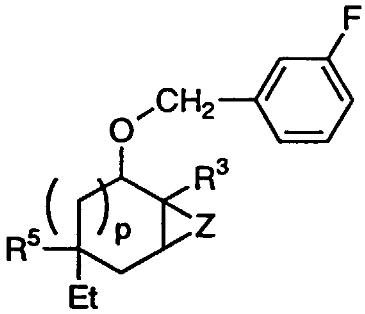
,
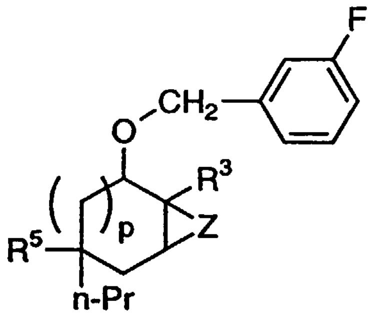
,

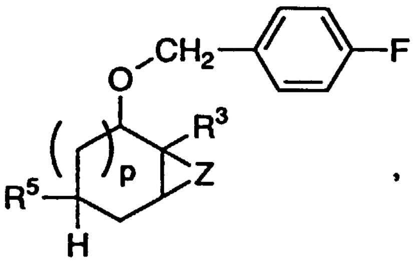
,
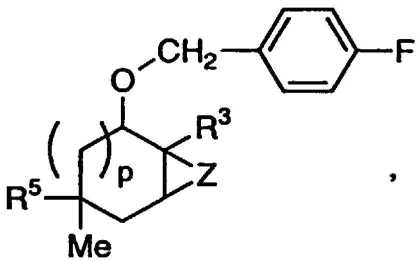
,

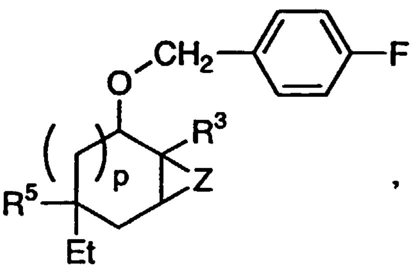
,
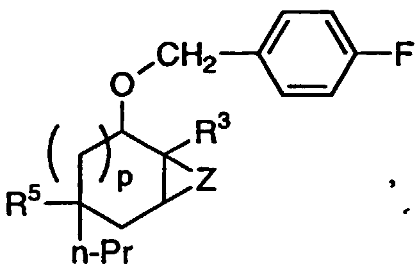
,

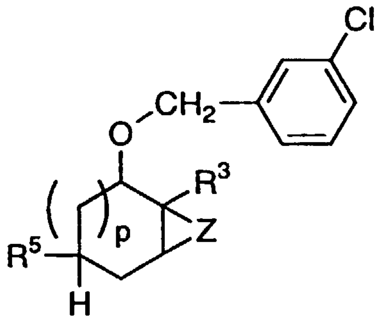 , 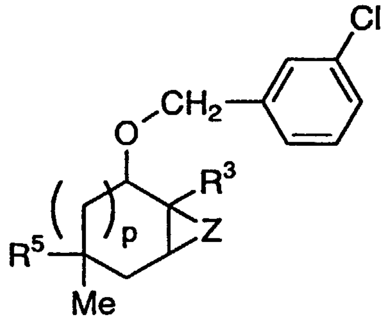 ,

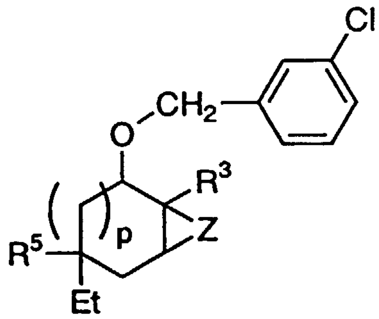 , 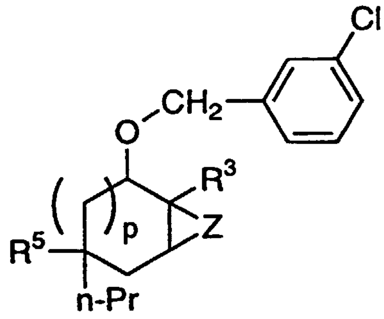 ,

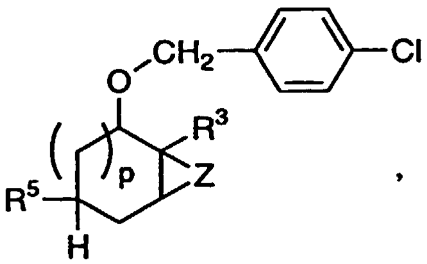 , 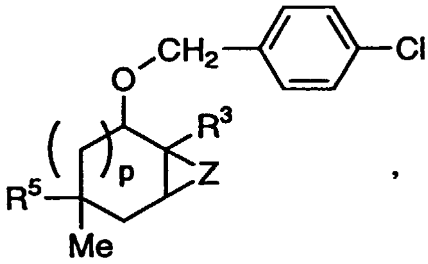 ,

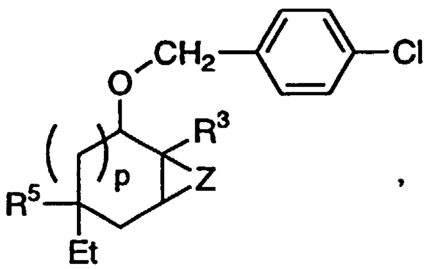 , 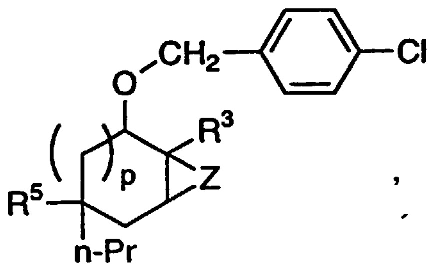 ,

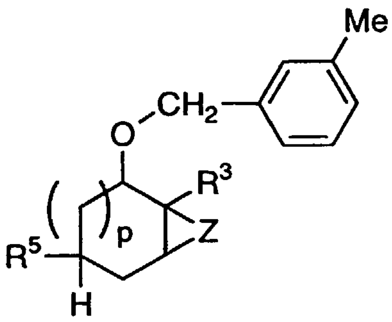 ,

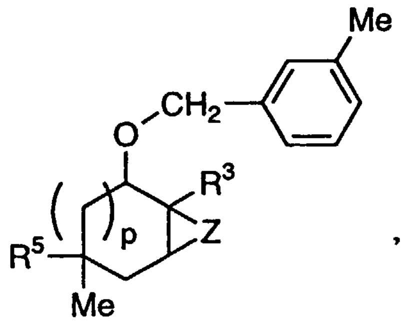 ,

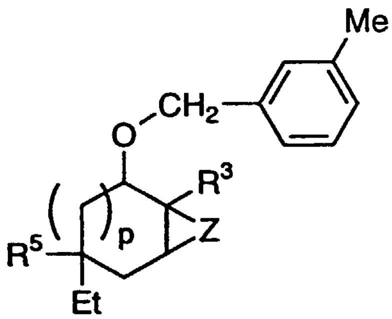 ,

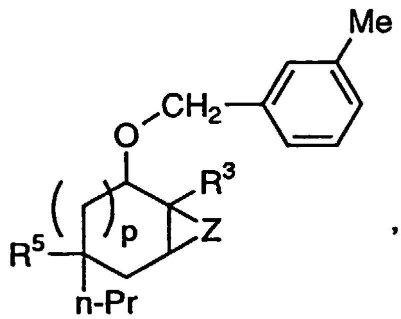 ,

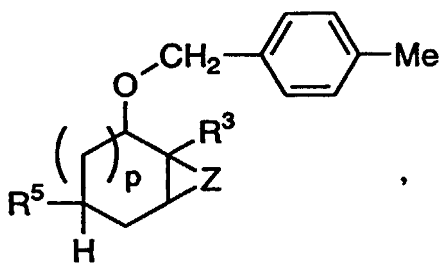 ,

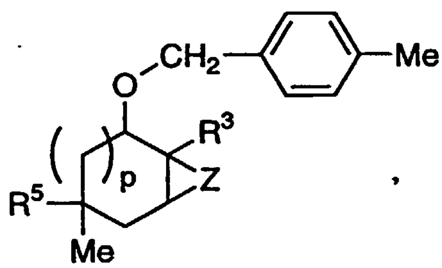 ,

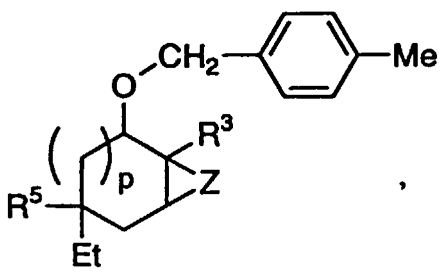 ,

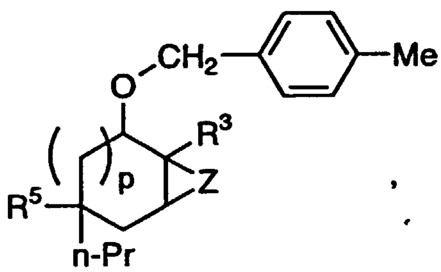 ,

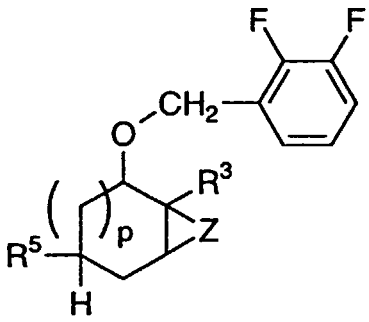 , 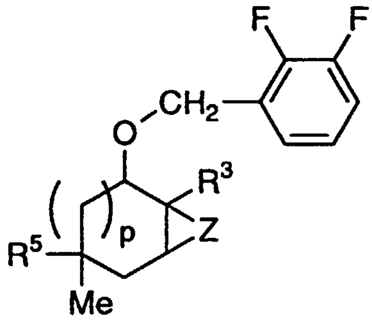 ,

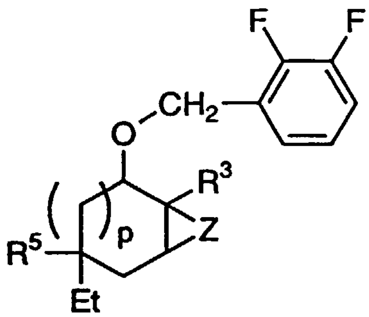 , 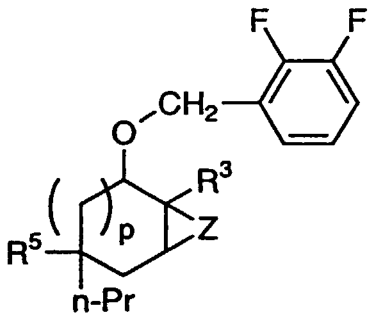 ,

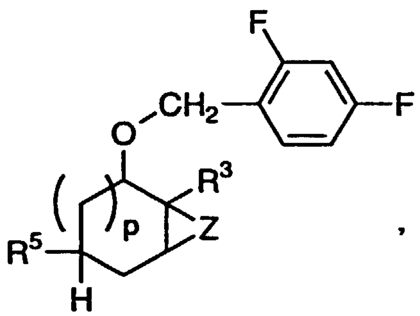 , 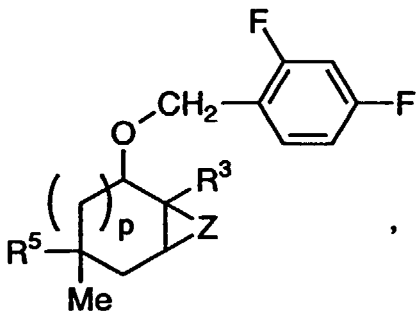 ,

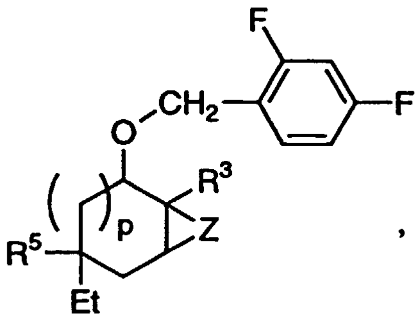 , 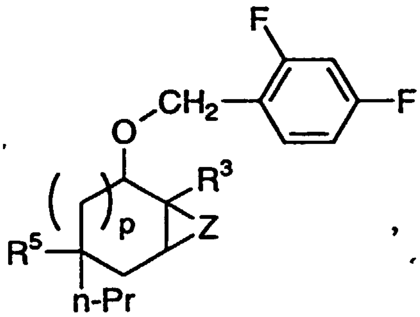 ,

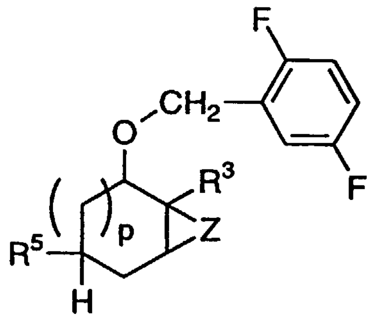
,
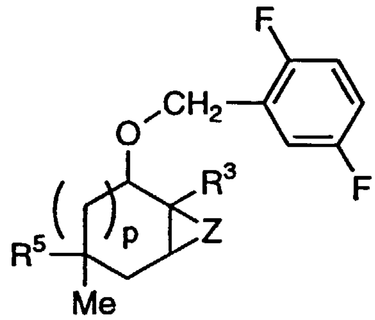
,
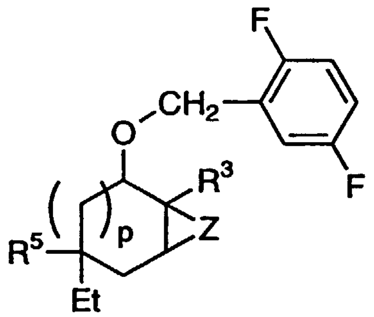
,
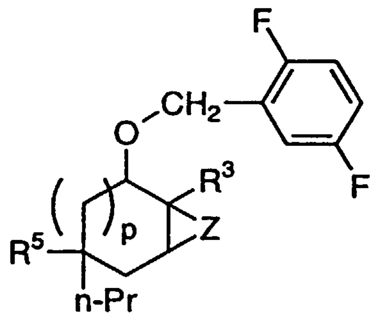
,
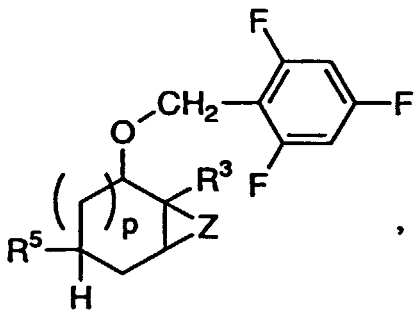
,
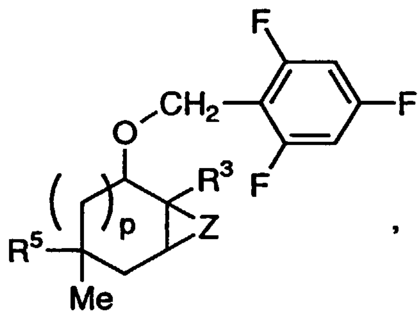
,
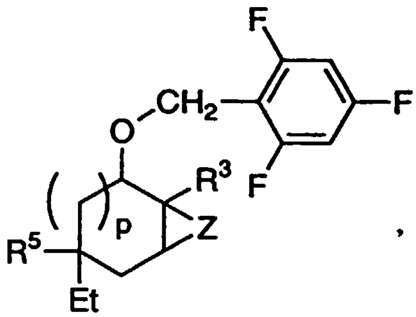
,
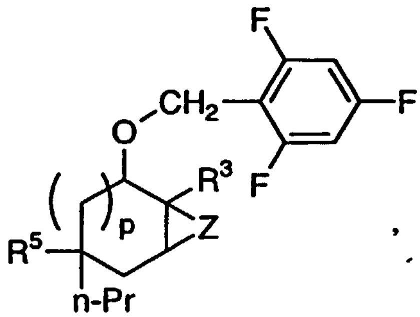
,

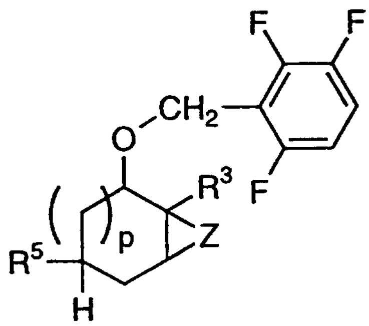 ,

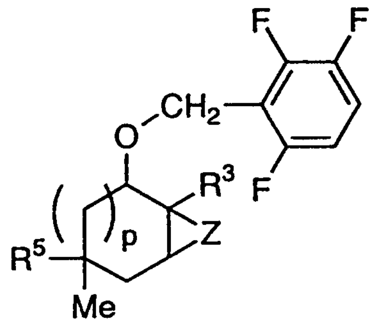 ,

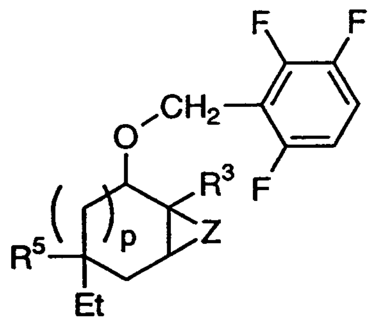 ,

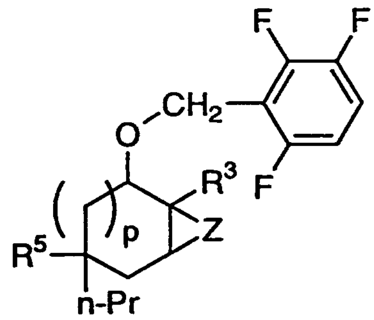 ,

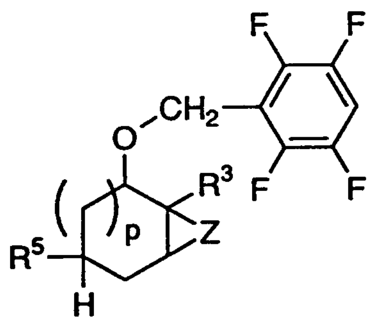 ,

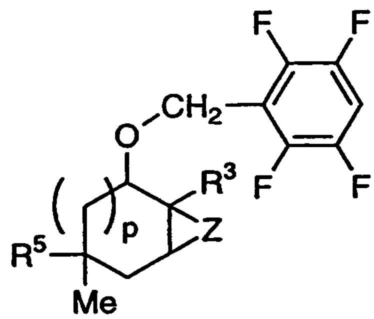 ,

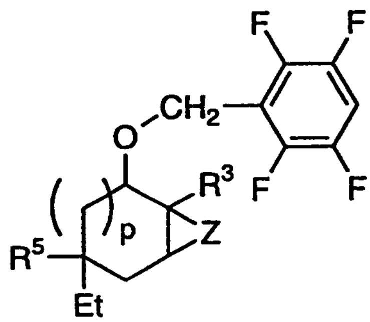 ,

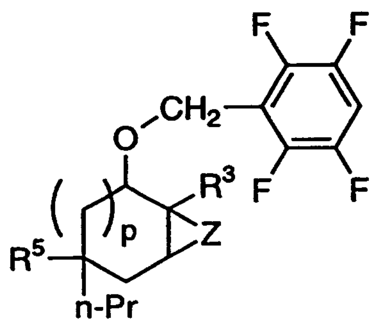 ,

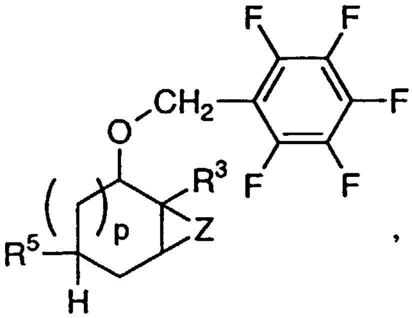

,

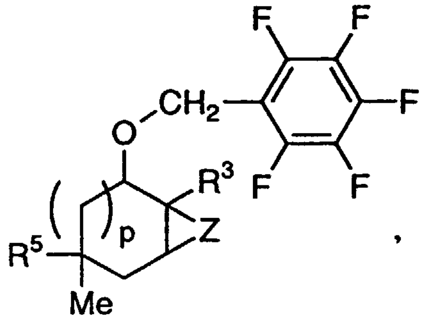

,

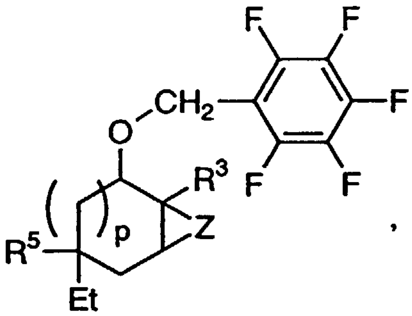

,

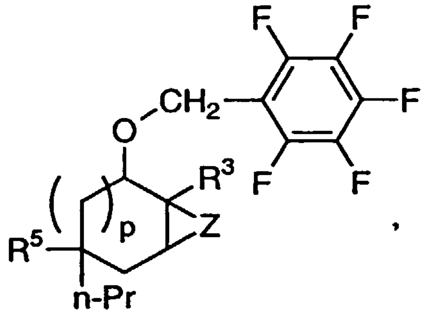

,

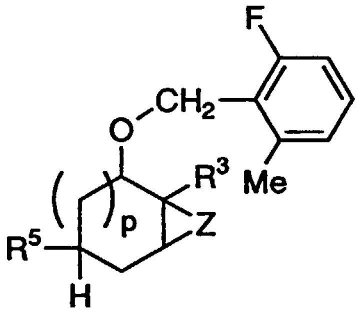

,

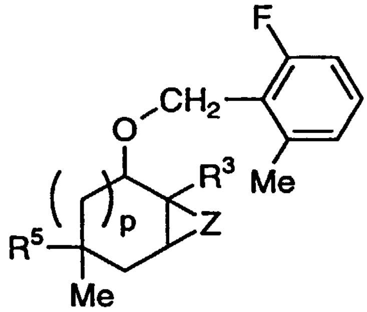

,

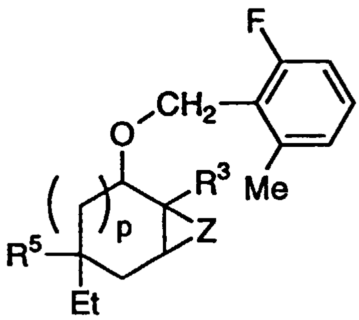

,

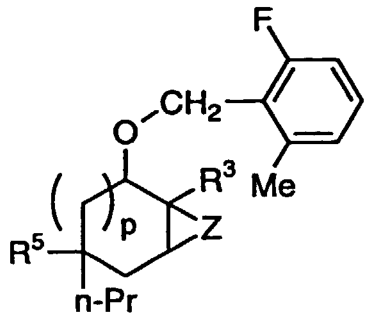

,

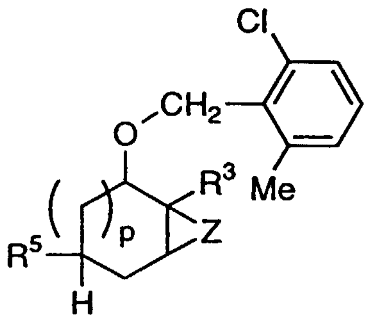

,

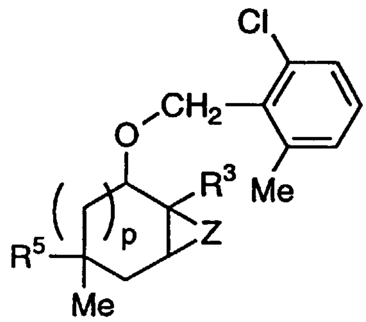

,

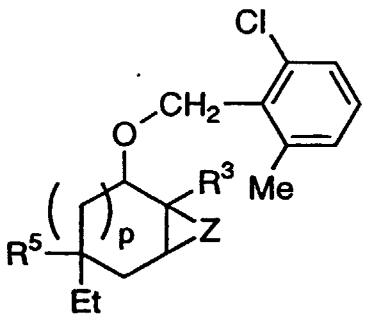

,

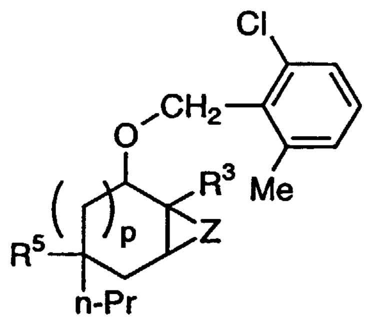

,

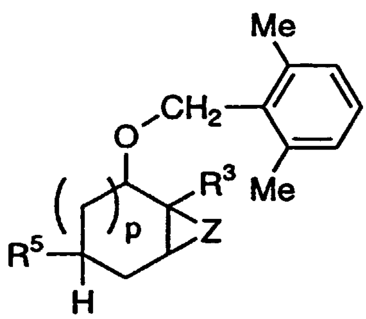

,

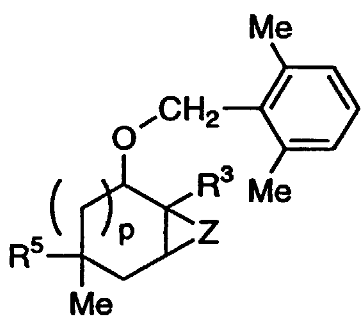

,

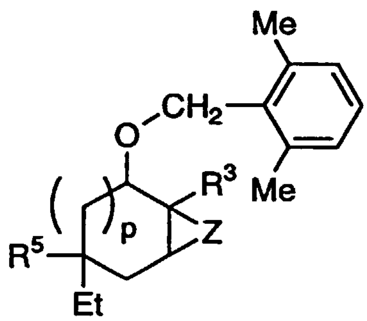

,

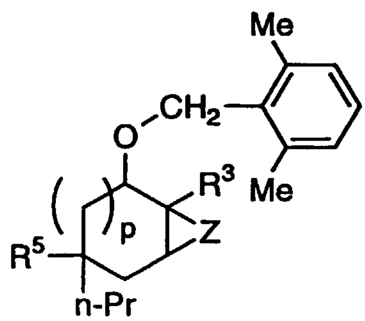

,

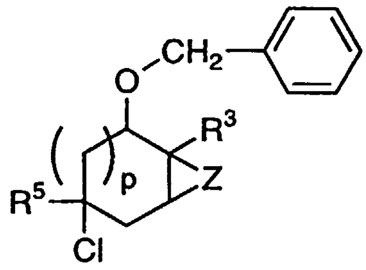
,
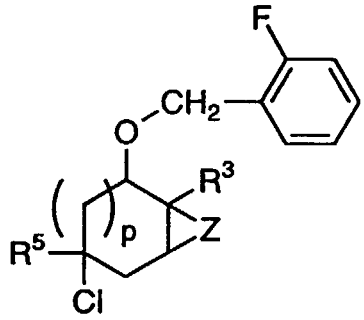
,

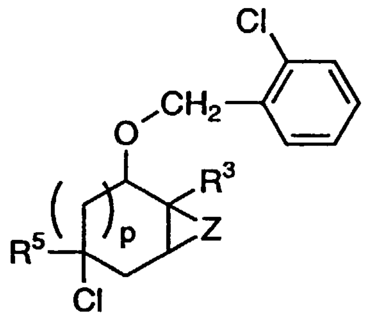
,
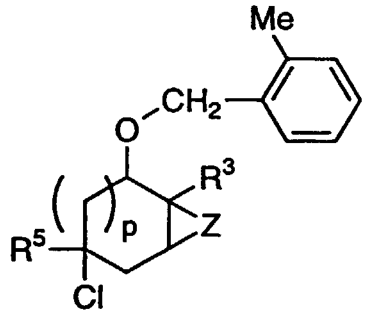
,

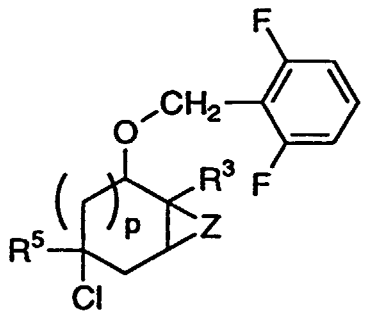
,
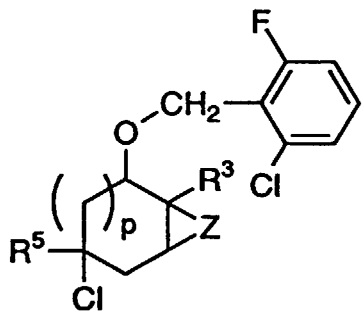
,

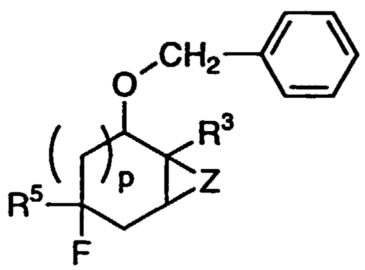
,
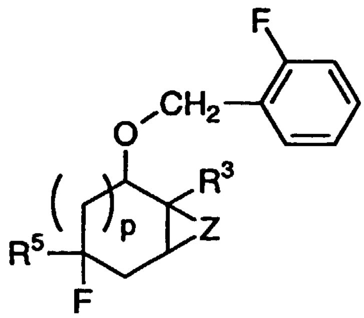
,

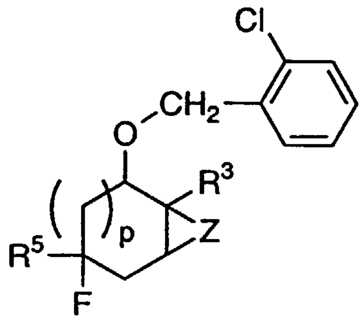 , 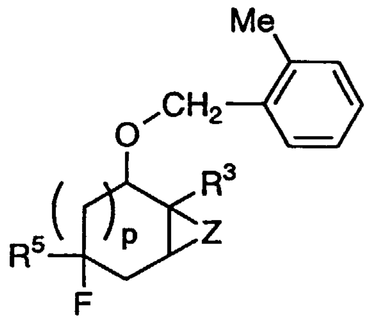 ,

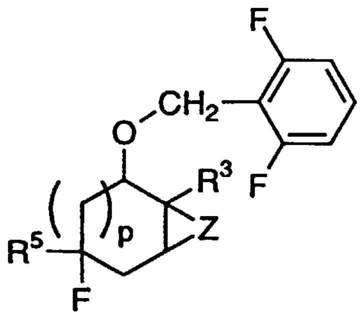 , 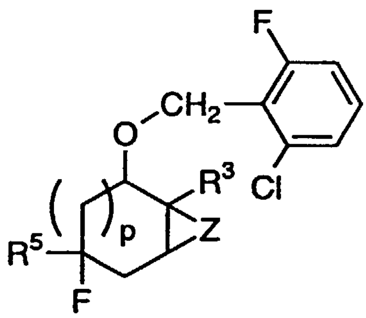 ,

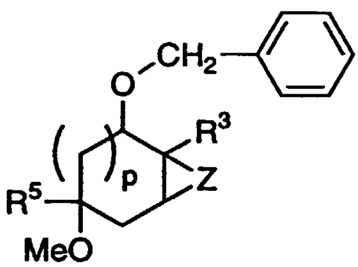 , 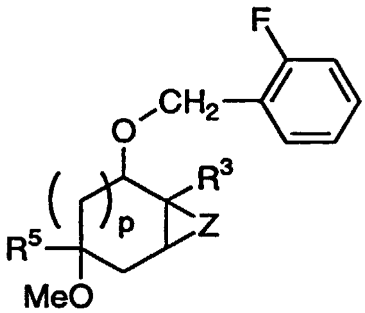 ,

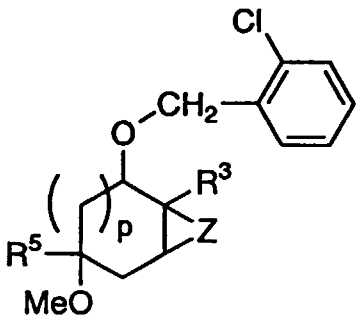 , 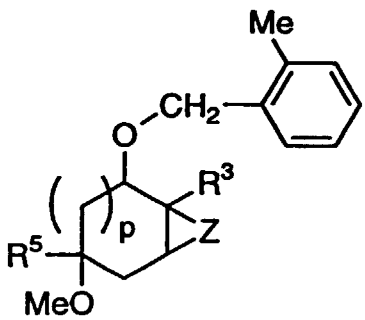 ,

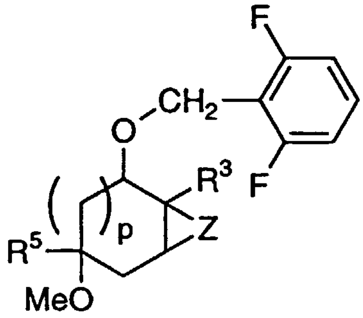 , 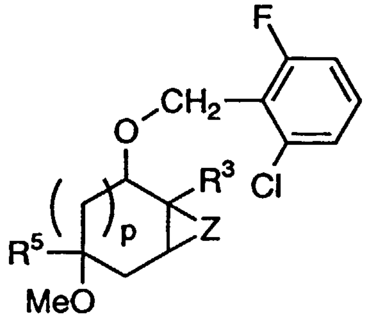 ,

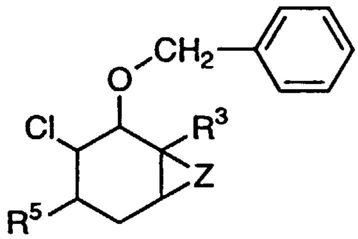 , 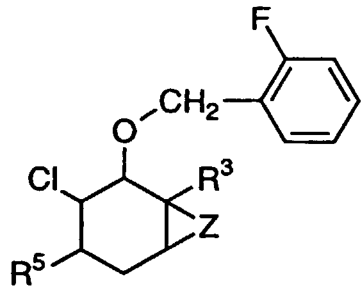 ,

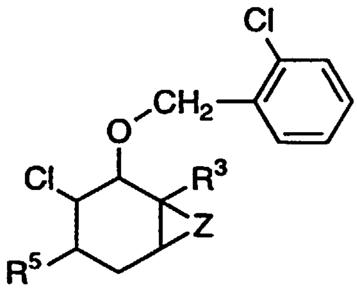 , 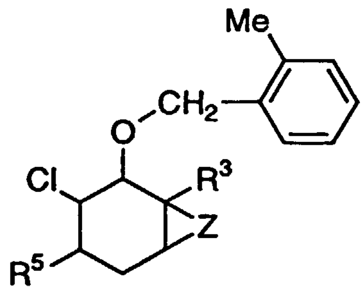 ,

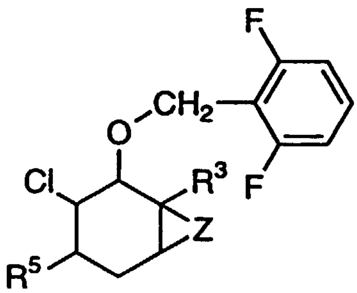 , 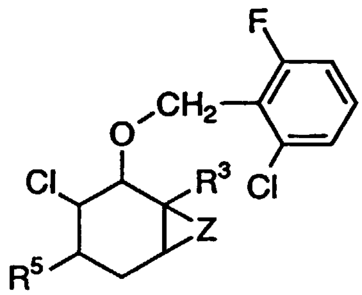 ,

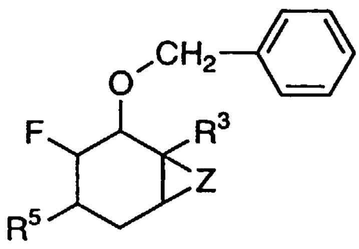 , 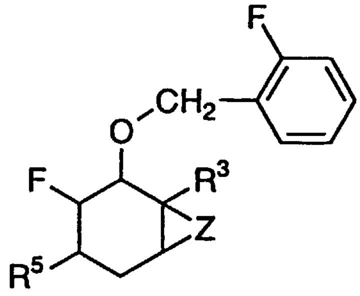 ,

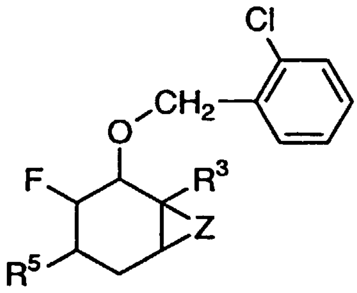 , 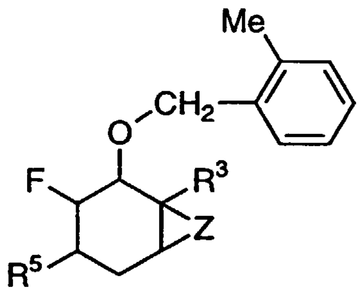 ,

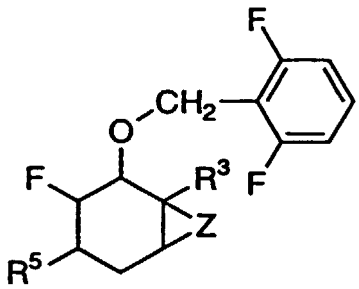 , 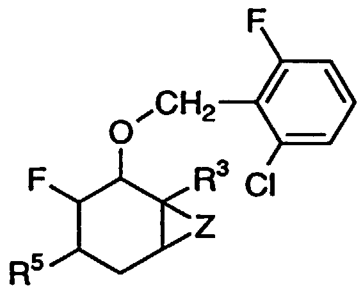 ,

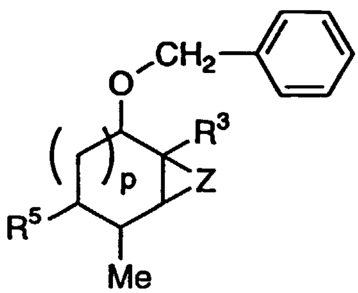 , 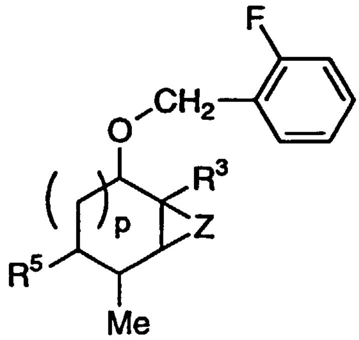 ,

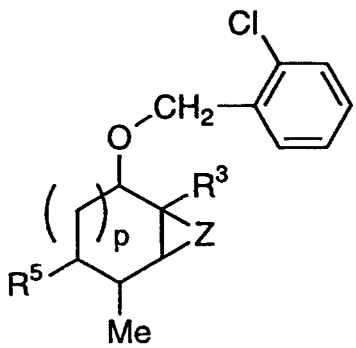

,

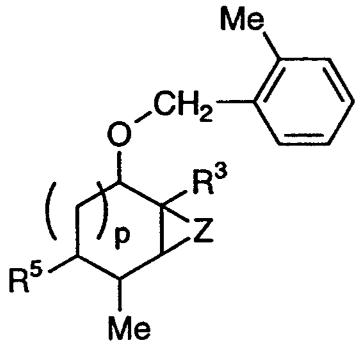

,

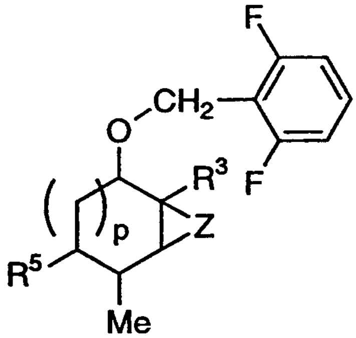

,

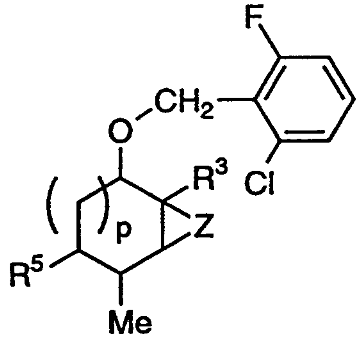

,

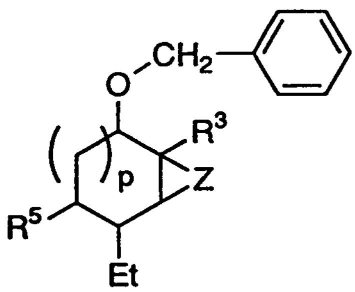

,

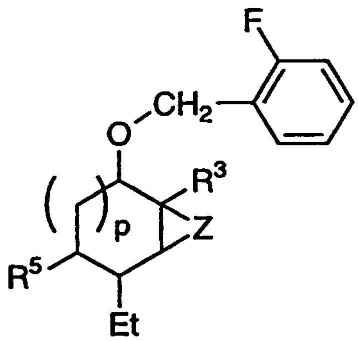

,

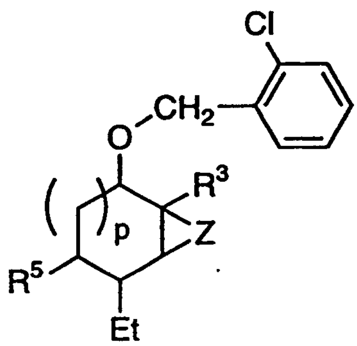

,

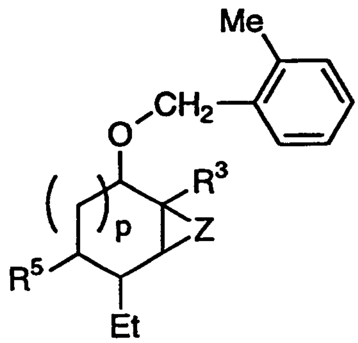

,

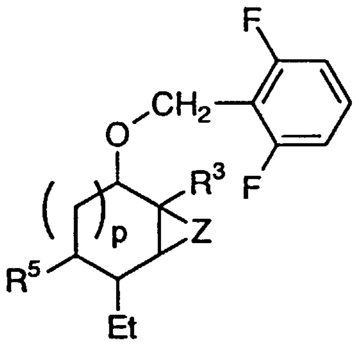

,

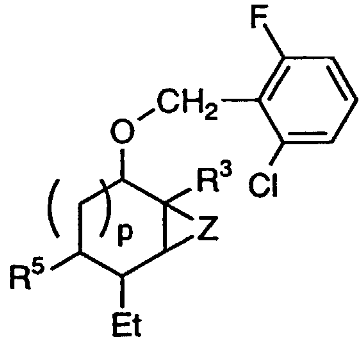

,

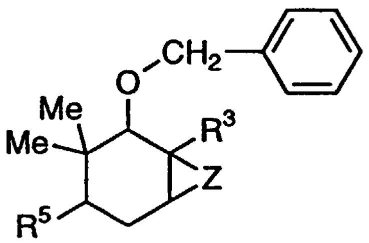

,

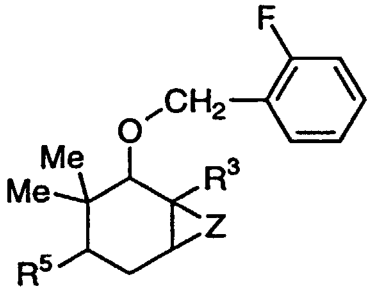

,

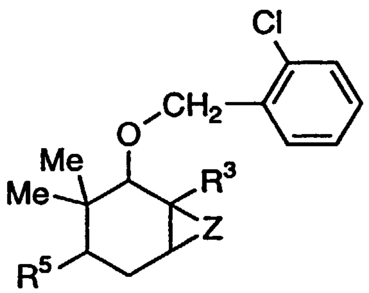

,

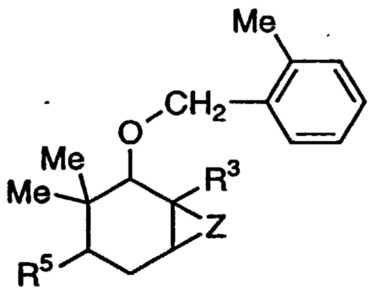

,

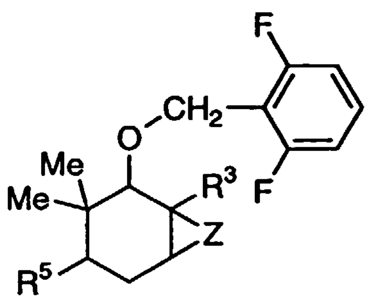

,

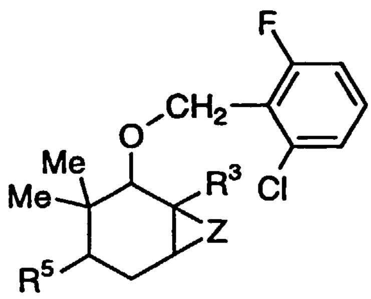

,

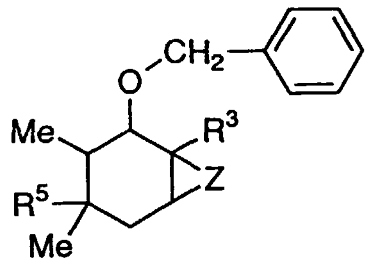

,

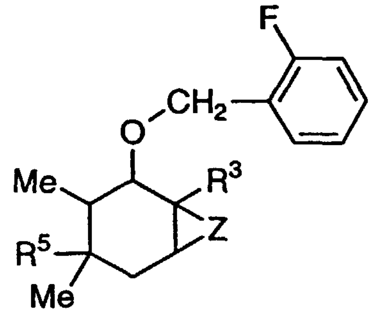

,

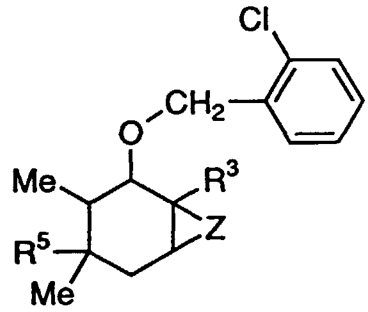

,

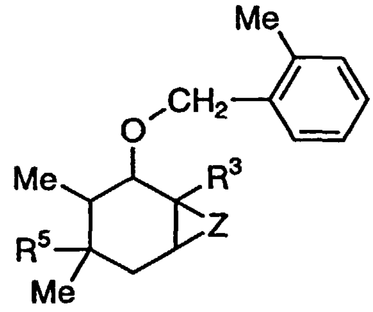

,

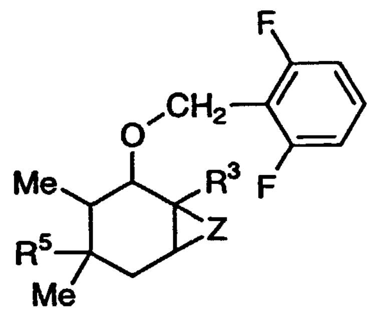

,

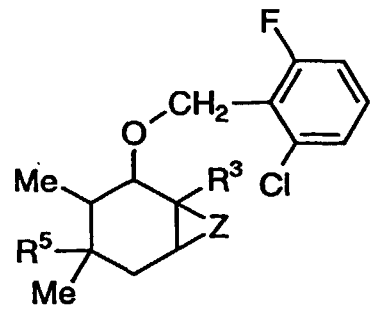

,

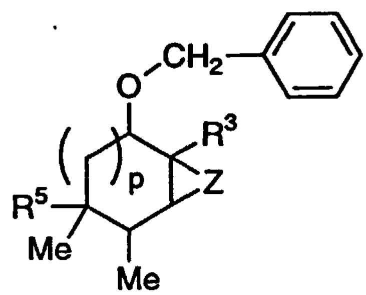

,

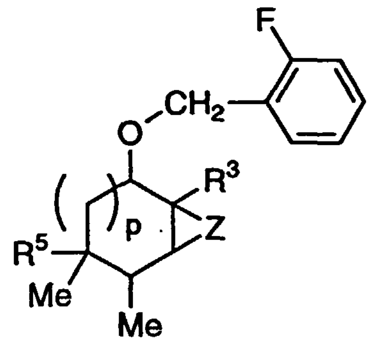

,

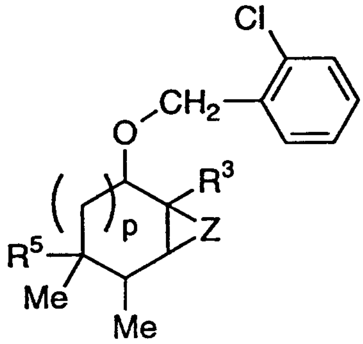 , 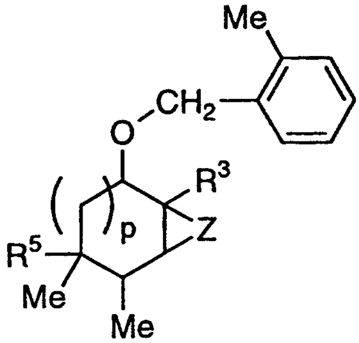 ,

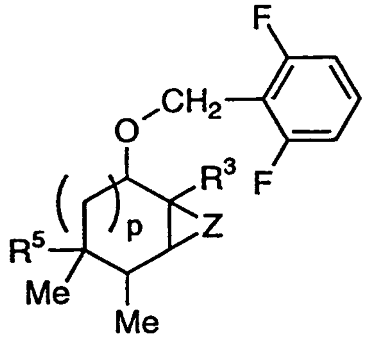 , 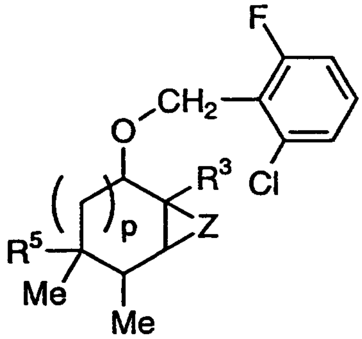 ,

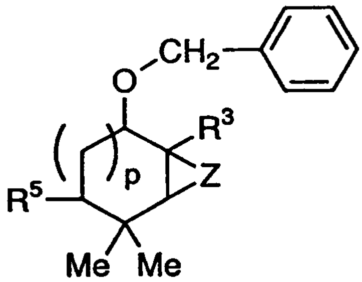 , 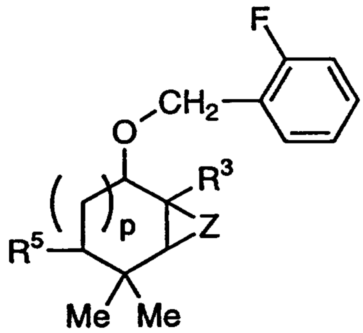 ,

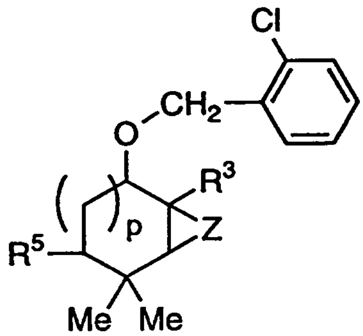 , 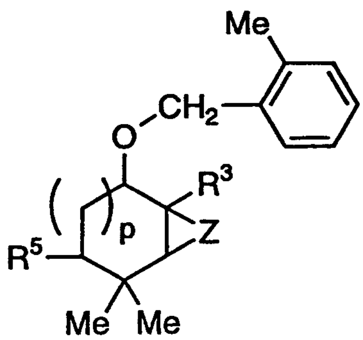 ,

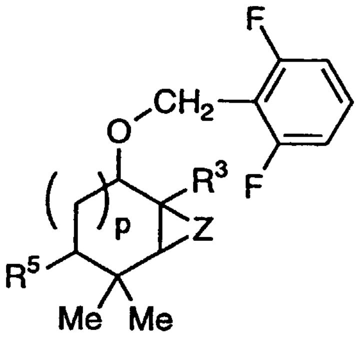

,

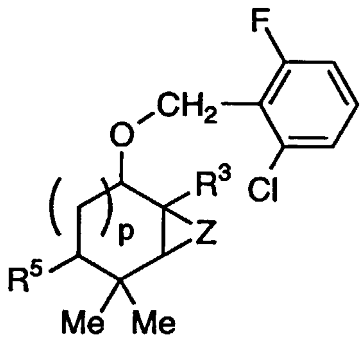

,

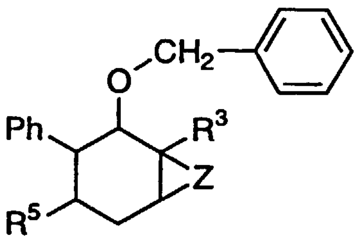

,

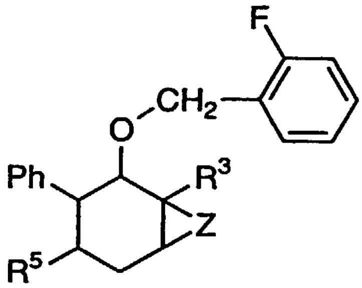

,

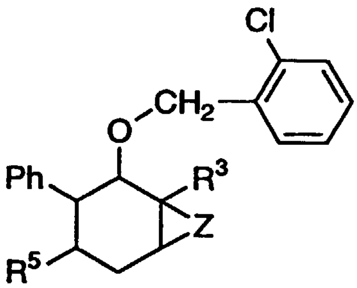

,

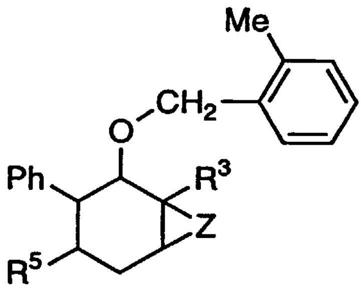

,

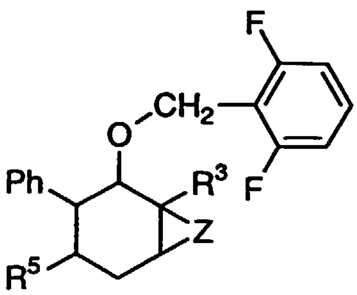

,

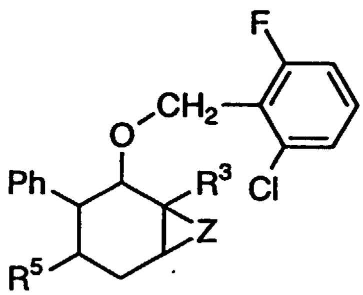

,

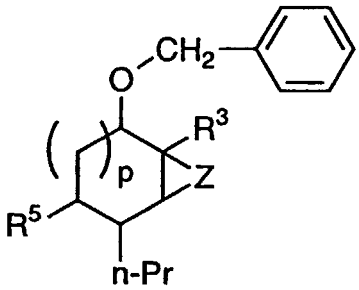 , 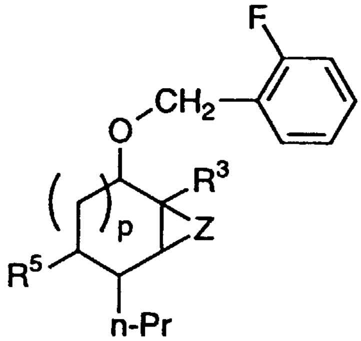 ,

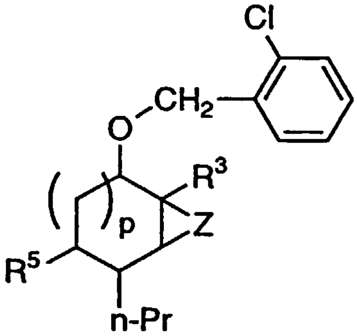 , 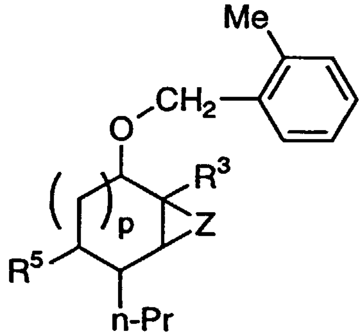 ,

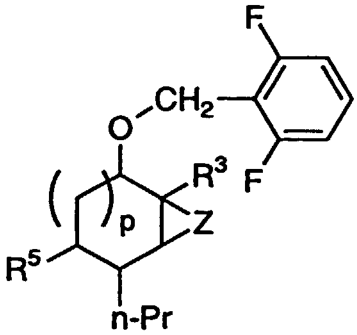 , 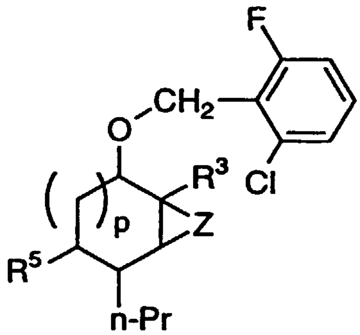 ,

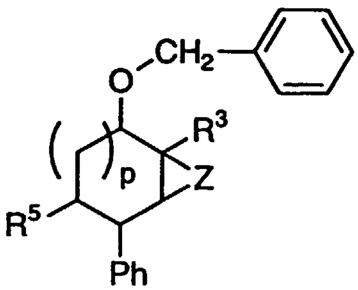 , 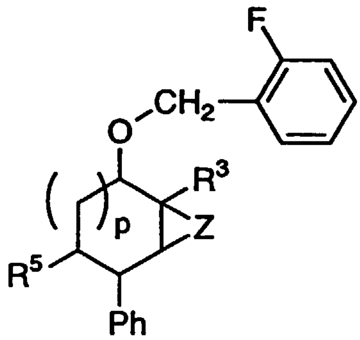 ,

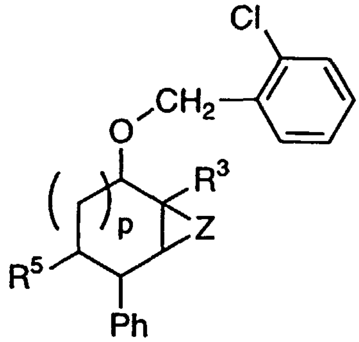 , 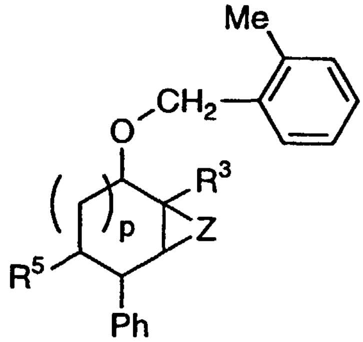 ,

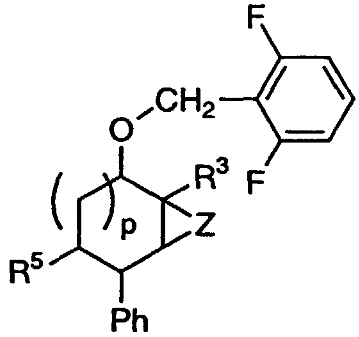 , 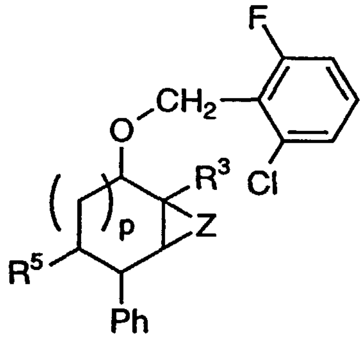 ,

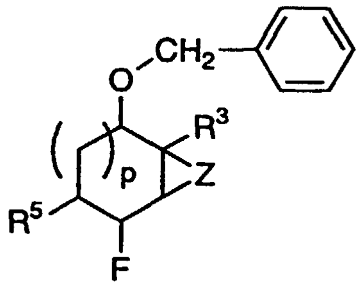 , 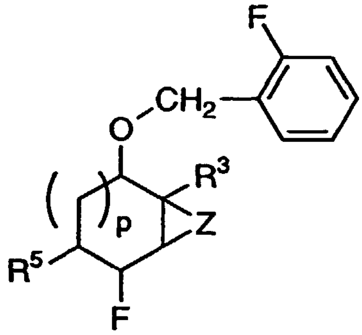 ,

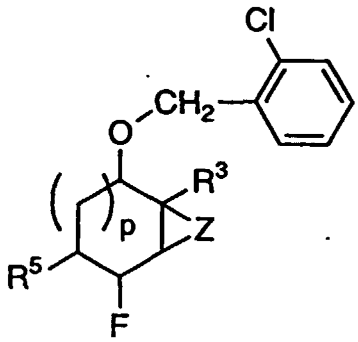 , 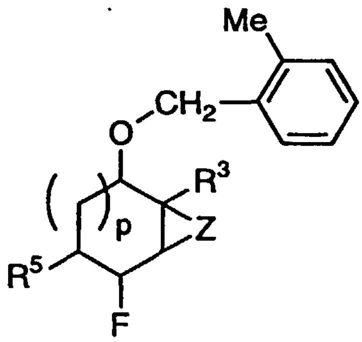 ,

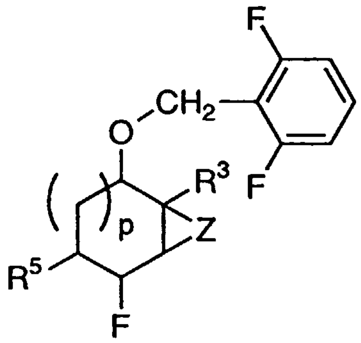

,

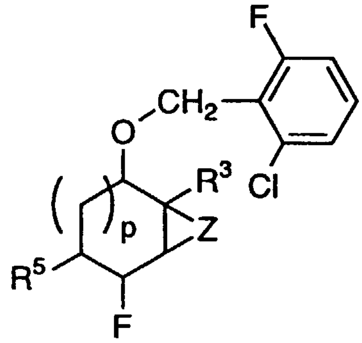

,

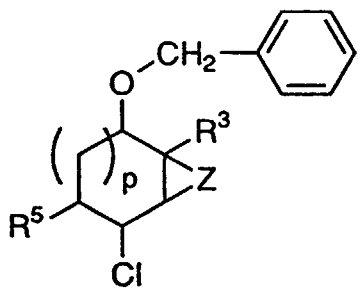

,

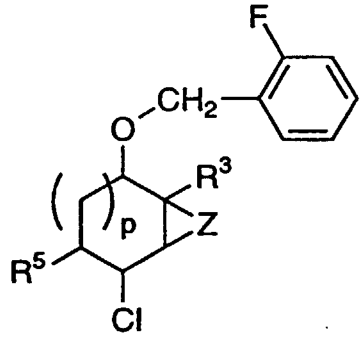

,

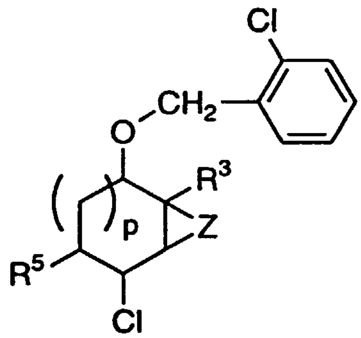

,

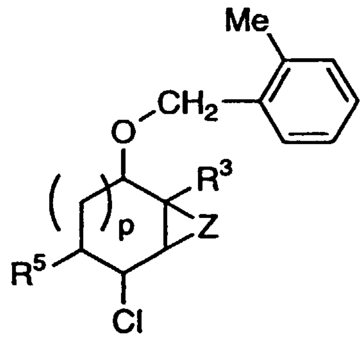

,

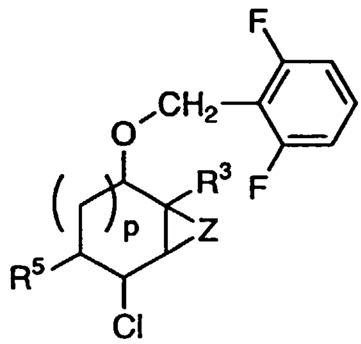

o r

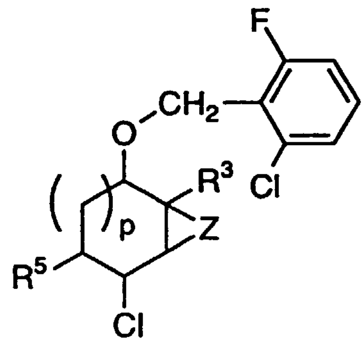

| $R^3$ | $R^5$ | p | Z |
|---|---|---|---|
| Me | Me | 1 | O |
| Me | Et | 1 | O |
| Me | n-Pr | 1 | O |
| Me | i-Pr | 1 | O |
| Me | n-Bu | 1 | O |
| Me | i-Bu | 1 | O |
| Me | s-Bu | 1 | O |
| Me | t-Bu | 1 | O |
| Me | n-Pen | 1 | O |
| Me | CHMe(n-Pr) | 1 | O |
| Me | $CH_2$s-Bu | 1 | O |
| Me | $(CH_2)_2$i-Pr | 1 | O |
| Me | $CH(Et)_2$ | 1 | O |
| Me | $C(Me)_2Et$ | 1 | O |
| Me | CHMe(i-Pr) | 1 | O |
| Me | $CH_2$t-Bu | 1 | O |
| Me | n-Hex | 1 | O |
| Me | CHMe(n-Bu) | 1 | O |
| Me | $CH_2$CHMe(n-Pr) | 1 | O |
| Me | $(CH_2)_2$s-Bu | 1 | O |
| Me | $(CH_2)_3$i-Pr | 1 | O |
| Me | $C(Me)_2$n-Pr | 1 | O |
| Me | CHMe(s-Bu) | 1 | O |
| Me | CHMe(i-Bu) | 1 | O |
| Me | $CH_2C(Me)_2Et$ | 1 | O |
| Me | $CH_2$CHMe(i-Pr) | 1 | O |
| Me | $(CH_2)_2$t-Bu | 1 | O |
| Me | CHEt(n-Pr) | 1 | O |
| Me | $CH_2CH(Et)_2$ | 1 | O |
| Me | $CMe(Et)_2$ | 1 | O |
| Me | CHEt(i-Pr) | 1 | O |
| Me | $C(Me)_2$i-Pr | 1 | O |
| Me | CHMe(t-Bu) | 1 | O |
| Me | $CH=CH_2$ | 1 | O |
| Me | $CMe=CH_2$ | 1 | O |
| Me | $CH_2CH=CH_2$ | 1 | O |
| Me | CH=CHMe | 1 | O |
| Me | $CEt=CH_2$ | 1 | O |
| Me | CMe=CHMe | 1 | O |

Table 3-a (continued)

| $R^3$ | $R^5$ | p | Z |
|---|---|---|---|
| Me | $CH=C(Me)_2$ | 1 | O |
| Me | $CHMeCH=CH_2$ | 1 | O |
| Me | CHMeCH=CHMe | 1 | O |
| Me | $CH_2CMe=C(Me)_2$ | 1 | O |
| Me | OMe | 1 | O |
| Me | Oi-Pr | 1 | O |
| Me | On-Hex | 1 | O |
| Me | $CH_2OMe$ | 1 | O |
| Me | $CH_2CH_2OMe$ | 1 | O |
| Me | CHMeOi-Pr | 1 | O |
| Me | $(CH_2)_3OEt$ | 1 | O |
| Me | $CHMeCH_2On$-Bu | 1 | O |
| Me | $C(Me)_2OMe$ | 1 | O |
| Me | $(CH_2)_4OEt$ | 1 | O |
| Me | $(CH_2)_5On$-Pr | 1 | O |
| Me | $(CH_2)_6OMe$ | 1 | O |
| Me | $CH_2Cl$ | 1 | O |
| Me | $CF_3$ | 1 | O |
| Me | $CH_2CH_2Br$ | 1 | O |
| Me | CHMeCl | 1 | O |
| Me | $(CH_2)_3I$ | 1 | O |
| Me | $CMeClCH_2Cl$ | 1 | O |
| Me | $CHMeCH_2Cl$ | 1 | O |
| Me | $CMeBrCH_2Br$ | 1 | O |
| Me | $(CH_2)_4Cl$ | 1 | O |
| Me | $(CH_2)_5F$ | 1 | O |
| Me | $(CH_2)_6Cl$ | 1 | O |
| Me | Ph | 1 | O |
| Me | 2-OMe-Ph | 1 | O |
| Me | 4-F-Ph | 1 | O |
| Me | $3-CF_3$-Ph | 1 | O |
| Me | 2-Me-4-Cl-Ph | 1 | O |
| Me | 4-On-Bu-Ph | 1 | O |
| Me | 4-CN-Ph | 1 | O |
| Me | $4-NO_2$-Ph | 1 | O |
| Me | $2-CO_2H$-Ph | 1 | O |
| Me | $4-CO_2Me$-Ph | 1 | O |
| Me | $4-CO_2t$-Bu-Ph | 1 | O |

Table 3-a (continued)

| $R^3$ | $R^5$ | p | Z |
|---|---|---|---|
| Me | 4-$CF_2CF_3$-Ph | 1 | O |
| Me | 2,3,4,5,6-$F_5$-Ph | 1 | O |
| Me | $CH_2Ph$ | 1 | O |
| Me | $CH_2$-4-Br-Ph | 1 | O |
| Me | $CH_2$-4-t-Br-Ph | 1 | O |
| Me | $(CH_2)_2Ph$ | 1 | O |
| Me | $(CH_2)_2$-2-Me-Ph | 1 | O |
| Me | CHMe-Ph | 1 | O |
| Me | CHMe-4-OMe-Ph | 1 | O |
| Me | $(CH_2)_3Ph$ | 1 | O |
| Me | $(CH_2)_3$-3-$CF_3$-Ph | 1 | O |
| Me | $(CH_2)_3$ -2,4,5-$Cl_3$-Ph | 1 | O |
| Me | $CH_2OH$ | 1 | O |
| Me | CH(OH)Me | 1 | O |
| Me | CH(OH)Et | 1 | O |
| Et | i-Pr | 1 | O |
| Et | $CMe=CH_2$ | 1 | O |
| Et | $CMeClCH_2Cl$ | 1 | O |
| Et | $CHMeCH_2Cl$ | 1 | O |
| Et | Ph | 1 | O |
| Et | $CH_2Ph$ | 1 | O |
| Et | CHMe-Ph | 1 | O |
| n-Pr | i-Pr | 1 | O |
| n-Pr | $CMe=CH_2$ | 1 | O |
| n-Pr | $CMeClCH_2Cl$ | 1 | O |
| n-Pr | $CHMeCH_2Cl$ | 1 | O |
| n-Pr | Ph | 1 | O |
| n-Pr | $CH_2Ph$ | 1 | O |
| n-Pr | CHMe-Ph | 1 | O |
| i-Pr | i-Pr | 1 | O |
| i-Pr | $CMe=CH_2$ | 1 | O |
| i-Pr | $CMeClCH_2Cl$ | 1 | O |
| i-Pr | $CHMeCH_2Cl$ | 1 | O |
| i-Pr | Ph | 1 | O |
| i-Pr | $CH_2Ph$ | 1 | O |
| i-Pr | CHMe-Ph | 1 | O |
| n-Bu | i-Pr | 1 | O |
| n-Bu | $CMe=CH_2$ | 1 | O |

Table 3-a (continued)

| $R^3$ | $R^5$ | p | Z |
|---|---|---|---|
| n-Bu | $CMeClCH_2Cl$ | 1 | O |
| n-Bu | $CHMeCH_2Cl$ | 1 | O |
| n-Bu | Ph | 1 | O |
| n-Bu | $CH_2Ph$ | 1 | O |
| n-Bu | CHMe-Ph | 1 | O |
| i-Bu | i-Pr | 1 | O |
| i-Bu | $CMe=CH_2$ | 1 | O |
| i-Bu | $CMeClCH_2Cl$ | 1 | O |
| i-Bu | $CHMeCH_2Cl$ | 1 | O |
| i-Bu | Ph | 1 | O |
| i-Bu | $CH_2Ph$ | 1 | O |
| i-Bu | CHMe-Ph | 1 | O |
| n-Pen | i-Pr | 1 | O |
| n-Pen | $CMe=CH_2$ | 1 | O |
| n-Pen | $CMeClCH_2Cl$ | 1 | O |
| n-Pen | $CHMeCH_2Cl$ | 1 | O |
| n-Pen | Ph | 1 | O |
| n-Pen | $CH_2Ph$ | 1 | O |
| n-Pen | CHMe-Ph | 1 | O |
| n-Hex | i-Pr | 1 | O |
| n-Hex | $CMe=CH_2$ | 1 | O |
| n-Hex | $CMeClCH_2Cl$ | 1 | O |
| n-Hex | $CHMeCH_2Cl$ | 1 | O |
| n-Hex | Ph | 1 | O |
| n-Hex | $CH_2Ph$ | 1 | O |
| n-Hex | CHMe-Ph | 1 | O |
| $CH_2CH=CH_2$ | i-Pr | 1 | O |
| $CH_2CH=CH_2$ | $CMe=CH_2$ | 1 | O |
| $CH_2CH=CH_2$ | $CMeClCH_2Cl$ | 1 | O |
| $CH_2CH=CH_2$ | $CHMeCH_2Cl$ | 1 | O |
| $CH_2CH=CH_2$ | Ph | 1 | O |
| $CH_2CH=CH_2$ | $CH_2Ph$ | 1 | O |
| $CH_2CH=CH_2$ | CHMe-Ph | 1 | O |
| $CH_2CH=CHMe$ | i-Pr | 1 | O |
| $CH_2CH=CHMe$ | $CMe=CH_2$ | 1 | O |
| $CH_2CH=CHMe$ | $CMeClCH_2Cl$ | 1 | O |
| $CH_2CH=CHMe$ | $CHMeCH_2Cl$ | 1 | O |
| $CH_2CH=CHMe$ | Ph | 1 | O |

Table 3-a (continued)

| $R^3$ | $R^5$ | p | Z |
|---|---|---|---|
| $CH_2CH=CHMe$ | $CH_2Ph$ | 1 | O |
| $CH_2CH=CHMe$ | CHMe-Ph | 1 | O |
| $CH_2CH=CHi$-Pr | i-Pr | 1 | O |
| $CH_2CH=CHi$-Pr | $CMe=CH_2$ | 1 | O |
| $CH_2CH=CHi$-Pr | $CMeClCH_2Cl$ | 1 | O |
| $CH_2CH=CHi$-Pr | $CHMeCH_2Cl$ | 1 | O |
| $CH_2CH=CHi$-Pr | Ph | 1 | O |
| $CH_2CH=CHi$-Pr | $CH_2Ph$ | 1 | O |
| $CH_2CH=CHi$-Pr | CHMe-Ph | 1 | O |
| $CH_2Cl$ | i-Pr | 1 | O |
| $CH_2Cl$ | $CMe=CH_2$ | 1 | O |
| $CH_2Cl$ | $CMeClCH_2Cl$ | 1 | O |
| $CH_2Cl$ | $CHMeCH_2Cl$ | 1 | O |
| $CH_2Cl$ | Ph | 1 | O |
| $CH_2Cl$ | $CH_2Ph$ | 1 | O |
| $CH_2Cl$ | CHMe-Ph | 1 | O |
| $(CH_2)_2Cl$ | i-Pr | 1 | O |
| $(CH_2)_2Cl$ | $CMe=CH_2$ | 1 | O |
| $(CH_2)_2Cl$ | $CMeClCH_2Cl$ | 1 | O |
| $(CH_2)_2Cl$ | $CHMeCH_2Cl$ | 1 | O |
| $(CH_2)_2Cl$ | Ph | 1 | O |
| $(CH_2)_2Cl$ | $CH_2Ph$ | 1 | O |
| $(CH_2)_2Cl$ | CHMe-Ph | 1 | O |
| $CH_2CHBrCH_2Br$ | i-Pr | 1 | O |
| $CH_2CHBrCH_2Br$ | $CMe=CH_2$ | 1 | O |
| $CH_2CHBrCH_2Br$ | $CMeClCH_2Cl$ | 1 | O |
| $CH_2CHBrCH_2Br$ | $CHMeCH_2Cl$ | 1 | O |
| $CH_2CHBrCH_2Br$ | Ph | 1 | O |
| $CH_2CHBrCH_2Br$ | $CH_2Ph$ | 1 | O |
| $CH_2CHBrCH_2Br$ | CHMe-Ph | 1 | O |
| $(CH_2)_3F$ | i-Pr | 1 | O |
| $(CH_2)_3F$ | $CMe=CH_2$ | 1 | O |
| $(CH_2)_3F$ | $CMeClCH_2Cl$ | 1 | O |
| $(CH_2)_3F$ | $CHMeCH_2Cl$ | 1 | O |
| $(CH_2)_3F$ | Ph | 1 | O |
| $(CH_2)_3F$ | $CH_2Ph$ | 1 | O |
| $(CH_2)_3F$ | CHMe-Ph | 1 | O |
| $(CH_2)_4Cl$ | i-Pr | 1 | O |

Table 3-a (continued)

| $R^3$ | $R^5$ | p | Z |
|---|---|---|---|
| $(CH_2)_4Cl$ | $CMe=CH_2$ | 1 | O |
| $(CH_2)_4Cl$ | $CMeClCH_2Cl$ | 1 | O |
| $(CH_2)_4Cl$ | $CHMeCH_2Cl$ | 1 | O |
| $(CH_2)_4Cl$ | Ph | 1 | O |
| $(CH_2)_4Cl$ | $CH_2Ph$ | 1 | O |
| $(CH_2)_4Cl$ | CHMe-Ph | 1 | O |
| $(CH_2)_5Br$ | i-Pr | 1 | O |
| $(CH_2)_5Br$ | $CMe=CH_2$ | 1 | O |
| $(CH_2)_5Br$ | $CMeClCH_2Cl$ | 1 | O |
| $(CH_2)_5Br$ | $CHMeCH_2Cl$ | 1 | O |
| $(CH_2)_5Br$ | Ph | 1 | O |
| $(CH_2)_5Br$ | $CH_2Ph$ | 1 | O |
| $(CH_2)_5Br$ | CHMe-Ph | 1 | O |
| $(CH_2)_6Cl$ | i-Pr | 1 | O |
| $(CH_2)_6Cl$ | $CMe=CH_2$ | 1 | O |
| $(CH_2)_6Cl$ | $CMeClCH_2Cl$ | 1 | O |
| $(CH_2)_6Cl$ | $CHMeCH_2Cl$ | 1 | O |
| $(CH_2)_6Cl$ | Ph | 1 | O |
| $(CH_2)_6Cl$ | $CH_2Ph$ | 1 | O |
| $(CH_2)_6Cl$ | CHMe-Ph | 1 | O |
| $CH_2OMe$ | i-Pr | 1 | O |
| $CH_2OMe$ | $CMe=CH_2$ | 1 | O |
| $CH_2OMe$ | $CMeClCH_2Cl$ | 1 | O |
| $CH_2OMe$ | $CHMeCH_2Cl$ | 1 | O |
| $CH_2OMe$ | Ph | 1 | O |
| $CH_2OMe$ | $CH_2Ph$ | 1 | O |
| $CH_2OMe$ | CHMe-Ph | 1 | O |
| $(CH_2)_2OEt$ | i-Pr | 1 | O |
| $(CH_2)_2OEt$ | $CMe=CH_2$ | 1 | O |
| $(CH_2)_2OEt$ | $CMeClCH_2Cl$ | 1 | O |
| $(CH_2)_2OEt$ | $CHMeCH_2Cl$ | 1 | O |
| $(CH_2)_2OEt$ | Ph | 1 | O |
| $(CH_2)_2OEt$ | $CH_2Ph$ | 1 | O |
| $(CH_2)_2OEt$ | CHMe-Ph | 1 | O |
| CHMeOn-Bu | i-Pr | 1 | O |
| CHMeOn-Bu | $CMe=CH_2$ | 1 | O |
| CHMeOn-Bu | $CMeClCH_2Cl$ | 1 | O |
| CHMeOn-Bu | $CHMeCH_2Cl$ | 1 | O |

Table 3-a (continued)

| $R^3$ | $R^5$ | p | Z |
|---|---|---|---|
| CHMeOn-Bu | Ph | 1 | O |
| CHMeOn-Bu | $CH_2Ph$ | 1 | O |
| CHMeOn-Bu | CHMe-Ph | 1 | O |
| $(CH_2)_3OMe$ | i-Pr | 1 | O |
| $(CH_2)_3OMe$ | $CMe=CH_2$ | 1 | O |
| $(CH_2)_3OMe$ | $CMeClCH_2Cl$ | 1 | O |
| $(CH_2)_3OMe$ | $CHMeCH_2Cl$ | 1 | O |
| $(CH_2)_3OMe$ | Ph | 1 | O |
| $(CH_2)_3OMe$ | $CH_2Ph$ | 1 | O |
| $(CH_2)_3OMe$ | CHMe-Ph | 1 | O |
| $(CH_2)_4Oi\text{-}Pr$ | i-Pr | 1 | O |
| $(CH_2)_4Oi\text{-}Pr$ | $CMe=CH_2$ | 1 | O |
| $(CH_2)_4Oi\text{-}Pr$ | $CMeClCH_2Cl$ | 1 | O |
| $(CH_2)_4Oi\text{-}Pr$ | $CHMeCH_2Cl$ | 1 | O |
| $(CH_2)_4Oi\text{-}Pr$ | Ph | 1 | O |
| $(CH_2)_4Oi\text{-}Pr$ | $CH_2Ph$ | 1 | O |
| $(CH_2)_4Oi\text{-}Pr$ | CHMe-Ph | 1 | O |
| $(CH_2)_5OEt$ | i-Pr | 1 | O |
| $(CH_2)_5OEt$ | $CMe=CH_2$ | 1 | O |
| $(CH_2)_5OEt$ | $CMeClCH_2Cl$ | 1 | O |
| $(CH_2)_5OEt$ | $CHMeCH_2Cl$ | 1 | O |
| $(CH_2)_5OEt$ | Ph | 1 | O |
| $(CH_2)_5OEt$ | $CH_2Ph$ | 1 | O |
| $(CH_2)_5OEt$ | CHMe-Ph | 1 | O |
| $(CH_2)_6OMe$ | i-Pr | 1 | O |
| $(CH_2)_6OMe$ | $CMe=CH_2$ | 1 | O |
| $(CH_2)_6OMe$ | $CMeClCH_2Cl$ | 1 | O |
| $(CH_2)_6OMe$ | $CHMeCH_2Cl$ | 1 | O |
| $(CH_2)_6OMe$ | Ph | 1 | O |
| $(CH_2)_6OMe$ | $CH_2Ph$ | 1 | O |
| $(CH_2)_6OMe$ | CHMe-Ph | 1 | O |
| Ph | i-Pr | 1 | O |
| Ph | $CMe=CH_2$ | 1 | O |
| Ph | $CMeClCH_2Cl$ | 1 | O |
| Ph | $CHMeCH_2Cl$ | 1 | O |
| Ph | Ph | 1 | O |
| Ph | $CH_2Ph$ | 1 | O |
| Ph | CHMe-Ph | 1 | O |

Table 3-a (continued)

| $R^3$ | $R^5$ | p | Z |
|---|---|---|---|
| 2-Me-Ph | i-Pr | 1 | O |
| 2-Me-Ph | $CMe=CH_2$ | 1 | O |
| 2-Me-Ph | $CMeClCH_2Cl$ | 1 | O |
| 2-Me-Ph | $CHMeCH_2Cl$ | 1 | O |
| 2-Me-Ph | Ph | 1 | O |
| 2-Me-Ph | $CH_2Ph$ | 1 | O |
| 2-Me-Ph | CHMe-Ph | 1 | O |
| 4-Br-Ph | i-Pr | 1 | O |
| 4-Br-Ph | $CMe=CH_2$ | 1 | O |
| 4-Br-Ph | $CMeClCH_2Cl$ | 1 | O |
| 4-Br-Ph | $CHMeCH_2Cl$ | 1 | O |
| 4-Br-Ph | Ph | 1 | O |
| 4-Br-Ph | $CH_2Ph$ | 1 | O |
| 4-Br-Ph | CHMe-Ph | 1 | O |
| $3-CF_3-Ph$ | i-Pr | 1 | O |
| $3-CF_3-Ph$ | $CMe=CH_2$ | 1 | O |
| $3-CF_3-Ph$ | $CMeClCH_2Cl$ | 1 | O |
| $3-CF_3-Ph$ | $CHMeCH_2Cl$ | 1 | O |
| $3-CF_3-Ph$ | Ph | 1 | O |
| $3-CF_3-Ph$ | $CH_2Ph$ | 1 | O |
| $3-CF_3-Ph$ | CHMe-Ph | 1 | O |
| 2-F-4-Cl-Ph | i-Pr | 1 | O |
| 2-F-4-Cl-Ph | $CMe=CH_2$ | 1 | O |
| 2-F-4-Cl-Ph | $CMeClCH_2Cl$ | 1 | O |
| 2-F-4-Cl-Ph | $CHMeCH_2Cl$ | 1 | O |
| 2-F-4-Cl-Ph | Ph | 1 | O |
| 2-F-4-Cl-Ph | $CH_2Ph$ | 1 | O |
| 2-F-4-Cl-Ph | CHMe-Ph | 1 | O |
| $2,3,4,5,6-F_5-Ph$ | i-Pr | 1 | O |
| $2,3,4,5,6-F_5-Ph$ | $CMe=CH_2$ | 1 | O |
| $2,3,4,5,6-F_5-Ph$ | $CMeClCH_2Cl$ | 1 | O |
| $2,3,4,5,6-F_5-Ph$ | $CHMeCH_2Cl$ | 1 | O |
| $2,3,4,5,6-F_5-Ph$ | Ph | 1 | O |
| $2,3,4,5,6-F_5-Ph$ | $CH_2Ph$ | 1 | O |
| $2,3,4,5,6-F_5-Ph$ | CHMe-Ph | 1 | O |
| $4-C_2F_5-Ph$ | i-Pr | 1 | O |
| $4-C_2F_5-Ph$ | $CMe=CH_2$ | 1 | O |
| $4-C_2F_5-Ph$ | $CMeClCH_2Cl$ | 1 | O |

Table 3-a (continued)

| $R^3$ | $R^6$ | p | Z |
|---|---|---|---|
| 4-$C_2F_5$-Ph | $CHMeCH_2Cl$ | 1 | O |
| 4-$C_2F_5$-Ph | Ph | 1 | O |
| 4-$C_2F_5$-Ph | $CH_2Ph$ | 1 | O |
| 4-$C_2F_5$-Ph | CHMe-Ph | 1 | O |
| 4-On-Bu-Ph | i-Pr | 1 | O |
| 4-On-Bu-Ph | $CMe=CH_2$ | 1 | O |
| 4-On-Bu-Ph | $CMeClCH_2Cl$ | 1 | O |
| 4-On-Bu-Ph | $CHMeCH_2Cl$ | 1 | O |
| 4-On-Bu-Ph | Ph | 1 | O |
| 4-On-Bu-Ph | $CH_2Ph$ | 1 | O |
| 4-On-Bu-Ph | CHMe-Ph | 1 | O |
| 4-CN-Ph | i-Pr | 1 | O |
| 4-CN-Ph | $CMe=CH_2$ | 1 | O |
| 4-CN-Ph | $CMeClCH_2Cl$ | 1 | O |
| 4-CN-Ph | $CHMeCH_2Cl$ | 1 | O |
| 4-CN-Ph | Ph | 1 | O |
| 4-CN-Ph | $CH_2Ph$ | 1 | O |
| 4-CN-Ph | CHMe-Ph | 1 | O |
| 4-$NO_2$-Ph | i-Pr | 1 | O |
| 4-$NO_2$-Ph | $CMe=CH_2$ | 1 | O |
| 4-$NO_2$-Ph | $CMeClCH_2Cl$ | 1 | O |
| 4-$NO_2$-Ph | $CHMeCH_2Cl$ | 1 | O |
| 4-$NO_2$-Ph | Ph | 1 | O |
| 4-$NO_2$-Ph | $CH_2Ph$ | 1 | O |
| 4-$NO_2$-Ph | CHMe-Ph | 1 | O |
| 2-$CO_2H$-Ph | i-Pr | 1 | O |
| 2-$CO_2H$-Ph | $CMe=CH_2$ | 1 | O |
| 2-$CO_2H$-Ph | $CMeClCH_2Cl$ | 1 | O |
| 2-$CO_2H$-Ph | $CHMeCH_2Cl$ | 1 | O |
| 2-$CO_2H$-Ph | Ph | 1 | O |
| 2-$CO_2H$-Ph | $CH_2Ph$ | 1 | O |
| 2-$CO_2H$-Ph | CHMe-Ph | 1 | O |
| 2-$CO_2$t-Bu-Ph | i-Pr | 1 | O |
| 2-$CO_2$t-Bu-Ph | $CMe=CH_2$ | 1 | O |
| 2-$CO_2$t-Bu-Ph | $CMeClCH_2Cl$ | 1 | O |
| 2-$CO_2$t-Bu-Ph | $CHMeCH_2Cl$ | 1 | O |
| 2-$CO_2$t-Bu-Ph | Ph | 1 | O |
| 2-$CO_2$t-Bu-Ph | $CH_2Ph$ | 1 | O |

Table 3-a (continued)

| $R^3$ | $R^5$ | p | Z |
|---|---|---|---|
| 2-$CO_2$t-Bu-Ph | CHMe-Ph | 1 | O |
| $CH_2$Ph | i-Pr | 1 | O |
| $CH_2$Ph | CMe=$CH_2$ | 1 | O |
| $CH_2$Ph | CMeCl$CH_2$Cl | 1 | O |
| $CH_2$Ph | CHMe$CH_2$Cl | 1 | O |
| $CH_2$Ph | Ph | 1 | O |
| $CH_2$Ph | $CH_2$Ph | 1 | O |
| $CH_2$Ph | CHMe-Ph | 1 | O |
| $CH_2$-4-t-Bu-Ph | i-Pr | 1 | O |
| $CH_2$-4-t-Bu-Ph | CMe=$CH_2$ | 1 | O |
| $CH_2$-4-t-Bu-Ph | CMeCl$CH_2$Cl | 1 | O |
| $CH_2$-4-t-Bu-Ph | CHMe$CH_2$Cl | 1 | O |
| $CH_2$-4-t-Bu-Ph | Ph | 1 | O |
| $CH_2$-4-t-Bu-Ph | $CH_2$Ph | 1 | O |
| $CH_2$-4-t-Bu-Ph | CHMe-Ph | 1 | O |
| CHMe-Ph | i-Pr | 1 | O |
| CHMe-Ph | CMe=$CH_2$ | 1 | O |
| CHMe-Ph | CMeCl$CH_2$Cl | 1 | O |
| CHMe-Ph | CHMe$CH_2$Cl | 1 | O |
| CHMe-Ph | Ph | 1 | O |
| CHMe-Ph | $CH_2$Ph | 1 | O |
| CHMe-Ph | CHMe-Ph | 1 | O |
| CHMe-4-Cl-Ph | i-Pr | 1 | O |
| CHMe-4-Cl-Ph | CMe=$CH_2$ | 1 | O |
| CHMe-4-Cl-Ph | CMeCl$CH_2$Cl | 1 | O |
| CHMe-4-Cl-Ph | CHMe$CH_2$Cl | 1 | O |
| CHMe-4-Cl-Ph | Ph | 1 | O |
| CHMe-4-Cl-Ph | $CH_2$Ph | 1 | O |
| CHMe-4-Cl-Ph | CHMe-Ph | 1 | O |
| $(CH_2)_2$Ph | i-Pr | 1 | O |
| $(CH_2)_2$Ph | CMe=$CH_2$ | 1 | O |
| $(CH_2)_2$Ph | CMeCl$CH_2$Cl | 1 | O |
| $(CH_2)_2$Ph | CHMe$CH_2$Cl | 1 | O |
| $(CH_2)_2$Ph | Ph | 1 | O |
| $(CH_2)_2$Ph | $CH_2$Ph | 1 | O |
| $(CH_2)_2$Ph | CHMe-Ph | 1 | O |
| $(CH_2)_2$-4-OMe-Ph | i-Pr | 1 | O |
| $(CH_2)_2$-4-OMe-Ph | CMe=$CH_2$ | 1 | O |

Table 3-a (continued)

| $R^3$ | $R^5$ | p | Z |
|---|---|---|---|
| $(CH_2)_2$-4-OMe-Ph | $CMeClCH_2Cl$ | 1 | O |
| $(CH_2)_2$-4-OMe-Ph | $CHMeCH_2Cl$ | 1 | O |
| $(CH_2)_2$-4-OMe-Ph | Ph | 1 | O |
| $(CH_2)_2$-4-OMe-Ph | $CH_2Ph$ | 1 | O |
| $(CH_2)_2$-4-OMe-Ph | CHMe-Ph | 1 | O |
| $(CH_2)_3Ph$ | i-Pr | 1 | O |
| $(CH_2)_3Ph$ | $CMe=CH_2$ | 1 | O |
| $(CH_2)_3Ph$ | $CMeClCH_2Cl$ | 1 | O |
| $(CH_2)_3Ph$ | $CHMeCH_2Cl$ | 1 | O |
| $(CH_2)_3Ph$ | Ph | 1 | O |
| $(CH_2)_3Ph$ | $CH_2Ph$ | 1 | O |
| $(CH_2)_3Ph$ | CHMe-Ph | 1 | O |
| $C(Me)_2Ph$ | i-Pr | 1 | O |
| $C(Me)_2Ph$ | $CMe=CH_2$ | 1 | O |
| $C(Me)_2Ph$ | $CMeClCH_2Cl$ | 1 | O |
| $C(Me)_2Ph$ | $CHMeCH_2Cl$ | 1 | O |
| $C(Me)_2Ph$ | Ph | 1 | O |
| $C(Me)_2Ph$ | $CH_2Ph$ | 1 | O |
| $C(Me)_2Ph$ | CHMe-Ph | 1 | O |
| Me | CH=CHCl | 1 | O |
| Me | CMe=CHBr | 1 | O |
| Me | CHMeCCl=CHCl | 1 | O |
| Me | $CMe_2CBr=CHBr$ | 1 | O |
| Me | CHn-PrCCl=CHCl | 1 | O |
| Me | $CH(OMe)CH_2Cl$ | 1 | O |
| Me | $CMe(OMe)CH_2Cl$ | 1 | O |
| Me | $CMe(OMe)CH_2Br$ | 1 | O |
| Me | $CMe(OMe)CH_2I$ | 1 | O |
| Me | $CMe(OEt)CH_2Cl$ | 1 | O |
| Me | $CMe(Oi-Pr)CH_2Br$ | 1 | O |
| Me | $CMe(On-Bu)CH_2Br$ | 1 | O |
| Me | $CEt(OMe)CH_2Cl$ | 1 | O |
| Me | c-Pr | 1 | O |
| Me | 1-Me-c-Pr | 1 | O |
| Me | 2-Me-c-Pr | 1 | O |
| Me | 1,2-$Me_2$-c-Pr | 1 | O |
| Me | 2,2-$Me_2$-c-Pr | 1 | O |
| Me | 2,3-$Me_2$-c-Pr | 1 | O |

Table 3-a (continued)

| $R^3$ | $R^5$ | p | Z |
|---|---|---|---|
| Me | c-Bu | 1 | O |
| Me | c-Pen | 1 | O |
| Me | 1-Me-c-Pen | 1 | O |
| Me | c-Hex | 1 | O |
| Me | 1-Me-c-Hex | 1 | O |
| Me | 2-Me-c-Hex | 1 | O |
| Me | c-Hep | 1 | O |
| Me | 1-Cl-c-Pr | 1 | O |
| Me | 1-Br-c-Pr | 1 | O |
| Me | 2,2-$F_2$-1-Me-c-Pr | 1 | O |
| Me | 2,2-$Cl_2$-1-Me-c-Pr | 1 | O |
| Me | 2,2-$Br_2$-1-Me-c-Pr | 1 | O |
| Me | 1-Cl-c-Bu | 1 | O |
| Me | 1-Cl-c-Pen | 1 | O |
| Me | 1-Cl-c-Hex | 1 | O |
| Me | 1-Br-c-Hex | 1 | O |
| Me | 1,2-$Br_2$-c-Hex | 1 | O |
| Me | 2-Cl-1-Me-c-Hex | 1 | O |
| Me | F | 1 | O |
| Me | Cl | 1 | O |
| Me | Br | 1 | O |
| Me | CN | 1 | O |
| Me | CMe=CHEt | 1 | O |
| Me | CMe=CHn-Pr | 1 | O |
| Me | CMe=CHi-Pr | 1 | O |
| Me | CMe=CHn-Bu | 1 | O |
| Me | $CMe_2CH=CH_2$ | 1 | O |
| Me | C≡CH | 1 | O |
| Me | C≡CMe | 1 | O |
| Me | $CH_2C≡CH$ | 1 | O |
| Me | $CH_2C≡CMe$ | 1 | O |
| Me | CHMeC≡CH | 1 | O |
| Me | CHMeC≡CMe | 1 | O |
| Me | $CMe_2C≡CH$ | 1 | O |
| Me | $CMe_2C≡CMe$ | 1 | O |
| Me | OH | 1 | O |
| Me | OEt | 1 | O |
| Me | On-Pr | 1 | O |

Table 3-a (continued)

| $R^3$ | $R^5$ | p | Z |
|---|---|---|---|
| Me | On-Bu | 1 | O |
| Me | Oi-Bu | 1 | O |
| Me | Os-Bu | 1 | O |
| Me | Ot-Bu | 1 | O |
| Me | On-Pen | 1 | O |
| Me | $OCH_2CH=CH_2$ | 1 | O |
| Me | $OCHMeCH=CH_2$ | 1 | O |
| Me | $OCMe_2CH=CH_2$ | 1 | O |
| Me | $OCH_2CH=CHMe$ | 1 | O |
| Me | $OCH_2C\equiv CH$ | 1 | O |
| Me | $OCHMeC\equiv CH$ | 1 | O |
| Me | $OMe_2C\equiv CH$ | 1 | O |
| Me | $OCH_2C\equiv CMe$ | 1 | O |
| Me | SH | 1 | O |
| Me | SMe | 1 | O |
| Me | SEt | 1 | O |
| Me | Sn-Pr | 1 | O |
| Me | Si-Pr | 1 | O |
| Me | Sn-Bu | 1 | O |
| Me | $SCH_2CH=CH_2$ | 1 | O |
| Me | $SCHMeCH=CH_2$ | 1 | O |
| Me | $SCMe_2CH=CH_2$ | 1 | O |
| Me | $SCH_2CH=CHMe$ | 1 | O |
| Me | $SCH_2C\equiv CH$ | 1 | O |
| Me | $SCHMeC\equiv CH$ | 1 | O |
| Me | $SCMe_2C\equiv CH$ | 1 | O |
| Me | $SCH_2C\equiv CMe$ | 1 | O |
| Me | $NH_2$ | 1 | O |
| Me | NHMe | 1 | O |
| Me | NHEt | 1 | O |
| Me | NHn-Pr | 1 | O |
| Me | $NMe_2$ | 1 | O |
| Me | NMe(Et) | 1 | O |
| Me | $NEt_2$ | 1 | O |
| Me | $C\equiv CCl$ | 1 | O |
| Me | $CH_2C\equiv CCl$ | 1 | O |
| Me | $CHClC\equiv CH$ | 1 | O |
| Me | $OCF_3$ | 1 | O |

Table 3-a (continued)

| $R^3$ | $R^5$ | p | Z |
|---|---|---|---|
| Me | $OCHF_2$ | 1 | O |
| Me | $OCHCl_2$ | 1 | O |
| Me | $OCH_2CF_3$ | 1 | O |
| Me | $OCH_2CCl_3$ | 1 | O |
| Me | $SCF_3$ | 1 | O |
| Me | $SCHF_2$ | 1 | O |
| Me | $SCHCl_2$ | 1 | O |
| Me | $SCH_2CF_3$ | 1 | O |
| Me | $SCH_2CCl_3$ | 1 | O |
| Me | CH(OMe)Me | 1 | O |
| Me | CH(OEt)Me | 1 | O |
| Me | $CMe_2OMe$ | 1 | O |
| Me | $CMe_2OEt$ | 1 | O |
| Me | $CMe(OEt)CH_2Br$ | 1 | O |
| Me | $CH_2OCH_2CH=CH_2$ | 1 | O |
| Me | $CHMeOCH_2CH=CH_2$ | 1 | O |
| Me | $CMe_2OCH_2CH=CH_2$ | 1 | O |
| Me | $CH_2OCH_2C\equiv CH$ | 1 | O |
| Me | $CHMeOCH_2C\equiv CH$ | 1 | O |
| Me | $CMe_2OCH_2C\equiv CH$ | 1 | O |
| Me | $CH_2OCF_3$ | 1 | O |
| Me | $CH_2OCH_2CF_3$ | 1 | O |
| Me | $CH_2OCH_2CCl_3$ | 1 | O |
| Me | $CHMeOCF_3$ | 1 | O |
| Me | $CHMeOCH_2CF_3$ | 1 | O |
| Me | $CHMeOCH_2CCl_3$ | 1 | O |
| Me | $CHMeOCHF_2$ | 1 | O |
| Me | $CHMeOCHCl_2$ | 1 | O |
| Me | $CHMeOCHBr_2$ | 1 | O |
| Me | $CMe_2OCH_2CF_3$ | 1 | O |
| Me | $CMe_2OCH_2CCl_3$ | 1 | O |
| Me | $CH_2SH$ | 1 | O |
| Me | CHMeSH | 1 | O |
| Me | $CMe_2SH$ | 1 | O |
| Me | $CH_2SMe$ | 1 | O |
| Me | $CH_2SEt$ | 1 | O |
| Me | CHMeSMe | 1 | O |
| Me | CHMeSEt | 1 | O |

Table 3-a (continued)

| $R^3$ | $R^5$ | p | Z |
|---|---|---|---|
| Me | $CMe_2SMe$ | 1 | O |
| Me | $CMe_2SEt$ | 1 | O |
| Me | $CH_2NH_2$ | 1 | O |
| Me | $CHMeNH_2$ | 1 | O |
| Me | $CMe_2NH_2$ | 1 | O |
| Me | $CH_2NHMe$ | 1 | O |
| Me | $CH_2NHEt$ | 1 | O |
| Me | CHMeNHMe | 1 | O |
| Me | CHMeNHEt | 1 | O |
| Me | $CMe_2NHMe$ | 1 | O |
| Me | $CMe_2NHEt$ | 1 | O |
| Me | $CH_2NMe_2$ | 1 | O |
| Me | $CH_2NMe(Et)$ | 1 | O |
| Me | $CH_2NEt_2$ | 1 | O |
| Me | $CHMeNMe_2$ | 1 | O |
| Me | CHMeNMe(Et) | 1 | O |
| Me | $CHMeNEt_2$ | 1 | O |
| Me | $CMe_2NMe_2$ | 1 | O |
| Me | $CMe_2NMe(Et)$ | 1 | O |
| Me | $CMe_2NEt_2$ | 1 | O |
| Me | $CH_2CN$ | 1 | O |
| Me | CHMeCN | 1 | O |
| Me | $CMe_2CN$ | 1 | O |
| Me | $CH_2C(O)H$ | 1 | O |
| Me | CHMeC(O)H | 1 | O |
| Me | $CMe_2C(O)H$ | 1 | O |
| Me | $CH_2CO_2H$ | 1 | O |
| Me | $CHMeCO_2H$ | 1 | O |
| Me | $CMe_2CO_2H$ | 1 | O |
| Me | $CH_2C(O)Me$ | 1 | O |
| Me | $CH_2C(O)Et$ | 1 | O |
| Me | CHMeC(O)Me | 1 | O |
| Me | CHMeC(O)Et | 1 | O |
| Me | $CMe_2C(O)Me$ | 1 | O |
| Me | $CMe_2C(O)Et$ | 1 | O |
| Me | $CH_2CO_2Me$ | 1 | O |
| Me | $CH_2CO_2Et$ | 1 | O |
| Me | $CHMeCO_2Me$ | 1 | O |

Table 3-a (continued)

| $R^3$ | $R^5$ | p | Z |
|---|---|---|---|
| Me | $CHMeCO_2Et$ | 1 | O |
| Me | $CMe_2CO_2Me$ | 1 | O |
| Me | $CMe_2CO_2Et$ | 1 | O |
| Me | $CH_2OCH_2OMe$ | 1 | O |
| Me | $CHMeOCH_2OMe$ | 1 | O |
| Me | $CHMeOCH_2OEt$ | 1 | O |
| Me | $CHMeO(CH_2)_2OMe$ | 1 | O |
| Me | $CMe_2CH_2OMe$ | 1 | O |
| Me | $CH_2OCH_2$c-Pr | 1 | O |
| Me | $CHMeOCH_2$c-Pr | 1 | O |
| Me | $CMe_2OCH_2$c-Pr | 1 | O |
| Me | $CH_2OCH_2$(2,2-$Cl_2$-c-Pr) | 1 | O |
| Me | $CHMeOCH_2$(2,2-$Cl_2$-c-Pr) | 1 | O |
| Me | $CMe_2OCH_2$(2,2-$Cl_2$-c-Pr) | 1 | O |
| Me | OC(O)Me | 1 | O |
| Me | OC(O)Et | 1 | O |
| Me | OC(O)n-Pr | 1 | O |
| Me | OC(O)i-Pr | 1 | O |
| Me | e-Et | 1 | O |
| Me | 1-Me-e-Et | 1 | O |
| Me | 2-Me-e-Et | 1 | O |
| Me | 1,2-$Me_2$-e-Et | 1 | O |
| Me | 2,3-$Me_2$-e-Et | 1 | O |
| Me | $CH_2$-e-Et | 1 | O |
| Me | CHMe-e-Et | 1 | O |
| Me | $CMe_2$-e-Et | 1 | O |
| Me | C(O)Et | 1 | O |
| Me | C(O)n-Pr | 1 | O |
| Me | $C(O)CF_3$ | 1 | O |
| Me | $CO_2Me$ | 1 | O |
| Me | $CO_2Et$ | 1 | O |
| Me | $CO_2CH_2CF_3$ | 1 | O |
| Me | $CO_2CH_2CCl_3$ | 1 | O |
| Me | C(O)SMe | 1 | O |
| Me | C(O)SEt | 1 | O |
| Me | C(O)NHMe | 1 | O |
| Me | C(O)NHEt | 1 | O |
| Me | C(O)NHt-Bu | 1 | O |

Table 3-a (continued)

| $R^3$ | $R^5$ | p | Z |
|---|---|---|---|
| Me | C(O)NMe$_2$ | 1 | O |
| Me | C(O)NMe(t-Bu) | 1 | O |
| Me | C(S)Et | 1 | O |
| Me | C(S)n-Pr | 1 | O |
| Me | C(S)CF$_3$ | 1 | O |
| Me | C(S)OMe | 1 | O |
| Me | C(S)OEt | 1 | O |
| Me | C(S)OCH$_2$CF$_3$ | 1 | O |
| Me | C(S)OCH$_2$CCl$_3$ | 1 | O |
| Me | C(S)SMe | 1 | O |
| Me | C(S)SEt | 1 | O |
| Me | C(S)NHMe | 1 | O |
| Me | C(S)NHEt | 1 | O |
| Me | C(S)NHt-Bu | 1 | O |
| Me | C(S)NMe$_2$ | 1 | O |
| Me | C(S)NMe(t-Bu) | 1 | O |
| Me | C(=NOMe)Me | 1 | O |
| Me | C(=NOMe)Et | 1 | O |
| Me | C(=NOMe)t-Bu | 1 | O |
| Me | C(=NOEt)Me | 1 | O |
| Me | C(=NOEt)Et | 1 | O |
| Me | C(=NOEt)t-Bu | 1 | O |
| Me | 2-Me-Ph | 1 | O |
| Me | 3-Me-Ph | 1 | O |
| Me | 4-Me-Ph | 1 | O |
| Me | 2-Cl-Ph | 1 | O |
| Me | 3-Cl-Ph | 1 | O |
| Me | 4-Cl-Ph | 1 | O |
| Me | 2-Br-Ph | 1 | O |
| Me | 3-Br-Ph | 1 | O |
| Me | 4-Br-Ph | 1 | O |
| Me | 2-F-Ph | 1 | O |
| Me | 3-F-Ph | 1 | O |
| Me | 2-NO$_2$-Ph | 1 | O |
| Me | 3-NO$_2$-Ph | 1 | O |
| Me | 3-MeO-Ph | 1 | O |
| Me | 4-MeO-Ph | 1 | O |
| Me | 2-CF$_3$-Ph | 1 | O |

Table 3-a (continued)

| $R^3$ | $R^5$ | p | Z |
|---|---|---|---|
| Me | 4-$CF_3$-Ph | 1 | O |
| Me | 2-CN-Ph | 1 | O |
| Me | 3-CN-Ph | 1 | O |
| Me | 2-MeS-Ph | 1 | O |
| Me | 3-MeS-Ph | 1 | O |
| Me | 4-MeS-Ph | 1 | O |
| Me | 2,3-$Cl_2$-Ph | 1 | O |
| Me | 2,4-$Cl_2$-Ph | 1 | O |
| Me | 2,5-$Cl_2$-Ph | 1 | O |
| Me | 2,6-$Cl_2$-Ph | 1 | O |
| Me | 2,3-$Me_2$-Ph | 1 | O |
| Me | 2,4-$Me_2$-Ph | 1 | O |
| Me | 2,5-$Me_2$-Ph | 1 | O |
| Me | 2,6-$Me_2$-Ph | 1 | O |
| Me | OPh | 1 | O |
| Me | O(2-Cl-Ph) | 1 | O |
| Me | O(3-Cl-Ph) | 1 | O |
| Me | O(4-Cl-Ph) | 1 | O |
| Me | O(2-Me-Ph) | 1 | O |
| Me | O(3-Me-Ph) | 1 | O |
| Me | O(4-Me-Ph) | 1 | O |
| Me | SPh | 1 | O |
| Me | S(2-Cl-Ph) | 1 | O |
| Me | S(3-Cl-Ph) | 1 | O |
| Me | S(4-Cl-Ph) | 1 | O |
| Me | S(2-Me-Ph) | 1 | O |
| Me | S(3-Me-Ph) | 1 | O |
| Me | S(4-Me-Ph) | 1 | O |
| Me | $OCH_2Ph$ | 1 | O |
| Me | $OCH_2$(2-Cl-Ph) | 1 | O |
| Me | $OCH_2$(3-Cl-Ph) | 1 | O |
| Me | $OCH_2$(4-Cl-Ph) | 1 | O |
| Me | $OCH_2$(2-Me-Ph) | 1 | O |
| Me | $OCH_2$(3-Me-Ph) | 1 | O |
| Me | $OCH_2$(4-Me-Ph) | 1 | O |
| Me | OCHMePh | 1 | O |
| Me | $SCH_2Ph$ | 1 | O |
| Me | $SCH_2$(2-Cl-Ph) | 1 | O |

Table 3-a (continued)

| $R^3$ | $R^5$ | p | Z |
|---|---|---|---|
| Me | $SCH_2$(3-Cl-Ph) | 1 | O |
| Me | $SCH_2$(4-Cl-Ph) | 1 | O |
| Me | $SCH_2$(2-Me-Ph) | 1 | O |
| Me | $SCH_2$(3-Me-Ph) | 1 | O |
| Me | $SCH_2$(4-Me-Ph) | 1 | O |
| Me | SCHMePh | 1 | O |
| Me | $CH_2$(2-Cl-Ph) | 1 | O |
| Me | $CH_2$(3-Cl-Ph) | 1 | O |
| Me | $CH_2$(4-Cl-Ph) | 1 | O |
| Me | $CH_2$(2-Me-Ph) | 1 | O |
| Me | $CH_2$(3-Me-Ph) | 1 | O |
| Me | $CH_2$(4-Me-Ph) | 1 | O |
| Me | CH(OH)Ph | 1 | O |
| Me | CH(OMe)Ph | 1 | O |
| Me | CHClPh | 1 | O |
| Me | CMe(OH)Ph | 1 | O |
| Me | CMe(OMe)Ph | 1 | O |
| Me | CMeClPh | 1 | O |
| Me | $CMe_2$Ph | 1 | O |
| Me | 2-pyridyl | 1 | O |
| Me | 3-pyridyl | 1 | O |
| Me | 4-pyridyl | 1 | O |
| Me | 3-Cl-2-pyridyl | 1 | O |
| Me | 2-Cl-3-pyridyl | 1 | O |
| Me | 4-Cl-3-pyridyl | 1 | O |
| Me | 3-Cl-4-pyridyl | 1 | O |
| Me | 3-Me-2-pyridyl | 1 | O |
| Me | 2-Me-3-pyridyl | 1 | O |
| Me | 4-Me-3-pyridyl | 1 | O |
| Me | 3-Me-4-pyridyl | 1 | O |
| Me | 2-pyrimidyl | 1 | O |
| Me | 4-pyrimidyl | 1 | O |
| Me | 5-pyrimidyl | 1 | O |
| Me | 5-Cl-4-pyrimidyl | 1 | O |
| Me | 4-Cl-5-pyrimidyl | 1 | O |
| Me | 5-Me-4-pyrimidyl | 1 | O |
| Me | 4-Me-5-pyrimidyl | 1 | O |
| Me | 2-thienyl | 1 | O |

Table 3-a (continued)

| $R^3$ | $R^5$ | p | Z |
|---|---|---|---|
| Me | 3-thienyl | 1 | O |
| Me | 3-Cl-2-thienyl | 1 | O |
| Me | 2-Cl-3-thienyl | 1 | O |
| Me | 4-Cl-3-thienyl | 1 | O |
| Me | 3-Me-2-thienyl | 1 | O |
| Me | 2-Me-3-thienyl | 1 | O |
| Me | 4-Me-3-thienyl | 1 | O |
| Me | 2-furyl | 1 | O |
| Me | 3-furyl | 1 | O |
| Me | 3-Cl-2-furyl | 1 | O |
| Me | 2-Cl-3-furyl | 1 | O |
| Me | 4-Cl-3-furyl | 1 | O |
| Me | 3-Me-2-furyl | 1 | O |
| Me | 2-Me-3-furyl | 1 | O |
| Me | 4-Me-3-furyl | 1 | O |
| Me | 1-Me-2-pyrrolyl | 1 | O |
| Me | 1-Me-3-pyrrolyl | 1 | O |
| Me | 3-Cl-(1-Me-2-pyrrolyl) | 1 | O |
| Me | 2-Cl-(1-Me-3-pyrrolyl) | 1 | O |
| Me | 4-Cl-(1-Me-3-pyrrolyl) | 1 | O |
| Me | 3-Me-(1-Me-2-pyrrolyl) | 1 | O |
| Me | 2-Me-(1-Me-3-pyrrolyl) | 1 | O |
| Me | 4-Me-(1-Me-3-pyrrolyl) | 1 | O |
| Me | 1-Me-3-pyrazolyl | 1 | O |
| Me | 1-Me-4-pyrazolyl | 1 | O |
| Me | 1-Me-5-pyrazolyl | 1 | O |
| Me | 4-Cl-(1-Me-3-pyrazolyl) | 1 | O |
| Me | 3-Cl-(1-Me-4-pyrazolyl) | 1 | O |
| Me | 5-Cl-(1-Me-4-pyrazolyl) | 1 | O |
| Me | 3,5-$Cl_2$-(1-Me-4-pyrazolyl) | 1 | O |
| Me | 4-Cl-(1-Me-5-pyrazolyl) | 1 | O |
| Me | 4-Me-(1-Me-3-pyrazolyl) | 1 | O |
| Me | 3-Me-(1-Me-4-pyrazolyl) | 1 | O |
| Me | 5-Me-(1-Me-4-pyrazolyl) | 1 | O |
| Me | 4-Me-(1-Me-5-pyrazolyl) | 1 | O |
| Et | Me | 1 | O |
| Et | Et | 1 | O |
| Et | n-Pr | 1 | O |

Table 3-a (continued)

| $R^3$ | $R^5$ | p | Z |
|---|---|---|---|
| Et | n-Bu | 1 | O |
| Et | i-Bu | 1 | O |
| Et | s-Bu | 1 | O |
| Et | t-Bu | 1 | O |
| Et | n-Pen | 1 | O |
| Et | c-Pr | 1 | O |
| Et | c-Bu | 1 | O |
| Et | c-pen | 1 | O |
| Et | 1-Me-c-Pr | 1 | O |
| Et | 2-Me-c-Pr | 1 | O |
| Et | 1,2-$Me_2$-c-Pr | 1 | O |
| Et | 2,2-$Me_2$-c-Pr | 1 | O |
| Et | 2,3-$Me_2$-c-Pr | 1 | O |
| Et | 1-Cl-c-Pr | 1 | O |
| Et | 1-Br-c-Pr | 1 | O |
| Et | 2,2-$F_2$-c-Pr | 1 | O |
| Et | 2,2-$Cl_2$-c-Pr | 1 | O |
| Et | 2,2-$Br_2$-c-Pr | 1 | O |
| Et | 2,2-$F_2$-1-Me-c-Pr | 1 | O |
| Et | 2,2-$Cl_2$-1-Me-c-Pr | 1 | O |
| Et | 2,2-$Br_2$-1-Me-c-Pr | 1 | O |
| Et | $CH_2$=$CH_2$ | 1 | O |
| Et | $CH_2$=CHMe | 1 | O |
| Et | $CH_2$=CHEt | 1 | O |
| Et | CHMe=CHMe | 1 | O |
| Et | 2-Cl-Ph | 1 | O |
| Et | 3-Cl-Ph | 1 | O |
| Et | 4-Cl-Ph | 1 | O |
| Et | 2-Me-Ph | 1 | O |
| Et | 3-Me-Ph | 1 | O |
| Et | 4-Me-Ph | 1 | O |
| Et | 2-$NO_2$-Ph | 1 | O |
| Et | 3-$NO_2$-Ph | 1 | O |
| Et | 4-$NO_2$-Ph | 1 | O |
| Et | 2-pyridyl | 1 | O |
| Et | 3-pyridyl | 1 | O |
| Et | 4-pyridyl | 1 | O |
| Et | 2-pyrimidyl | 1 | O |

Table 3-a (continued)

| $R^3$ | $R^5$ | p | Z |
|---|---|---|---|
| Et | 4-pyrimidyl | 1 | O |
| Et | 5-pyrimidyl | 1 | O |
| Et | 2-thienyl | 1 | O |
| Et | 3-thienyl | 1 | O |
| Et | 2-furyl | 1 | O |
| Et | 3-furyl | 1 | O |
| Et | 1-Me-2-pyrrolyl | 1 | O |
| Et | 1-Me-3-pyrrolyl | 1 | O |
| Et | 1-Me-3-pyrazolyl | 1 | O |
| Et | 1-Me-4-pyrazolyl | 1 | O |
| Et | 1-Me-5-pyrazolyl | 1 | O |
| Et | 1-Me-3-Cl-4-pyrazolyl | 1 | O |
| Et | 1-Me-3,5-$Cl_2$-4-pyrazlyl | 1 | O |
| Me | F | 0 | O |
| Me | Cl | 0 | O |
| Me | Br | 0 | O |
| Me | I | 0 | O |
| Me | CN | 0 | O |
| Me | Me | 0 | O |
| Me | Et | 0 | O |
| Me | n-Pr | 0 | O |
| Me | i-Pr | 0 | O |
| Me | n-Bu | 0 | O |
| Me | i-Bu | 0 | O |
| Me | s-Bu | 0 | O |
| Me | t-Bu | 0 | O |
| Me | n-Pen | 0 | O |
| Me | $CH=CH_2$ | 0 | O |
| Me | $CH_2CH=CH_2$ | 0 | O |
| Me | $C\equiv CH$ | 0 | O |
| Me | $CH_2C\equiv CH$ | 0 | O |
| Me | OH | 0 | O |
| Me | OMe | 0 | O |
| Me | OEt | 0 | O |
| Me | On-Pr | 0 | O |
| Me | Oi-Pr | 0 | O |
| Me | On-Bu | 0 | O |
| Me | $OCH_2CH=CH_2$ | 0 | O |

Table 3-a (continued)

| $R^3$ | $R^5$ | p | Z |
|---|---|---|---|
| Me | $OCH_2C{\equiv}CH$ | 0 | O |
| Me | SH | 0 | O |
| Me | SMe | 0 | O |
| Me | SEt | 0 | O |
| Me | Sn-Pr | 0 | O |
| Me | $SCH_2CH{=}CH_2$ | 0 | O |
| Me | $SCH_2C{\equiv}CH$ | 0 | O |
| Me | $NH_2$ | 0 | O |
| Me | NHMe | 0 | O |
| Me | NHEt | 0 | O |
| Me | $NMe_2$ | 0 | O |
| Me | $CH_2Cl$ | 0 | O |
| Me | $CF_3$ | 0 | O |
| Me | $CHClCH_2Cl$ | 0 | O |
| Me | $CH_2CHClCH_2Cl$ | 0 | O |
| Me | $CH{=}CHCl$ | 0 | O |
| Me | $CH_2CH{=}CHCl$ | 0 | O |
| Me | $C{\equiv}CCl$ | 0 | O |
| Me | $CH_2C{\equiv}CCl$ | 0 | O |
| Me | $OCF_3$ | 0 | O |
| Me | $OCHF_2$ | 0 | O |
| Me | $OCHCl_2$ | 0 | O |
| Me | $OCH_2CF_3$ | 0 | O |
| Me | $SCF_3$ | 0 | O |
| Me | $SCHF_2$ | 0 | O |
| Me | $SCHCl_2$ | 0 | O |
| Me | $SCH_2CF_3$ | 0 | O |
| Me | $CH_2OH$ | 0 | O |
| Me | CH(OH)Me | 0 | O |
| Me | $CH_2OMe$ | 0 | O |
| Me | CH(OMe)Me | 0 | O |
| Me | $CH_2OCH_2C{=}CH_2$ | 0 | O |
| Me | $CH(OCH_2C{=}CH_2)Me$ | 0 | O |
| Me | $CH_2OCH_2C{\equiv}CH$ | 0 | O |
| Me | $CH(OCH_2C{\equiv}CH)Me$ | 0 | O |
| Me | $CH_2OCHF_2$ | 0 | O |
| Me | $CH(OCHF_2)Me$ | 0 | O |
| Me | $CH_2OCH_2CF_3$ | 0 | O |

Table 3-a (continued)

| $R^3$ | $R^5$ | p | Z |
|---|---|---|---|
| Me | $CH(OCH_2CF_3)Me$ | 0 | O |
| Me | $CH_2SH$ | 0 | O |
| Me | $CH_2SMe$ | 0 | O |
| Me | $CH_2NH_2$ | 0 | O |
| Me | $CH_2NHMe$ | 0 | O |
| Me | $CH_2NMe_2$ | 0 | O |
| Me | $CH_2CN$ | 0 | O |
| Me | $CH_2C(O)H$ | 0 | O |
| Me | $CH_2CO_2H$ | 0 | O |
| Me | $CH_2C(O)Me$ | 0 | O |
| Me | $CH_2CO_2Me$ | 0 | O |
| Me | $CH_2OCH_2OMe$ | 0 | O |
| Me | $CH_2OCH_2OEt$ | 0 | O |
| Me | $CH_2O(CH_2)_2OMe$ | 0 | O |
| Me | $CH_2OCH_2$c-Pr | 0 | O |
| Me | $CH_2OCH_2$(2,2-$Cl_2$-c-Pr) | 0 | O |
| Me | $CH(OMe)CH_2Cl$ | 0 | O |
| Me | $CH(OMe)CH_2Br$ | 0 | O |
| Me | c-Pr | 0 | O |
| Me | 1-Me-c-Pr | 0 | O |
| Me | 2-Me-c-Pr | 0 | O |
| Me | c-Bu | 0 | O |
| Me | c-Pen | 0 | O |
| Me | c-Hex | 0 | O |
| Me | 1-Cl-c-Pr | 0 | O |
| Me | 2-Cl-c-Pr | 0 | O |
| Me | 2,2-$F_2$-c-Pr | 0 | O |
| Me | 2,2-$Cl_2$-c-Pr | 0 | O |
| Me | 2,2-$Br_2$-c-Pr | 0 | O |
| Me | e-Et | 0 | O |
| Me | $CH_2$-e-Et | 0 | O |
| Me | C(O)Et | 0 | O |
| Me | $C(O)CF_3$ | 0 | O |
| Me | $CO_2Me$ | 0 | O |
| Me | C(O)SMe | 0 | O |
| Me | C(O)NHMe | 0 | O |
| Me | $C(O)NMe_2$ | 0 | O |
| Me | C(S)Et | 0 | O |

Table 3-a (continued)

| $R^3$ | $R^5$ | p | Z |
|---|---|---|---|
| Me | C(S)OMe | 0 | O |
| Me | C(NOMe)Et | 0 | O |
| Me | Ph | 0 | O |
| Me | 2-Cl-Ph | 0 | O |
| Me | 3-Cl-Ph | 0 | O |
| Me | 4-Cl-Ph | 0 | O |
| Me | 2-Me-Ph | 0 | O |
| Me | 3-Me-Ph | 0 | O |
| Me | 4-Me-Ph | 0 | O |
| Me | $CH_2Ph$ | 0 | O |
| Me | CHMe-Ph | 0 | O |
| Me | OPh | 0 | O |
| Me | SPh | 0 | O |
| Me | $OCH_2Ph$ | 0 | O |
| Me | OCHMePh | 0 | O |
| Me | $SCH_2Ph$ | 0 | O |
| Me | SCHMePh | 0 | O |
| Me | 2-pyridyl | 0 | O |
| Me | 3-pyridyl | 0 | O |
| Me | 4-pyridyl | 0 | O |
| Me | 2-pyrimidyl | 0 | O |
| Me | 4-pyrimidyl | 0 | O |
| Me | 5-pyrimidyl | 0 | O |
| Me | 2-thienyl | 0 | O |
| Me | 3-thienyl | 0 | O |
| Me | 2-furyl | 0 | O |
| Me | 3-furyl | 0 | O |
| Me | 1-Me-2-pyrrolyl | 0 | O |
| Me | 1-Me-3-pyrrolyl | 0 | O |
| Me | 1-Me-3-pyrazoyl | 0 | O |
| Me | 1-Me-4-pyrazoyl | 0 | O |
| Me | 1-Me-5-pyrazoyl | 0 | O |
| Me | 1-Me-3-Cl-4-pyrazoyl | 0 | O |
| Me | 1-Me-3,5-$Cl_2$-4-pyrazoyl | 0 | O |
| Et | F | 0 | O |
| Et | Cl | 0 | O |
| Et | Br | 0 | O |
| Et | CN | 0 | O |

Table 3-a (continued)

| $R^3$ | $R^5$ | p | Z |
|---|---|---|---|
| Et | Me | 0 | O |
| Et | Et | 0 | O |
| Et | n-Pr | 0 | O |
| Et | i-Pr | 0 | O |
| Et | n-Bu | 0 | O |
| Et | i-Bu | 0 | O |
| Et | s-Bu | 0 | O |
| Et | t-Bu | 0 | O |
| Et | n-Pen | 0 | O |
| Et | OH | 0 | O |
| Et | OMe | 0 | O |
| Et | SH | 0 | O |
| Et | SMe | 0 | O |
| Et | $NH_2$ | 0 | O |
| ET | NHMe | 0 | O |
| Et | $NMe_2$ | 0 | O |
| Et | $CH_2Cl$ | 0 | O |
| Et | $CF_3$ | 0 | O |
| Et | $CHClCH_2Cl$ | 0 | O |
| Et | $CH_2OH$ | 0 | O |
| Et | CH(OH)Me | 0 | O |
| Et | $CH_2OMe$ | 0 | O |
| Et | CH(OMe)Me | 0 | O |
| Et | $CH_2SH$ | 0 | O |
| Et | $CH_2SMe$ | 0 | O |
| Et | $CH_2NH_2$ | 0 | O |
| Et | $CH_2NHMe$ | 0 | O |
| Et | $CH_2NMe_2$ | 0 | O |
| Et | $CH_2CN$ | 0 | O |
| Et | $CH_2C(O)H$ | 0 | O |
| Et | $CH_2CO_2H$ | 0 | O |
| Et | $CH_2C(O)Me$ | 0 | O |
| Et | $CH_2CO_2Me$ | 0 | O |
| Et | $CH_2OCH_2OMe$ | 0 | O |
| Et | $CH_2OCH_2$c-Pr | 0 | O |
| Et | c-Pr | 0 | O |
| Et | c-Bu | 0 | O |
| Et | c-Pen | 0 | O |

Table 3-a (continued)

| $R^3$ | $R^5$ | p | Z |
|---|---|---|---|
| Et | c-Hex | 0 | O |
| Et | e-Et | 0 | O |
| Et | $CH_2$-e-Et | 0 | O |
| Et | C(O)Et | 0 | O |
| Et | $CO_2Me$ | 0 | O |
| Et | Ph | 0 | O |
| Et | $CH_2Ph$ | 0 | O |
| Et | CHMe-Ph | 0 | O |
| Et | OPh | 0 | O |
| Et | SPh | 0 | O |
| Et | $OCH_2Ph$ | 0 | O |
| Et | $SCH_2Ph$ | 0 | O |
| n-Pr | F | 0 | O |
| n-Pr | Cl | 0 | O |
| n-Pr | Br | 0 | O |
| n-Pr | Me | 0 | O |
| n-Pr | Et | 0 | O |
| n-Pr | n-Pr | 0 | O |
| n-Pr | i-Pr | 0 | O |
| n-Pr | n-Bu | 0 | O |
| n-Pr | i-Bu | 0 | O |
| n-Pr | s-Bu | 0 | O |
| n-Pr | t-Bu | 0 | O |
| n-Pr | n-Pen | 0 | O |
| n-Pr | OH | 0 | O |
| n-Pr | OMe | 0 | O |
| n-Pr | SH | 0 | O |
| n-Pr | SMe | 0 | O |
| n-Pr | $NH_2$ | 0 | O |
| n-Pr | NHMe | 0 | O |
| n-Pr | $NMe_2$ | 0 | O |
| n-Pr | Ph | 0 | O |
| n-Pr | $CH_2Ph$ | 0 | O |
| n-Pr | OPh | 0 | O |
| n-Pr | SPh | 0 | O |
| n-Pr | $OCH_2Ph$ | 0 | O |
| n-Pr | $SCH_2Ph$ | 0 | O |
| i-Pr | F | 0 | O |

Table 3-a (continued)

| $R^3$ | $R^5$ | p | Z |
|---|---|---|---|
| i-Pr | Cl | 0 | O |
| i-Pr | Br | 0 | O |
| i-Pr | Me | 0 | O |
| i-Pr | Et | 0 | O |
| i-Pr | n-Pr | 0 | O |
| i-Pr | i-Pr | 0 | O |
| i-Pr | n-Bu | 0 | O |
| i-Pr | i-Bu | 0 | O |
| i-Pr | s-Bu | 0 | O |
| i-Pr | t-Bu | 0 | O |
| i-Pr | n-Pen | 0 | O |
| i-Pr | OH | 0 | O |
| i-Pr | OMe | 0 | O |
| i-Pr | SH | 0 | O |
| i-Pr | SMe | 0 | O |
| i-Pr | $NH_2$ | 0 | O |
| i-Pr | NHMe | 0 | O |
| i-Pr | $NMe_2$ | 0 | O |
| i-Pr | Ph | 0 | O |
| i-Pr | $CH_2Ph$ | 0 | O |
| i-Pr | OPh | 0 | O |
| i-Pr | SPh | 0 | O |
| i-Pr | $OCH_2Ph$ | 0 | O |
| i-Pr | $SCH_2Ph$ | 0 | O |
| n-Bu | F | 0 | O |
| n-Bu | Cl | 0 | O |
| n-Bu | Br | 0 | O |
| n-Bu | Me | 0 | O |
| n-Bu | Et | 0 | O |
| n-Bu | n-Pr | 0 | O |
| n-Bu | i-Pr | 0 | O |
| n-Bu | n-Bu | 0 | O |
| n-Bu | i-Bu | 0 | O |
| n-Bu | s-Bu | 0 | O |
| n-Bu | t-Bu | 0 | O |
| n-Bu | n-Pen | 0 | O |
| n-Bu | OH | 0 | O |
| n-Bu | OMe | 0 | O |

Table 3-a (continued)

| $R^3$ | $R^5$ | p | Z |
|---|---|---|---|
| n-Bu | SH | 0 | O |
| n-Bu | SMe | 0 | O |
| n-Bu | $NH_2$ | 0 | O |
| n-Bu | NHMe | 0 | O |
| n-Bu | $NMe_2$ | 0 | O |
| n-Bu | Ph | 0 | O |
| n-Bu | $CH_2Ph$ | 0 | O |
| n-Bu | OPh | 0 | O |
| n-Bu | SPh | 0 | O |
| n-Bu | $OCH_2Ph$ | 0 | O |
| n-Bu | $SCH_2Ph$ | 0 | O |
| i-Bu | F | 0 | O |
| i-Bu | Cl | 0 | O |
| i-Bu | Br | 0 | O |
| i-Bu | Me | 0 | O |
| i-Bu | Et | 0 | O |
| i-Bu | n-Pr | 0 | O |
| i-Bu | i-Pr | 0 | O |
| i-Bu | n-Bu | 0 | O |
| i-Bu | i-Bu | 0 | O |
| i-Bu | s-Bu | 0 | O |
| i-Bu | t-Bu | 0 | O |
| i-Bu | n-Pen | 0 | O |
| i-Bu | OH | 0 | O |
| i-Bu | OMe | 0 | O |
| i-Bu | SH | 0 | O |
| i-Bu | SMe | 0 | O |
| i-Bu | $NH_2$ | 0 | O |
| i-Bu | NHMe | 0 | O |
| i-Bu | $NMe_2$ | 0 | O |
| i-Bu | Ph | 0 | O |
| i-Bu | $CH_2Ph$ | 0 | O |
| i-Bu | OPh | 0 | O |
| i-Bu | SPh | 0 | O |
| i-Bu | $OCH_2Ph$ | 0 | O |
| i-Bu | $SCH_2Ph$ | 0 | O |
| n-Pen | F | 0 | O |
| n-Pen | Cl | 0 | O |

Table 3-a (continued)

| $R^3$ | $R^5$ | p | Z |
|---|---|---|---|
| n-Pen | Br | 0 | O |
| n-Pen | Me | 0 | O |
| n-Pen | Et | 0 | O |
| n-Pen | n-Pr | 0 | O |
| n-Pen | i-Pr | 0 | O |
| n-Pen | n-Bu | 0 | O |
| n-Pen | i-Bu | 0 | O |
| n-Pen | s-Bu | 0 | O |
| n-Pen | t-Bu | 0 | O |
| n-Pen | n-Pen | 0 | O |
| n-Pen | OH | 0 | O |
| n-Pen | OMe | 0 | O |
| n-Pen | SH | 0 | O |
| n-Pen | SMe | 0 | O |
| n-Pen | $NH_2$ | 0 | O |
| n-Pen | NHMe | 0 | O |
| n-Pen | $NMe_2$ | 0 | O |
| n-Pen | Ph | 0 | O |
| n-Pen | $CH_2Ph$ | 0 | O |
| n-Pen | OPh | 0 | O |
| n-Pen | SPh | 0 | O |
| n-Pen | $OCH_2Ph$ | 0 | O |
| n-Pen | $SCH_2Ph$ | 0 | O |
| Me | Me | 1 | $CH_2$ |
| Me | Et | 1 | $CH_2$ |
| Me | n-Pr | 1 | $CH_2$ |
| Me | i-Pr | 1 | $CH_2$ |
| Me | n-Bu | 1 | $CH_2$ |
| Me | i-Bu | 1 | $CH_2$ |
| Me | s-Bu | 1 | $CH_2$ |
| Me | t-Bu | 1 | $CH_2$ |
| Me | n-Pen | 1 | $CH_2$ |
| Me | c-Pr | 1 | $CH_2$ |
| Me | c-Bu | 1 | $CH_2$ |
| Me | c-pen | 1 | $CH_2$ |
| Me | 1-Me-c-Pr | 1 | $CH_2$ |
| Me | 2-Me-c-Pr | 1 | $CH_2$ |
| Me | 1,2-$Me_2$-c-Pr | 1 | $CH_2$ |

Table 3-a (continued)

| $R^3$ | $R^5$ | p | Z |
|---|---|---|---|
| Me | 2,2-$Me_2$-c-Pr | 1 | $CH_2$ |
| Me | 2,3-$Me_2$-c-Pr | 1 | $CH_2$ |
| Me | 1-Cl-c-Pr | 1 | $CH_2$ |
| Me | 1-Br-c-Pr | 1 | $CH_2$ |
| Me | 2,2-$F_2$-c-Pr | 1 | $CH_2$ |
| Me | 2,2-$Cl_2$-c-Pr | 1 | $CH_2$ |
| Me | 2,2-$Br_2$-c-Pr | 1 | $CH_2$ |
| Me | 2,2-$F_2$-1-Me-c-Pr | 1 | $CH_2$ |
| Me | 2,2-$Cl_2$-1-Me-c-Pr | 1 | $CH_2$ |
| Me | 2,2-$Br_2$-1-Me-c-Pr | 1 | $CH_2$ |
| Me | $CH_2$=$CH_2$ | 1 | $CH_2$ |
| Me | $CH_2$=CHMe | 1 | $CH_2$ |
| Me | CHMe=$CH_2$ | 1 | $CH_2$ |
| Me | $CH_2$=CHEt | 1 | $CH_2$ |
| Me | CHMe=CHMe | 1 | $CH_2$ |
| Me | Ph | 1 | $CH_2$ |
| Me | 2-Cl-Ph | 1 | $CH_2$ |
| Me | 3-Cl-Ph | 1 | $CH_2$ |
| Me | 4-Cl-Ph | 1 | $CH_2$ |
| Me | 2-Me-Ph | 1 | $CH_2$ |
| Me | 3-Me-Ph | 1 | $CH_2$ |
| Me | 4-Me-Ph | 1 | $CH_2$ |
| Me | 2-$NO_2$-Ph | 1 | $CH_2$ |
| Me | 3-$NO_2$-Ph | 1 | $CH_2$ |
| Me | 4-$NO_2$-Ph | 1 | $CH_2$ |
| Me | Me | 0 | $CH_2$ |
| Me | Et | 0 | $CH_2$ |
| Me | n-Pr | 0 | $CH_2$ |
| Me | i-Pr | 0 | $CH_2$ |
| Me | n-Bu | 0 | $CH_2$ |
| Me | i-Bu | 0 | $CH_2$ |
| Me | s-Bu | 0 | $CH_2$ |
| Me | t-Bu | 0 | $CH_2$ |
| Me | n-Pen | 0 | $CH_2$ |
| Me | c-Pr | 0 | $CH_2$ |
| Me | c-Bu | 0 | $CH_2$ |
| Me | c-pen | 0 | $CH_2$ |
| Me | 1-Me-c-Pr | 0 | $CH_2$ |

Table 3-a (continued)

| $R^3$ | $R^5$ | p | Z |
|---|---|---|---|
| Me | 2-Me-c-Pr | 0 | $CH_2$ |
| Me | 1,2-$Me_2$-c-Pr | 0 | $CH_2$ |
| Me | 2,2-$Me_2$-c-Pr | 0 | $CH_2$ |
| Me | 2,3-$Me_2$-c-Pr | 0 | $CH_2$ |
| Me | 1-Cl-c-Pr | 0 | $CH_2$ |
| Me | 1-Br-c-Pr | 0 | $CH_2$ |
| Me | 2,2-$F_2$-c-Pr | 0 | $CH_2$ |
| Me | 2,2-$Cl_2$-c-Pr | 0 | $CH_2$ |
| Me | 2,2-$Br_2$-c-Pr | 0 | $CH_2$ |
| Me | 2,2-$F_2$-1-Me-c-Pr | 0 | $CH_2$ |
| Me | 2,2-$Cl_2$-1-Me-c-Pr | 0 | $CH_2$ |
| Me | 2,2-$Br_2$-1-Me-c-Pr | 0 | $CH_2$ |
| Me | $CH_2$=$CH_2$ | 0 | $CH_2$ |
| Me | $CH_2$=CHMe | 0 | $CH_2$ |
| Me | CHMe=$CH_2$ | 0 | $CH_2$ |
| Me | $CH_2$=CHEt | 0 | $CH_2$ |
| Me | CHMe=CHMe | 0 | $CH_2$ |
| Me | Ph | 0 | $CH_2$ |
| Me | 2-Cl-Ph | 0 | $CH_2$ |
| Me | 3-Cl-Ph | 0 | $CH_2$ |
| Me | 4-Cl-Ph | 0 | $CH_2$ |
| Me | 2-Me-Ph | 0 | $CH_2$ |
| Me | 3-Me-Ph | 0 | $CH_2$ |
| Me | 4-Me-Ph | 0 | $CH_2$ |
| Me | 2-$NO_2$-Ph | 0 | $CH_2$ |
| Me | 3-$NO_2$-Ph | 0 | $CH_2$ |
| Me | 4-$NO_2$-Ph | 0 | $CH_2$ |
| Me | CHMe$CH_2$Br | 1 | O |
| Me | CHMe$CH_2$I | 1 | O |
| Me | $CMe_2$Cl | 1 | O |
| Me | $CMe_2$Br | 1 | O |
| Me | $CMe_2$I | 1 | O |
| Me | $CMe_2$OEt | 1 | O |
| Me | C($CH_2$Cl)=$CH_2$ | 1 | O |
| Me | C($CH_2$Br)=$CH_2$ | 1 | O |
| Me | C($CH_2$I)=$CH_2$ | 1 | O |
| Me | $N_3$ | 0 | O |
| Me | $N_3$ | 1 | O |

Table 3-a (continued)

| $R^3$ | $R^5$ | p | Z |
|---|---|---|---|
| Me | $N_3$ | 0 | $CH_2$ |
| Me | $N_3$ | 1 | $CH_2$ |
| Me | $CHMeCH_2N_3$ | 0 | O |
| Me | $CHMeCH_2N_3$ | 1 | O |
| Me | $CHMeCH_2N_3$ | 0 | $CH_2$ |
| Me | $CHMeCH_2N_3$ | 1 | $CH_2$ |
| Me | $CHMeCH_2F$ | 0 | O |
| Me | $CHMeCH_2F$ | 1 | O |
| Me | $CHMeCH_2F$ | 0 | $CH_2$ |
| Me | $CHMeCH_2F$ | 1 | $CH_2$ |
| Me | $CHMeCH_2OH$ | 0 | O |
| Me | $CHMeCH_2OH$ | 1 | O |
| Me | $CHMeCH_2OH$ | 0 | $CH_2$ |
| Me | $CHMeCH_2OH$ | 1 | $CH_2$ |
| Me | $CHMeCH_2OMe$ | 0 | O |
| Me | $CHMeCH_2OMe$ | 1 | O |
| Me | $CHMeCH_2OMe$ | 0 | $CH_2$ |
| Me | $CHMeCH_2OMe$ | 1 | $CH_2$ |
| Me | $CHMeCH=CBr_2$ | 0 | O |
| Me | $CHMeCH=CBr_2$ | 1 | O |
| Me | $CHMeCH=CCl_2$ | 0 | O |
| Me | $CHMeCH=CCl_2$ | 1 | O |
| Me | $CHMeCH_2CN$ | 0 | O |
| Me | $CHMeCH_2CN$ | 1 | O |

Table 3-b

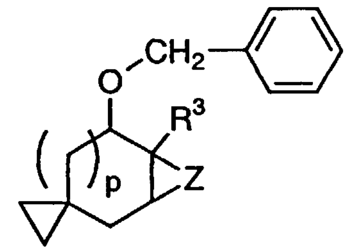 , 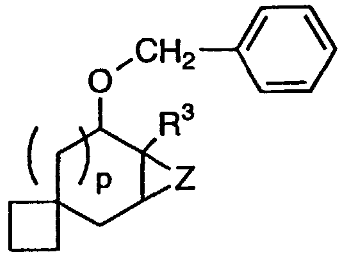 ,

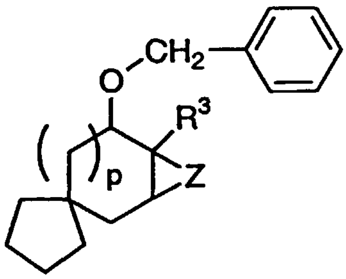 , 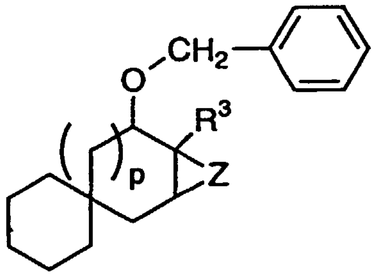 ,

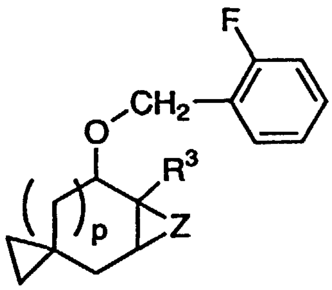 , 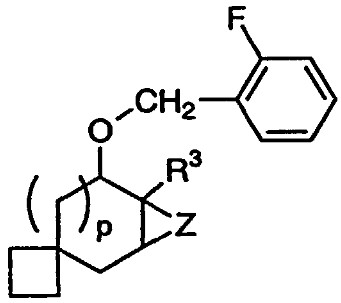 ,

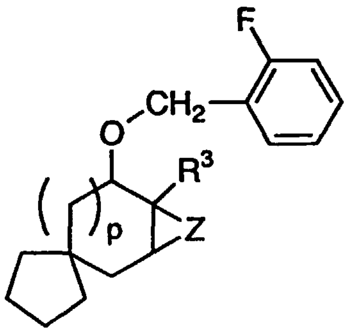 , 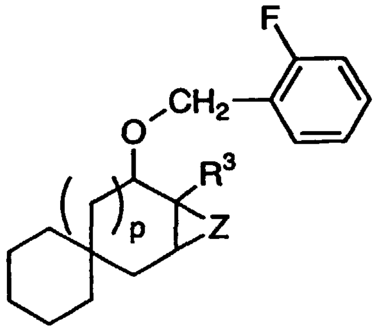 ;

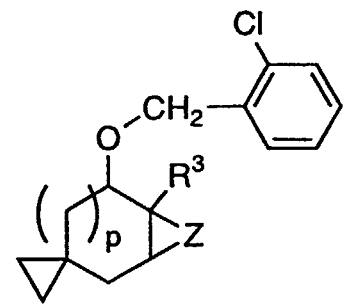

,

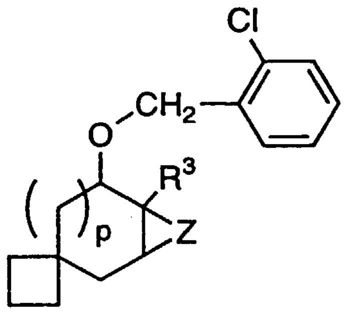

,

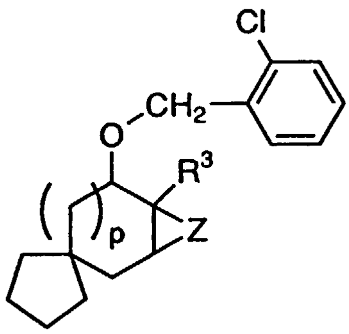

,

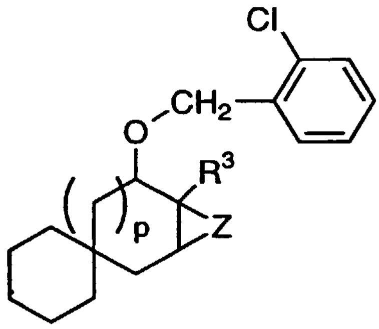

,

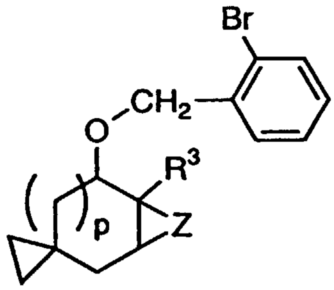

,

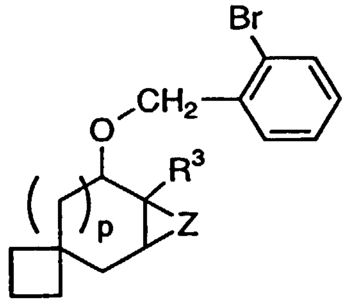

,

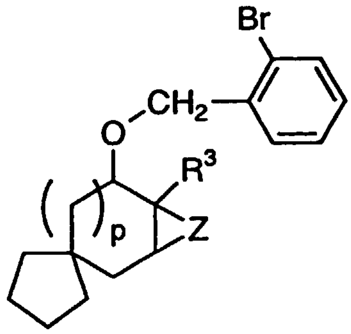

,

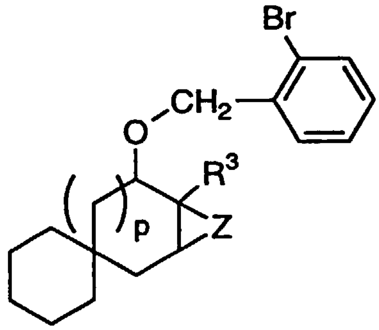

;

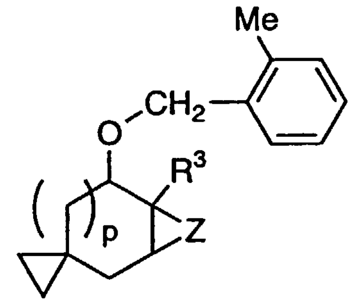

,

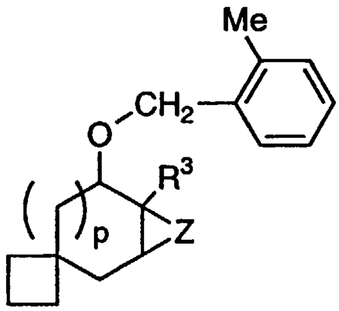

,

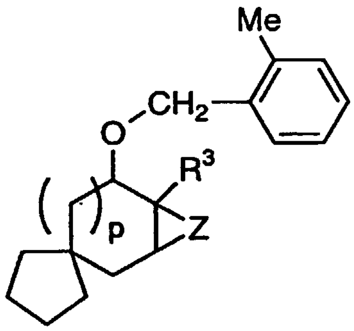

,

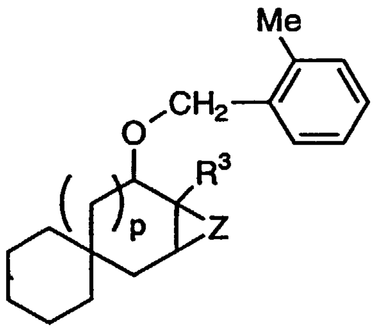

,

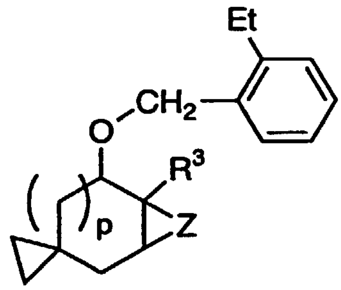

,

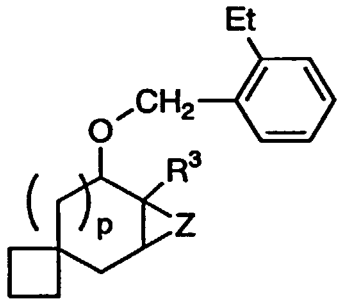

,

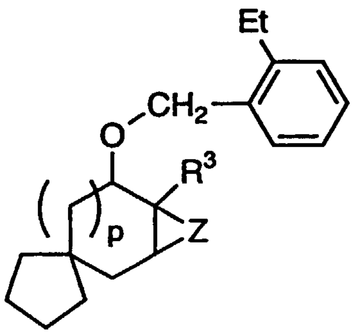

,

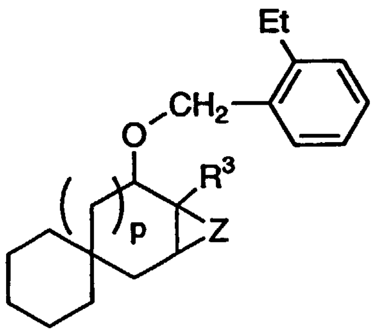

;

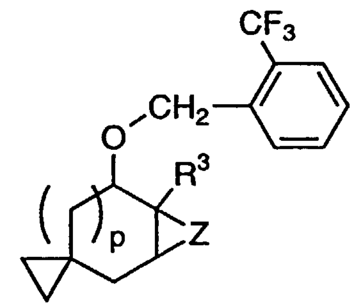 , 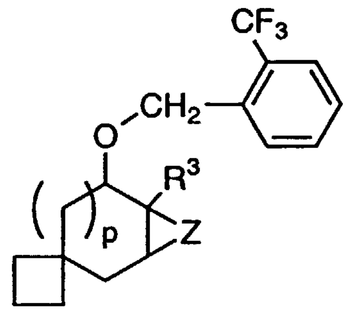 ,

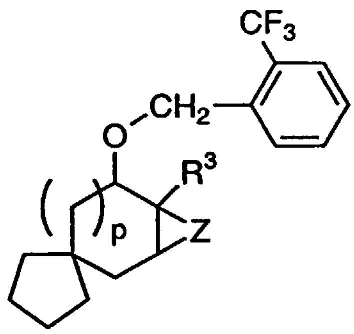 , 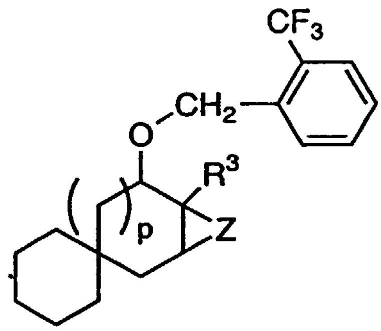 ,

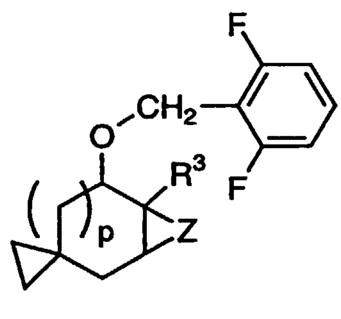 , 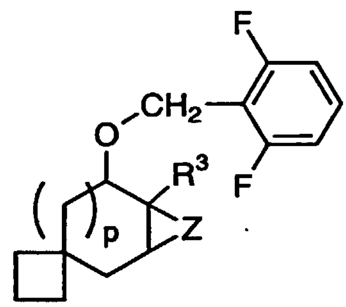 ,

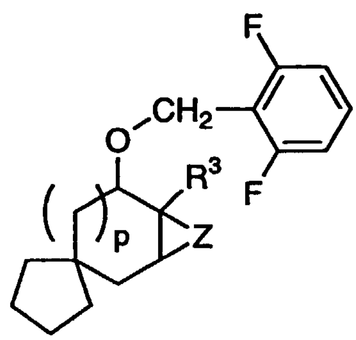 , 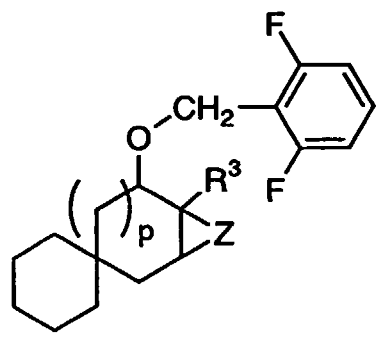 ,

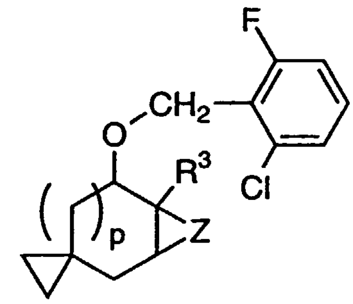 ,

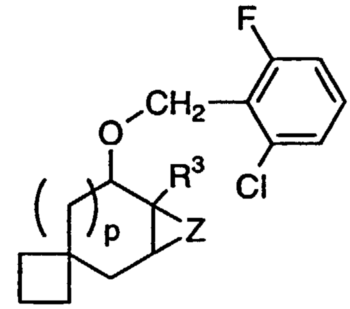 ,

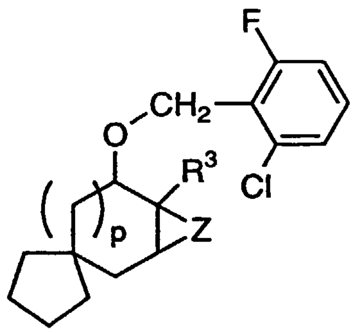 ,

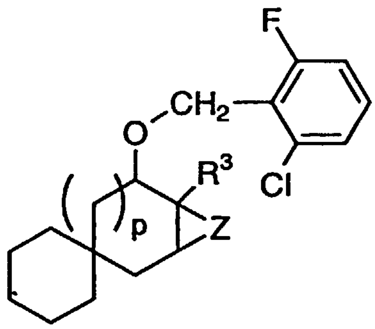 ,

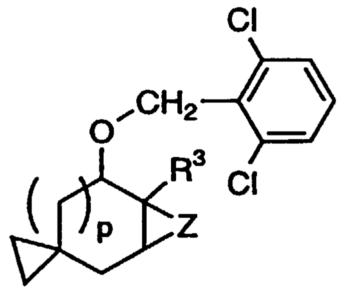 ,

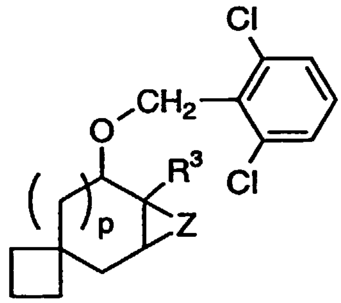 ,

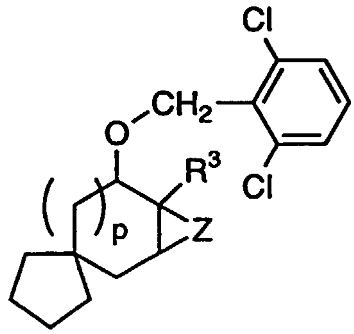 ,

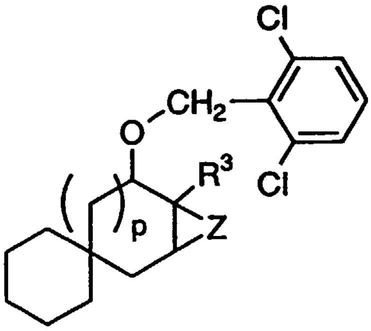 ,

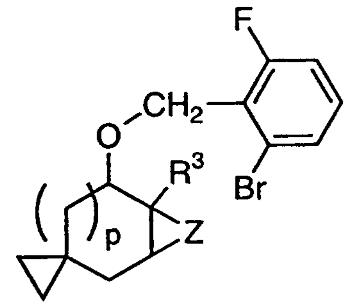

,

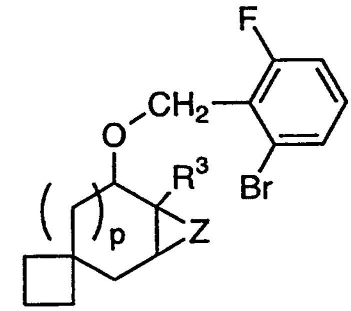

,

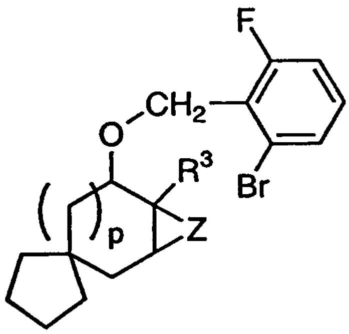

,

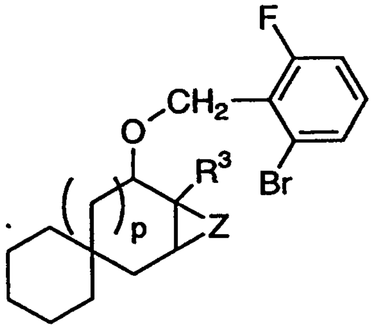

,

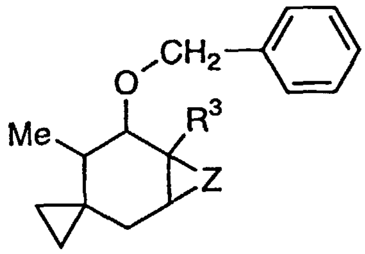
,
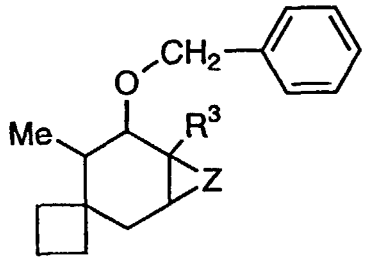
,

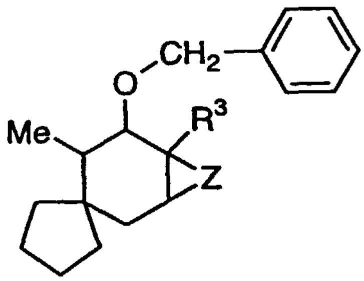
,
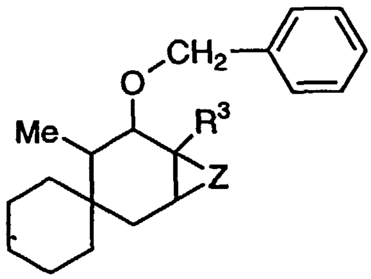
,

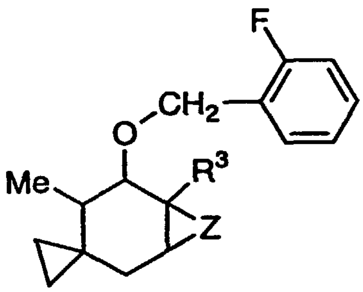
,
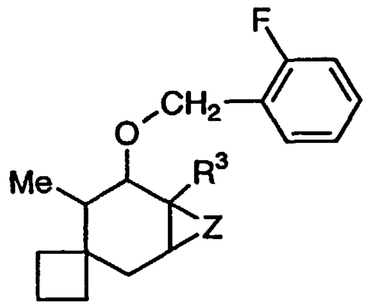
,

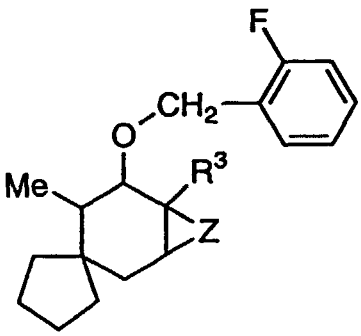
,
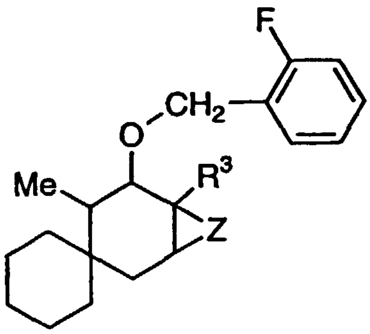
,

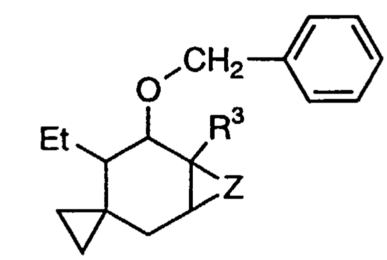
,
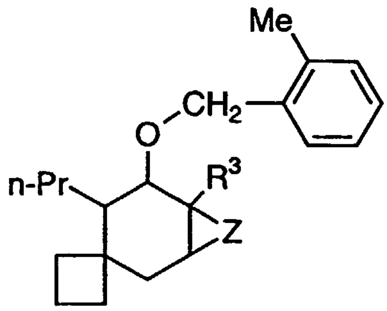
,

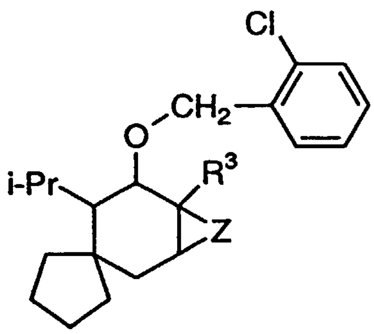
,
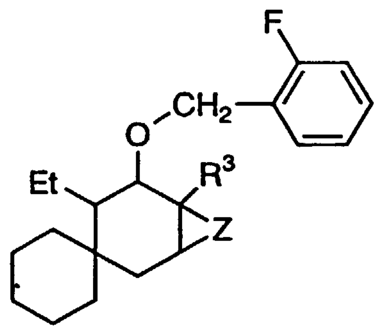
,

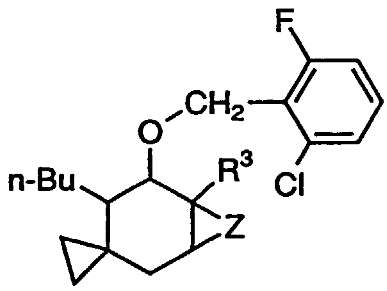
,
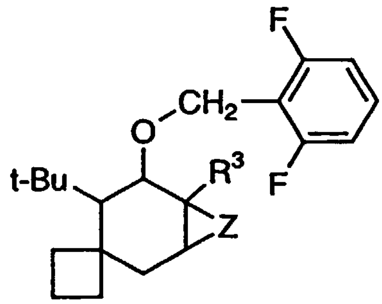
,

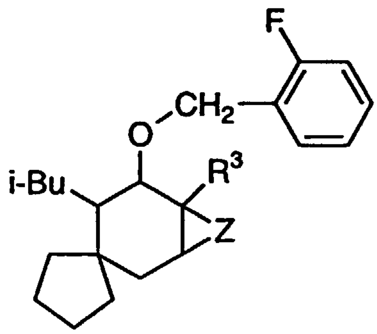
,
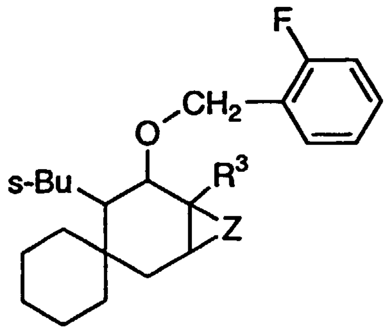
,

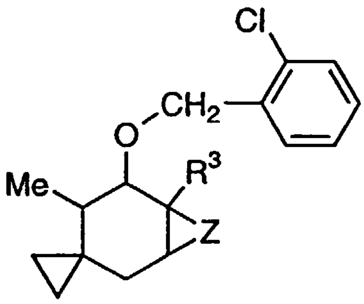 , 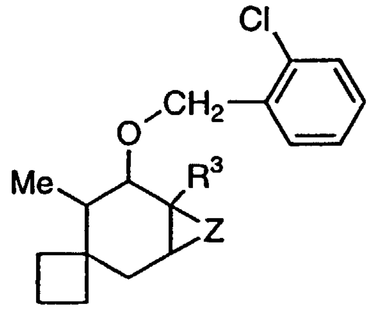 ,

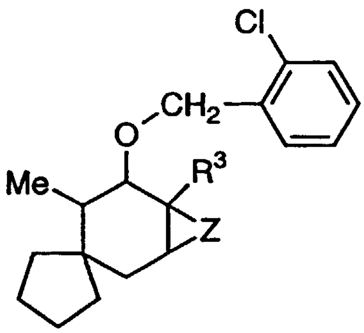 , 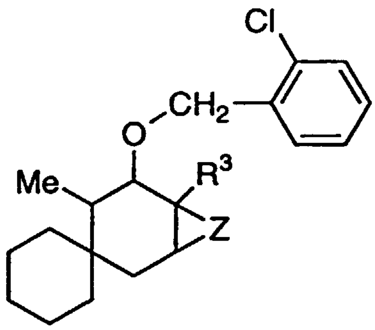 ,

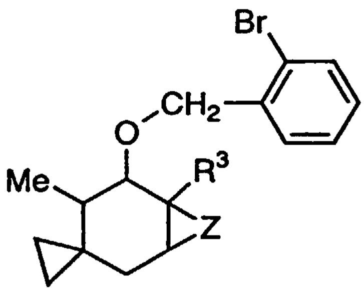 , 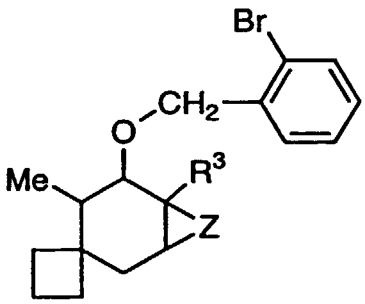 ,

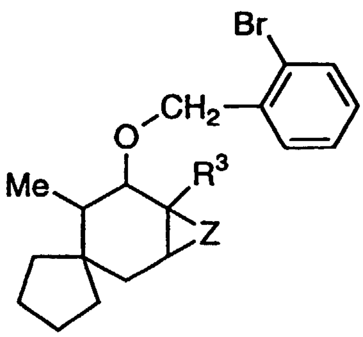 , 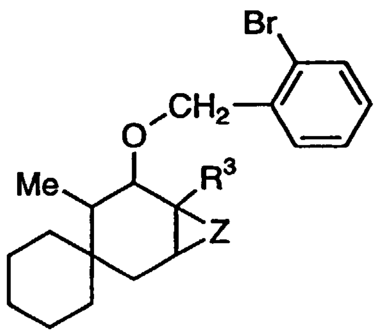 ;

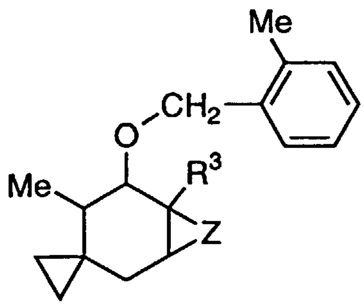

,

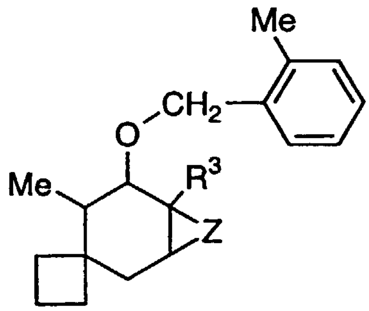

,

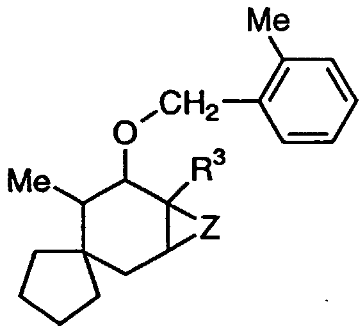

,

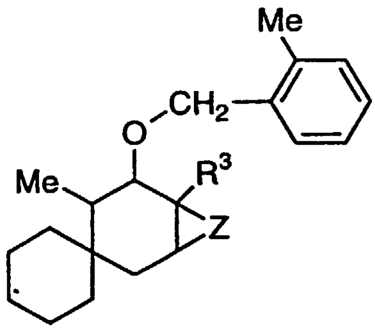

,

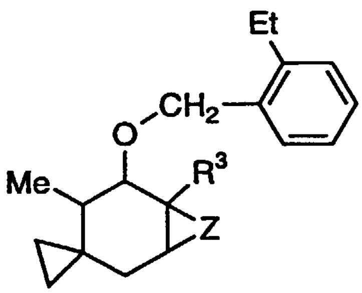

,

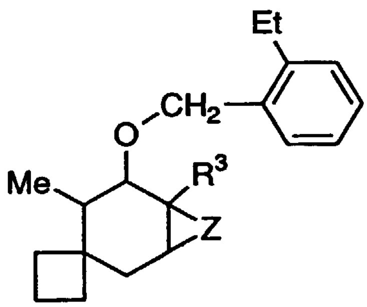

,

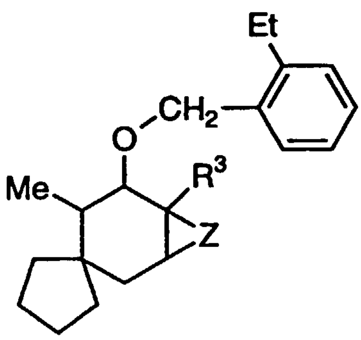

,

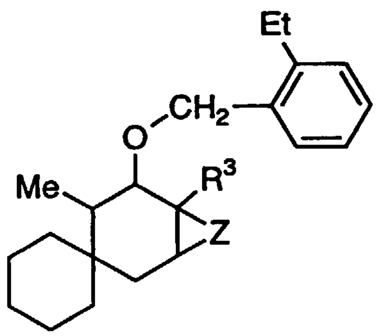

;

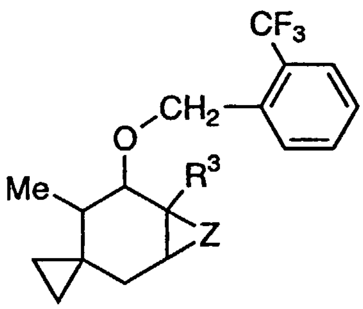 , 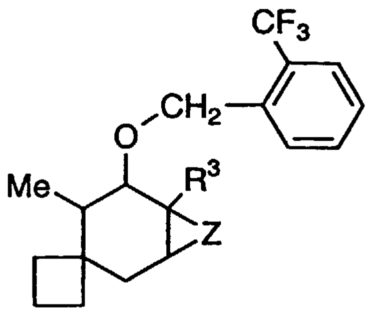 ,

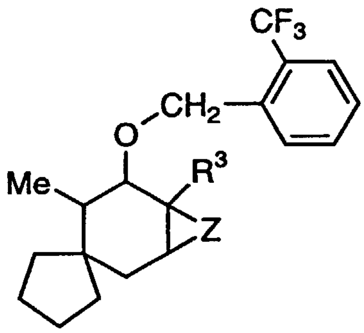 , 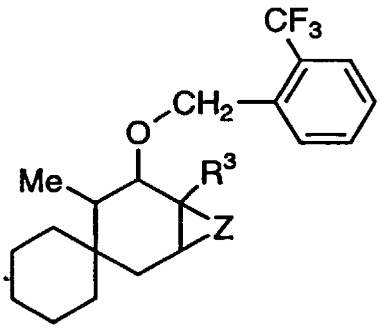 ,

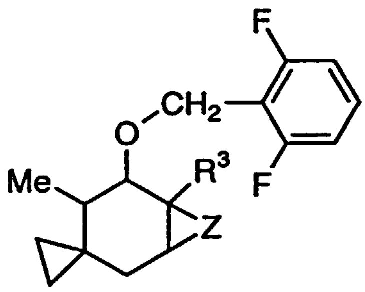 , 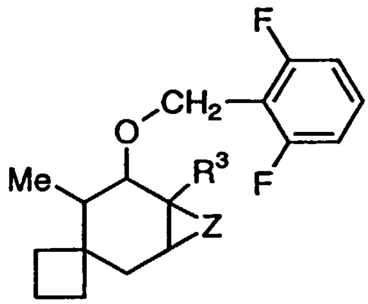 ,

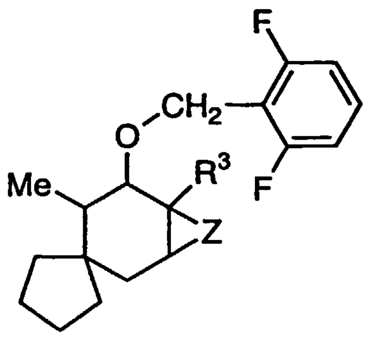 , 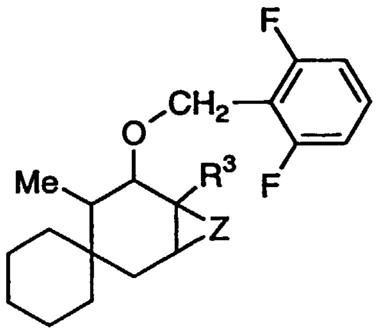 ,

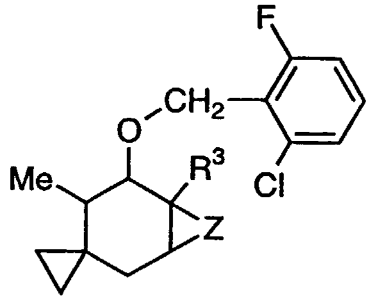 , 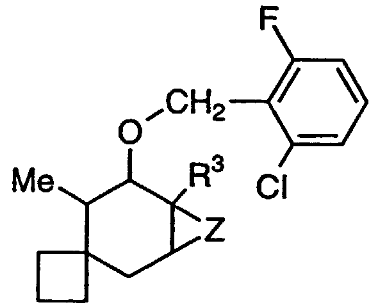 ,

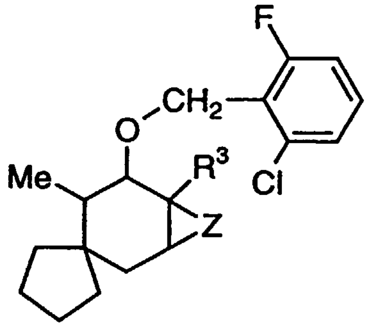 , 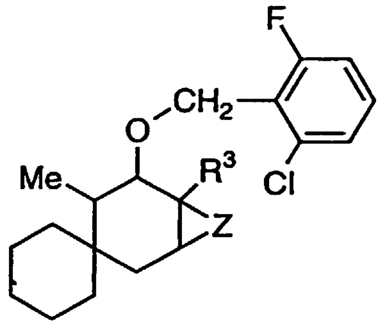 ,

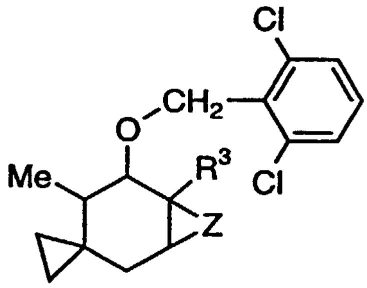 , 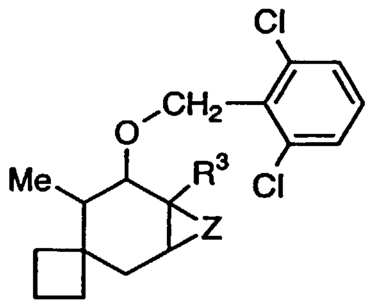 ,

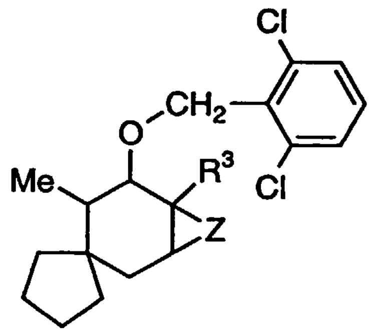 , 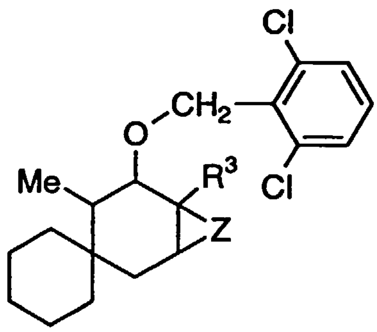 ,

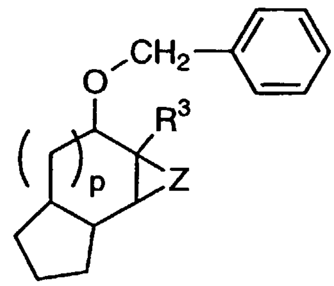 , 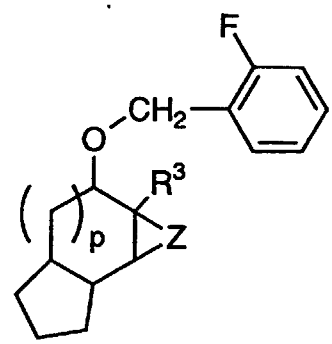 ,

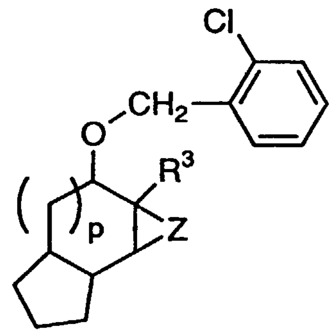 , 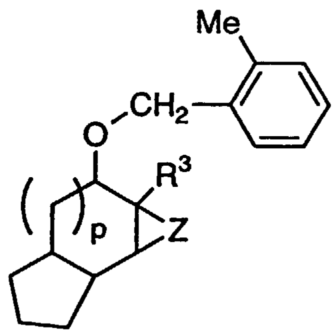 ,

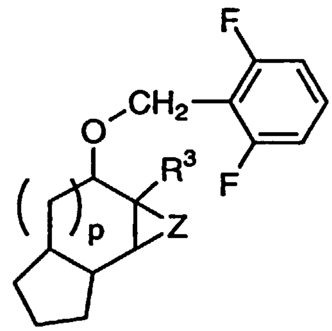 , 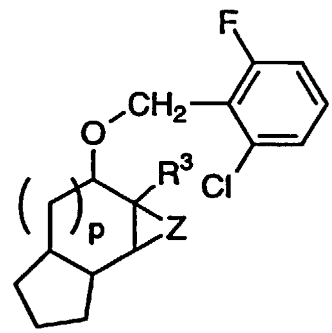 ,

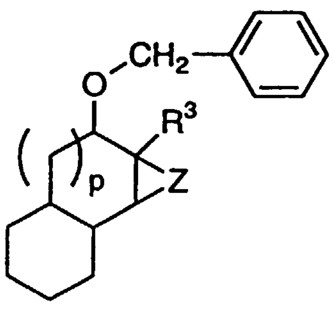 , 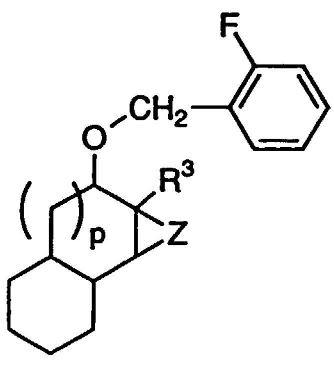 ;

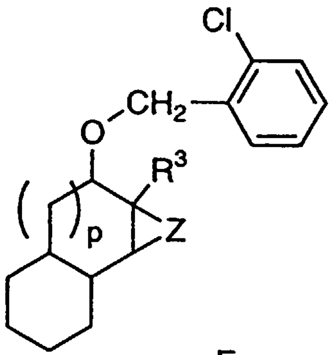

,

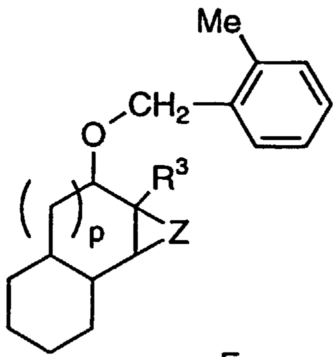

,

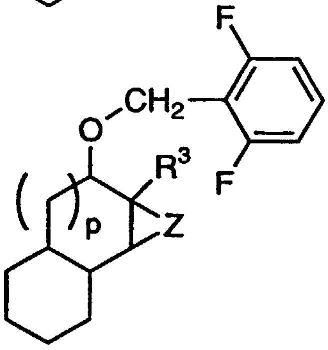

,

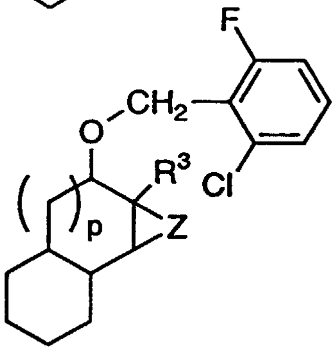

,

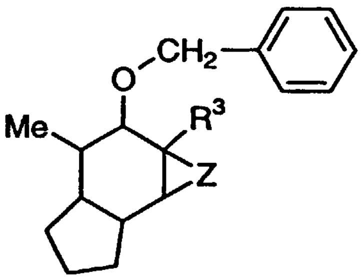

,

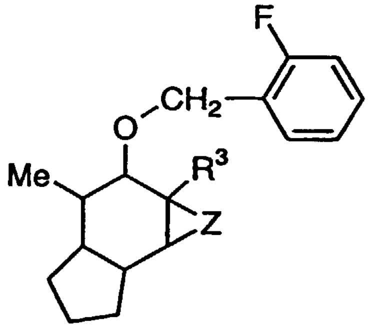

,

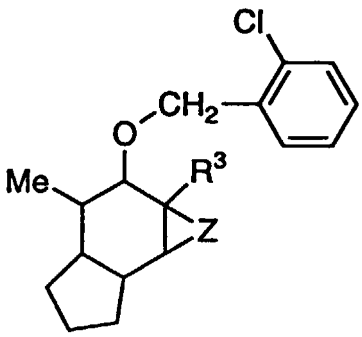

,

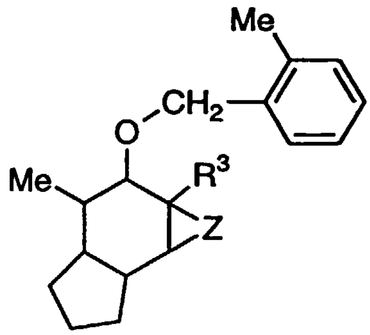

;

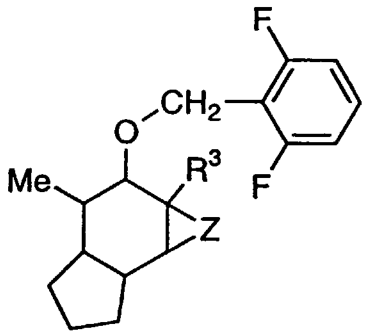 , 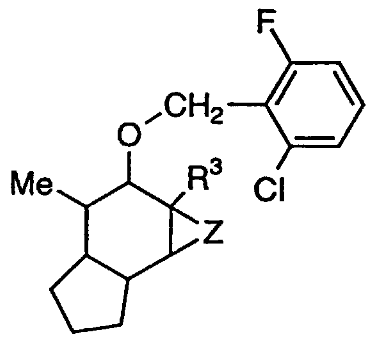 ,

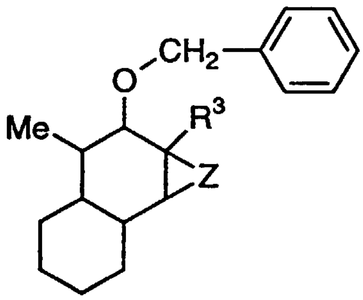 , 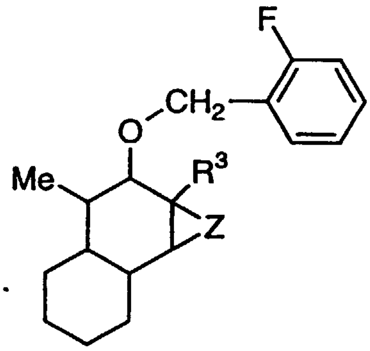 ,

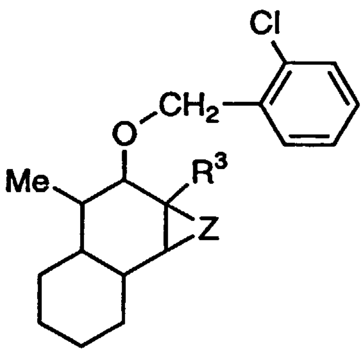 , 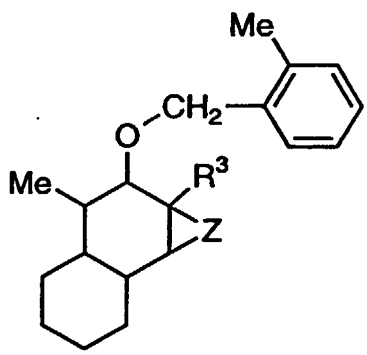 ,

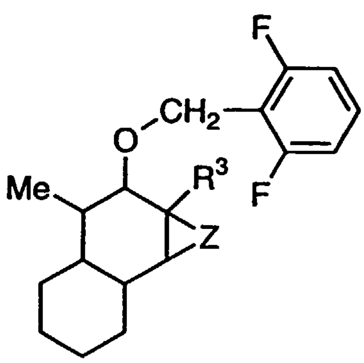 , 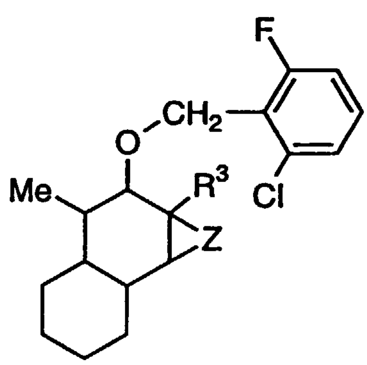 ;

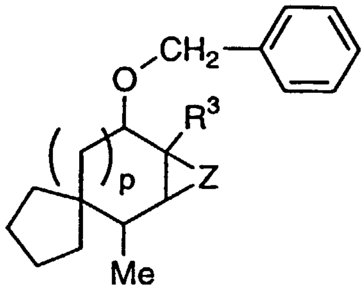 , 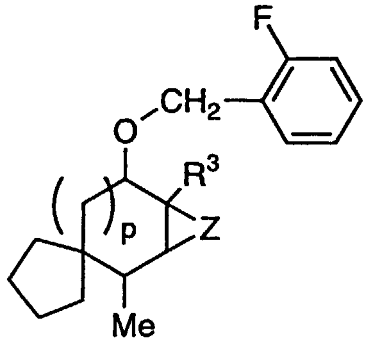 ,

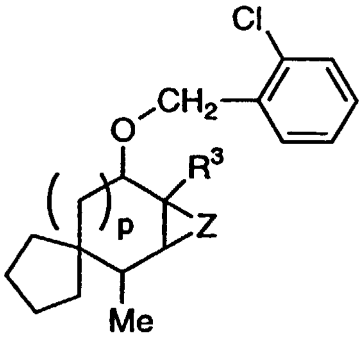 , 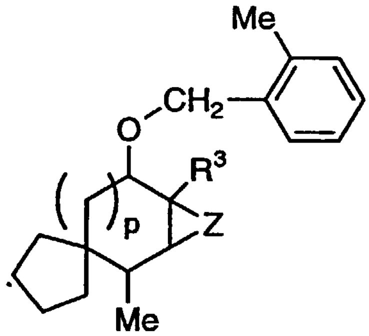 ,

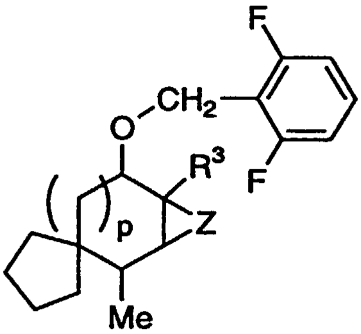 , 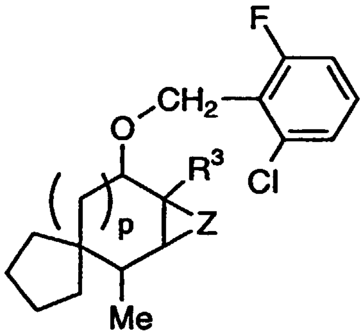 ,

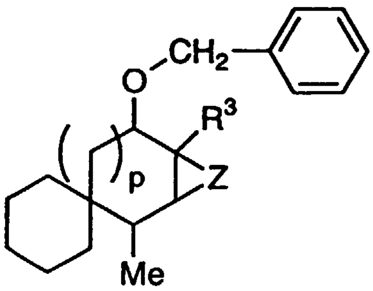 , 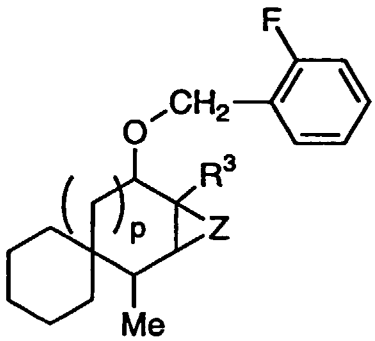 ,

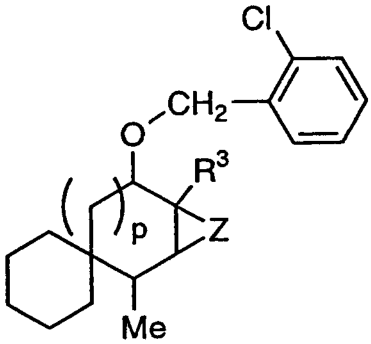

,

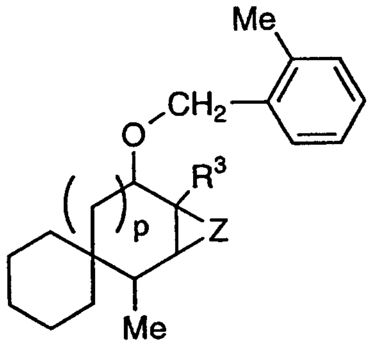

,

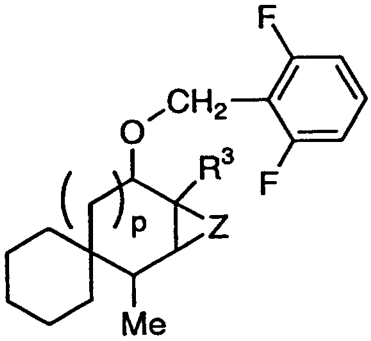

,

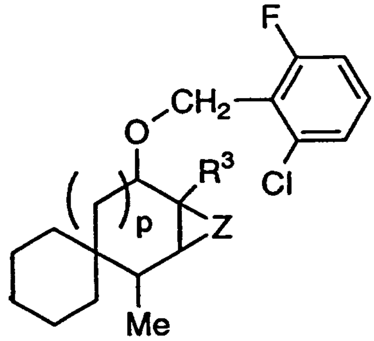

,

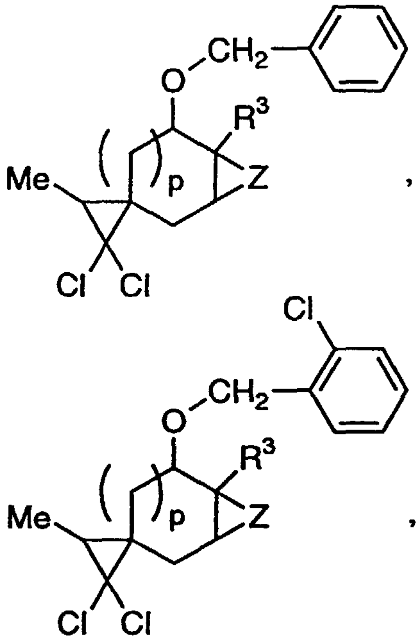

,

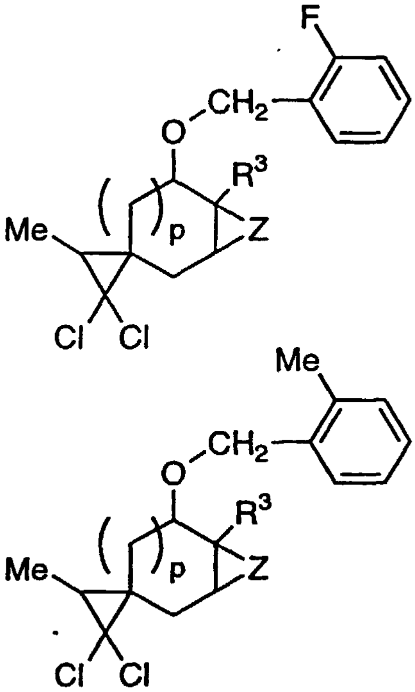

,

,

,

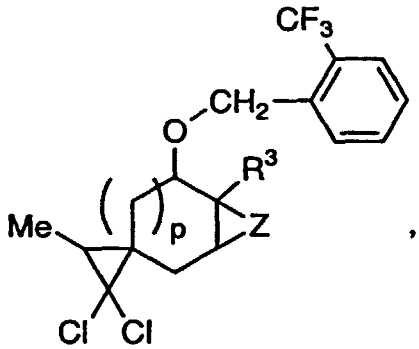

,

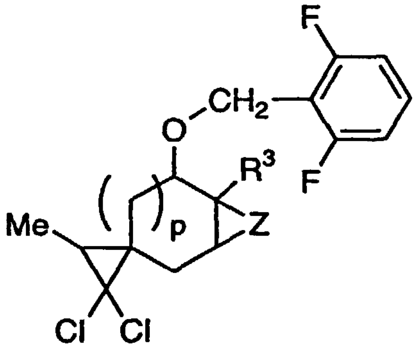

,

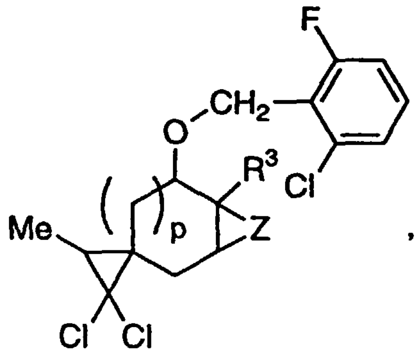

,

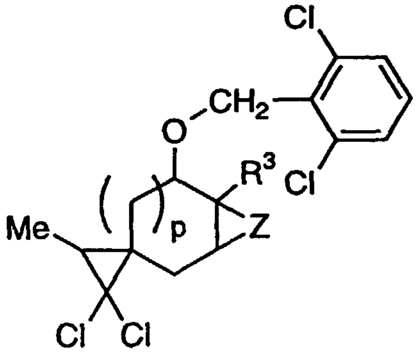

,

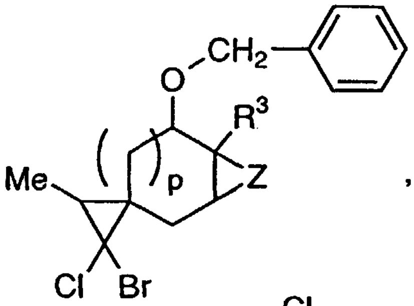 , 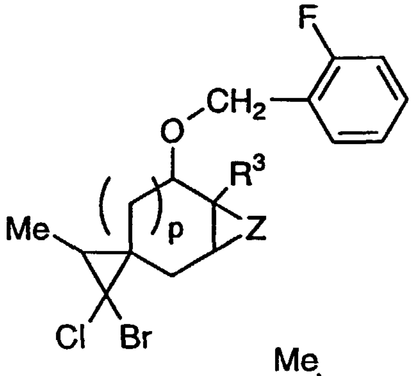 ,

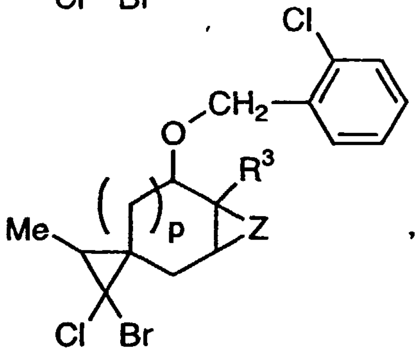 , 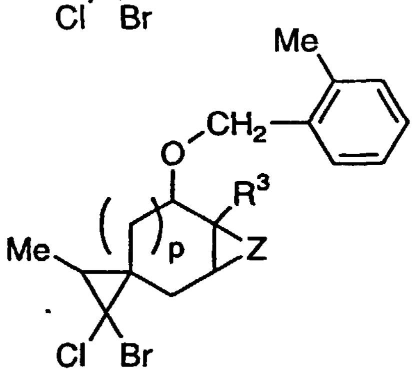 ,

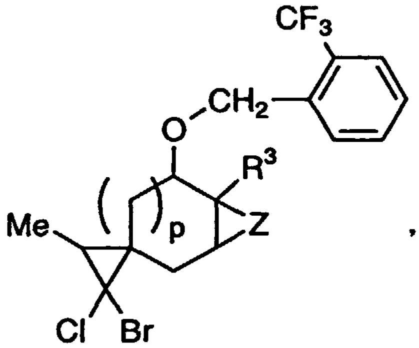 , 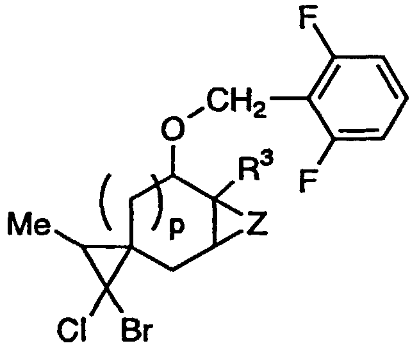 ,

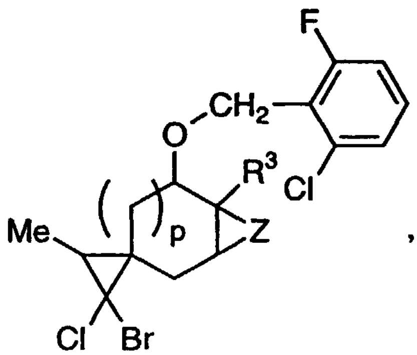 , 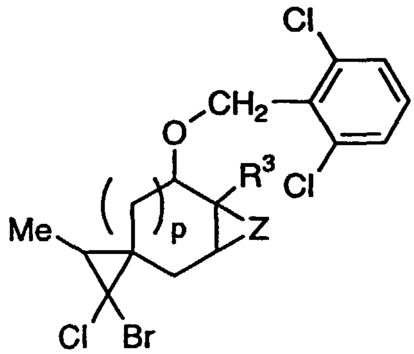 ;

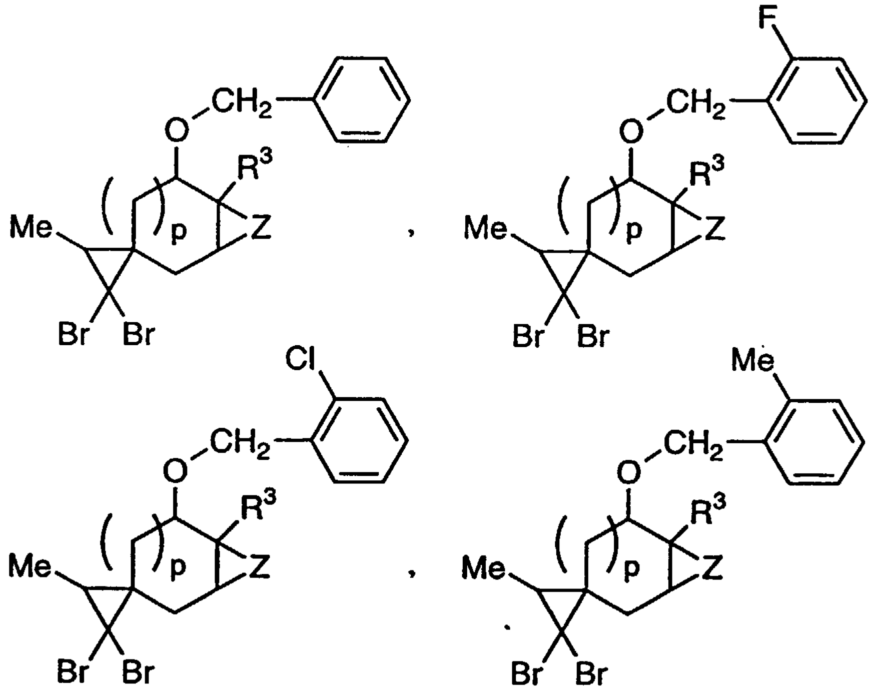

, , , ,

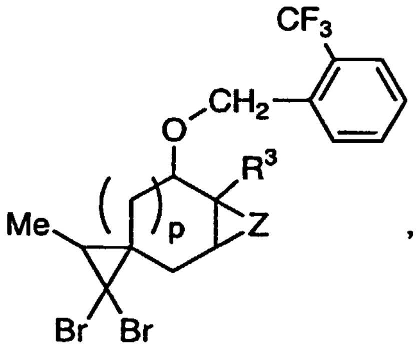

,

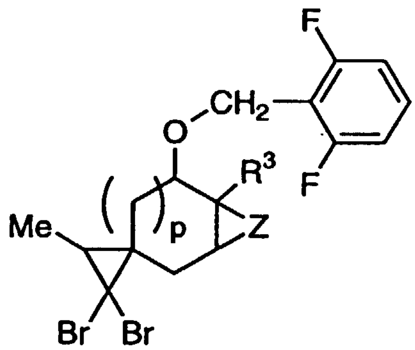

,

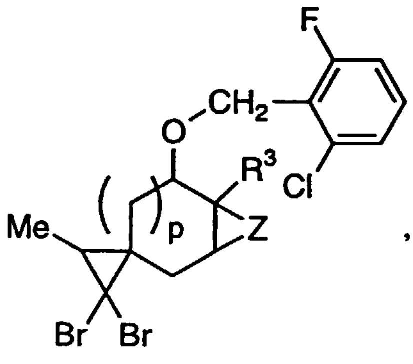

,

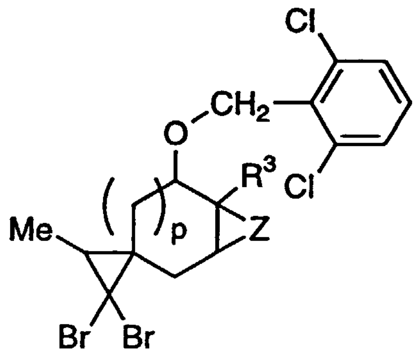

,

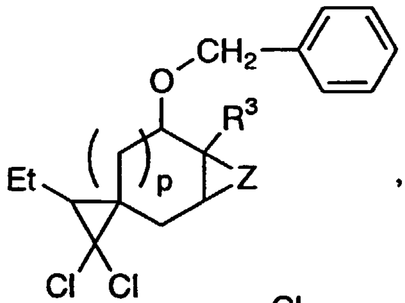 ,

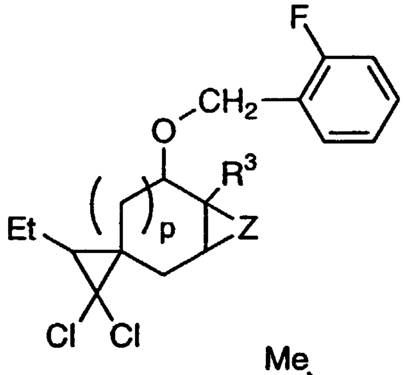 ,

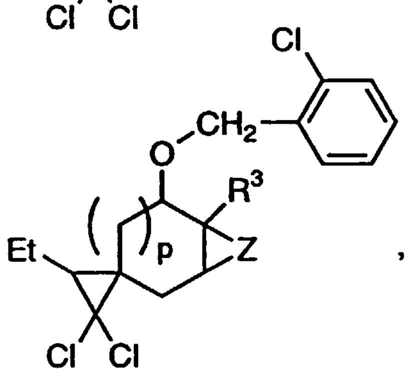 ,

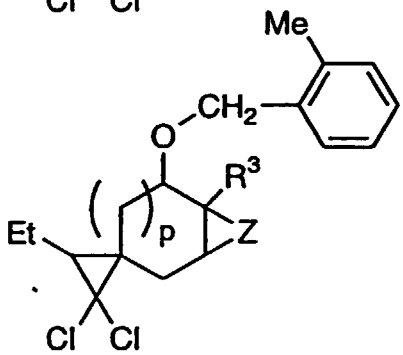 ,

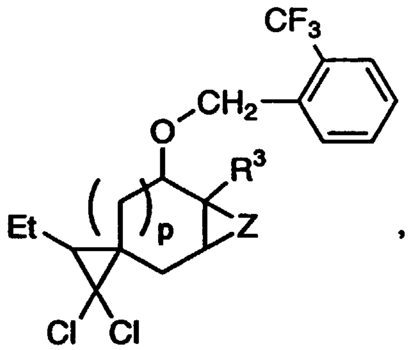 ,

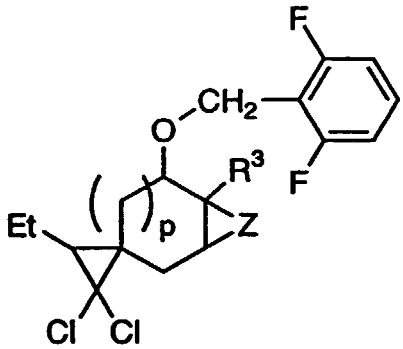 ,

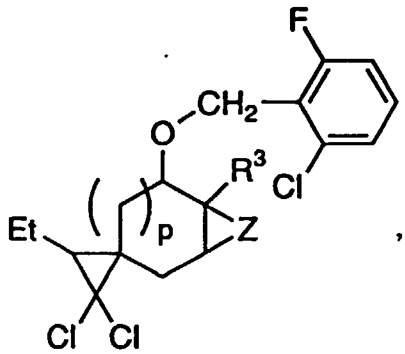 ,

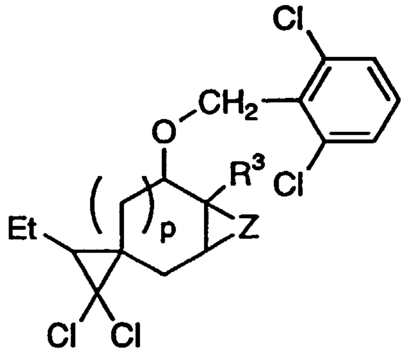 ;

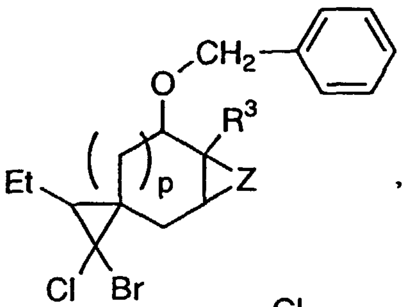
,
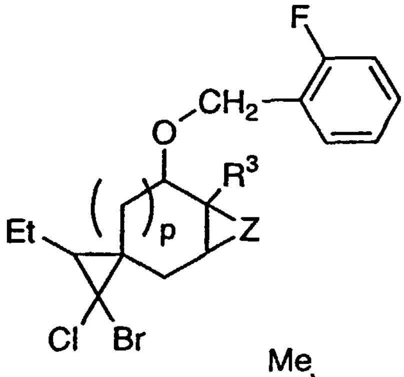
,
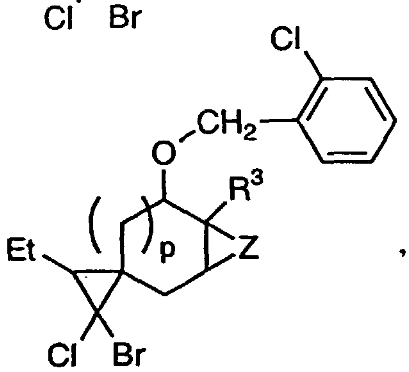
,
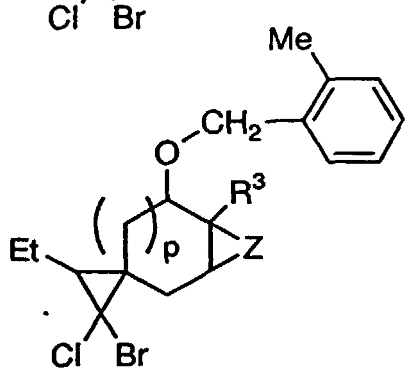
,
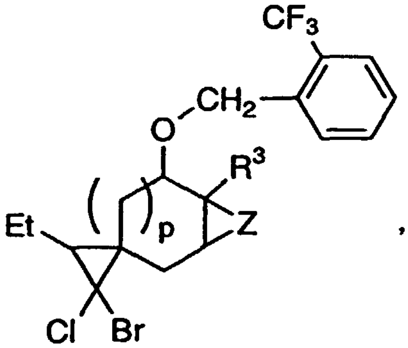
,
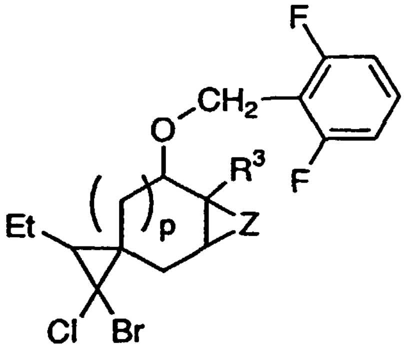
,
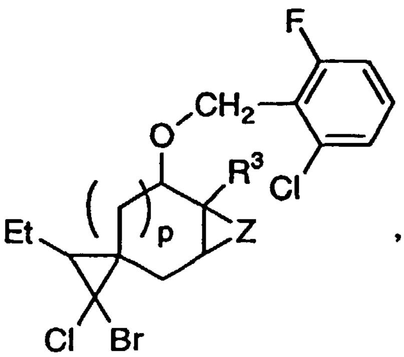
,
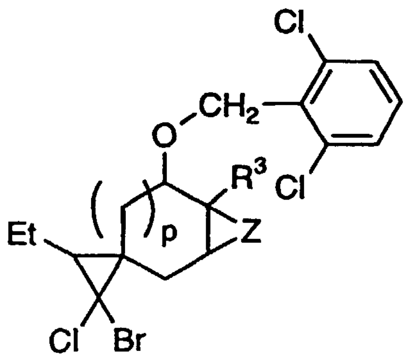
;

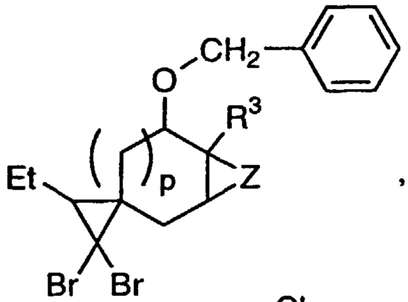 , 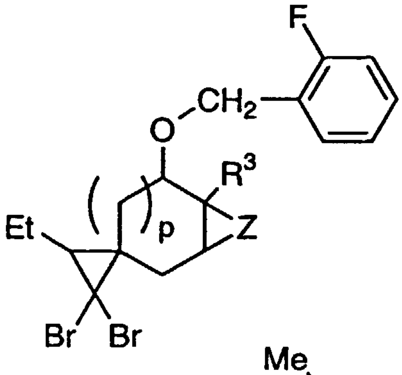 ,

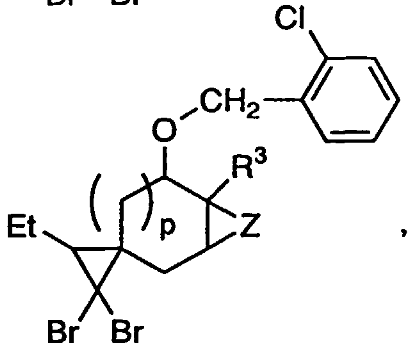 , 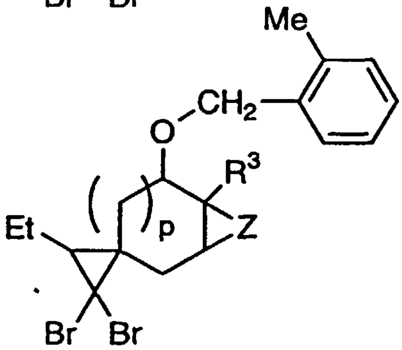 ,

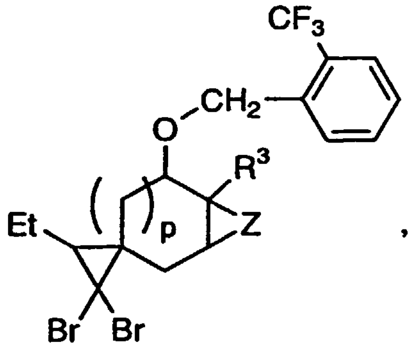 , 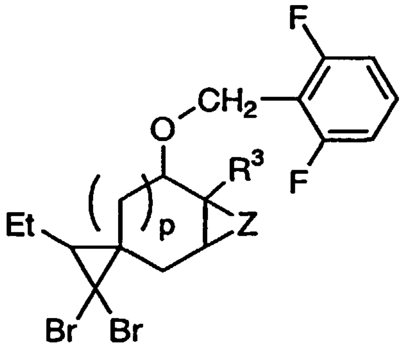 ,

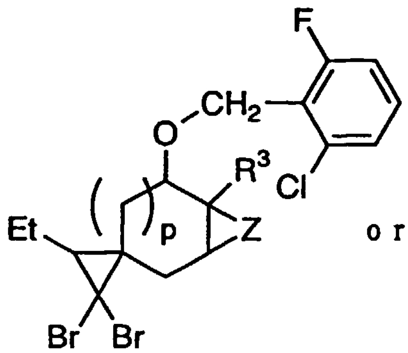 or 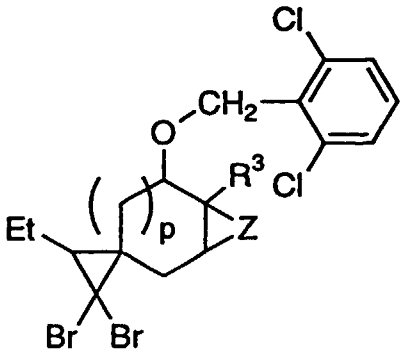 ,

| $R^3$ | p | Z |
|---|---|---|
| Me | 1 | O |
| Et | 1 | O |
| n-Pr | 1 | O |
| i-Pr | 1 | O |
| n-Bu | 1 | O |
| i-Bu | 1 | O |
| n-Pen | 1 | O |
| n-Hex | 1 | O |
| $CH_2CH=CH_2$ | 1 | O |
| $CH_2CH=CHMe$ | 1 | O |
| $CH_2CH=CHi\text{-}Pr$ | 1 | O |
| $CH_2Cl$ | 1 | O |
| $(CH_2)_2Cl$ | 1 | O |
| $CH_2CHBrCH_2Br$ | 1 | O |
| $(CH_2)_3F$ | 1 | O |
| $(CH_2)_4Cl$ | 1 | O |
| $(CH_2)_5Br$ | 1 | O |
| $(CH_2)_6Cl$ | 1 | O |
| $CH_2OMe$ | 1 | O |
| $(CH_2)_2OEt$ | 1 | O |
| CHMeOn-Bu | 1 | O |
| $(CH_2)_3OMe$ | 1 | O |
| $(CH_2)_4Oi\text{-}Pr$ | 1 | O |

| $R^3$ | p | Z |
|---|---|---|
| $(CH_2)_5OEt$ | 1 | O |
| $(CH_2)_6OMe$ | 1 | O |
| Ph | 1 | O |
| 2-Me-Ph | 1 | O |
| 4-Br-Ph | 1 | O |
| 3-$CF_3$-Ph | 1 | O |
| 2-F-4-Cl-Ph | 1 | O |
| 2,3,4,5,6-$F_5$-Ph | 1 | O |
| 4-$C_2F_5$-Ph | 1 | O |
| 4-On-Bu-Ph | 1 | O |
| 4-CN-Ph | 1 | O |
| 4-$NO_2$-Ph | 1 | O |
| 2-$CO_2H$-Ph | 1 | O |
| 2-$CO_2$t-Bu-Ph | 1 | O |
| $CH_2Ph$ | 1 | O |
| $CH_2$-4-t-Bu-Ph | 1 | O |
| CHMe-Ph | 1 | O |
| CHMe-4-Cl-Ph | 1 | O |
| $(CH_2)_2Ph$ | 1 | O |
| $(CH_2)_2$-4-OMe-Ph | 1 | O |
| $(CH_2)_3Ph$ | 1 | O |
| $C(Me)_2Ph$ | 1 | O |

Table 3-b (continued)

| $R^3$ | p | Z | $R^3$ | p | Z |
|---|---|---|---|---|---|
| Me | 0 | O | $(CH_2)_5OEt$ | 0 | O |
| Et | 0 | O | $(CH_2)_6OMe$ | 0 | O |
| n-Pr | 0 | O | Ph | 0 | O |
| i-Pr | 0 | O | 2-Me-Ph | 0 | O |
| n-Bu | 0 | O | 4-Br-Ph | 0 | O |
| i-Bu | 0 | O | 3-$CF_3$-Ph | 0 | O |
| n-Pen | 0 | O | 2-F-4-Cl-Ph | 0 | O |
| n-Hex | 0 | O | 2,3,4,5,6-$F_5$-Ph | 0 | O |
| $CH_2CH=CH_2$ | 0 | O | 4-$C_2F_5$-Ph | 0 | O |
| $CH_2CH=CHMe$ | 0 | O | 4-On-Bu-Ph | 0 | O |
| $CH_2CH=CH$i-Pr | 0 | O | 4-CN-Ph | 0 | O |
| $CH_2Cl$ | 0 | O | 4-$NO_2$-Ph | 0 | O |
| $(CH_2)_2Cl$ | 0 | O | 2-$CO_2H$-Ph | 0 | O |
| $CH_2CHBrCH_2Br$ | 0 | O | 2-$CO_2$t-Bu-Ph | 0 | O |
| $(CH_2)_3F$ | 0 | O | $CH_2Ph$ | 0 | O |
| $(CH_2)_4Cl$ | 0 | O | $CH_2$-4-t-Bu-Ph | 0 | O |
| $(CH_2)_5Br$ | 0 | O | CHMe-Ph | 0 | O |
| $(CH_2)_6Cl$ | 0 | O | CHMe-4-Cl-Ph | 0 | O |
| $CH_2OMe$ | 0 | O | $(CH_2)_2Ph$ | 0 | O |
| $(CH_2)_2OEt$ | 0 | O | $(CH_2)_2$-4-OMe-Ph | 0 | O |
| CHMeOn-Bu | 0 | O | $(CH_2)_3Ph$ | 0 | O |
| $(CH_2)_3OMe$ | 0 | O | $C(Me)_2Ph$ | 0 | O |
| $(CH_2)_4$Oi-Pr | 0 | O | | | |

Table 3-b (continued)

| $R^3$ | p | Z | $R^3$ | p | Z |
|---|---|---|---|---|---|
| Me | 1 | $CH_2$ | $(CH_2)_5OEt$ | 1 | $CH_2$ |
| Et | 1 | $CH_2$ | $(CH_2)_6OMe$ | 1 | $CH_2$ |
| n-Pr | 1 | $CH_2$ | Ph | 1 | $CH_2$ |
| i-Pr | 1 | $CH_2$ | 2-Me-Ph | 1 | $CH_2$ |
| n-Bu | 1 | $CH_2$ | 4-Br-Ph | 1 | $CH_2$ |
| i-Bu | 1 | $CH_2$ | $3-CF_3-Ph$ | 1 | $CH_2$ |
| n-Pen | 1 | $CH_2$ | 2-F-4-Cl-Ph | 1 | $CH_2$ |
| n-Hex | 1 | $CH_2$ | $2,3,4,5,6-F_5-Ph$ | 1 | $CH_2$ |
| $CH_2CH=CH_2$ | 1 | $CH_2$ | $4-C_6F_5-Ph$ | 1 | $CH_2$ |
| $CH_2CH=CHMe$ | 1 | $CH_2$ | 4-On-Bu-Ph | 1 | $CH_2$ |
| $CH_2CH=CHi-Pr$ | 1 | $CH_2$ | 4-CN-Ph | 1 | $CH_2$ |
| $CH_2Cl$ | 1 | $CH_2$ | $4-NO_2-Ph$ | 1 | $CH_2$ |
| $(CH_2)_2Cl$ | 1 | $CH_2$ | $2-CO_2H-Ph$ | 1 | $CH_2$ |
| $CH_2CHBrCH_2Br$ | 1 | $CH_2$ | $2-CO_2t-Bu-Ph$ | 1 | $CH_2$ |
| $(CH_2)_3F$ | 1 | $CH_2$ | $CH_2Ph$ | 1 | $CH_2$ |
| $(CH_2)_4Cl$ | 1 | $CH_2$ | $CH_2-4-t-Bu-Ph$ | 1 | $CH_2$ |
| $(CH_2)_5Br$ | 1 | $CH_2$ | CHMe-Ph | 1 | $CH_2$ |
| $(CH_2)_6Cl$ | 1 | $CH_2$ | CHMe-4-Cl-Ph | 1 | $CH_2$ |
| $CH_2OMe$ | 1 | $CH_2$ | $(CH_2)_2Ph$ | 1 | $CH_2$ |
| $(CH_2)_2OEt$ | 1 | $CH_2$ | $(CH_2)_2-4-OMe-Ph$ | 1 | $CH_2$ |
| CHMeOn-Bu | 1 | $CH_2$ | $(CH_2)_3Ph$ | 1 | $CH_2$ |
| $(CH_2)_3OMe$ | 1 | $CH_2$ | $C(Me)_2Ph$ | 1 | $CH_2$ |
| $(CH_2)_4Oi-Pr$ | 1 | $CH_2$ | | | |

Table 3-b (continued)

| $R^3$ | p | Z | $R^3$ | p | Z |
|---|---|---|---|---|---|
| Me | 0 | $CH_2$ | $(CH_2)_5OEt$ | 0 | $CH_2$ |
| Et | 0 | $CH_2$ | $(CH_2)_6OMe$ | 0 | $CH_2$ |
| n-Pr | 0 | $CH_2$ | Ph | 0 | $CH_2$ |
| i-Pr | 0 | $CH_2$ | 2-Me-Ph | 0 | $CH_2$ |
| n-Bu | 0 | $CH_2$ | 4-Br-Ph | 0 | $CH_2$ |
| i-Bu | 0 | $CH_2$ | $3-CF_3-Ph$ | 0 | $CH_2$ |
| n-Pen | 0 | $CH_2$ | 2-F-4-Cl-Ph | 0 | $CH_2$ |
| n-Hex | 0 | $CH_2$ | $2,3,4,5,6-F_5-Ph$ | 0 | $CH_2$ |
| $CH_2CH=CH_2$ | 0 | $CH_2$ | $4-C_2F_5-Ph$ | 0 | $CH_2$ |
| $CH_2CH=CHMe$ | 0 | $CH_2$ | 4-On-Bu-Ph | 0 | $CH_2$ |
| $CH_2CH=CHi-Pr$ | 0 | $CH_2$ | 4-CN-Ph | 0 | $CH_2$ |
| $CH_2Cl$ | 0 | $CH_2$ | $4-NO_2-Ph$ | 0 | $CH_2$ |
| $(CH_2)_2Cl$ | 0 | $CH_2$ | $2-CO_2H-Ph$ | 0 | $CH_2$ |
| $CH_2CHBrCH_2Br$ | 0 | $CH_2$ | $2-CO_2t-Bu-Ph$ | 0 | $CH_2$ |
| $(CH_2)_3F$ | 0 | $CH_2$ | $CH_2Ph$ | 0 | $CH_2$ |
| $(CH_2)_4Cl$ | 0 | $CH_2$ | $CH_2-4-t-Bu-Ph$ | 0 | $CH_2$ |
| $(CH_2)_5Br$ | 0 | $CH_2$ | CHMe-Ph | 0 | $CH_2$ |
| $(CH_2)_6Cl$ | 0 | $CH_2$ | CHMe-4-Cl-Ph | 0 | $CH_2$ |
| $CH_2OMe$ | 0 | $CH_2$ | $(CH_2)_2Ph$ | 0 | $CH_2$ |
| $(CH_2)_2OEt$ | 0 | $CH_2$ | $(CH_2)_2-4-OMe-Ph$ | 0 | $CH_2$ |
| CHMeOn-Bu | 0 | $CH_2$ | $(CH_2)_3Ph$ | 0 | $CH_2$ |
| $(CH_2)_3OMe$ | 0 | $CH_2$ | $C(Me)_2Ph$ | 0 | $CH_2$ |
| $(CH_2)_4Oi-Pr$ | 0 | $CH_2$ | | | |

When the compound of the present invention is applied as a herbicide, it can generally be applied as a mixture with a suitable carrier, for example, a solid carrier such as clay, talc, bentonite, diatomaceous earth, and white carbon, or a liquid carrier such as water, alcohols (isopropanol, butanol, benzyl alcohol, furfuryl alcohol, etc.), aromatic hydrocarbons (toluene, xylene, etc.), ethers (anisole, etc.), ketones (cyclohexanone, isophorone, etc.), esters (butyl acetate, etc.) , acid amides (N-methylpyrrolidone, etc.), or halogenated hydrocarbons (chlorobenzene, etc.). If desired, surfactants, emulsifiers, dispersants, penetrants, spreaders, thickeners, antifreezing agents, antisolidifying agents, stabilizers, etc. can be added and it can be put into practical use in any formulation such as liquid formulation, emulsifiable concentrate, wettable powder, dry flowable powder, flowable concentrate, dust, granule, etc.

The compound of the present invention may, if desired, be mixed with other herbicides, various types of insecticides, bactericides, plant growth regulators, synergists, etc. upon formulation or application.

In particular, by application as mixtures with other herbicides, there can be expected reduction in cost due to a decrease in application rate, expansion of herbicidal spectrum due to synergism between mixed chemicals, or higher herbicidal effect. It is expected that some combinations might alleviate phytotoxicity on crop. In this case, it is possible to combine the compound of the present invention with a plurality of known herbicides simultaneously. The kind of the herbicide to be combined with the compound of the present invention includes, for example, compounds described in Farm Chemicals Handbook, 1997.

As the preferable herbicide to be used after being mixed with the compound of the present invention, the following ones can be mentioned:

pyrazosulfuron ethyl (common name), halosulfuron methyl (common name), bensulfuron methyl (common name), cinosulfuron (common name), azimsulfuron (common name), imazosulfuron (common name), cyclosulfamuron (common name), ethoxysulfuron (common name), pretilachlor (common name), esprocarb (common name), pyrazolate (common name), pyrazoxyfen (common name), benzofenap (common name), daimuron (common name), bromobutide (common name), naproanilide (common name), clomeprop (common name), CNP (chlornitrofen (common name)), chlomethoxynil (common name), bifenox (common name), oxadiazon (common name), oxadiargyl (common name), cafenstrole (common name), MY-100 (test name), indanofan (common name), pentoxazone (common name), pyriminobac meytyl (common name), cyhalofop butyl (common name), NBA-061 (test name), mefenacet (common name), butachlor (common name), butenachlor (common name), thenylchlor (common name), dithiopyr (common name), benfuresate (common name), pyributicarb (common name), benthiocarb (common name), dimepiperate (common name), molinate (common name), butamifos (common name), quinclorac (common name), cinmethylin (common name), simetryn (common name), SAP (common name), dimethametryn (common name), MCPA (common name), MCPB (common name), etobenzanid (common name), cumyluron (common name), SB-500 (test name), MT-147 (test name), KUH-913 (test name), anilofos (common name), bentazone (common name), carfentrazone ethyl) and quinoclamine (common name), etc..

The application dosage of the compound of the present invention as a herbicide may vary depending on the situation of application, time of application, method of application, cultivated crop, etc. but, generally, it is suitably on the order of 0.0001 to 10 kg, preferably on the order of 0.001 to 5 kg as the amount of effective ingredient per hectare (ha).

Next, composition examples for formulation in which the compound of the present invention (in the formulae examples, referred to as "Inventive Compound")is used will be shown concretely. However, the composition example of the present invention is not limited thereto. In the following formulae, " part(s)" means part(s) by weight.

| [Wettable powder] | |
|---|---|
| Inventive Compound | 5 to 80 parts |
| Solid carrier | 10 to 85 parts |
| Surfactant | 1 to 10 parts |
| Others | 1 to 5 parts |
| Others include, for example, an antisolidifying agent. | |

| [Emulsifiable concentrate] | |
|---|---|
| Inventive Compound | 1 to 30 parts |

(continued)

| [Emulsifiable concentrate] | |
|---|---|
| Liquid carrier | 55 to 95 parts |
| Surfactant | 4 to 15 parts |

| [Flowable concentrate] | |
|---|---|
| Inventive Compound | 5 to 70 parts |
| Liquid carrier | 15 to 65 parts |
| Surfactant | 5 to 12 parts |
| Others | 5 to 30 parts |
| Others include, for example, an antifreezing agent, a thickener, etc. | |

| [Granulate wettable powder (dry flowable powder)] | |
|---|---|
| Inventive Compound | 20 to 90 parts |
| Solid carrier | 9 to 60 parts |
| Surfactant | 1 to 20 parts |

| [Granule] | |
|---|---|
| Inventive Compound | 0.01 to 10 parts |
| Solid carrier | 90 to 99.99 parts |
| Others | 0 to 5 parts |

| [Composition Example 1] Wettable powder | |
|---|---|
| Inventive Compound A-5 | 50 parts |
| Zeeklite PF (Kaolin type clay: Zeeklite Kogyo Co., Ltd., trade name) | 43 parts |
| Sorpol 5050 (Anionic surfactant: Toho Chemical Industry Co., Ltd., trade name) | 2 parts |
| Runox 1000C (Anionic surfactant: Toho Chemical Industry Co., Ltd., trade name) | 3 parts |

(continued)

| [Composition Example 1] Wettable powder | |
|---|---|
| Carplex #80 (antisolidifying agent) (White carbon: Shionogi & Co., Ltd., trade name) | 2 parts |
| The above are uniformly mixed and pulverized to form a wettable powder. | |

| [Composition Example 2] Emulsifiable concentrate | |
|---|---|
| Inventive Compound A-4 | 3 parts |
| Xylene | 76 parts |
| Isophorone | 15 parts |
| Horpol 3005X (a mixture of nonionic surfactant and anionic surfactant: Toho Chemical Industry Co., Ltd., trade name) | 6 parts |
| The above are uniformly mixed to form an emulsifiable concentrate. | |

| [Composition Example 3] Flowable powder | |
|---|---|
| Inventive Compound A-7 | 35 parts |
| Agrisol S-711 (nonionic surfactant: Kao Corporation, trade name) | 8 parts |
| Runox 1000C (anionic surfactant: Toho Chemical Industry Co., Ltd., trade name) | 0.5 part |
| 1% Rodopol water (thickener: Rhone Poulenc Co., trade name) | 20 parts |
| Ethylene glycol (antifreezing agent) | 8 parts |
| Water | 28.5 parts |
| The above are uniformly mixed to form flowable powder. | |

| [Composition Example 4] Granulate wettable powder (dry flowable powder) | |
|---|---|
| Inventive Compound A-9 | 75 parts |
| Isoban No. 1 (anionic surfactant: Kuraray Isoprene Chemical Co.,Ltd., trade name) | 10 parts |
| Banilex N (anionic surfactant: Sanyo Kokusaku Pulp Co., Ltd., trade name) | 5 parts |
| Carplex #80 (White carbon: Shionogi & Co., Ltd., trade name) | 10 parts |
| The above are uniformly mixed and pulverized to form dry flowable powder. | |

| [Composition Example 5] Granule | |
|---|---|
| Inventive Compound A-8 | 0.1 part |
| Bentonite | 55.0 parts |
| Talc | 44.9 parts |

After the above are mixed and pulverized, a small amount of water is added and the mixture is stirred, mixed and kneaded, granulated in an extrusion type granulator and then dried to form dust.

Upon use, the above-described wettable powder, emulsifiable concentrate, flowable powder, and granulate wettable powder are diluted 50 to 1,000 folds with water such that the active ingredient is in a concentration of from 1 to 10,000 ppm or the active ingredient is applied in a rate of from 0.0001 to 10 kg per hectare (ha).

Next, usefulness of the compound of the present invention as a herbicide will be explained concretely by the following test examples.

[Test Example 1] Herbicidal effect test by pre-emergence treatment to weeds under submerged conditions

In a 1/10,000-are Wagner pot was filled alluvial soil, which was mixed with water introduced therein to establish submerged conditions at a water depth of 4 cm. Respective seeds of Echinochloa crus-galli var. crus-galli, Monochoria vaginalis, Rotala indica and Scirpus juncoides were sowed mixedly in the pot and then 2.5 leaf stage rice seedlings were transplanted. The pot was placed in a greenhouse at 25 to 30°C to grow the plant. One day after the sowing, the compound of the present invention prepared according to one of Composition Examples was applied onto the water surface at a predetermined application rate. Three weeks after the treatment, the herbicidal effect on the rice plant and each kind of the weeds was examined. There was used five rating evaluation, with 0 indicating no influence and 5 indicating complete withering. Table 4 shows the results.

[Test Example 2] Herbicidal effect test by post-emergence treatment to weeds under submerged conditions

In a 1/10,000-are Wagner pot was filled alluvial soil, which was mixed with water introduced therein to establish submerged conditions at a water depth of 4 cm. Respective seeds of Echinochloa crus-galli var. crus-galli, Monochoria vaginalis, Rotala indica and Scirpus juncoides were sowed mixedly in the pot and then 2.5 leaf stage rice seedlings were transplanted. The pot was placed in a greenhouse at 25 to 30°C to grow the plant. When Echinochloa crus-galli var. crus-galli, Monochoria vaginalis, Rotala indica and Scirpus juncoides reached 1 to 2 leaf stage and the rice plant reached 4 leaf stage, the compound of the present invention prepared according to one of Composition Examples was applied onto the water surface at a predetermined application rate. Three weeks after the treatment, the herbicidal effect on each kind of the weeds was examined in accordance with the criteria of judgment in Test Example 1. Table 5 shows the results.

[Test Example 3] Herbicidal effect test by soil treatment

In a plastic box of 21 cm in length, 13 cm in width and 7 cm in depth was filled diluvial soil and seeds of Echinochloa crus-galli var. crus-galli, Setaria viridis, soy bean and cotton were sowed respectively in a spot-like fashion and were covered with soil at a height of about 1.5 cm. Then, the compound of the present invention was uniformly applied onto the surface of the soil using a small sprayer so that the amount of the active ingredient was in a predetermined application rate. The liquid used upon the application was obtained by diluting with water the wettable powder prepared appropriately according to one of the above-described Composition Examples. The liquid was applied all over the surface. Three weeks after the application of the liquid, the herbicidal effect on each kind of the weeds was examined in accordance with the criteria of judgment in Test Example 1. Table 6 shows the results.

[Test Example 4] Phytotoxicity test under shallow planting conditions for rice plant

In a 1/5,000-are Wagner pot was filled alluvial soil, which was mixed with water introduced therein to establish submerged conditions at a water depth of 4 cm. Rice seedlings at 2.5 leaf stage were transplanted at a depth of 0.5 cm and the roots were exposed to the air. On the day when the transplantation was made, the compound of the present invention prepared according to one of Composition Examples was applied. Three weeks after the treatment, the live

weight of the aerial part of the plant was measured and inhibition ratio (%) was obtained according to the following equation.

$$\text{Inhibition ratio (\%)} = (1 - \frac{\text{live weight of aerial part of treated plant}}{\text{live weight of aerial part of non-treated plant}}) \times 100$$

Table 7 shows the results.

[Test Example 5] Herbicidal effect test under submerged conditions

In a 1/10,000-are Wagner pot was filled alluvial soil, which was mixed with water introduced therein to establish submerged conditions at a water depth of 4 cm. Respective seeds of Monochoria vaginalis, Rotala indica and Scirpus juncoides were sowed mixedly in the pot and then treated with the compound of the present invention prepared according to one of Composition Examples. Three weeks after the treatment, the herbicidal effect on the various weeds was examined. Table 8 shows the results.

As will be apparent from Tables 7 and 8, a combination of the compound of the present invention and other herbicides remarkably alleviated the phytotoxicity on the transplanted paddy rice without decreasing the herbicidal effect.

[Test Example 6] Herbicidal effect test under submerged conditions

In a 1/5,000-are Wagner pot was filled alluvial soil, which was mixed with water introduced therein to establish submerged conditions at a water depth of 4 cm. After seeds of Monochoria vaginalis and Scirpus juncoides were sowed mixedly in the pot, 2.5 leaf stage rice seedlings were transplanted at a depth of 2 cm. On the next day of transplantation of the rice, the compound of the present invention prepared according to one of Composition Examples was applied. Three weeks after the treatment, the live weights of aerial parts of each kind of the weeds and rice were measured and inhibition ratios (%) were obtained according to Test Example 4. Tables 9 and 10 show the results. In the tables, (E) indicates expected values obtained by the equation of Colby and (F) indicates found values. Left side values and right side values in the column of application rate for each active ingredient indicate corresponding dosages of compounds in the column of compound name.

Hereafter, the equation of Colby will be explained.

Synergistic herbicidal effect of herbicide mixtures will be explained as follows. That is, individual active compounds in many cases may be deficient in their herbicidal activity. In such a case, if the herbicidal activity of two active compounds in combination is higher than the simple sum of the activities of the compounds (expected activity), then this is called synergistic action. The expected activity from the stable combination of two herbicides, can be calculated as follows. (cf. Colby, S. R., Calculation of Synergistic and Antagonistic Reactions of Combination of Herbicides, Weed, vol. 15, pages 20-22 (1967))

$$E = \alpha + \beta - ( \alpha \cdot \beta / 100)$$

$\alpha$ : Inhibition ratio when herbicide A is applied in a rate of a kg/ha

$\beta$ : Inhibition ratio when herbicide B is applied in a rate of b kg/ha

E : Inhibition ratio expected when herbicide A is applied in a rate of a kg/ha and herbicide B is applied in a rate of b kg/ha.

That is, if the actual inhibition ratio is larger than the above-calculated value, it can be said that the activity as a result of combination shows synergism.

Therefore, as will be apparent from Tables 9 and 10, combination of Inventive Compound No. A-5 and daimuron gave rise to synergistic action without increasing phytotoxicity to rice.

The similar results were obtained for the following compounds.

A-6 to A-8, A-28 to A-31 ("-1 and -2" is attached to each number), A-34 to A-40 ("-1 and -2" is attached to each number), A-51 to A-52, A-54 to A-55, A-57 to A-60, A-63 to A-64 ("-1 and -2" is attached to each number), A-72, A-77, A-78-1 and -2, A-81, A-85 to A-87 ("-1 and -2" is attached to each number), A-88 to A-93, A-95 to 97, A-99, A-101 to 107, A-109 to 112 and A-117.

[Comparative examples]

Comparative tests between the compound of the formula:

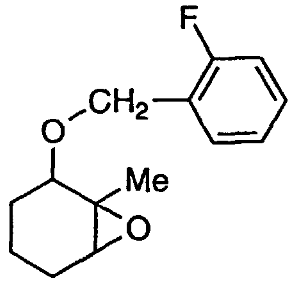

(diastreomer-(1) and (2)) involved in the claims of WO 97/02258 and the compound No. A-5 of the present invention were carried out in manners similar to that in test example 1 and 2. And the results of the tests are shown in Table 11 and 12.

As shown below, each marks in the following tables have meanings in the parentheses:( ) next to the mark.

The Kind of the Plants: A(Echinochloa crus-galli var. crus-galli), B (Scirpus juncoides ), C (Monochoria vaginalis), D (Rotala indica ), E (Setaria viridis ), a (transplanted rice), b (soy bean), c (cotton).

Compound No. : R-1 (daimuron), R-2 (pyrazosulfuron ethyl), R-3 (halosulfuron methyl), R-4 (imazosulfuron), R-5 (azimsulfuron) R-6 (cyclosulfamuron), R-7 (ethoxysulfuron), R-8 (cinosulfuron), R-9 (bensulfuron methyl).

Table 4

| compound No. | dosage (kg/ha) | A | B | C | D | a |
|---|---|---|---|---|---|---|
| A-1 | 2.0 | 5 | 5 | 5 | 5 | 0 |
| A-2 | 2.0 | 5 | 5 | 5 | 5 | 0 |
| A-3 | 2.0 | 5 | 5 | 5 | 5 | 0 |
| A-4 | 2.0 | 5 | 5 | 5 | 5 | 0 |
| A-5 | 0.25 | 5 | 5 | 5 | 4 | 0 |
| A-6 | 0.25 | 5 | 5 | 5 | 4 | 1 |
| A-7 | 1.0 | 5 | 5 | 5 | 5 | 0 |
| A-8 | 1.0 | 5 | 5 | 5 | 5 | 0 |
| A-9 | 1.0 | 5 | 5 | 5 | 5 | 0 |
| A-10 | 1.0 | 5 | 5 | 5 | 5 | 0 |
| A-11 | 1.0 | 5 | 5 | 5 | 5 | 0 |
| A-12 | 0.25 | 5 | 5 | 5 | 5 | 0 |
| A-13 | 0.25 | 5 | 5 | 5 | 5 | 0 |
| A-14 | 0.25 | 5 | 5 | 5 | 5 | 0 |
| A-15 | 0.25 | 5 | 5 | 5 | 5 | 0 |
| A-16 | 0.25 | 5 | 5 | 5 | 5 | 0 |
| A-17 | 0.25 | 5 | 5 | 5 | 5 | 0 |
| A-18 | 1.0 | 5 | 5 | 5 | 5 | 0 |
| A-19 | 0.25 | 5 | 4 | 5 | 1 | 0 |
| A-20 | 0.25 | 5 | 5 | 5 | 2 | 0 |
| A-21 | 0.25 | 5 | 5 | 5 | 2 | 0 |
| A-22 | 0.25 | 5 | 5 | 5 | 5 | 0 |
| A-23-1 + A-23-2 | 0.25 | 5 | 5 | 5 | 5 | 0 |
| A-24 | 2.0 | 5 | 5 | 5 | 5 | 0 |
| A-26 | 0.25 | 5 | 5 | 5 | - | 0 |
| A-27 | 0.25 | 4 | 3 | 5 | - | 0 |
| A-28-1 | 0.25 | 5 | 5 | 5 | - | 0 |
| A-28-2 | 0.25 | 5 | 5 | 5 | - | 0 |
| A-29-1 | 0.25 | 5 | 5 | 5 | - | 0 |
| A-29-2 | 0.25 | 5 | 5 | 5 | - | 0 |
| A-30-1 | 0.25 | 5 | 5 | 5 | - | 0 |
| A-30-2 | 0.25 | 5 | 5 | 5 | - | 0 |
| A-31-1 | 0.25 | 5 | 5 | 5 | - | - |
| A-31-2 | 0.25 | 5 | 5 | 5 | - | 0 |
| A-32 | 0.25 | 5 | 0 | 5 | - | 0 |
| A-33 | 0.25 | 5 | 3 | 5 | - | 0 |

Table 4 (continued)

| compound No. | dosage (kg/ha) | A | B | C | D | a |
|---|---|---|---|---|---|---|
| A-34-1 | 0.25 | 5 | 5 | 5 | - | 4 |
| A-34-2 | 0.25 | 5 | 5 | 5 | - | 0 |
| A-34-1 + A-34-2 | 1.0 | 5 | 5 | 5 | 5 | 0 |
| A-35-1 | 0.25 | 5 | 5 | 5 | - | 0 |
| A-35-2 | 0.25 | 5 | 5 | 5 | - | 0 |
| A-35-1 + A-35-2 | 0.25 | 5 | 5 | 5 | 5 | 0 |
| A-36-1 | 0.25 | 5 | 4 | 5 | - | 0 |
| A-36-2 | 0.25 | 5 | 3 | 5 | - | 0 |
| A-36-1 + A-36-2 | 0.25 | 5 | 5 | 5 | 5 | 0 |
| A-37-1 | 0.25 | 5 | 4 | 5 | - | 0 |
| A-37-2 | 0.25 | 5 | 4 | 5 | - | 0 |
| A-38-1 | 0.25 | 5 | 5 | 5 | - | 0 |
| A-38-2 | 0.25 | 5 | 4 | 5 | - | 0 |
| A-39-1 | 0.25 | 5 | 4 | 5 | - | 0 |
| A-39-2 | 0.25 | 5 | 4 | 5 | - | 0 |
| A-40-1 | 0.25 | 5 | 5 | 5 | - | 1 |
| A-40-2 | 0.25 | 5 | 4 | 5 | - | 0 |
| A-43 | 1.0 | 5 | 5 | 5 | - | 0 |
| A-44 | 0.25 | 0 | 0 | 5 | - | 0 |
| A-45 | 0.25 | 5 | 0 | 5 | - | 0 |
| A-46 | 0.25 | 5 | 5 | 5 | - | 2 |
| A-47 | 0.25 | 4 | 4 | 5 | - | 0 |
| A-48 | 0.25 | 5 | 5 | 5 | - | 0 |
| A-49 | 0.25 | 5 | 4 | 5 | - | 0 |
| A-50 | 2.0 | 5 | 4 | 5 | - | 0 |
| A-51 | 0.25 | 5 | 5 | 5 | - | 0 |
| A-52 | 0.25 | 5 | 5 | 5 | - | 0 |
| A-54 | 0.25 | 5 | 5 | 5 | - | 0 |
| A-55 | 0.25 | 5 | 5 | 5 | - | 2 |
| A-56 | 1.0 | 2 | 0 | 4 | - | 0 |
| A-57 | 1.0 | 5 | 5 | 5 | - | 3 |
| A-58 | 1.0 | 5 | 5 | 5 | - | 2 |
| A-59 | 0.25 | 5 | 5 | 5 | - | 0 |
| A-60 | 0.25 | 5 | 5 | 5 | - | 0 |

Table 4 (continued)

| compound No. | dosage (kg/ha) | A | B | C | D | a |
|---|---|---|---|---|---|---|
| A-61-1 | 0.25 | 5 | 4 | 5 | - | 0 |
| A-61-2 | 0.25 | 5 | 4 | 5 | - | 0 |
| A-62 | 1.0 | 5 | 5 | 5 | - | 0 |
| A-63-1 | 0.25 | 5 | 5 | 5 | - | 2 |
| A-63-2 | 0.25 | 5 | 5 | 5 | - | 0 |
| A-64-1 | 0.25 | 5 | 5 | 5 | - | 0 |
| A-64-2 | 0.25 | 5 | 5 | 4 | - | 0 |
| A-65 | 0.25 | 5 | 4 | 4 | - | 0 |
| A-67 | 1.0 | 0 | - | 2 | - | 0 |
| A-68 | 0.25 | 2 | 3 | 3 | - | 0 |
| A-69 | 0.25 | 3 | 0 | 3 | - | - |
| A-70 | 0.25 | 4 | 0 | 3 | - | 0 |
| A-71 | 0.25 | 5 | 2 | 4 | - | 0 |
| A-72 | 0.25 | 5 | 5 | 5 | - | - |
| A-73 | 0.25 | 5 | 2 | 3 | - | 0 |
| A-74 | 0.25 | 5 | 4 | 4 | - | 0 |
| A-75 | 1.0 | 5 | 5 | 5 | - | 0 |
| A-76 | 1.0 | 5 | 5 | 5 | - | 0 |
| A-77 | 0.25 | 5 | 5 | 5 | - | 0 |
| A-78-1 | 0.25 | 5 | 5 | 5 | - | 0 |
| A-78-2 | 0.25 | 5 | 5 | 5 | - | 0 |
| A-79 | 0.25 | 5 | 0 | 5 | - | 0 |
| A-80 | 0.25 | 5 | 0 | 5 | - | 0 |
| A-81 | 0.25 | 5 | 5 | 5 | - | 0 |
| A-82 | 0.25 | 5 | 5 | 5 | - | 0 |
| A-83 | 0.25 | 3 | 0 | 0 | - | 0 |
| A-85-1 | 0.25 | 5 | 4 | 5 | - | 1 |
| A-85-2 | 0.25 | 5 | 5 | 5 | - | 0 |
| A-86-1 | 0.25 | 5 | 5 | 5 | - | 0 |
| A-86-2 | 0.25 | 5 | 5 | 5 | - | 0 |
| A-87-1 | 0.25 | 5 | 5 | 5 | - | - |
| A-87-2 | 0.25 | 5 | 5 | 5 | - | 0 |
| A-88 | 0.25 | 5 | 5 | 5 | - | 0 |
| A-89 | 0.25 | 5 | 5 | 5 | - | - |
| A-90 | 0.25 | 5 | 5 | 5 | - | 0 |
| A-91 | 0.25 | 5 | 5 | 5 | - | 0 |
| A-92 | 0.25 | 5 | 5 | 5 | - | - |

Table 4 (continued)

| compound No. | dosage (kg/ha) | A | B | C | D | a |
|---|---|---|---|---|---|---|
| A-93 | 0.25 | 5 | 5 | 5 | - | 0 |
| A-94 | 1 | 5 | 5 | 5 | - | 0 |
| A-95 | 0.25 | 5 | 5 | 5 | - | 0 |
| A-96 | 0.25 | 5 | 5 | 5 | - | 0 |
| A-97 | 0.25 | 5 | 4 | 5 | - | 0 |
| A-98 | 0.25 | 5 | 4 | 4 | - | 0 |
| A-99 | 0.25 | 5 | 5 | 5 | - | 0 |
| A-100 | 0.25 | 0 | 0 | 2 | - | 0 |
| A-101 | 0.25 | 5 | 5 | 5 | - | 0 |
| A-102 | 0.25 | 5 | 5 | 5 | - | 0 |
| A-103 | 0.25 | 5 | 5 | 5 | - | 0 |
| A-104 | 0.25 | 5 | 5 | 5 | - | 0 |
| A-105 | 0.25 | 5 | 5 | 5 | - | 0 |
| A-106 | 0.25 | 5 | 5 | 1 | - | 0 |
| A-107 | 0.25 | 5 | 5 | 4 | - | 0 |
| A-108 | 0.25 | 5 | 5 | 4 | - | 0 |
| A-109 | 0.25 | 5 | 5 | 1 | - | 0 |
| A-110 | 0.25 | 5 | 4 | 5 | - | 0 |
| A-111 | 0.25 | 5 | 4 | 5 | - | 0 |
| A-112 | 0.25 | 5 | 5 | 5 | - | 0 |
| A-113 | 0.25 | 0 | 0 | 4 | - | 0 |
| A-114 | 0.25 | 5 | - | 5 | - | 0 |
| A-115 | 0.25 | 5 | 5 | 5 | - | 0 |
| A-116 | 0.25 | 5 | 0 | 4 | - | 0 |
| A-117 | 0.25 | 5 | 5 | 5 | - | 0 |
| B-1 | 1.0 | 5 | 5 | 5 | - | 0 |
| B-2-1 | 1.0 | 1 | 2 | 0 | - | 0 |
| B-2-2 | 1.0 | 5 | 5 | 5 | - | 0 |
| B-3-1 | 0.25 | 0 | 0 | 2 | - | 0 |
| B-4-2 | 1.0 | 5 | 5 | 4 | - | 0 |
| B-4-4 | 1.0 | 5 | 5 | 4 | - | 5 |
| B-5-1 | 1.0 | 2 | 0 | 2 | - | 0 |
| B-5-2 | 0.25 | 5 | 5 | 5 | - | 0 |
| B-5-3 | 0.25 | 4 | 5 | 4 | - | 0 |
| C-1 | 2.0 | 4 | 2 | 4 | - | 0 |

Table 5

| compound No. | dosage (kg/ha) | A | B | C | D |
|---|---|---|---|---|---|
| A-1 | 2.0 | 5 | 5 | 5 | 5 |
| A-2 | 2.0 | 5 | 5 | 5 | 5 |
| A-3 | 2.0 | 5 | 4 | 5 | 5 |
| A-4 | 2.0 | 5 | 4 | 5 | 5 |
| A-5 | 0.25 | 5 | 5 | 5 | 4 |
| A-6 | 0.25 | 3 | 3 | 4 | 5 |
| A-7 | 1.0 | 5 | 3 | 5 | 5 |
| A-8 | 1.0 | 5 | 3 | 5 | 5 |
| A-9 | 1.0 | 3 | 1 | 4 | 4 |
| A-10 | 1.0 | 4 | 3 | 4 | 4 |
| A-11 | 1.0 | 4 | 4 | 4 | 4 |
| A-12 | 0.25 | 5 | 5 | 5 | 5 |
| A-13 | 0.25 | 5 | 4 | 5 | 5 |
| A-14 | 0.25 | 5 | 3 | 5 | 5 |
| A-15 | 0.25 | 5 | 4 | 5 | 5 |
| A-16 | 0.25 | 5 | 1 | 5 | 5 |
| A-17 | 0.25 | 5 | 5 | 5 | 4 |
| A-18 | 1.0 | 5 | 5 | 5 | 5 |
| A-19 | 0.25 | 1 | 1 | 5 | 4 |
| A-20 | 0.25 | 4 | 3 | 5 | 4 |
| A-22 | 0.25 | 3 | 0 | 4 | 3 |
| A-23-1 + A-23-2 | 0.25 | 5 | 1 | 4 | 5 |
| A-24 | 2.0 | 5 | 3 | 3 | 3 |
| A-26 | 0.25 | 0 | 3 | 3 | - |
| A-27 | 0.25 | 2 | 0 | 1 | - |
| A-28-1 | 0.25 | 5 | 3 | 5 | - |
| A-28-2 | 0.25 | 5 | 3 | 5 | - |
| A-29-1 | 0.25 | 5 | 3 | 5 | - |
| A-29-2 | 0.25 | 5 | 3 | 4 | - |
| A-30-1 | 0.25 | 5 | 4 | 5 | - |
| A-30-2 | 0.25 | 5 | 4 | 5 | - |
| A-31-1 | 0.25 | 5 | 5 | 5 | - |
| A-31-2 | 0.25 | 5 | 4 | 5 | - |
| A-32 | 0.25 | 2 | 0 | 2 | - |
| A-33 | 0.25 | 3 | 3 | 5 | - |
| A-34-1 | 0.25 | 5 | 3 | 5 | - |

Table 5 (continued)

| compound No. | dosage (kg/ha) | A | B | C | D |
|---|---|---|---|---|---|
| A-34-2 | 0.25 | 5 | 2 | 5 | - |
| A-34-1 + A-34-2 | 1.0 | 5 | 4 | 5 | 4 |
| A-35-1 | 0.25 | 5 | 4 | 5 | - |
| A-35-2 | 0.25 | 5 | 4 | 5 | - |
| A-35-1 + A-35-2 | 0.25 | 5 | 1 | 4 | 4 |
| A-36-1 | 0.25 | 5 | 4 | 5 | - |
| A-36-2 | 0.25 | 5 | 4 | 5 | - |
| A-36-1 + A-36-2 | 0.25 | 5 | 3 | 5 | 5 |
| A-37-1 | 0.25 | 5 | 4 | 5 | - |
| A-37-2 | 0.25 | 5 | 4 | 5 | - |
| A-38-1 | 0.25 | 5 | 3 | 5 | - |
| A-38-2 | 0.25 | 3 | 3 | 5 | - |
| A-39-1 | 0.25 | 5 | 4 | 5 | - |
| A-39-2 | 0.25 | 5 | 4 | 5 | - |
| A-40-1 | 0.25 | 5 | 5 | 5 | - |
| A-40-2 | 0.25 | 5 | 4 | 5 | - |
| A-43 | 1.0 | 5 | 3 | 5 | - |
| A-45 | 0.25 | 3 | 3 | 3 | - |
| A-46 | 0.25 | 5 | 0 | 5 | - |
| A-48 | 0.25 | 5 | 0 | 5 | - |
| A-50 | 2.0 | 0 | 0 | 5 | - |
| A-51 | 0.25 | 5 | 0 | 5 | - |
| A-52 | 0.25 | 5 | 1 | 5 | - |
| A-54 | 0.25 | 5 | 2 | 4 | - |
| A-55 | 0.25 | 5 | 3 | 5 | - |
| A-57 | 1.0 | 5 | 4 | 5 | - |
| A-58 | 1.0 | 5 | 5 | 5 | - |
| A-59 | 0.25 | 5 | 4 | 2 | - |
| A-60 | 0.25 | 5 | 2 | 3 | - |
| A-61-1 | 0.25 | 3 | 2 | 4 | - |
| A-61-2 | 0.25 | 1 | 0 | 2 | - |
| A-62 | 1.0 | 2 | 2 | 4 | - |
| A-63-1 | 0.25 | 5 | 3 | 4 | - |
| A-63-2 | 0.25 | 5 | 0 | 3 | - |

Table 5 (continued)

| compound No. | dosage (kg/ha) | A | B | C | D |
|---|---|---|---|---|---|
| A-64-1 | 0.25 | 5 | 3 | 4 | - |
| A-64-2 | 0.25 | 5 | 2 | 3 | - |
| A-65 | 0.25 | 4 | 2 | 4 | - |
| A-68 | 0.25 | 2 | 0 | 1 | - |
| A-69 | 0.25 | 3 | 0 | 3 | - |
| A-70 | 0.25 | 1 | - | 2 | - |
| A-71 | 0.25 | 2 | - | 2 | - |
| A-72 | 0.25 | 4 | - | 4 | - |
| A-73 | 0.25 | 2 | 3 | 4 | - |
| A-74 | 0.25 | 5 | 3 | 4 | - |
| A-75 | 1.0 | 2 | 0 | 3 | - |
| A-76 | 1.0 | 5 | 1 | 5 | - |
| A-77 | 0.25 | 5 | 4 | 5 | - |
| A-78-1 | 0.25 | 4 | 0 | 3 | - |
| A-78-2 | 0.25 | 5 | 5 | 5 | - |
| A-79 | 0.25 | 0 | 0 | 5 | - |
| A-80 | 0.25 | 0 | 0 | 5 | - |
| A-81 | 0.25 | 5 | 2 | 2 | - |
| A-82 | 0.25 | 3 | 0 | 2 | - |
| A-85-1 | 0.25 | 4 | - | 4 | - |
| A-85-2 | 0.25 | 2 | 0 | 3 | - |
| A-86-1 | 0.25 | 4 | 0 | 3 | - |
| A-86-2 | 0.25 | 4 | 0 | 3 | - |
| A-87-1 | 0.25 | 4 | 2 | 5 | - |
| A-87-2 | 0.25 | 4 | 0 | 3 | - |
| A-88 | 0.25 | 4 | 2 | 5 | - |
| A-89 | 0.25 | 5 | 0 | 3 | - |
| A-90 | 0.25 | 4 | 0 | 2 | - |
| A-91 | 0.25 | 5 | 0 | 4 | - |
| A-92 | 0.25 | 5 | 0 | 3 | - |
| A-93 | 0.25 | 5 | 0 | 4 | - |
| A-94 | 1 | 5 | - | 4 | - |
| A-95 | 0.25 | 5 | - | 4 | - |
| A-96 | 0.25 | 5 | - | 5 | - |
| A-97 | 0.25 | 5 | - | 3 | - |
| A-98 | 0.25 | 0 | - | 2 | - |
| A-99 | 0.25 | 5 | - | 5 | - |

Table 5 (continued)

| compound No. | dosage (kg/ha) | A | B | C | D |
|---|---|---|---|---|---|
| A-101 | 0.25 | 5 | - | 4 | - |
| A-102 | 0.25 | 5 | - | 5 | - |
| A-103 | 0.25 | 5 | - | 4 | - |
| A-104 | 0.25 | 5 | - | 4 | - |
| A-105 | 0.25 | 5 | - | 4 | - |
| A-106 | 0.25 | 5 | - | 4 | - |
| A-107 | 0.25 | 5 | - | 4 | - |
| A-108 | 0.25 | 0 | - | 4 | - |
| A-109 | 0.25 | 5 | - | 2 | - |
| A-110 | 0.25 | 5 | - | 5 | - |
| A-111 | 0.25 | 5 | - | 0 | - |
| A-112 | 0.25 | 5 | - | 5 | - |
| A-114 | 0.25 | 1 | - | 4 | - |
| A-115 | 0.25 | 1 | - | 5 | - |
| A-116 | 0.25 | 1 | - | 4 | - |
| A-117 | 0.25 | 5 | - | 5 | - |
| B-1 | 1.0 | 2 | 3 | 5 | - |
| B-2-1 | 1.0 | 5 | 5 | 5 | - |
| B-4-2 | 1.0 | 4 | 3 | 5 | - |
| B-4-4 | 1.0 | 1 | 1 | 4 | - |
| B-5-1 | 1.0 | 0 | 0 | 2 | - |
| B-5-2 | 0.25 | 3 | 2 | 4 | - |
| B-5-3 | 0.25 | 2 | 0 | 4 | - |

Table 6

| compound No. | dosage (kg/ha) | A | E | b | c |
|---|---|---|---|---|---|
| A-1 | 5.0 | 5 | 5 | 0 | 0 |
| A-2 | 5.0 | 5 | 5 | 0 | 0 |
| A-3 | 5.0 | 5 | 4 | 0 | 0 |
| A-4 | 5.0 | 5 | 5 | 0 | 0 |
| A-5 | 0.63 | 5 | 5 | 0 | 0 |
| A-6 | 0.63 | 4 | 0 | 0 | 0 |

Table 6 (continued)

| compound No. | dosage (kg/ha) | A | E | b | c |
|---|---|---|---|---|---|
| A-7 | 25 | 5 | 5 | 3 | 0 |
| A-8 | 2.5 | 5 | 5 | 2 | 0 |
| A-9 | 2.5 | 5 | 5 | 0 | 3 |
| A-10 | 2.5 | 5 | 4 | 0 | 0 |
| A-11 | 2.5 | 5 | 5 | 0 | 0 |
| A-12 | 0.63 | 5 | 5 | 0 | 0 |
| A-13 | 0.63 | 5 | 5 | 0 | 0 |
| A-14 | 0.63 | 5 | 5 | 0 | 0 |
| A-15 | 0.63 | 5 | 5 | 0 | 0 |
| A-16 | 0.63 | 5 | 5 | 0 | 0 |
| A-17 | 0.63 | 5 | 5 | 0 | 0 |
| A-18 | 2.5 | 5 | 5 | 0 | 0 |
| A-19 | 2.5 | 5 | 4 | 0 | 0 |
| A-20 | 0.63 | 5 | 5 | 0 | 0 |
| A-21 | 0.63 | 5 | 4 | 0 | 0 |
| A-22 | 0.63 | 5 | 4 | 0 | 0 |
| A-23-1 + A-23-2 | 0.63 | 5 | 5 | 0 | 0 |
| A-24 | 5.0 | 5 | 5 | 0 | 0 |
| A-26 | 0.63 | - | 5 | 0 | 0 |
| A-27 | 0.63 | - | 4 | 0 | 0 |
| A-28-1 | 0.63 | - | 5 | 0 | 0 |
| A-28-2 | 0.63 | - | 5 | 0 | 0 |
| A-29-1 | 0.63 | - | 5 | 0 | 0 |
| A-29-2 | 5.0 | - | 5 | 0 | 0 |
| A-30-1 | 0.63 | - | 5 | 0 | 0 |
| A-30-2 | 0.63 | - | 5 | 0 | 0 |
| A-31-1 | 0.63 | - | 5 | 0 | 0 |
| A-31-2 | 0.63 | - | 5 | 0 | 0 |
| A-34-1 | 0.63 | - | 5 | 0 | 0 |
| A-34-2 | 0.63 | - | 5 | 0 | 0 |
| A-34-1 + A-34-2 | 2.5 | 5 | 5 | 0 | 0 |
| A-35-1 | 0.63 | - | 5 | 0 | 0 |
| A-35-2 | 0.63 | - | 5 | 0 | 0 |
| A-35-1 + A-35-2 | 0.63 | 5 | 5 | 0 | 0 |

Table 6 (continued)

| compound No. | dosage (kg/ha) | A | E | b | c |
|---|---|---|---|---|---|
| A-36-1 | 0. 63 | - | 5 | 0 | 0 |
| A-36-2 | 0. 63 | - | 5 | 0 | 0 |
| A-36-1 + A-36-2 | 0. 63 | 5 | 5 | 0 | 0 |
| A-37-1 | 0. 63 | - | 5 | 0 | 0 |
| A-37-2 | 0. 63 | - | 4 | 0 | 0 |
| A-38-1 | 0. 63 | - | 5 | 0 | 0 |
| A-38-2 | 0. 63 | - | 3 | 0 | 0 |
| A-39-1 | 0. 63 | - | 5 | 0 | 0 |
| A-39-2 | 0. 63 | - | 5 | 0 | 0 |
| A-40-1 | 0. 63 | - | 5 | 0 | 0 |
| A-40-2 | 0. 63 | - | 2 | 0 | 0 |
| A-41 | 0. 63 | - | 2 | 0 | 0 |
| A-43 | 2. 5 | - | 1 | 0 | 0 |
| A-46 | 0. 63 | - | 3 | 0 | 0 |
| A-48 | 0. 63 | - | 5 | 0 | 0 |
| A-49 | 0. 63 | - | 2 | 0 | 0 |
| A-51 | 0. 63 | - | 5 | 0 | 0 |
| A-52 | 0. 63 | - | 5 | 0 | 0 |
| A-54 | 0. 63 | - | 4 | 0 | 0 |
| A-55 | 0. 63 | - | 4 | 0 | 0 |
| A-57 | 0. 63 | - | 5 | 0 | 0 |
| A-58 | 0. 63 | - | 5 | 0 | 0 |
| A-59 | 0. 63 | - | 5 | 0 | 0 |
| A-60 | 0. 63 | - | 5 | 0 | 0 |
| A-61-1 | 0. 63 | - | 5 | 0 | 0 |
| A-61-2 | 0. 63 | - | 2 | 0 | 0 |
| A-62 | 0. 63 | - | 4 | 0 | 0 |
| A-63-1 | 0. 63 | - | 5 | 0 | 0 |
| A-63-2 | 0. 63 | - | 5 | 0 | 0 |
| A-64-1 | 0. 63 | - | 5 | 0 | 0 |
| A-64-2 | 0. 63 | - | 5 | 0 | 0 |
| A-65 | 0. 63 | - | 4 | 0 | 0 |
| A-66 | 0. 63 | - | 3 | 0 | 0 |
| A-69 | 0. 63 | - | 2 | 0 | 0 |
| A-70 | 0. 63 | - | 2 | 0 | 0 |
| A-71 | 0. 63 | - | 2 | 0 | 0 |

Table 6 (continued)

| compound No. | dosage (kg/ha) | A | E | b | c |
|---|---|---|---|---|---|
| A-72 | 0.63 | - | 5 | 0 | 0 |
| A-74 | 0.63 | - | 5 | 0 | 0 |
| A-75 | 2.5 | - | 2 | 0 | 0 |
| A-76 | 2.5 | - | 5 | 0 | 0 |
| A-77 | 2.5 | - | 5 | 0 | 0 |
| A-78-1 | 0.63 | - | 5 | 0 | 0 |
| A-78-2 | 0.63 | - | 4 | 0 | 0 |
| A-79 | 0.63 | - | 5 | 0 | 0 |
| A-81 | 0.63 | - | 5 | 0 | 0 |
| A-82 | 0.63 | - | 2 | 0 | 0 |
| A-85-1 | 0.63 | - | 4 | 0 | 0 |
| A-85-2 | 0.63 | - | 4 | 0 | 0 |
| A-86-1 | 0.63 | - | 5 | 0 | 0 |
| A-86-2 | 0.63 | - | 2 | 0 | 0 |
| A-87-1 | 0.63 | - | 1 | 0 | 0 |
| A-87-2 | 0.63 | - | 2 | 0 | 0 |
| A-88 | 0.63 | - | 5 | 0 | 0 |
| A-89 | 0.63 | - | 5 | 0 | 0 |
| A-90 | 0.63 | - | 5 | 0 | 0 |
| A-91 | 0.63 | - | 5 | 0 | 0 |
| A-92 | 0.63 | - | 5 | 0 | 0 |
| A-93 | 0.63 | - | 5 | 0 | 0 |
| A-94 | 2.5 | - | 5 | 0 | 0 |
| A-95 | 0.63 | - | 4 | 0 | 0 |
| A-96 | 0.63 | - | 5 | 0 | 0 |
| A-97 | 0.63 | - | 4 | 0 | 0 |
| A-98 | 0.63 | - | 3 | 0 | 0 |
| A-99 | 0.63 | - | 5 | 0 | 0 |
| A-101 | 0.63 | - | 5 | 0 | 0 |
| A-102 | 0.63 | - | 5 | 0 | 0 |
| A-103 | 0.63 | - | 5 | 0 | 0 |
| A-104 | 0.63 | - | 5 | 0 | 0 |
| A-105 | 0.63 | - | 5 | 0 | 0 |
| A-106 | 0.63 | - | 3 | 0 | 0 |
| A-107 | 0.63 | - | 5 | 0 | 0 |
| A-108 | 0.63 | - | 1 | 0 | 0 |
| A-109 | 0.63 | - | 3 | 0 | 0 |

Table 6 (continued)

| compound No. | dosage (kg/ha) | A | E | b | c |
|---|---|---|---|---|---|
| A-110 | 0.63 | - | 5 | 0 | 0 |
| A-111 | 0.63 | - | 5 | 0 | 0 |
| A-112 | 0.63 | - | 5 | 0 | 0 |
| A-117 | 0.63 | - | 5 | 0 | 0 |
| B-2-2 | 2.5 | - | 5 | 0 | 0 |
| B-4-1 | 2.5 | - | 1 | 0 | 0 |
| B-4-2 | 2.5 | - | 5 | 0 | 0 |
| B-4-4 | 2.5 | - | 5 | 0 | 0 |
| B-5-2 | 0.63 | - | 5 | 0 | 0 |
| B-5-3 | 0.63 | - | 5 | 0 | 0 |

Table 7

| compound No. | dosage(g/ha) | the ratio of live weight to the non-treated area (%) |
|---|---|---|
| A-5 | 125 | 86 |
| A-5 | 250 | 67 |
| R-1 | 500 | 97 |
| A-5 + R-1 | 125 + 500 | 100 |
| A-5 + R-1 | 250 + 500 | 99 |

Table 8

| compound No. | dosage (g/ha) | B | C | D |
|---|---|---|---|---|
| A-5 | 125 | 4 | 3 | 2 |
| A-5 | 250 | 5 | 5 | 4 |
| A-5 | 500 | 5 | 5 | 5 |
| R-1 | 500 | 5 | 0 | 1 |
| A-5 + R-1 | 125 + 500 | 5 | 4 | 4 |
| A-5 + R-1 | 250 + 500 | 5 | 5 | 5 |
| A-5 + R-1 | 500 + 500 | 5 | 5 | 5 |

Table 9

| compound No. | dosage (g/ha) | B | C | a |
|---|---|---|---|---|
| A-5 | 25 | 52 | 59 | 0 |
| R-2 | 2 | 71 | 80 | 0 |
| R-3 | 4 | 66 | 71 | 0 |
| R-4 | 4 | 60 | 65 | 0 |
| R-5 | 2 | 61 | 75 | 0 |
| R-6 | 4 | 78 | 77 | 0 |
| R-7 | 2 | 72 | 63 | 0 |
| R-8 | 4 | 70 | 69 | 0 |
| R-9 | 4 | 68 | 69 | 0 |

Table 10

| | | B | | C | | a | |
|---|---|---|---|---|---|---|---|
| compound No. | dosage (g/ha) | (F) | (E) | (F) | (E) | (F) | (E) |
| A-5 + R-2 | 25 + 2 | 100 | 86 | 100 | 92 | 0 | 0 |
| A-5 + R-3 | 25 + 4 | 100 | 84 | 100 | 88 | 0 | 0 |
| A-5 + R-4 | 25 + 4 | 100 | 81 | 100 | 86 | 0 | 0 |
| A-5 + R-5 | 25 + 2 | 100 | 81 | 100 | 90 | 0 | 0 |
| A-5 + R-6 | 25 + 4 | 100 | 89 | 100 | 91 | 0 | 0 |
| A-5 + R-7 | 25 + 2 | 100 | 87 | 100 | 85 | 0 | 0 |
| A-5 + R-8 | 25 + 4 | 100 | 86 | 100 | 87 | 0 | 0 |
| A-5 + R-9 | 25 + 4 | 100 | 85 | 100 | 87 | 0 | 0 |

Table 11

| compound No. | dosage (g/ha) | A | B | C | D | a |
|---|---|---|---|---|---|---|
| A-5 (the present invention) | 12.5 | 5 | 2 | 2 | 2 | 0 |
| | 50 | 5 | 3 | 5 | 5 | 0 |
| | 200 | 5 | 5 | 5 | 5 | 0 |
| comparative compound diastereomer (1) (WO 97/02258) | 12.5 | 0 | 0 | 0 | 0 | 0 |
| | 50 | 0 | 0 | 0 | 0 | 0 |
| | 200 | 0 | 0 | 0 | 0 | 0 |
| comparative compound diastereomer (2) (WO 97/02258) | 12.5 | 0 | 0 | 0 | 0 | 0 |
| | 50 | 0 | 0 | 0 | 0 | 0 |
| | 200 | 1 | 1 | 2 | 4 | 0 |

Table 12

| compound No. | dosage (g/ha) | A | B | C | D |
|---|---|---|---|---|---|
| A-5 (the present invention) | 12.5 | 1 | 1 | 1 | 1 |
| | 50 | 5 | 3 | 4 | 3 |
| | 200 | 5 | 5 | 5 | 4 |
| comparative compound diastereomer (1) (WO 97/02258) | 12.5 | 0 | 0 | 0 | 0 |
| | 50 | 0 | 0 | 0 | 0 |
| | 200 | 0 | 0 | 0 | 0 |
| comparative compound diastereomer (2) (WO 97/02258) | 12.5 | 0 | 0 | 0 | 0 |
| | 50 | 0 | 0 | 0 | 0 |
| | 200 | 0 | 0 | 0 | 0 |

UTILITY FOR INDUSTRY

The compound of the formula (1) is useful as an active ingredient for an agricultural chemical, especially a herbicide.

**Claims**

**1.** A compound of cycloalkyl benzyl ether of the formula (1):

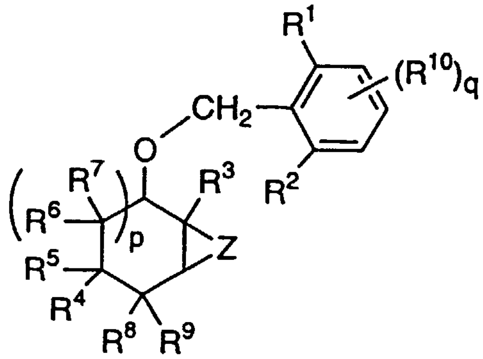

(1)

wherein $R^1$ and $R^2$ each represents independently hydrogen, halogen, $C_1$-$C_6$alkyl, $C_1$-$C_6$haloalkyl, $C_1$-$C_6$alkoxy, $C_1$-$C_6$haloalkoxy, $C_1$-$C_6$alkylthio, $C_1$-$C_6$haloalkylthio, nitro or cyano;
$R^3$ represents $C_1$-$C_6$alkyl, $C_3$-$C_6$alkenyl, $C_1$-$C_6$haloalkyl, ($C_1$-$C_4$alkoxy)$C_1$-$C_6$alkyl or the formula:

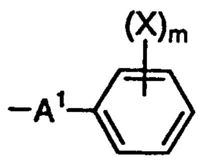

($A^1$ represents single bond or $C_1$-$C_3$alkylen chain;
X represents halogen, $C_1$-$C_6$alkyl, $C_1$-$C_6$haloalkyl, $C_1$-$C_6$alkoxy, cyano, nitro, carboxyl or ($C_1$-$C_6$alkoxy)carbonyl;
m represents an integer of 1 to 5, and
X each may be identical with or different from the other when m represents an integer 2 to 5);
$R^4$ represents hydrogen, halogen, $C_1$-$C_6$alkyl, $C_1$-$C_6$alkoxy or phenyl;
$R^5$ represents halogen, cyano, azido or $C_1$-$C_6$alkyl unsubstituted or substituted by azido, $C_2$-$C_6$alkenyl, $C_2$-$C_6$alkynyl, hydroxy, $C_1$-$C_6$alkoxy, $C_2$-$C_6$alkenyloxy, $C_2$-$C_6$alkynyloxy, mercapto, $C_1$-$C_6$alkylthio, $C_2$-$C_6$alkenylthio, $C_2$-$C_6$alkynylthio, amino, $C_1$-$C_6$alkylamino, di$C_1$-$C_6$alkylamino, $C_1$-$C_6$haloalkyl, $C_2$-$C_6$haloalkenyl, $C_2$-$C_6$haloalkynyl, $C_1$-$C_6$haloalkoxy, $C_1$-$C_6$haloalkylthio, hydroxy$C_1$-$C_6$alkyl, ($C_1$-$C_6$alkoxy)$C_1$-$C_6$alkyl, ($C_2$-$C_6$alkenyloxy)$C_1$-$C_6$alkyl, ($C_2$-$C_6$alkynyloxy)$C_1$-$C_6$alkyl, ($C_1$-$C_6$haloalkoxy)$C_1$-$C_6$alkyl, mercapto$C_1$-$C_6$alkyl, ($C_1$-$C_6$alkylthio)$C_1$-$C_6$alkyl, amino$C_1$-$C_6$alkyl, ($C_1$-$C_6$alkylamino)$C_1$-$C_6$alkyl, (di$C_1$-$C_6$alkylamino)$C_1$-$C_6$alkyl, cyano$C_1$-$C_6$alkyl, formyl$C_1$-$C_6$alkyl, (hydroxycarbonyl)$C_1$-$C_6$alkyl, ($C_1$-$C_6$alkylcarbonyl)$C_1$-$C_6$alkyl, ($C_1$-$C_6$alkoxycarbonyl)$C_1$-$C_6$alkyl, ($C_1$-$C_6$alkoxy) ($C_1$-$C_6$alkoxy)$C_1$-$C_6$alkyl, ($C_3$-$C_7$cycloalkyl) ($C_1$-$C_6$alkoxy)$C_1$-$C_6$alkyl, ($C_3$-$C_7$halocycloalkyl) ($C_1$-$C_6$alkoxy)$C_1$-$C_6$alkyl, ($C_1$-$C_6$alkoxy)$C_1$-$C_6$haloalkyl, $C_3$-$C_7$cycloalkyl, $C_3$-$C_7$halocycloalkyl, $C_1$-$C_6$alkylcarboxy, epoxyethyl unsubstituted or substituted by halogen or $C_1$-$C_3$alkyl, (epoxyethyl unsubstituted or substituted by halogen or $C_1$-$C_3$alkyl)$C_1$-$C_6$alkyl, C(=W)$R^{11}$ ($R^{11}$ represents $C_2$-$C_6$alkyl, $C_1$-$C_6$haloalkyl, $C_1$-$C_6$alkoxy, $C_1$-$C_6$haloalkoxy, $C_1$-$C_6$alkylthio, $C_1$-$C_6$alkylamino or di$C_1$-$C_6$alkylamino; and W represents oxygen, sulfur or NOR$^{12}$ ($R^{12}$ represents $C_1$-$C_6$alkyl or $C_1$-$C_6$haloalkyl)), the formula:

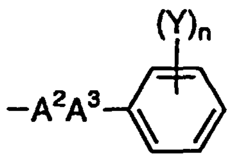

($A^2$ represents an single bond, oxygen or sulfur;
$A^3$ represents a single bond, $C_1$-$C_3$alkylene chain or $C_1$-$C_3$alkylene chain substituted by hydroxy, halogen, $C_1$-$C_6$alkyl or $C_1$-$C_6$alkoxy, with a proviso that $A^2A^3$ means a single bond when $A^2$ and $A^3$ represent a single bond at the same time;
Y represents halogen, $C_1$-$C_6$alkyl, $C_1$-$C_6$haloalkyl, $C_1$-$C_6$alkoxy, $C_1$-$C_6$alkylthio, cyano, nitro, carboxyl or ($C_1$-$C_4$alkoxy) carbonyl;
n represents an integer 0 to 5, and
Y each may be identical with or different from the other when n represents an integer 2 to 5) or the formula:

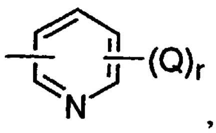 , 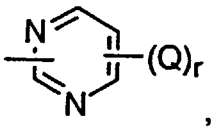 , 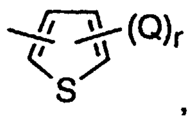 , 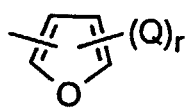 ,

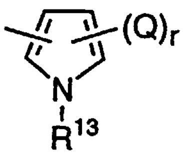 or 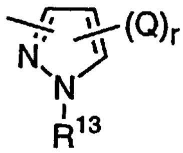 .

($R^{13}$ represents $C_1$-$C_6$alkyl;
Q represents halogen, $C_1$-$C_6$alkyl, $C_1$-$C_6$haloalkyl, $C_1$-$C_6$alkoxy, alkylthio, cyano, nitro, carboxyl or ($C_1$-$C_6$alkoxy)carbonyl;
r represents an integer 0 to 4, and

Q each may be identical with or different from the other when r represents an integer 2 to 4);
$R^4$ and $R^5$ together may form $C_2$-$C_7$ alkylene chain unsubstiuted or substituted by $C_1$-$C_6$alkyl, $C_1$-$C_6$alkoxy or halogen;
$R^6$, $R^7$, $R^8$ and $R^9$ each represents independently hydrogen, halogen, $C_1$-$C_6$alkyl or phenyl;
$R^5$ and $R^8$ together may form $C_1$-$C_7$alkylene chain unsubstiuted or substituted by $C_1$-$C_6$alkyl;
$R^{10}$ represents halogen, $C_1$-$C_6$alkyl, $C_1$-$C_6$haloalkyl, $C_1$-$C_6$alkoxy or $C_1$-$C_6$haloalkoxy;
p is 0 or 1;
q represents an integer 0 to 3, and
$R^{10}$ each may be identical with or different from the other when q represents an integer 2 to 3; and
Z represents oxygen or $CH_2$.

**2.** An agricultural chemical containing at least one compound of cycloalkyl benzyl ether of the formula (1) according to claim 1.

**3.** A herbicide containing at least one compound of cycloalkyl benzyl ether of the formula (1) according to claim 1.

**INTERNATIONAL SEARCH REPORT**

International application No.
PCT/JP98/01541

A. CLASSIFICATION OF SUBJECT MATTER
Int.Cl$^6$ C07C43/18, C07D207/33, 213/30, 231/12, 239/26, 303/22, C07D307/42, 333/16, 405/04, 407/04, 409/04, A01N31/14, 43/20 //

According to International Patent Classification (IPC) or to both national classification and IPC

B. FIELDS SEARCHED

Minimum documentation searched (classification system followed by classification symbols)
Int.Cl$^6$ C07C43/18, C07D207/33, 213/30, 231/12, 239/26, 303/22, C07D307/42, 333/16, 405/04, 407/04, 409/04, A01N31/14, 43/20 //

Documentation searched other than minimum documentation to the extent that such documents are included in the fields searched

Electronic data base consulted during the international search (name of data base and, where practicable, search terms used)
CAS ONLINE

C. DOCUMENTS CONSIDERED TO BE RELEVANT

| Category* | Citation of document, with indication, where appropriate, of the relevant passages | Relevant to claim No. |
|---|---|---|
| A | WO, 97/02258, A1 (HOECHST SCHERING AGREVO GMBH.), January 23, 1997 (23. 01. 97) & DE, 19524143, A1 | 1-3 |
| A | JP, 8-81310, A (Sagami Chemical Research Center), March 26, 1996 (26. 03. 96) & WO, 96/08481, A1 | 1-3 |
| A | JP, 2-108681, A (Shell International Research Maatschappy B.V), April 20, 1990 (20. 04. 90) & EP, 361560, A1 & US, 5006552, A | 1-3 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| June 17, 1998 (17. 06. 98) | June 30, 1998 (30. 06. 98) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP98/01541

A. (Continuation) CLASSIFICATION OF SUBJECT MATTER

A01N43/40, 43/54, 43/56

B. (Continuation) FIELDS SEARCHED

A01N43/40, 43/54, 43/56

Form PCT/ISA/210 (extra sheet) (July 1992)